# EUROPÄISCHE PATENTSCHRIFT

(11) **EP 1 453 810 B1**
(45) Veröffentlichungstag und Bekanntmachung des Hinweises auf die Patenterteilung: **18.03.2009**
(21) Anmeldenummer: 02804183.8
(22) Anmeldetag: 20.11.2002
(51) Int. Cl.: C07D 217/22, C07D 401/12, C07D 403/12, C07D 417/12, C07D 409/12, A61K 31/47, A61P 9/12

(54) **SUBSTITUIERTE 4-PHENYLTETRAHYDROISOCHINOLINE, VERFAHREN ZU IHRER HERSTELLUNG, IHRE VERWENDUNG ALS MEDIKAMENT, SOWIE SIE ENTHALTENDES MEDIKAMENT**
SUBSTITUTED 4-PHENYLTETRAHYDROISOQUINOLINES, METHOD FOR THE PRODUCTION THEREOF, THE USE THEREOF AS MEDICAMENTS, IN ADDITION TO A MEDICAMENT CONTAINING SAME
4-PHENYLTETRAHYDROISOQUINOLEINE, SON PROCEDE DE PRODUCTION, SON UTILISATION COMME MEDICAMENT, ET MEDICAMENT LA CONTENANT

(30) Priorität: 05.12.2001 DE 10159714
(43) Veröffentlichungstag der Anmeldung: 08.09.2004
(73) Patentinhaber: Sanofi-Aventis Deutschland GmbH, 65929 Frankfurt am Main (DE)
(72) Erfinder: HOFMEISTER, Armin, 55276 Oppenheim (DE); HEINELT, Uwe, 65187 Wiesbaden (DE); LANG, Hans-Jochen, 65719 Hofheim (DE); BLEICH, Markus, 65597 Hünfelden-Dauborn (DE); WIRTH, Klaus, 65830 Kriftel (DE); GEKLE, Michael, 97072 Würzburg (DE)
(86) Internationale Anmeldenummer: PCT/EP2002/012990
(87) Internationale Veröffentlichungsnummer: WO 2003/048129

(56) Entgegenhaltungen:
- WO-A-01/32624
- WO-A-01/79186
- SATTELKAU T ET AL: "AN EFFICIENT SYNTHESIS OF THE POTENT DOPAMINE D1 AGONIST DINAPSOLINE BY CONSTRUCTION AND SELECTIVE REDUCTION OF 2'-AZADIMETHOXYBENZANTHRONE" SYNTHESIS, GEORG THIEME VERLAG. STUTTGART, DE, Nr. 2, 2001, Seiten 262-266, XP002949177 ISSN: 0039-7881

## Beschreibung

Die Erfindung betrifft Verbindungen der Formel I 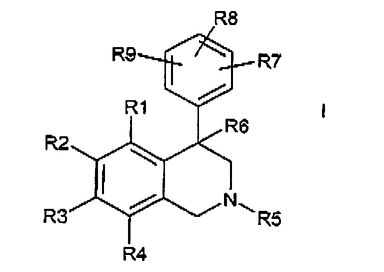 worin bedeuten:
- d: 1, 2, 3, oder 4, wobei ein oder mehrere H-Atome durch F-Atome ersetzt sein dürfen;
oder
- R1, R2, R3 und R4: unabhängig voneinander Heteroaryl, wobei Null, 1, 2, 3 oder 4 N-Atome, Null oder 1 Sauerstoff-Atom oder Null oder 1 S-Atom als Ringatome enthalten sein können;
oder
- R1, R2, R3 und R4: unabhängig voneinander CONR11R12 oder NR11R12;
R11 und R12 unabhängig voneinander H, CₑH₂ₑ₊₁, CᵣᵣH₂ᵣᵣ₋₁;
e 1, 2, 3, 4, 5, 6, 7 oder 8,
rr 3, 4, 5, 6, 7, oder 8,
wobei in den Gruppen CₑH₂ₑ₊₁ und CᵣᵣH₂ᵣᵣ₋₁ ein oder mehrere H-Atome durch F-Atome ersetzt sein dürfen und ein oder mehrere CH₂-Gruppen durch O oder NR13 ersetzt sein können;
R13 H oder C_{f}H_{2f+1};
f 1, 2, 3, oder 4, wobei ein oder mehrere H-Atome durch F-Atome ersetzt sein dürfen; oder
R13 und eine CH₂-Gruppe von R11 oder R12 gemeinsam mit dem N-Atom, an das sie gebunden sind, einen 5- oder 6-gliedrigen Ring; oder
R11 und R12 gemeinsam mit dem N-Atom, an das sie gebunden sind, einen 5-, 6- oder 7-gliedrigen Ring;
oder
R11 und R12 unabhängig voneinander COR14, CSR14 oder SO₂R14;
R14 C_{g}H_{2g+1};
g 1, 2, 3, 4, 5, 6, 7 oder 8, wobei ein oder mehrere H-Atome durch F-Atome ersetzt sein können und ein oder mehrere CH₂-Gruppen durch O oder NR13 ersetzt sein können,
oder
- R1, R2, R3 und R4: unabhängig voneinander -Oₕ-SOⱼ-R15, wobei
h Null oder 1;
j Null, 1 oder 2;
R15 CₖH₂ₖ₊₁, OH, OCₗH₂ₗ₊₁ oder NR17R18;
k 1, 2, 3, 4, 5, 6, 7 oder 8, wobei ein oder mehrere H-Atome durch F-Atome ersetzt sein können;
l 1, 2, 3, 4, 5, 6,7 oder 8, wobei ein oder mehrere H-Atome durch F-Atome ersetzt sein können;
R17 und R18 unabhängig voneinander H oder CₘH₂ₘ₊₁;
m 1, 2, 3, 4, 5, 6, 7 oder 8, wobei in der Gruppe CₘH₂ₘ₊₁ ein oder mehrere H-Atome durch F-Atome ersetzt sein können und ein oder mehrere CH₂-Gruppen durch O, CO, CS oder NR19 ersetzt sein können;
R19 H oder CₙH₂ₙ₊₁;
n 1, 2, 3 oder 4;
wobei in CₙH₂ₙ₊₁ ein oder mehrere H-Atome durch F-Atome ersetzt sein können; oder
R17 und R18 gemeinsam mit dem N-Atom, an das sie gebunden sind, einen 5-, 6- oder 7-gliedrigen Ring;
oder
R19 und eine CH₂-Gruppe von R17 oder R18 gemeinsam mit dem N-Atom, an das sie gebunden sind, einen 5- oder 6-gliedrigen Ring;
wobei jedoch immer R2 ungleich H sein muss,
- R5: H, CₚH₂ₚ₊₁, CₛₛH₂ₛₛ₋₁, COR20 oder SO₂R20;
p 1, 2, 3, 4, 5, 6, 7 oder 8,
ss 3, 4, 5, 6, 7 oder 8,
R20 C_{q}H_{2q+1};
q 1, 2, 3, 4, 5, 6, 7 oder 8,
wobei in den Gruppen CₚH₂ₚ₊₁, CₛₛH₂ₛₛ₋₁ und C_{q}H_{2q+1} ein oder mehrere H-Atome durch F-Atome ersetzt sein können, und eine oder mehrere CH₂-Gruppen durch O oder NR21 ersetzt sein können;
R21 H oder CᵣH₂ᵣ₊₁;
r 1, 2, 3 oder 4; wobei in CᵣH₂ᵣ₊₁ ein oder mehrere H-Atome durch F-Atome ersetzt sein können;
- R6: H, F, Cl, Br, I, CₛH₂ₛ₊₁, C_{dd}H_{2dd-1}, OH, OCₜH₂ₜ₊₁ oder OCOR22; s und t
unabhängig voneinander 1, 2, 3, 4, 5, 6, 7 oder 8;
dd 3, 4, 5, 6, 7 oder 8, wobei in CₛH₂ₛ₊₁, C_{dd}H_{2dd-1} und OCₜH₂ₜ₊₁ ein oder mehrere H-Atome durch F-Atome ersetzt sein können;
R22 CᵤH₂ᵤ₊₁;
u 1, 2, 3 oder 4;
wobei in CᵤH₂ᵤ₊₁ ein oder mehrere H-Atome durch F-Atome ersetzt sein können;
- R7, R8 und R9: unabhängig voneinander -Oᵥ-SO_{w}-R23;
v Null oder 1;
w Null, 1 oder 2;
R23 CₙₙH₂ₙₙ₊₁, CₘₘH₂ₘₘ₋₁, OH, OCₚₚH₂ₚₚ₊₁ oder NR25R26;
nn und pp unabhängig voneinander 1, 2, 3, 4, 5, 6, 7 oder 8,
mm 3, 4, 5, 6, 7 oder 8,
wobei in CₙₙH₂ₙₙ₊₁, CₘₘH₂ₘₘ₋₁ und OCₚₚH₂ₚₚ₊₁ ein oder mehrere H-Atome durch F-Atome ersetzt sein können;
R25 und R26 unabhängig voneinander H, CN oder C_{z}H_{2z+1}, C_{zz}H_{2zz-1};
z 1, 2, 3, 4, 5, 6, 7 oder 8;
zz 3, 4, 5, 6, 7 oder 8, wobei ein oder mehrere H-Atome durch F-Atome ersetzt sein dürfen und
in C_{z}H_{2z+1} ein oder mehrere H-Atome durch F-Atome ersetzt sein können und ein oder mehrere CH₂-Gruppen durch O, CO, CS oder NR27 ersetzt sein können;
R27 H oder CₐₐH2ₐₐ₊₁;
aa 1, 2, 3 oder 4;
wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5-, 6- oder 7-gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
- R7, R8 und R9: unabhängig voneinander NR32COR30, NR32CSR30 oder NR32SO_{bb}R30;
R30 H, C_{cc}H_{2cc+1}, C_{yy}H_{2yy-1}, Pyrrolidinyl oder Piperidinyl, in welchen Ringen eine CH₂-Gruppe durch O oder NR33 ersetzt sein kann;
R32 und R33 unabhängig voneinander H oder CₕH₂ₕ₊₁ ;
bb 2 oder 3;
cc 1, 2, 3, 4, 5, 6, 7 oder 8;
yy 3, 4, 5, 6, 7 oder 8;
h 1, 2, 3, 4, 5, 6, 7 oder 8, wobei in CₕH₂ₕ₊₁ ein oder mehrere H-Atome durch F-Atome ersetzt sein dürfen und in den Gruppen C_{cc}H_{2cc+1} und C_{yy}H_{2yy-1} ein oder mehrere H-Atome durch F-Atome ersetzt sein können und eine oder mehrere CH₂- Gruppen durch NR31 ersetzt sein können und eine CH2-Gruppe durch O ersetzt sein kann ;
R31 H, CₖₖH₂ₖₖ₊₁, COR65 oder SO₂R65;
kk 1, 2, 3, oder 4;
wobei ein oder mehrere H-Atome durch F-Atome ersetzt sein können,
R65 H, CₓₓH₂ₓₓ₊₁;
xx 1, 2, 3 oder 4, wobei ein oder mehrere H-Atome durch F-Atome ersetzt sein dürfen; oder
R31 zusammen mit einer CH₂-Gruppe von R30 einen 5-, 6- oder 7-gliedrigen Ring bildet; oder
R30 ein 5- oder 6-gliedriges Heteroaryl mit 1, 2, 3 oder 4 N-Atomen, Null oder 1 S-Atomen und Null oder 1 O-Atomen,
das unsubstituiert ist oder substituiert mit bis zu drei Substituenten ausgewählt aus der Gruppe bestehend aus F, Cl, Br, I, CₒₒH₂ₒₒ₊₁, NR70R71;
R70 und R71 unabhängig voneinander H, CᵤᵤH₂ᵤᵤ₊₁ und COR72;
R72 H, CᵥᵥH₂ᵥᵥ₊₁;
oo, uu und w unabhängig voneinander 1, 2, 3, 4, 5, 6, 7 oder 8; wobei in den Gruppen CₒₒH₂ₒₒ₊₁, CᵤᵤH₂ᵤᵤ₊₁ oder CᵥᵥH₂ᵥᵥ₊₁ ein oder mehrere H-Atome durch F-Atome ersetzt sein dürfen;
oder
- R7, R8 und R9: unabhängig voneinander H, F, Cl, Br, I, NO₂, CN, OH, NH₂, CₑₑH₂ₑₑ₊₁, C_{ww}H_{2ww-1}, OC_{ff}H_{2ff+1}, NR40R41, CONR40R41, COOR42, COR42 oder OCOR42,
ee und ff unabhängig voneinander 1, 2, 3, 4, 5, 6, 7 oder 8;
ww 3, 4, 5, 6, 7 oder 8; wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H, CₜₜH₂ₜₜ₊₁ oder C(NH)NH_{2;}
tt 1, 2, 3, 4, 5, 6, 7 oder 8; wobei in der Gruppe CₜₜH₂ₜₜ₊₁ ein oder mehrere H-Atome durch F-Atome ersetzt sein dürfen und eine oder mehrere CH₂-Gruppen durch O- oder NR44 ersetzt sein können;
R44 H oder C_{gg}H_{2gg+1};
gg 1, 2, 3, 4, 5, 6, 7 oder 8; wobei in der Gruppe C_{gg}H_{2gg+1} ein oder mehrere H-Atome durch F-Atome ersetzt sein können und R44 zusammen mit einer (CH₂)-Gruppe von R40 oder R41 und dem N-Atom, an das sie gemeinsam gebunden sind, einen 5 oder 6-gliedrigen Ring bilden können; oder
R40 und R41 mit dem N-Atom, an das sie gebunden sind, einen 5- oder 6-gliedrigen Ring;
R42 H oder CₕₕH₂ₕₕ₊₁;
hh 1, 2, 3, 4, 5, 6, 7 oder 8; wobei in der Gruppe CₕₕH₂ₕₕ₊₁ ein oder mehrere H-Atome durch F-Atome ersetzt sein können; wobei jedoch nicht zwei Substituenten aus der Gruppe R7, R8 und R9 gleichzeitig OH oder OCH₃ sein dürfen
und wobei mindestens einer der Reste R7, R8 oder R9 ausgewählt sein muss aus der Gruppe bestehend aus CONR40R41, -OᵥSO_{w}R23, NR32COR30, NR32CSR30 und NR32SO_{bb}R30;
sowie deren pharmazeutisch verträgliche Salze und Trifluoracetate.

Bevorzugt sind Verbindungen der Formel I, worin bedeuten:
- R1, R2, R3 und R4: unabhängig voneinander H, F, Cl, Br, I, CN, NO₂, OH, NH₂, CₐH₂ₐ₊₁, Cycloalkyl mit 3, 4, 5 oder 6 C-Atomen, OC_{b}H_{2b+1}, COOR10;
a und b unabhängig voneinander 1, 2, 3 oder 4, wobei ein oder mehrere H-Atome durch F-Atome ersetzt sein können;
R10 H oder C_{c}H_{2c+1};
c 1, 2, 3 oder 4, wobei ein oder mehrere H-Atome durch F-Atome ersetzt sein dürfen;
oder
- R1, R2, R3 und R4: unabhängig voneinander 5- oder 6-gliedriges Heteroaryl, ausgewählt aus der Gruppe bestehend aus Pyridyl, Imidazolyl, Pyrazolyl, Pyrrolyl, Triazolyl, Tetrazolyl, Thiazolyl und Oxazolyl;
oder
- R1, R2, R3 und R4: unabhängig voneinander CONR11R12 oder NR11R12;
R11 und R12 unabhängig voneinander H, CₑH₂ₑ₊₁, CᵣᵣH₂ᵣᵣ₋₁;
e 1, 2, 3 oder 4,
rr 3, 4, 5 oder 6,
wobei in den Gruppen CₑH₂ₑ₊₁ und CᵣᵣH₂ᵣᵣ₋₁ ein oder mehrere H-Atome durch F-Atome ersetzt sein dürfen oder
R11 und R12 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl; oder
R11 und R12 gemeinsam mit dem N-Atom, an das sie gebunden sind, einen Pyrrolidin-, Piperidin-, N-Methylpiperazin-, Piperazin- oder Morpholinring; oder
R11 und R12 unabhängig voneinander COR14, CSR14, CONHR14, CSNHR14 oder SO₂R14;
R14 C_{g}H_{2g+1};
g 1, 2, 3 oder 4, wobei ein oder mehrere H-Atome durch F-Atome ersetzt sein;
oder
- R1, R2, R3 und R4: unabhängig voneinander OSO₃H, SO₃H, SO₂R₁₅, wobei
R15 CₖH₂ₖ₊₁, OCₗH₂ₗ₊₁ oder NR17R18;
k 1, 2, 3 oder 4, wobei ein oder mehrere H-Atome durch F-Atome ersetzt sein können;
l 1, 2, 3 oder 4, wobei ein oder mehrere H-Atome durch F-Atome ersetzt sein können;
R17 und R18 unabhängig voneinander H, CₘH₂ₘ₊₁, in welchem die an den Stickstoff gebundene erste CH₂-Gruppe durch CO und die zweite CH₂-Gruppe durch NR19 ersetzt ist;
m 1, 2, 3, 4 oder 5, wobei in der Gruppe CₘH₂ₘ₊₁ ein oder mehrere H-Atome durch F-Atome ersetzt sein können;
R19 H oder CₙH₂ₙ₊₁;
n 1, 2, 3 oder 4; wobei in CₙH₂ₙ₊₁ ein oder mehrere H-Atome durch F-Atome ersetzt sein können; oder
R17 und R18 gemeinsam mit dem N-Atom, an das sie gebunden sind, einen 5- oder, 6-gliedrigen Ring;
wobei jedoch immer R2 ungleich H sein muss,
- R5: H, CₚH₂ₚ₊₁;
p 1, 2, 3 oder 4;
wobei in CₚH₂ₚ₊₁ ein oder mehrere H-Atome durch F-Atome ersetzt sein können;
- R6: H, CₛH₂ₛ₊₁, OCₜH₂ₜ₊₁ oder OCOR22;
s und t unabhängig voneinander 1, 2, 3 oder 4;
wobei in CₛH₂ₛ₊₁ und OCₜH₂ₜ₊₁ ein oder mehrere H-Atome durch F-Atome ersetzt sein können;
R22 CᵤH₂ᵤ₊₁;
u 1, 2, 3 oder 4; wobei in CᵤH₂ᵤ₊₁ ein oder mehrere H-Atome durch F-Atome ersetzt sein können;

- R7, R8 und R9: unabhängig voneinander OSO₃H, SO₃H oder SO₂R23;
R23 CₙₙH₂ₙₙ₊₁, CₘₘH₂ₘₘ₋₁, OCₚₚH₂ₚₚ₊₁ oder NR25R26;
nn und pp unabhängig voneinander 1, 2, 3, 4 oder 5,
mm 3, 4, 5 oder 6, wobei in CₙₙH₂ₙₙ₊₁, CₘₘH₂ₘₘ₋₁ und OCₚₚH₂ₚₚ₊₁ ein oder mehrere H-Atome durch F-Atome ersetzt sein können;
R25 und R26 unabhängig voneinander H, CN, C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atome durch F-Atome ersetzt sein;
R27 H oder CₐₐH₂ₐₐ₊₁;
aa 1, 2, 3 oder 4; wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
- R7, R8 und R9: unabhängig voneinander NR32COR30, NR32CSR30 oder NR32SO₂R30;
R30 H, OH, C_{cc}H_{2cc+1}, C_{yy}H_{2yy-1}; Pyrrolidinyl oder Piperidinyl, in welchen Ringen eine CH₂-Gruppe durch O oder NR33 ersetzt sein kann;
R32 und R33 unabhängig von einander H oder CₕH₂ₕ₊₁;
cc 1, 2, 3, 4, 5, 6, 7 oder 8;
yy 3, 4, 5 oder 6;
h 1, 2, 3 oder 4; wobei in CₕH₂ₕ₊₁ ein oder mehrere H-Atome durch F-Atome ersetzt sein dürfen und in den Gruppen C_{cc}H_{2cc+1} und C_{yy}H_{2yy-1} ein oder mehrere H-Atome durch F-Atome ersetzt sein können und eine oder mehrere CH₂-Gruppen durch NR31 ersetzt sein können und eine CH₂-Gruppe durch O ersetzt sein kann;
R31 H, CₖₖH₂ₖₖ₊₁, COR65 oder SO₂R65;
kk 1, 2, 3, oder 4; wobei ein oder mehrere H-Atome durch F-Atome ersetzt sein können,
R65 H, CₓₓH₂ₓₓ₊₁;
xx 1, 2, 3 oder 4, wobei ein oder mehrere H-Atome durch F-Atome ersetzt sein dürfen; oder
R31 zusammen mit einer CH₂-Gruppe von R30 und dem N-Atom, an das sie gemeinsam gebunden sind einen 5- oder 6-gliedrigen Ring bilden; oder
R30 einen 5- oder 6-gliedrigen Heteroaromaten ausgewählt aus der Gruppe bestehend aus Pyridyl, Imidazolyl, Pyrazolyl, Pyrrolyl, Triazolyl, Tetrazolyl, Thienyl, Thiazolyl und Oxazolyl,
die unsubstituiert sind oder substituiert mit bis zu drei Substituenten ausgewählt aus der Gruppe bestehend aus F, Cl, Br, I, CₒₒH₂ₒₒ₊₁, NR70R71,
R70 und R71 unabhängig voneinander H, CᵤᵤH₂ᵤᵤ₊₁ oder COR72,;
R72 H, CᵥᵥH₂ᵥᵥ₊₁;
oo, uu und vv unabhängig voneinander 1, 2, 3 oder 4; wobei in den Gruppen CₒₒH₂ₒₒ₊₁, CᵤᵤH₂ᵤᵤ₊₁ oder
CᵥᵥH₂ᵥᵥ₊₁ ein oder mehrere H-Atome durch F-Atome ersetzt sein dürfen;
oder
- R7, R8 und R9: unabhängig voneinander H, F, Cl, Br, I, NO₂, CN, OH, NH₂, CₑₑH₂ₑₑ₊₁, C_{ww}H_{2ww-1}, OC_{ff}H_{2ff+1}, NR40R41, CONR40R41, COOR42, COR42 oder OCOR42;
ee und ff unabhängig voneinander 1, 2, 3 oder 4;
ww 3, 4, 5 oder 6, wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H, CₜₜH₂ₜₜ₊₁ oder C(NH)NH₂
tt 1, 2, 3, 4, 5, 6, 7 oder 8; wobei in der Gruppe CₜₜH₂ₜₜ₊₁ ein oder mehrere H-Atome durch F-Atome ersetzt sein dürfen; oder
R40 und R41 unabhängig voneinander auszuwählen Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl; oder
R40 und R41 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin;
R42 H oder CₕₕH₂ₕₕ₊₁;
hh 1, 2, 3 oder 4; wobei in der Gruppe CₕₕH₂ₕₕ₊₁ ein oder mehrere H-Atome durch F-Atome ersetzt sein können; wobei jedoch nicht zwei Substituenten aus der Gruppe R7, R8 und R9 gleichzeitig OH oder OCH₃ sein dürfen
und wobei mindestens einer der Reste R7, R8 oder R9 ausgewählt sein muss aus der Gruppe bestehend aus CONR40R41, -OᵥSO_{w}R23, NR32COR30, NR32CSR30 und NR32SO_{bb}R30;
sowie deren pharmazeutisch verträgliche Salze und Trifluoracetate.

Besonders bevorzugt sind Verbindungen der Formel I, worin bedeuten:
- R1, R2, R3 und R4: unabhängig voneinander H, F, Cl, Br, OH, NH₂, CₐH₂ₐ₊₁, Cycloalkyl mit 3, 4, 5 oder 6 C-Atomen, OC_{b}H_{2b+1};
a und b in den Gruppen CₐH₂ₐ₊₁ und OC_{b}H_{2b+1} unabhängig voneinander 1, 2, 3 oder 4, wobei ein oder mehrere H-Atome durch F-Atome ersetzt sein können;
oder
- R1,R2,R3undR4: unabhängig voneinander NR11R12;
R11 und R12 unabhängig voneinander H, CₑH₂ₑ₊₁, CᵣᵣH₂ᵣᵣ₋₁;
e 1, 2, 3 oder 4,
rr 3, 4, 5 oder 6,
wobei in den Gruppen CₑH₂ₑ₊₁ und CᵣᵣH₂ᵣᵣ₋₁ ein oder mehrere H-Atome durch F-Atome ersetzt sein dürfen; oder
R11 und R12 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin; oder
R11 und R12 unabhängig voneinander COR14, CSR14, CONHR14, CSNHR14 oder SO₂R14;
R14 C_{g}H_{2g+1};
g 1, 2, 3 oder 4, wobei ein oder mehrere H-Atome durch F-Atome ersetzt sein können;
oder
- R1, R2, R3 und R4: unabhängig voneinander OSO₃H, SO₃H, SO₂R15;
R15 CₖH₂ₖ₊₁ oder NR17R18;
k 1, 2, 3 oder 4, wobei ein oder mehrere H-Atome durch F-Atome ersetzt sein können;
R17 und R18 unabhängig voneinander H oder CₘH₂ₘ₊₁;
m 1, 2, 3, 4 oder 5, wobei in der Gruppe CₘH₂ₘ₊₁ ein oder mehrere H-Atome durch F-Atome ersetzt sein können; oder
R17 und R18 gemeinsam mit dem N-Atom, an das sie gebunden sind, einen 5- oder, 6-gliedrigen Ring;
wobei jedoch immer R2 ungleich H sein muss;
- R5: Methyl oder Trifluormethyl;
- R6: H;
- R7, R8 und R9: unabhängig voneinander OSO₃H, SO₃H oder SO₂R23;
R23 CₙₙH₂ₙₙ₊₁ oder NR25R26;
nn 1, 2, 3, 4 oder 5,
wobei in CₙₙH₂ₙₙ₊₁ ein oder mehrere H-Atome durch F-Atome ersetzt sein können;
R25 und R26 unabhängig voneinander H, CN oder C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atome durch F-Atome ersetzt sein können;
R27 H oder CₐₐH₂ₐₐ₊₁;
aa 1, 2, 3 öder 4;
wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
- R7, R8 und R9: unabhängig voneinander NR32COR30, NR32CSR30 oder NR32SO₂R30;
R30 H, OH, C_{cc}H_{2cc+1}, C_{yy}H_{2yy-1}, Pyrrolidinyl oder Piperidinyl, in welchen Ringe eine CH₂-Gruppe durch O oder NR33 ersetzt sein kann;
R32 und R33 H, Methyl oder CF₃;
cc 1, 2, 3, 4, 5, 6 7 oder 8;
yy 3, 4, 5 oder 6; wobei in den Gruppen C_{cc}H_{2cc+1} und C_{yy}H_{2yy-1} ein oder mehrere H-Atome durch F-Atome ersetzt sein können und eine oder mehrere CH₂-Gruppen durch NR31 ersetzt sein können und eine CH₂-Gruppe durch O ersetzt sein kann;
R31 H, Methyl, Ethyl, CF₃, CH₂CF₃, Acetyl, Propinyl, Methansulfonyl oder Ethansulfonyl; oder
R31 zusammen mit einer CH₂-Gruppe von R30 und dem N-Atom, an das sie gemeinsam gebunden sind, einen 5- oder 6-gliedrigen Ring bilden; oder
R30 Pyridyl, Imidazolyl, Pyrazolyl, Pyrrolyl, Triazolyl, Tetrazolyl, Thiazolyl oder Oxazolyl, die unsubstituiert sind oder substituiert mit maximal 3 Substituenten ausgewählt aus der Gruppe bestehend aus F, Cl, Methyl, Ethyl, Trifluormethyl, NH₂, NHAcetyl;
oder
- R7, R8 und R9: unabhängig voneinander H, F, Cl, OH, NH₂, CₑₑH₂ₑₑ₊₁, C_{ww}H_{2ww-1}, OC_{ff}H_{2ff+1}, NR40R41, CONR40R41, COOR42 oder OCOR42,
ee und ff unabhängig voneinander 1, 2, 3 oder 4;
ww 3, 4, 5 oder 6,
wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H, CₜₜH₂ₜₜ₊₁ oder C(NH)NH₂;
tt 1, 2, 3 oder 4; wobei in der Gruppe CₜₜH₂ₜₜ₊₁ ein oder mehrere H-Atome durch F-Atome ersetzt sein dürfen; oder
R40 und R41
- R1, R2, R3 und R4: unabhängig voneinander H, F, Cl, Br, OH, NH₂, CₐH₂ₐ₊₁, Cycloalkyl mit 3, 4, 5 oder 6 C-Atomen, OC_{b}H_{2b+1};
a und b in den Gruppen CₐH₂ₐ₊₁ und OC_{b}H_{2b+1} unabhängig voneinander 1, 2, 3 oder 4, wobei ein oder mehrere H-Atome durch F-Atome ersetzt sein können;
oder
- R1, R2, R3 und R4: unabgängig voneinander NR11R12;
R11 und R12 unabhängig voneinander H, CₑH₂ₑ₊₁, CᵣᵣH₂ᵣᵣ₋₁;
e 1, 2, 3 oder 4,
rr 3, 4, 5 oder 6,
wobei in den Gruppen CₑH₂ₑ₊₁ und CᵣᵣH₂ᵣᵣ₋₁ ein oder mehrere H-Atome durch F-Atome ersetzt sein dürfen; oder
R11 und R12 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin; oder
R11 und R12 unabhängig voneinander COR14, CSR14, CONHR14, CSNHR14 oder SO₂R14;
R14 C_{g}H_{2g+1};
g 1, 2, 3 oder 4, wobei ein oder mehrere H-Atome durch F-Atome ersetzt sein können;
oder
- R1, R2, R3 und R4: unabhängig voneinander OSO₃H, SO₃H, SO₂R15;
R15 CₖH₂ₖ₊₁ oder NR17R18;
k 1, 2, 3 oder 4, wobei ein oder mehrere H-Atome durch F-Atome ersetzt sein können;
R17 und R18 unabhängig voneinander H oder CₘH₂ₘ₊₁;
m 1, 2, 3, 4 oder 5, wobei in der Gruppe CₘH₂ₘ₊₁ ein oder mehrere H-Atome durch F-Atome ersetzt sein können; oder
R17 und R18 gemeinsam mit dem N-Atom, an das sie gebunden sind, einen 5- oder, 6-gliedrigen Ring;
wobei jedoch immer R2 ungleich H sein muss;
- R5: Methyl oder Trifluormethyl;
- R6: H;
- R7, R8 und R9: unabhängig voneinander OSO₃H, SO₃H oder SO₂R23;
R23 CₙₙH₂ₙₙ₊₁ oder NR25R26;
nn 1, 2, 3, 4 oder 5,
wobei in CₙₙH₂ₙₙ₊₁ ein oder mehrere H-Atome durch F-Atome ersetzt sein können;
R25 und R26 unabhängig voneinander H, CN oder C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atöme durch F-Atome ersetzt sein können;
R27 H oder CₐₐH₂ₐₐ₊₁;
aa 1, 2, 3 oder 4;
wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
- R7, R8 und R9: unabhängig voneinander NR32COR30, NR32CSR30 oder NR32SO₂R30;
R30 H, OH, C_{cc}H_{2cc+1}, C_{yy}H_{2yy-1}, Pyrrolidinyl oder Piperidinyl, in welchen Ringen eine CH₂-Gruppe durch O oder NR33 ersetzt sein kann;
R32 und R33 H, Methyl oder CF₃;
cc 1, 2, 3, 4, 5, 6, 7 oder 8;
yy 3, 4, 5, 6; wobei in den Gruppen C_{cc}H_{2cc+1} und C_{yy}H_{2yy-1} ein oder mehrere H-Atome durch F-Atome ersetzt sein können und eine oder mehrere CH₂-Gruppen durch NR31 ersetzt sein können und eine CH₂-Gruppe durch O ersetzt sein kann;
R31 H, Methyl, Ethyl, CF₃, CH₂CF₃, Acetyl, Propionyl, Methansulfonyl oder Ethansulfonyl; oder
R31 zusammen mit einer CH₂-Gruppe von R30 und dem N-Atom, an das sie gemeinsam gebunden sind, einen 5- oder 6-gliedrigen Ring bilden; oder
R30 Pyridyl, Imidazolyl, Pyrazolyl, Pyrrolyl, Triazolyl, Tetrazolyl, Thiazolyl oder Oxazolyl, die unsubstituiert sind oder substituiert mit maximal 3 Substituenten ausgewählt aus der Gruppe bestehend aus F, Cl, Methyl, Ethyl, Trifluormethyl, NH₂, NHAcetyl;
oder
- R7, R8 und R9: unabhängig voneinander H, F, Cl, OH, NH₂, CₑₑH₂ₑₑ₊₁, C_{ww}H_{2ww-1}, OC_{ff}H_{2ff+1}, NR40R41, CONR40R41, COOR42 oderOCOR42,
ee und ff unabhängig voneinander 1, 2, 3 oder 4;
ww 3, 4, 5 oder 6,
wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H, CₜₜH₂ₜₜ₊₁ oder C(NH)NH₂;
tt 1, 2, 3 oder 4; wobei in der Gruppe CₜₜH₂ₜₜ₊₁ein oder mehrere H-Atome durch F-Atome ersetzt sein dürfen; oder
R40 und R41 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl; oder
R40 und R41 gemeinsam mit dem N-Atom, an das sie gebunden sind, einen Pyrrolidin-, Piperidin-, N-Methylpiperazin-, Piperazin- oder Morpholinring;
R42 H oder CₕₕH₂ₕₕ₊₁;
hh 1, 2, 3 oder 4; wobei in der Gruppe CₕₕH₂ₕₕ₊₁ ein oder mehrere H-Atome durch F-Atome ersetzt sein können;
wobei jedoch nicht zwei Substituenten aus der Gruppe R7, R8 und R9 gleichzeitig OH oder OCH₃ sein dürfen
und wobei mindestens einer der Reste R7, R8 oder R9 ausgewählt sein muss aus der Gruppe bestehend aus -OᵥSO_{w}R23, NR32COR30, NR32CSR30 und NR32SO_{bb}R30; sowie deren pharmazeutisch verträgliche Salze und Trifluoracetate.

Ganz besonders speziell bevorzugt sind Verbindungen
1) N-[4-(6,8-Dichloro-2-methyl-1,2,3,4-tetrahydro-isochinolin-4-yl)-phenyl]-acetamid;
2) 4-(6,8-Dichloro-2-methyl-1,2,3,4-tetrahydro-isochinolin-4-yl)-benzolsulfonamid;
3) 3-(6,8-Dichloro-2-methyl-1,2,3,4-tetrahydro-isochinolin-4-yl)-benzolsulfonamid;
4) 4-(6,8-Dichloro-2-methyl-1,2,3,4-tetrahydro-isochinolin-4-yl)-N,N-dimethylbenzol-sulfonamid;
5) 4-(4-Bromo-phenyl)-6,8-dichloro-2-methyl-1,2,3,4-tetrahydroisochinolin;
6) 4-(6,8-Dichloro-2-methyl-1,2,3,4-tetrahydro-isochinolin-4-yl)-benzoesäure;
7) 4-(6,8-Dichloro-2-methyl-1,2,3,4-tetrahydroisochinolin-4-yl)-N-ethyl-benzamid;
8) 4-(6,8-Dichloro-2-methyl-1,2,3,4-tetrahydroisochinolin-4-yl)-N-propyl-benzamid;
9) 4-(6,8-Dichloro-2-methyl-1,2,3,4-tetrahydro-isochinolin-4-yl)-N-(2-dimethylamino-ethyl)-benzamid;
10) 6,8-Dichloro-2-methyl-4-(4-morpholin-4-yl-phenyl)-1,2,3,4-tetrahydroisochinolin;
11) [4-(6,8-Dichloro-2-methyl-1,2,3,4-tetrahydroisochinolin-4-yl)-phenyl]-diethyl-amin
12) 6,8-Dichloro-2-methyl-4-(4-piperidin-1-yl-phenyl)-1,2,3,4-tetrahydroisochinolin;
13) 6,8-Dichloro-2-methyl-4-(4-pyrrolidin-1-yl-phenyl)-1,2,3,4-tetrahydroisochinolin;
14) 6,8-Dichloro-2-methyl-4-[4-(4-methyl-piperazin-1 -yl)-phenyl]-1,2,3,4-tetrahydroisochinolin;
15) 6,8-Dichloro-2-cyclopropyl-4-phenyl-1,2,3,4-tetrahydroisochinolin;
16) 4-(6,8-Dichloro-2-methyl-1,2,3,4-tetrahydro-isochinolin-4-yl)-phenylamin;
17) 1-[4-(6,8-Dichloro-2-methyl-1,2,3,4-tetrahydro-isochinolin-4-yl)-phenyl]-3-propyl-harnstoff;
18) 1-[4-(6,8-Dichloro-2-methyl-1,2,3,4-tetrahydro-isochinolin-4-yl)-phenyl]-3-methyl-thioharnstoff;
19) 1-[4-(6,8-Dichloro-2-methyl-1,2,3,4-tetrahydro-isochinolin-4-yl)-phenyl]-3-ethyl-harnstoff;
20) N-[4-(6-Methansulfonyl-2-methyl-1,2,3,4-tetrahydro-isochinolin-4-yl)-phenyl]-acetamid;
21) N-[4-(2,6,8-Trimethyl-1,2,3,4-tetrahydro-isochinolin-4-yl)-phenyl]-acetamid;
22) N-[4-(6-Bromo-8-chloro-2-methyl-1,2,3,4-tetrahydro-isochinolin-4-yl)-phenyl]-acetamid;
23) N-[4-(8-Chloro-2-methyl-6-pyrrolidin-1-yl-1,2,3,4-tetrahydro-isochinolin-4-yl)-phenyl]- acetamid;
24) N-[4-(8-Chloro-2-methyl-6-morpholin-4-yl-1,2,3,4-tetrahydro-isochinolin-4-yl)-phenyl]- acetamid;
25) N-{4-[8-Chloro-2-methyl-6-(4-methyl-piperazin-1-yl)-1,2,3,4-tetrahydroisochinolin-4- yl]-phenyl}-acetamid;
26) N-{4-[8-Chloro-6-(cyclopropylmethyl-amino)-2-methyl-1,2,3,4-tetrahydroisochinolin-4- yl]-phenyl}-acetamid;
27) 5-(6,8-Dichloro-2-methyl-1,2,3,4-tetrahydro-isochinolin-4-yl)-2-hydroxybenzoesäure;
28) 5-(6,8-Dichloro-2-methyl-1,2,3,4-tetrahydro-isochinolin-4-yl)-2-hydroxy-N-methyl-benzamid;
29) 5-(6,8-Dichloro-2-methyl-1,2,3,4-tetrahydro-isochinolin-4-yl)-N-ethyl-2-hydroxy-benzamid;
30) 5-(6,8-Dichloro-2-methyl-1,2,3,4-tetrahydro-isochinolin-4-yl)-N-(2-dimethylamino-ethyl)-2-hydroxy-benzamid;
31) N-[5-(6,8-Dichloro-2-methyl-1,2,3,4-tetrahydro-isochinolin-4-yl)-2-hydroxy-benzoyl]- guanidin;
32) N-[3-(6,8-Dichloro-2-methyl-1,2,3,4-tetrahydro-isochinolin-4-yl)-phenyl]-acetamid;
33) 3-(6,8-Dichloro-2-methyl-1,2,3,4-tetrahydro-isochinolin-4-yl)-phenylamin;
34) 2-(6,8-Dichloro-2-methyl-1,2,3,4-tetrahydro-isochinolin-4-yl)-phenylamin;
35) N-[4-(6,8-Dichloro-2-methyl-1,2,3,4-tetrahydro-isochinolin-4-yl)-phenyl]-propionamid;
36) N-[4-(6,8-Dichloro-2-methyl-1,2,3,4-tetrahydro-isochinolin-4-yl)-phenyl]-butyramid;
37) Pentansäure-[4-(6,8-dichloro-2-methyl-1,2,3,4-tetrahydro-isochinolin-4-yl)-phenyl]-amid;
38) N-[4-(6,8-Dichloro-2-methyl-1,2,3,4-tetrahydro-isochinolin-4-yl)-phenyl]-isobutyramid;
39) N-[4-(6,8-Dichloro-2-methyl-1,2,3,4-tetrahydro-isochinolin-4-yl)-phenyl]-2,2-dimethyl- propionamid;
40) Cyclopropancarbonsäure-[4-(6,8-dichloro-2-methyl-1,2,3,4-tetrahydro-isochinolin-4-yl)-phenyl]-amid;
41) Cyclobutancarbonsäure-[4-(6,8-dichloro-2-methyl-1,2,3,4-tetrahydro-isochinolin-4-yl)- phenyl]-amid;
42) Cyclopentancarbonsäure-[4-(6,8-dichloro-2-methyl-1,2,3,4-tetrahydro-isochinolin-4-yl)-phenyl]-amid;
43) N-[4-(6,8-Dichloro-2-methyl-1,2,3,4-tetrahydro-isochinolin-4-yl)-phenyl]-2,2,2-trifluoro-acetamid;
44) 1-Acetyl-piperidin-4-carbonsäure-[4-(6,8-dichloro-2-methyl-1,2,3,4-tetrahydroisochinolin-4-yl)-phenyl]-amid;
45) N-[4-(6,8-Dichloro-2-methyl-1,2,3,4-tetrahydro-isochinolin-4-yl)-phenyl]-nicotinamid;
46) N-[4-(6,8-Dichloro-2-methyl-1,2,3,4-tetrahydro-isochinolin-4-yl)-phenyl]-methansulfonamid;
47) Ethansulfonsäure-[4-(6,8-dichloro-2-methyl-1,2,3,4-tetrahydro-isochinolin-4-yl)-phenyl]-amid;
48) N',N'-Dimethylamino-N-[4-(6,8-Dichloro-2-methyl-1,2,3,4-tetrahydro-isochinolin-4-yl)-phenyl]-sulfamid;
49) N-[3-(6,8-Dichloro-2-methyl-1,2,3,4-tetrahydro-isochinolin-4-yl)-phenyl]-propionamid;
50) N-[3-(6,8-Dichloro-2-methyl-1,2,3,4-tetrahydro-isochinolin-4-yl)-phenyl]-butyramid;
51) Pentansäure-[3-(6,8-dichloro-2-methyl-1,2,3,4-tetrahydro-isochinolin-4-yl)-phenyl]-amid;
52) N-[3-(6,8-Dichloro-2-methyl-1,2,3,4-tetrahydro-isochinolin-4-yl)-phenyl]-isobutyramid;
53) N-[3-(6,8-Dichloro-2-methyl-1,2,3,4-tetrahydro-isöchinolin-4-yl)-phenyl]-2,2-dimethyl-propionamid;
54) Cyclopropancarbonsäure-[3-(6,8-dichloro-2-methyl-1,2,3,4-tetrahydro-isochinolin-4-yl)-phenyl]-amid;
55) Cyclobutancarbonsäure-[3-(6,8-dichloro-2-methyl-1,2,3,4-tetrahydro-isochinolin-4-yl)- phenyl]-amid;
56) Cyclopentancarbonsäure-[3-(6,8-dichloro-2-methyl-1,2,3,4-tetrahydro-isochinolin-4-yl)-phenyl]-amid;
57) N-[3-(6,8-Dichloro-2-methyl-1,2,3,4-tetrahydro-isochinolin-4-yl)-phenyl]-2,2,2-trifluoro-acetamid;
58) 1-Acetyl-piperidin-4-carbonsäure-[3-(6,8-dichloro-2-methyl-1,2,3,4-tetrahydroisochinolin-4-yl)-phenyl]-amid;
59) N-[3-(6,8-Dichloro-2-methyl-1,2,3,4-tetrahydro-isochinolin-4-yl)-phenyl]-nicotinamid;
60) N-[3-(6,8-Dichloro-2-methyl-1,2,3,4-tetrahydro-isochinolin-4-yl)-phenyl]-methansulfonamid;
61) Ethansulfonsäure-[3-(6,8-dichloro-2-methyl-1,2,3,4-tetrahydro-isochinolin-4-yl)-phenyl]-amid;
62) N',N'-Dimethylamino-N-[3-(6,8-Dichloro-2-methyl-1,2,3,4-tetrahydro-isochinolin-4-yl)-phenyl]-sulfamid;
63) N-[2-(6,8-Dichloro-2-methyl-1,2,3,4-tetrahydro-isochinolin-4-yl)-phenyl]-propionamid;
64) N-[2-(6,8-Dichloro-2-methyl-1,2,3,4-tetrahydro-isochinolin-4-yl)-phenyl]-butyramid;
65) Pentansäure-[2-(6,8-dichloro-2-methyl-1,2,3,4-tetrahydro-isochinolin-4-yl)-phenyl]-amid;
66) N-[2-(6,8-Dichloro-2-methyl-1,2,3,4-tetrahydro-isochinolin-4-yl)-phenyl]-isobutyramid;
67) N-[2-(6,8-Dichloro-2-methyl-1,2,3,4-tetrahydro-isochinolin-4-yl)-phenyl]-2,2-dimethyl- propionamid;
68) Cyclopropancarbonsäure-[2-(6,8-dichloro-2-methyl-1,2,3,4-tetrahydro-isochinolin-4-yl)-phenyl]-amid;
69) Cyclobutancarbonsäure-[2-(6,8-dichloro-2-methyl-1,2,3,4-tetrahydro-isochinolin-4-yl)- phenyl]-amid;
70) Cyclopentancarbonsäure-[2-(6,8-dichloro-2-methyl-1,2,3,4-tetrahydro-isochinolin-4-yl)-phenyl]-amid;
71) N-[2-(6,8-Dichloro-2-methyl-1,2,3,4-tetrahydro-isochinolin-4-yl)-phenyl]-2,2,2-trifluoro-acetamid;
72) 1-Acetyl-piperidin-4-carbonsäure-[2-(6,8-dichloro-2-methyl-1,2,3,4-tetrahydroisochinolin-4-yl)-phenyl]-amid;
73) N-[2-(6,8-Dichloro-2-methyl-1,2,3,4-tetrahydro-isochinolin-4-yl)-phenyl]-methansulfonamid;
74) Ethansulfonsäure-[2-(6,8-dichloro-2-methyl-1,2,3,4-tetrahydro-isochinolin-4-yl)-phenyl]-amid;
75) N',N'-Dimethylamino-N-[2-(6,8-Dichloro-2-methyl-1,2,3,4-tetrahydro-isochinolin-4-yl)-phenyl]-sulfamid;
76) 1-[3-(6,8-Dichloro-2-methyl-1,2,3,4-tetrahydro-isochinolin-4-yl)-phenyl]-3-ethyl-harnstoff;
77) 1-[3-(6,8-Dichloro-2-methyl-1,2,3,4-tetrahydro-isochinolin-4-yl)-phenyl]-3-methyl-thioharnstoff;
78) 1-[2-(6,8-Dichloro-2-methyl-1,2,3,4-tetrahydro-isochinolin-4-yl)-phenyl]-3-ethyl-harnstoff;
79) 1-[2-(6,8-Dichloro-2-methyl-1,2,3,4-tetrahydro-isochinolin-4-yl)-phenyl]-3-methyl-thioharnstoff;
80) N-{5-[4-(6,8-Dichloro-2-methyl-1,2,3,4-tetrahydro-isochinolin-4-yl)-phenylsulfamoyl]-4-methyl-thiazol-2-yl}-acetamid;
81) N-{5-[3-(6,8-Dichloro-2-methyl-1,2,3,4-tetrahydro-isochinolin-4-yl)-phenylsulfamoyl]-4-methyl-thiazol-2-yl}-acetamid;
82) 1,2-Dimethyl-1H-imidazole-4-sulfonsäure-[4-(6,8-dichloro-2-methyl-1,2,3,4-tetrahydro- isochinolin-4-yl)-phenyl]-amid;
83) 1,2-Dimethyl-1H-imidazole-4-sulfonsäure-[3-(6,8-dichloro-2-methyl-1,2,3,4-tetrahydro- isochinolin-4-yl)-phenyl]-amid;
84) 5-Chloro-1,3-dimethyl-1H-pyrazol-4-sulfonsäure-[4-(6,8-dichloro-2-methyl-1,2,3,4-tetrahydro-isochinolin-4-yl)-phenyl]-amid;
85) 5-Chloro-1,3-dimethyl-1 H-pyrazol-4-sulfonsäure-[3-(6,8-dichloro-2-methyl-1,2,3,4-tetrahydro-isochinolin-4-yl)-phenyl]-amid;
86) 5-Bromo-thiophen-2-sulfonsäure-[4-(6,8-dichloro-2-methyl-1,2,3,4-tetrahydro-isochinolin-4-yl)-phenyl]-amid;
87) 5-Bromo-thiophen-2-sulfonsäure-[3-(6,8-dichloro-2-methyl-1,2,3,4-tetrahydroisochinolin-4-yl)-phenyl]-amid;
88) N-[4-(6,8-Dichloro-2-methyl-1,2,3,4-tetrahydro-isochinolin-4-yl)-phenyl]-C,C,C-trifluoro-methanesulfonamid;
89) N-[3-(6,8-Dichloro-2-methyl-1,2,3,4-tetrahydro-isochinolin-4-yl)-phenyl]-C,C,C-trifluoro-methanesulfonamid;
90) 2,2,2-Trifluoro-ethansulfonsäure-4-(6,8-dichloro-2-methyl-1,2,3,4-tetrahydroisochinolin-4-yl)-phenyl]-amid;
91) 2,2,2-Trifluoro-ethansulfonsäure-3-(6,8-dichloro-2-methyl-1,2,3,4-tetrahydroisochinolin-4-yl)-phenyl]-amid;
92) N-Ethyl-N'-4-(6,8-Dichloro-2-methyl-1,2,3,4-tetrahydro-isochinolin-4-yl)-benzolsulfonyl-harnstoff;
93) 2-Chloro-5-(6,8-dichloro-2-methyl-1,2,3,4-tetrahydro-isochinolin-4-yl)-benzolsulfonamid;
94) 2-Methyl-4-phenyl-6,8-bis-trifluoromethyl-1,2,3,4-tetrahydro-isochinolin;
95) 2-Amino-N-[4-(6,8-dichloro-2-methyl-1,2,3,4-tetrahydro- isochinolin -4-yl)-phenyl]-acetamid;
96) N-[4-(6,8-Dichloro-2-methyl-1,2,3,4-tetrahydro- isochinolin -4-yl)-phenyl]-2-methylamino- acetamid;
97) N-[4-(6,8-Dichloro-2-methyl-1,2,3,4-tetrahydro- isochinolin -4-yl)-phenyl]-2-dimethylamino-acetamid;
98) 2-Amino-N-[4-(6,8-dichloro-2-methyl-1,2,3,4-tetrahydro- isochinolin -4-yl)-phenyl]-propionamid;
99) 2-Amino-N-[4-(6,8-dichloro-2-methyl-1,2,3,4-tetrahydro- isochinolin -4-yl)-phenyl]-butyramid;
100) 2,6-Diamino-hexansäure-[4-(6,8-dichloro-2-methyl-1,2,3,4-tetrahydro- isochinolin -4-yl)- phenyl]-amid;
101) Pyrrolidine-2-carbonsäure-[4-(6,8-dichloro-2-methyl-1,2,3,4-tetrahydro-isochinolin -4- yl)-phenyl]-amid;
102) N-[4-(6,8-Dichloro-2-methyl-1,2,3,4-tetrahydro- isochinolin-4-yl)-phenyl]-isonicotinamid;
103) 1 H-Pyrrole-3-carbonsäure-[4-(6,8-dichloro-2-methyl-1,2,3,4-tetrahydroisochinolin-4- yl)-phenyl]-amid;
104) 1H-Pyrrol-2- carbonsäure-[4-(6,8-dichloro-2-methyl-1,2,3,4,-tetrahydroisochinolin-4- yl)-phenyl]-amid;
105) 1-Methyl-piperidin-4-carbonsäure-[4-(6,8-dichloro-2-methyl-1,2,3,4-tetrahydroisochinolin-4-yl)-phenyl]-amid;
106) 1,4-Dimethyl-1H-pyrrol-2-carbonsäure-[4-(6,8-dichloro-2-methyl-1,2,3,4-tetrahydro- isochinolin-4-yl)-phenyl]-amid;
107) 4-Nitro-1H-pyrrol-2-carbonsäure-[4-(6,8-dichloro-2-methyl-1,2,3,4-tetrahydroisochinolin-4-yl)-phenyl]-amid;
108) 2,5-Dimethyl-1H-pyrrol-3-carbonsäure-[4-(6,8-dichloro-2-methyl-1,2,3,4-tetrahydro- isochinolin-4-yl)-phenyl]-amid;
109) 1H-Imidazol-4-carbonsäure-[4-(6,8-dichloro-2-methyl-1,2,3,4-tetrahydroisochinolin-4-yl)-phenyl]-amid;
110) 1-Methansulfonyl-piperidin-4-carbonsäure-[4-(6,8-dichloro-2-methyl-1,2,3,4-tetrahydro- isochinolin-4-yl)-phenyl]-amid;
111) 3,5-Dimethyl-1H-pyrazol-4-carbonsäure-[4-(6,8-dichloro-2-methyl-1,2,3,4-tetrahydro- isochinolin-4-yl)-phenyl]-amid;
112) 1H-Pyrazol-4- carbonsäure-[4-(6,8-dichloro-2-methyl-1,2,3,4-tetrahydroisochinolin- 4-yl)-phenyl]-amid;
113) 3-Trifluoromethyl-1H-pyrazol-4- carbonsäure-[4-(6,8-dichloro-2-methyl-1,2,3,4-tetrahydro-isochinolin-4-yl)-phenyl]-amid;
114) N-[3-(6,8-Dichloro-2-methyl-1,2,3,4-tetrahydro-isochinolin-4-yl)-phenyl]-2-methylamino-acetamid;
115) N-[3-(6,8-Dichloro-2-methyl-1,2,3,4-tetrahydro-isochinolin-4-yl)-phenyl]-2-dimethylamino-acetamid;
116) 2-Amino-N-[3-(6,8-dichloro-2-methyl-1,2,3,4-tetrahydro-isochinolin-4-yl)-phenyl]-propionamid;
117) 2-Amino-N-[3-(6,8-dichloro-2-methyl-1,2,3,4-tetrahydro-isochinolin-4-yl)-phenyl]-butyramid;
118) 2,6-Diamino-hexansäure-[3-(6,8-dichloro-2-methyl-1,2,3,4-tetrahydro-isochinolin-4-yl)- phenyl]-amid;
119) Pyrrolidin-2- carbonsäure-[3-(6,8-dichloro-2-methyl-1,2,3,4-tetrahydro-isochinolin-4- yl)-phenyl]-amid;
120) N-[3-(6,8-Dichloro-2-methyl-1,2,3,4-tetrahydro-isochinolin-4-yl)-phenyl]-isonicotinamid;
121) 1H-Pyrrol-3- carbonsäure-[3-(6,8-dichloro-2-methyl-1,2,3,4-tetrahydro-isochinolin-4- yl)-phenyl]-amid;
122) 1H-Pyrrol-2-carbonsäure-[3-(6,8-dichloro-2-methyl-1,2,3,4-tetrahydro-isochinolin-4- yl)-phenyl]-amid;
123) 1-Methyl-piperidin-4- carbonsäure-[3-(6,8-dichloro-2-methyl-1,2,3,4-tetrahydroisochinolin-4-yl)-phenyl]-amid;
124) 1,4-Dimethyl-1H-pyrrol-2- carbonsäure-[3-(6,8-dichloro-2-methyl-1,2,3,4-tetrahydro- isochinolin-4-yl)-phenyl]-amid;
125) 4-Nitro-1H-pyrrol-2- carbonsäure-[3-(6,8-dichloro-2-methyl-1,2,3,4-tetrahydroisochinolin-4-yl)-phenyl]-amid;
126) 2,5-Dimethyl-1H-pyrrol-3- carbonsäure-[3-(6,8-dichloro-2-methyl-1,2,3,4-tetrahydro- isochuinolin-4-yl)-phenyl]-amid;
127) 1H-Imidazol-4- carbonsäure-[3-(6,8-dichloro-2-methyl-1,2,3,4-tetrahydro-isochuinolin- 4-yl)-phenyl]-amid;
128) 1-Methansulfonyl-piperidin-4- carbonsäure-[3-(6,8-dichloro-2-methyl-1,2,3,4-tetrahydro-isochinolin-4-yl)-phenyl]-amid;
129) 3,5-Dimethyl-1H-pyrazol-4- carbonsäure-[3-(6,8-dichloro-2-methyl-1,2,3,4-tetrahydro-isochinolin-4-yl)-phenyl]-amid;
130) 1H-Pyrazol-4- carbonsäure-[3-(6,8-dichloro-2-methyl-1,2,3,4-tetrahydroisochinolin- 4-yl)-phenyl]-amid;
131) 3-Trifluoromethyl-1H-pyrazol-4- carbonsäure-[3-(6,8-dichloro-2-methyl-1,2,3,4-tetrahydro-isochinolin-4-yl)-phenyl]-amid;
132) 1-[2-(6,8-Dichloro-2-methyl-1,2,3,4-tetrahydro- isochinolin-4-yl)-phenyl]-3-ethyl-thioharnstoff;
133) 1-[4-(6,8-Dichloro-2-methyl-1,2,3,4-tetrahydro-isochinolin-4-yl)-phenyl]-3-ethyl-thioharnstoff;
134) 1-[3-(6,8-Dichloro-2-methyl-1,2,3,4-tetrahydro-isochinolin-4-yl)-phenyl]-3-ethyl-thioharnstoff;
135) 3-[3-(6,8-Dichloro-2-methyl-1,2,3,4-tetrahydro-isochinolin-4-yl)-phenyl]-1,1-dimethyl-harnstoff;
136) 4-Methyl-piperazin-1-carbonsäure-[3-(6,8-dichloro-2-methyl-1,2,3,4-tetrahydroisochinolin-4-yl)-phenyl]-amid;
137) Piperidin-1-carbonsäure-[3-(6,8-dichloro-2-methyl-1,2,3,4-tetrahydro-isochinolin-4- yl)-phenyl]-amid;
138) Morpholin-4-carbonsäure-[3-(6,8-dichloro-2-methyl-1,2,3,4-tetrahydro-isochinolin-4- yl)-phenyl]-amid;
139) Pyrrolidin-1-carbonsäure-[3-(6,8-dichloro-2-methyl-1,2,3,4-tetrahydro-isochinolin-4- yl)-phenyl]-amid;
140) 3-[3-(6,8-Dichloro-2-methyl-1,2,3,4-tetrahydro-isochinolin-4-yl)-phenyl]-1,1-diethyl-harnstoff;
141) 1-[3-(6,8-Dichloro-2-methyl-1,2,3,4-tetrahydro-isochinolin-4-yl)-phenyl]-3-methyl-harnstoff;
142) 1-[3-(6,8-Dichloro-2-methyl-1,2,3,4-tetrahydro-isochinolin-4-yl)-phenyl]-3-(2-dimethylamino-ethyl)-harnstoff;
143) 1-[3-(6,8-Dichloro-2-methyl-1,2,3,4-tetrahydro-isochinolin-4-yl)-phenyl]-3-(tetrahydro-furan-3-yl)-harnstoff;
144) 1-[3-(6,8-Dichloro-2-methyl-1,2,3,4-tetrahydro-isochinolin-4-yl)-phenyl]-3-(tetrahydro-pyran-4-yl)-harnstoff;
145) 3-[3-(6,8-Dichloro-2-methyl-1,2,3,4-tetrahydro-isochinolin-4-yl)-phenyl]-1-methyl-1-(1-methyl-piperidin-4-yl)-hamstoff;
146) 3-[3-(6,8-Dichloro-2-methyl-1,2,3,4-tetrahydro-isochinolin-4-yl)-phenyl]-1-(3-dimethylamino-propyl)-1-methyl-harnstoff;
147) 3-[3-(6,8-Dichloro-2-methyl-1,2,3,4-tetrahydro-isochinolin-4-yl)-phenyl]-1-(2-dimethylamino-ethyl)-1-methyl-harnstoff;
148) 1-[3-(6,8-Dichloro-2-methyl-1,2,3,4-tetrahydro-isochinolin-4-yl)-phenyl]-3-(3-dimethylamino-propyl)-harnstoff;
149) 1-[3-(6,8-Dichloro-2-methyl-1,2,3,4-tetrahydro-isochinolin-4-yl)-phenyl]-3-(2-methoxy-ethyl)-harnstoff;
150) 1-[3-(6,8-Dichloro-2-methyl-1,2,3,4-tetrahydro-isochinolin-4-yl)-phenyl]-3-pyridin-3-yl-harnstoff;
151) 1-[3-(6,8-Dichloro-2-methyl-1,2,3,4-tetrahydro-isochinolin-4-yl)-phenyl]-3-pyridin-4-yl-harnstoff;
152) 4-Methyl-piperazin-1-carbonsäure-[2-(6,8-dichloro-2-methyl-1,2,3,4-tetrahydro-isoquinolin-4-yl)-phenyl]-amid;
153) 1-[2-(6,8-Dichloro-2-methyl-1,2,3,4-tetrahydro-isochinolin-4-yl)-phenyl]-3-methyl-harnstoff;
154) 3-[2-(6,8-Dichloro-2-methyl-1,2,3,4-tetrahydro-isochinolin-4-yl)-phenyl]-1,1-dimethyl-harnstoff;
155) 3-[2-(6,8-Dichloro-2-methyl-1,2,3,4-tetrahydro-isochinolin-4-yl)-phenyl]-1,1-diethyl-harnstoff;
156) Piperidin-1-carbonsäure-[2-(6,8-dichloro-2-methyl-1,2,3,4-tetrahydro-isochinolin-4- yl)-phenyl]-amid;
157) Morpholin-4-carbonsäure-[2-(6,8-dichloro-2-methyl-1,2,3,4-tetrahydro-isochinolin-4- yl)-phenyl]-amid;
158) Pyrrolidin-1-carbonsäure-[2-(6,8-dichloro-2-methyl-1,2,3,4-tetrahydro-isochinolin-4- yl)-phenyl]-amid;
159) 1-[2-(6,8-Dichloro-2-methyl-1,2,3,4-tetrahydro-isochinolin-4-yl)-phenyl]-3-(2-dimethylamino-ethyl)-harnstoff;
160) 4-Methyl-piperazin-1-carbonsäure-[4-(6,8-dichloro-2-methyl-1,2,3,4-tetrahydroisochinolin-4-yl)-phenyl]-amid;
161) Pyrrolidin-1-carbonsäure-[4-(6,8-dichloro-2-methyl-1,2,3,4-tetrahydro-isochinolin-4- yl)-phenyl]-amid;
162) 1-[4-(6,8-Dichloro-2-methyl-1,2,3,4-tetrahydro-isochinolin-4-yl)-phenyl]-3-methyl-harnstoff;
163) 3-[4-(6,8-Dichloro-2-methyl-1,2,3,4-tetrahydro-isochinolin-4-yl)-phenyl]-1,1-dimethyl-harnstoff;
164) 3-[4-(6,8-Dichloro-2-methyl-1,2,3,4-tetrahydro-isochinolin-4-yl)-phenyl]-1,1-diethyl-harnstoff;
165) 1-[4-(6,8-Dichloro-2-methyl-1,2,3,4-tetrahydro-isochinolin-4-yl)-phenyl]-3-(2-dimethylamino-ethyl)-harnstoff;
166) Piperidin-1-carbonsäure-[4-(6,8-dichloro-2-methyl-1,2,3,4-tetrahydro-isochinolin-4- yl)-phenyl]-amid;
167) Morpholin-4-carbonsäure-[4-(6,8-dichloro-2-methyl-1,2,3,4-tetrahydro-isochinolin-4- yl)-phenyl]-amid;
168) N-[4-(6,8-Dichloro-2-methyl-1,2,3,4-tetrahydro-isochinolin-4-yl)-phenyl]-formamid;
169) [4-(6,8-Dichloro-2-methyl-1,2,3,4-tetrahydro-isochinolin-4-yl)-phenyl]-methylamin;
170) 1-[4-(6,8-Dichloro-2-methyl-1,2,3,4-tetrahydro-isochinolin-4-yl)-phenyl]-1,3-dimethyl-harnstoff;
171) 4-Methyl-piperazin-1-carbonsäure-[4-(6,8-dichloro-2-methyl-1,2,3,4-tetrahydroisochinolin-4-yl)-phenyl]-methyl-amid;
172) 1-[4-(6,8-Dichloro-2-methyl-1,2,3,4-tetrahydro-isochinolin-4-yl)-phenyl]-1,3,3-trimethyl-harnstoff;
173) Piperidin-1-carbonsäure-[4-(6,8-dichloro-2-methyl-1,2,3,4-tetrahydro-isochinolin-4-yl)-phenyl]-methyl-amid;
174) Morpholin-4-carbonsäure-[4-(6,8-dichloro-2-methyl-1,2,3,4-tetrahydro-isochinolin-4- yl)-phenyl]-methyl-amid;
175) Pyrrolidin-1-carbonsäure-[4-(6,8-dichloro-2-methyl-1,2,3,4-tetrahydro-isochinolin-4- yl)-phenyl]-methyl-amid;
176) 1-[4-(6,8-Dichloro-2-methyl-1,2,3,4-tetrahydro-isochinolin-4-yl)-phenyl]-3-(2-dimethylamino-ethyl)-1-methyl-harnstoff;
177) 1-[4-(6,8-Dichloro-2-methyl-1,2,3,4-tetrahydro-isochinolin-4-yl)-phenyl]-3,3-diethyl-1-methyl-harnstoff;
178) N-[3-(6,8-Dichloro-2-methyl-1,2,3,4-tetrahydro-isochinolin-4-yl)-phenyl]-formamid;
179) [3-(6,8-Dichloro-2-methyl-1,2,3,4-tetrahydro-isochinolin-4-yl)-phenyl]-methyl-amin;
180) Pyrrolidin-1-carbonsäure-[3-(6,8-dichloro-2-methyl-1,2,3,4-tetrahydro-isochinolin-4- yl)-phenyl]-methyl-amid;
181) Piperidin-1-carbonsäure-[3-(6,8-dichloro-2-methyl-1,2,3,4-tetrahydro-isochinolin-4- yl)-phenyl]-methyl-amid;
182) 1-[3-(6,8-Dichloro-2-methyl-1,2,3,4-tetrahydro-isochinolin-4-yl)-phenyl]-1,3,3-trimethyl-harnstoff;
183) 1-[3-(6,8-Dichloro-2-methyl-1,2,3,4-tetrahydro-isochinolin-4-yl)-phenyl]-1,3-dimethyl-harnstoff;
184) Morpholin-4-carbonsäure-[3-(6,8-dichloro-2-methyl-1,2,3,4-tetrahydro-isochinolin-4- yl)-phenyl]-methyl-amid;
185) 4-Methyl-piperazin-1-carbonsäure-[3-(6,8-dichloro-2-methyl-1,2,3,4-tetrahydroisochinolin-4-yl)-phenyl]-methyl-amid;
186) 1-[3-(6,8-Dichloro-2-methyl-1,2,3,4-tetrahydro-isochinolin-4-yl)-phenyl]-3-(2-dimethylamino-ethyl)-1-methyl-harnstoff;
187) 1-[3-(6,8-Dichloro-2-methyl-1,2,3,4-tetrahydro-isochinolin-4-yl)-phenyl]-3,3-diethyl-1-methyl-harnstoff;
188) [3-(6,8-Dichloro-2-methyl-1,2,3,4-tetrahydro-isochinolin-4-yl)-phenyl]-carbaminsäure-2-dimethylamino-ethyl-ester;
189) [4-(6,8-Dichloro-2-methyl-1,2,3,4-tetrahydro-isochinolin-4-yl)-phenyl]-carbaminsäure-2-dimethylamino-ethyl-ester;
190) [2-(6,8-Dichloro-2-methyl-1,2,3,4-tetrahydro-isochinolin-4-yl)-phenyl]-carbaminsäure-2-dimethylamino-ethyl-ester;
191) [3-(6,8-Dichloro-2-methyl-1,2,3,4-tetrahydro-isochinolin-4-yl)-phenyl]-carbaminsäure-methyl-ester;
192) [3-(6,8-Dichloro-2-methyl-1,2,3,4-tetrahydro-isochinolin-4-yl)-phenyl]-carbaminsäure-ethyl-ester;
193) [3-(6,8-Dichloro-2-methyl-1,2,3,4-tetrahydro-isochinolin-4-yl)-phenyl]-carbaminsäure-isopropyl-ester;
194) [3-(6,8-Dichloro-2-methyl-1,2,3,4-tetrahydro-isochinolin-4-yl)-phenyl]-carbaminsäure-2,2- dimethyl-propyl-ester;
195) [4-(6,8-Dichloro-2-methyl-1,2,3,4-tetrahydro-isochinolin-4-yl)-phenyl]-carbaminsäure-methyl-ester;
196) [4-(6,8-Dichloro-2-methyl-1,2,3,4-tetrahydro-isochinolin-4-yl)-phenyl]-carbaminsäu re-isopropyl-ester;
197) [4-(6,8-Dichloro-2-methyl-1,2,3,4-tetrahydro-isochinolin-4-yl)-phenyl]-carbaminsäure-2,2- dimethyl-propyl-ester;
198) [4-(6,8-Dichloro-2-methyl-1,2,3,4-tetrahydro-isochinolin-4-yl)-phenyl]-carbaminsäure-ethyl-ester;
199) (R)-N-[3-(6,8-Dichloro-2-methyl-1,2,3,4-tetrahydro-isochinolin-4-yl)-phenyl]-methansulfonamid;
200) (S)-N-[3-(6,8-Dichloro-2-methyl-1,2,3,4-tetrahydro-isochinolin-4-yl)-phenyl]-methansulfonamid;
201) (R)-1-[2-(6,8-Dichloro-2-methyl-1,2,3,4-tetrahydro-isochinolin-4-yl)-phenyl]-3-ethyl-harnstoff;
202) (S)-1-[2-(6,8-Dichloro-2-methyl-1,2,3,4-tetrahydro-isochinolin-4-yl)-phenyl]-3-ethyl-harnstoff;
203) N-[3-(6,8-Difluoro-2-methyl-1,2,3,4-tetrahydro-isochinolin-4-yl)-phenyl]-acetamid;
204) 4-(3-Bromo-phenyl)-6,8-dichloro-2-methyl-1,2,3,4-tetrahydro-isochinolin;
205) 1-[3-(6,8-Dichloro-2-methyl-1,2,3,4-tetrahydro-isochinolin-4-yl)-phenyl]-3-(2-hydroxy-ethyl)-harnstoff;
206) 3-(6,8-Dichloro-2-methyl-1,2,3,4-tetrahydro-isochinolin-4-yl)-benzoesäure-ethylester;
207) 3-(6,8-Dichloro-2-methyl-1,2,3,4-tetrahydro-isochinolin-4-yl)-benzoesäure. sowie deren pharmazeutisch verträgliche Salze.

Außerordentlich ganz besonders bevorzugt sind Verbindungen aus der Gruppe
1) N-[4-(6,8-Dichloro-2-methyl-1,2,3,4-tetrahydro-isochinolin-4-yl)-phenyl]-acetamid;
2) 4-(6,8-Dichloro-2-methyl-1,2,3,4-tetrahydro-isochinolin-4-yl)-benzolsulfonamid;
3) 3-(6,8-Dichloro-2-methyl-1,2,3,4-tetrahydro-isochinolin-4-yl)-benzolsulfonamid;
4) 1-[4-(6,8-Dichloro-2-methyl-1,2,3,4-tetrahydro-isochinolin-4-yl)-phenyl]-3-methyl-thioharnstoff;
5) 1-[4-(6,8-Dichloro-2-methyl-1,2,3,4-tetrahydro-isochinolin-4-yl)-phenyl]-3-ethyl-harnstoff;
6) N-[4-(6-Bromo-8-chloro-2-methyl-1,2,3,4-tetrahydro-isochinolin-4-yl)-phenyl]-acetamid;
7) N-[3-(6,8-Dichloro-2-methyl-1,2,3,4-tetrahydro-isochinolin-4-yl)-phenyl]-acetamid;
8) N-[4-(6,8-Dichloro-2-methyl-1,2,3,4-tetrahydro-isochinolin-4-yl)-phenyl]-propionamid;
9) 1-Acetyl-piperidin-4-carbonsäure-[4-(6,8-dichloro-2-methyl-1,2,3,4-tetrahydroisochinolin-4-yl)-phenyl]-amid;
10) N-[4-(6,8-Dichloro-2-methyl-1,2,3,4-tetrahydro-isochinolin-4-yl)-phenyl]-methansulfonamid;
11) Ethansulfonsäure-[4-(6,8-dichloro-2-methyl-1,2,3,4-tetrahydro-isochinolin-4-yl)-phenyl]-amid;
12) N',N'-Dimethylamino-N-[4-(6,8-Dichloro-2-methyl-1,2,3,4-tetrahydro-isochinolin-4-yl)-phenyl]-sulfamid;
13) N-[3-(6,8-Dichloro-2-methyl-1,2,3,4-tetrahydro-isochinolin-4-yl)-phenyl]-propionamid;
14) N-[3-(6,8-Dichloro-2-methyl-1,2,3,4-tetrahydro-isochinolin-4-yl)-phenyl]-butyramid;
15) N-[3-(6,8-Dichloro-2-methyl-1,2,3,4-tetrahydro-isochinolin-4-yl)-phenyl]-isobutyramid;
16) Cyclopropancarbonsäure-[3-(6,8-dichloro-2-methyl-1,2,3,4-tetrahydro-isochinolin-4-yl)-phenyl]-amid;
17) Cyclobutancarbonsäure-[3-(6,8-dichloro-2-methyl-1,2,3,4-tetrahydro-isochinolin-4-yl)- phenyl]-amid;
18) N-[3-(6,8-Dichloro-2-methyl-1,2,3,4-tetrahydro-isochinolin-4-yl)-phenyl]-2,2,2-trifluoro-acetamid;
19) 1-Acetyl-piperidin-4-carbonsäure-[3-(6,8-dichloro-2-methyl-1,2,3,4-tetrahydroisochinolin-4-yl)-phenyl]-amid;
20) N-[3-(6,8-Dichloro-2-methyl-1,2,3,4-tetrahydro-isochinolin-4-yl)-phenyl]-nicotinamid;
21) N-[3-(6,8-Dichloro-2-methyl-1,2,3,4-tetrahydro-isochinolin-4-yl)-phenyl]-methansulfonamid;
22) Ethansulfonsäure-[3-(6,8-dichloro-2-methyl-1,2,3,4-tetrahydro-isochinolin-4-yl)-phenyl]-amid;
23) N',N'-Dimethylamino-N-[3-(6,8-Dichloro-2-methyl-1,2,3,4-tetrahydro-isochinolin-4-yl)-phenyl]-sulfamid;
24) Cyclopropancarbonsäure-[2-(6,8-dichloro-2-methyl-1,2,3,4-tetrahydro-isochinolin-4-yl)-phenyl]-amid;
25) 1-Acetyl-piperidin-4-carbonsäure-[2-(6,8-dichloro-2-methyl-1,2,3,4-tetrahydroisochinolin-4-yl)-phenyl]-amid;
26) 1-[3-(6,8-Dichloro-2-methyl-1,2,3,4-tetrahydro-isochinolin-4-yl)-phenyl]-3-ethyl-harnstoff;
27) 1-[3-(6,8-Dichloro-2-methyl-1,2,3,4-tetrahydro-isochinolin-4-yl)-phenyl]-3-methyl-thioharnstoff;
28) 1-[2-(6,8-Dichloro-2-methyl-1,2,3,4-tetrahydro-isochinolin-4-yl)-phenyl]-3-ethyl-harnstoff;
29) 1-[2-(6,8-Dichloro-2-methyl-1,2,3,4-tetrahydro-isochinolin-4-yl)-phenyl]-3-methyl-thioharnstoff;
30) N-{5-[3-(6,8-Dichloro-2-methyl-1,2,3,4-tetrahydro-isochinolin-4-yl)-phenylsulfamoyl]-4-methyl-thiazol-2-yl}-acetamid;
31) 1,2-Dimethyl-1 H-imidazole-4-sulfonsäure-[3-(6,8-dichloro-2-methyl-1,2,3,4-tetrahydro- isochinolin-4-yl)-phenyl]-amid;
32) N-[4-(6,8-Dichloro-2-methyl-1,2,3,4-tetrahydro-isochinolin-4-yl)-phenyl]-C,C,C-trifluoro-methanesulfonamid;
33) N-[3-(6,8-Dichloro-2-methyl-1,2,3,4-tetrahydro-isochinolin-4-yl)-phenyl]-C,C,C-trifluoro-methanesulfonamid;
34) N-Ethyl-N'-4-(6,8-Dichloro-2-methyl-1,2,3,4-tetrahydro-isochinolin-4-yl)-benzolsulfonyl-harnstoff;
35) N-[4-(6,8-Dichloro-2-methyl-1,2,3,4-tetrahydro- isochinolin -4-yl)-phenyl]-2-dimethylamino-acetamid;
36) 2,6-Diamino-hexansäure-[4-(6,8-dichloro-2-methyl-1,2,3,4-tetrahydro-isochinolin -4-yl)- phenyl]-amid;
37) 1 H-Pyrrole-3-carbonsäure-[4-(6,8-dichloro-2-methyl-1,2,3,4-tetrahydroisochinolin-4-yl)-phenyl]-amid;
38) 1-Methyl-piperidin-4-carbonsäure-[4-(6,8-dichloro-2-methyl-1,2,3,4-tetrahydroisochinolin-4-yl)-phenyl]-amid;
39) 1-Methansulfonyl-piperidin-4-carbonsäure-[4-(6,8-dichloro-2-methyl-1,2,3,4-tetrahydro- isochinolin-4-yl)-phenyl]-amid;
40) 1 H-Pyrazol-4- carbonsäure-[4-(6,8-dichloro-2-methyl-1,2,3,4-tetrahydroisochinolin- 4-yl)-phenyl]-amid;
41) N-[3-(6,8-Dichloro-2-methyl-1,2,3,4-tetrahydro-isochinolin-4-yl)-phenyl]-2-methylamino-acetamid;
42) N-[3-(6,8-Dichloro-2-methyl-1,2,3,4-tetrahydro-isochinolin-4-yl)-phenyl]-2-dimethylamino-acetamid;
43) 2-Amino-N-[3-(6,8-dichloro-2-methyl-1,2,3,4-tetrahydro-isochinolin-4-yl)-phenyl]-propionamid;
44) 2-Amino-N-[3-(6,8-dichloro-2-methyl-1,2,3,4-tetrahydro-isochinolin-4-yl)-phenyl]-butyramid;
45) 2,6-Diamino-hexansäure-[3-(6,8-dichloro-2-methyl-1,2,3,4-tetrahydro-isochinolin-4-yl)- phenyl]-amid;
46) 1-Methyl-piperidin-4- carbonsäure-[3-(6,8-dichloro-2-methyl-1,2,3,4-tetrahydroisochinolin-4-yl)-phenyl]-amid;
47) 1H-Imidazol-4-carbonsäure-[3-(6,8-dichloro-2-methyl-1,2,3,4-tetrahydro-isochuinolin- 4-yl)-phenyl]-amid;
48) 1-Methansulfonyl-piperidin-4-carbonsäure-[3-(6,8-dichloro-2-methyl-1,2,3,4-tetrahydro-isochinolin-4-yl)-phenyl]-amid;
49) 3,5-Dimethyl-1H-pyrazol-4-carbonsäure-[3-(6,8-dichloro-2-methyl-1,2,3,4-tetrahydro-isochinolin-4-yl)-phenyl]-amid;
50) 1 H-Pyrazol-4- carbonsäure-[3-(6,8-dichloro-2-methyl-1,2,3,4-tetrahydroisochinolin- 4-yl)-phenyl]-amid;
51) 3-[3-(6,8-Dichloro-2-methyl-1,2,3,4-tetrahydro-isochinolin-4-yl)-phenyl]-1,1-dimethyl-harnstoff;
52) 4-Methyl-piperazin-1-carbonsäure-[3-(6,8-dichloro-2-methyl-1,2,3,4-tetrahydroisochinolin-4-yl)-phenyl]-amid;
53) Piperidin-1-carbonsäure-[3-(6,8-dichloro-2-methyl-1,2,3,4-tetrahydro-isochinolin-4- yl)-phenyl]-amid;
54) Morpholin-4-carbonsäure-[3-(6,8-dichloro-2-methyl-1,2,3,4-tetrahydro-isochinolin-4- yl)-phenyl]-amid;
55) Pyrrolidin-1-carbonsäure-[3-(6,8-dichloro-2-methyl-1,2,3,4-tetrahydro-isochinolin-4- yl)-phenyl]-amid;
56) 3-[3-(6,8-Dichloro-2-methyl-1,2,3,4-tetrahydro-isochinolin-4-yl)-phenyl]-1,1-diethyl-harnstoff;
57) 1-[3-(6,8-Dichloro-2-methyl-1,2,3,4-tetrahydro-isochinolin-4-yl)-phenyl]-3-methyl-harnstoff;
58) 1-[3-(6,8-Dichloro-2-methyl-1,2,3,4-tetrahydro-isochinolin-4-yl)-phenyl]-3-(2-dimethylamino-ethyl)- harnstoff;
59) 1-[3-(6,8-Dichloro-2-methyl-1,2,3,4-tetrahydro-isochinolin-4-yl)-phenyl]-3-(tetrahydro-furan-3-yl)-harnstoff;
60) 1-[3-(6,8-Dichloro-2-methyl-1,2,3,4-tetrahydro-isochinolin-4-yl)-phenyl]-3-(tetrahydro-pyran-4-yl)-harnstoff;
61) 3-[3-(6,8-Dichloro-2-methyl-1,2,3,4-tetrahydro-isochinolin-4-yl)-phenyl]-1-methyl-1-(1-methyl-piperidin-4-yl)-harnstoff;
62) 3-[3-(6,8-Dichloro-2-methyl-1,2,3,4-tetrahydro-isochinolin-4-yl)-phenyl]-1-(3-dimethylamino-propyl)-1-methyl-harnstoff;
63) 3-[3-(6,8-Dichloro-2-methyl-1,2,3,4-tetrahydro-isochinolin-4-yl)-phenyl]-1-(2-dimethylamino-ethyl)-1-methyl-harnstoff;
64) 1-[3-(6,8-Dichloro-2-methyl-1,2,3,4-tetrahydro-isochinolin-4-yl)-phenyl]-3-(3-dimethylamino-propyl)-harnstoff;
65) 1-[3-(6,8-Dichloro-2-methyl-1,2,3,4-tetrahydro-isochinolin-4-yl)-phenyl]-3-(2-methoxy-ethyl)-harnstoff;
66) 1-[3-(6,8-Dichloro-2-methyl-1,2,3,4-tetrahydro-isochinolin-4-yl)-phenyl]-3-pyridin-3-yl-harnstoff;
67) 1-[3-(6,8-Dichloro-2-methyl-1,2,3,4-tetrahydro-isochinolin-4-yl)-phenyl]-3-pyridin-4-yl-harnstoff;
68) 4-Methyl-piperazin-1-carbonsäure-[2-(6,8-dichloro-2-methyl-1,2,3,4-tetrahydro-isoquinolin-4-yl)-phenyl]-amid;
69) 1-[2-(6,8-Dichloro-2-methyl-1,2,3,4-tetrahydro-isochinolin-4-yl)-phenyl]-3-methyl-harnstoff;
70) 1-[2-(6,8-Dichloro-2-methyl-1,2,3,4-tetrahydro-isochinolin-4-yl)-phenyl]-3-(2-dimethylamino-ethyl)-harnstoff;
71) 4-Methyl-piperazin-1-carbonsäure-[4-(6,8-dichloro-2-methyl-1,2,3,4-tetrahydroisochinolin-4-yl)-phenyl]-amid;
72) 1-[4-(6,8-Dichloro-2-methyl-1,2,3,4-tetrahydro-isochinolin-4-yl)-phenyl]-3,methyl-harnstoff;
73) 3-[4-(6,8-Dichloro-2-methyl-1,2,3,4-tetrahydro-isochinolin-4-yl)-phenyl]-1,1-dimethyl-harnstoff;
74) 3-[4-(6,8-Dichloro-2-methyl-1,2,3,4-tetrahydro-isochinolin-4-yl)-phenyl]-1,1-diethyl-harnstoff;
75) 1-[4-(6,8-Dichloro-2-methyl-1,2,3,4-tetrahydro-isochinolin-4-yl)-phenyl]-3-(2-dimethylamino-ethyl)-harnstoff;
76) Morpholin-4-carbonsäure-[4-(6,8-dichloro-2-methyl-1,2,3,4-tetrahydro-isochinolin-4- yl)-phenyl]-amid;
77) N-[4-(6,8-Dichloro-2-methyl-1,2,3,4-tetrahydro-isochinolin-4-yl)-phenyl]-formamid;
78) N-[3-(6,8-Dichloro-2-methyl-1,2,3,4-tetrahydro-isochmolin-4-yl)-phenyl]-formamid;
79) 1-[3-(6,8-Dichloro-2-methyl-1,2,3,4-tetrahydro-isochinolin-4-yl)-phenyl]-1,3,3-trimethyl-harnstoff;
80) 1-[3-(6,8-Dichloro-2-methyl-1,2,3,4-tetrahydro-isochinolin-4-yl)-phenyl]-1,3-dimethyl-harnstoff;
81) Morpholin-4-carbonsäure-[3-(6,8-dichloro-2-methyl-1,2,3,4-tetrahydro-isochinolin-4- yl)-phenyl]-methyl-amid;
82) 4-Methyl-piperazin-1-carbonsäure-[3-(6,8-dichloro-2-methyl-1,2,3,4-tetrahydroisochinolin-4-yl)-phenyl]-methyl-amid;
83) 1-[3-(6,8-Dichioro-2-methyl-1,2,3,4-tetrahydro-isochinolin-4-yl)-phenyl]-3-(2-dimethylamino-ethyl)-1-methyl-harnstoff;
84) [3-(6,8-Dichloro-2-methyl-1,2,3,4-tetrahydro-isochinolin-4-yl)-phenyl]-carbaminsäure-2-dimethylamino-ethyl-ester;
85) [4-(6,8-Dichloro-2-methyl-1,2,3,4-tetrahydro-isochinolin-4-yl)-phenyl]-carbaminsäure-2-dimethylamino-ethyl-ester;
86) [2-(6,8-Dichloro-2-methyl-1,2,3,4-tetrahydro-isochinolin-4-yl)-phenyl]-carbaminsäure-2-dimethylamino-ethyl-ester;
87) [3-(6,8-Dichloro-2-methyl-1,2,3,4-tetrahydro-isochinolin-4-yl)-phenyl]-carbaminsäure-methyl-ester;
88) (R oder S)-N-[3-(6,8-Dichloro-2-methyl-1,2,3,4-tetrahydro-isochinolin-4-yl)-phenyl]- methansulfonamid;
89) (R oder S)-1-[2-(6,8-Dichloro-2-methyl-1,2,3,4-tetrahydro-isochinolin-4-yl)-phenyl]-3-ethyl-hamstoff;
90) 1-[3-(6,8-Dichloro-2-methyl-1,2,3,4-tetrahydro-isochinolin-4-yl)-phenyl]-3-(2-hydroxy-ethyl)-harnstoff;
sowie deren pharmazeutisch verträgliche Salze.

Inhibierung des Natrium-Protonen-Austauschers Subtyp III (NHE3) beeinflusst werden können, worin bedeuten:
- R1, R2, R3 und R4: unabhängig voneinander H, F, Cl, Br, I, CN, NO₂, OH, NH₂, CₐH₂ₐ₊₁, C_{qq}H_{2qq-1}, OC_{b}H_{2b+1}, COOR10, OCOR10, COR10 oder Oₓ-(CH₂)_{y}-Phenyl;
a und b in den Gruppen CₐH₂ₐ₊₁, und OC_{b}H_{2b+1} unabhängig voneinander 1, 2, 3, 4, 5, 6, 7 oder 8, wobei ein oder mehrere H-Atome durch F-Atome ersetzt sein können;
qq 3, 4, 5, 6, 7 oder 8, wobei ein oder mehrere H-Atome durch F-Atome ersetzt sein können;
R10 H oder C_{c}H_{2c+1};
c 1, 2, 3, 4, 5, 6, 7 oder 8, wobei ein oder mehrere H-Atome durch F-Atome ersetzt sein dürfen,
x Null oder 1;
y Null, 1, 2, 3 oder 4;
wobei der Phenylring in der Gruppe Oₓ-(CH₂)_{y}-Phenyl unsubstituiert ist oder substituiert mit 1 - 3 Substituenten ausgewählt aus der Gruppe bestehend aus F, Cl, Br, CN, NO₂, OH, NH₂ oder C_{d}H_{2d+1};
d 1, 2, 3, oder 4, wobei ein oder mehrere H-Atome durch F-Atome ersetzt sein dürfen;
oder
- R1, R2, R3 und R4: unabhängig voneinander Heteroaryl, wobei Null, 1, 2, 3 oder 4 N-Atome, Null oder 1 Sauerstoff-Atom oder Null oder 1 S-Atom als Ringatome enthalten sein können;
oder
- R1, R2, R3 und R4: unabhängig voneinander CONR11R12 oder NR11R12;
R11 und R12 unabhängig voneinander H, CₑH₂ₑ₊₁, CᵣᵣH₂ᵣᵣ₋₁;
e 1, 2, 3, 4, 5, 6, 7 oder 8,
rr 3, 4, 5, 6, 7, oder 8,
wobei in den Gruppen CₑH₂ₑ₊₁ und CᵣᵣH₂ᵣᵣ₋₁ ein oder mehrere H-Atome durch F-Atome ersetzt sein dürfen und ein oder mehrere CH₂-Gruppen durch O oder NR13 ersetzt sein können;
R13 H oder C_{f}H_{2f+1};
f 1, 2, 3, oder 4, wobei ein oder mehrere H-Atome durch F-Atome ersetzt sein dürfen; oder
R13 und eine CH₂-Gruppe von R11 oder R12 gemeinsam mit dem N-Atom, an das sie gebunden sind, einen 5- oder 6-gliedrigen Ring; oder
R11 und R12 gemeinsam mit dem N-Atom, an das sie gebunden sind, einen 5-, 6- oder 7-gliedrigen Ring; oder
R11 und R12 unabhängig voneinander COR14, CSR14 oder SO₂R14;
R14 C_{g}H_{2g+1};
g 1, 2, 3, 4, 5, 6, 7 oder 8, wobei ein oder mehrere H-Atome durch F-Atome ersetzt sein können und ein oder mehrere CH₂-Gruppen durch O oder NR13 ersetzt sein können,
oder
- R1, R2, R3 und R4: unabhängig voneinander -Oₕ-SOⱼ-R15, wobei
h Null oder 1;
j Null, 1 oder 2;
R15 CₖH₂ₖ₊₁, OH, OCₗH₂ₗ₊₁ oder NR17R18;
k 1, 2, 3, 4, 5, 6, 7 oder 8, wobei ein oder mehrere H-Atome durch F-Atome ersetzt sein können;
l 1, 2, 3, 4, 5, 6,7 oder 8, wobei ein oder mehrere H-Atome durch F-Atome ersetzt sein können;
R17 und R18 unabhängig voneinander H oder CₘH₂ₘ₊₁;
m 1, 2, 3, 4, 5, 6, 7 oder 8, wobei in der Gruppe CₘH₂ₘ₊₁ ein oder mehrere H-Atome durch F-Atome ersetzt sein können und ein oder mehrere CH₂-Gruppen durch O, CO, CS oder NR19 ersetzt sein können;
R19 H oder CₙH₂ₙ₊₁;
n 1, 2, 3 oder 4;
wobei in CₙH₂ₙ₊₁ ein oder mehrere H-Atome durch F-Atome ersetzt sein können; oder
R17 und R18 gemeinsam mit dem N-Atom, an das sie gebunden sind, einen 5-, 6- oder 7-gliedrigen Ring;
oder
R19 und eine CH₂-Gruppe von R17 oder R18 gemeinsam mit dem N-Atom, an das sie gebunden sind, einen 5- oder 6-gliedrigen Ring;
- R5: H, CₚH₂ₚ₊₁, CₛₛH₂ₛₛ₋₁, COR20 oder SO₂R20;
p 1, 2, 3, 4, 5, 6, 7 oder 8,
ss 3, 4, 5, 6, 7 oder 8, wobei in CₚH₂ₚ₊₁ und CₛₛH₂ₛₛ₋₁ ein oder mehrere H-Atome durch F-Atome ersetzt sein können;
R20 C_{q}H_{2q+1};
q 1, 2, 3, 4, 5, 6, 7 oder 8, wobei in C_{q}H_{2q+1} ein oder mehrere H-Atome durch F-Atome ersetzt sein können, und eine oder mehrere CH₂-Gruppen durch O oder
NR21 ersetzt sein können;
R21 H oder CᵣH₂ᵣ₊₁;
r 1, 2, 3 oder 4; wobei in CᵣH₂ᵣ₊₁ ein oder mehrere H-Atome durch F-Atome ersetzt sein können;
- R6: H, F, Cl, Br, I, CₛH₂ₛ₊₁, C_{dd}H_{2dd-1}, OH, OCₜH₂ₜ₊₁ oder OCOR22;
s und t unabhängig voneinander 1, 2, 3, 4, 5, 6, 7 oder 8;
dd 3, 4, 5, 6, 7 oder 8, wobei in CₛH₂ₛ₊₁, C_{dd}H_{2dd-1} und OCₜH₂ₜ₊₁ ein oder mehrere H-Atome durch F-Atome ersetzt sein können;
R22 CᵤH₂ᵤ₊₁;
u 1, 2, 3 oder 4;
wobei in CᵤH₂ᵤ₊₁ ein oder mehrere H-Atome durch F-Atome ersetzt sein können;
- R7, R8 und R9: unabhängig voneinander -Oᵥ-SO_{w}-R23;
v Null oder 1;
w Null, 1 oder 2;
R23 CₙₙH₂ₙₙ₊₁, CₘₘH₂ₘₘ₋₁, OH, OCₚₚH₂ₚₚ₊₁ oder NR25R26;
nn und pp unabhängig voneinander 1, 2, 3, 4, 5, 6, 7 oder 8,
mm 3, 4, 5, 6, 7 oder 8,
wobei in CₙₙH₂ₙₙ₊₁, CₘₘH₂ₘₘ₋₁ und OCₚₚH₂ₚₚ₊₁ ein oder mehrere H-Atome durch F-Atome ersetzt sein können;
R25 und R26 unabhängig voneinander H, CN oder C_{z}H_{2z+1}, C_{zz}H_{2zz-1};
z 1, 2, 3, 4, 5, 6, 7 oder 8;
zz 3, 4, 5, 6, 7 oder 8, wobei ein oder mehrere H-Atome durch F-Atome ersetzt sein dürfen und in C_{z}H_{2z+1} ein oder mehrere H-Atome durch F-Atome ersetzt sein können und ein oder mehrere CH₂-Gruppen durch O, CO, CS oder NR27 ersetzt sein können;
R27 H oder CₐₐH₂ₐₐ₊₁;
aa 1, 2, 3 oder 4;
wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5-, 6- oder 7-gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
- R7, R8 und R9: unabhängig voneinander NR32COR30, NR32CSR30 oder NR32SO_{bb}R30;
R30 H, C_{cc}H_{2cc+1}, C_{yy}H_{2yy-1}, Pyrrolidinyl oder Piperidinyl, in welchen Ringen eine CH₂-Gruppe durch O oder NR33 ersetzt sein kann;
R32 und R33 unabhängig voneinander H oder CₕH₂ₕ₊₁;
bb 2 oder 3;
cc 1, 2, 3, 4, 5, 6, 7 oder 8;
yy 3, 4, 5, 6, 7 oder 8;
h 1, 2, 3, 4, 5, 6, 7 oder 8,
wobei in CₕH₂ₕ₊₁ ein oder mehrere H-Atome durch F-Atome ersetzt sein dürfen und in den Gruppen C_{cc}H_{2cc+1} und C_{yy}H_{2yy-1} ein oder mehrere H-Atome durch F-Atome ersetzt sein können und eine oder mehrere (CH₂)- Gruppen durch NR31 ersetzt sein können und eine (CH₂)- Gruppe durch O ersetzt sein kann;
R31 H, CₖₖH₂ₖₖ₊₁, COR65 oder SO₂R65;
kk 1, 2, 3, oder 4;
wobei ein oder mehrere H-Atome durch F-Atome ersetzt sein können,
R65 H, CₓₓH₂ₓₓ₊₁;
xx 1, 2, 3 oder 4, wobei ein oder mehrere H-Atome durch F-Atome ersetzt sein dürfen; oder
R31 zusammen mit einer CH₂-Gruppe von R30 einen 5-, 6- oder 7-gliedrigen Ring bildet; oder
R30 ein 5- oder 6-gliedriges Heteroaryl mit 1, 2, 3 oder 4 N-Atomen, Null oder 1 S-Atomen und Null oder 1 O-Atomen,
das unsubstituiert ist oder substituiert mit bis zu drei Substituenten ausgewählt aus der Gruppe bestehend aus F, Cl, Br, I, CₒₒH₂ₒₒ₊₁, NR70R71;
R70 und R71 unabhängig voneinander H, CᵤᵤH₂ᵤᵤ₊₁ und COR72;
R72 H, CᵥᵥH₂ᵥᵥ₊₁;
oo, uu und vv unabhängig voneinander 1, 2, 3, 4, 5, 6, 7 oder 8; wobei in den Gruppen CₒₒH₂ₒₒ₊₁, CᵤᵤH₂ᵤᵤ₊₁ oder CᵥᵥH₂ᵥᵥ₊₁ ein oder mehrere H-Atome durch F-Atome ersetzt sein dürfen;
oder
- R7, R8 und R9: unabhängig voneinander H, F, Cl, Br, I, NO₂, CN, OH, NH₂, CₑₑH₂ₑₑ₊₁, C_{ww}H_{2ww-1}, OC_{ff}H_{2ff+1}, NR40R41, CONR40R41, COOR42, COR42 oder OCOR42,
ee und ff unabhängig voneinander 1, 2, 3, 4, 5, 6, 7 oder 8;
ww 3, 4, 5, 6, 7 oder 8, wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H, CₜₜH₂ₜₜ₊₁ oder C(NH)NH₂;
tt 1, 2, 3, 4, 5, 6, 7 oder 8; wobei in der Gruppe CₜₜH₂ₜₜ₊₁ ein oder mehrere H-Atome durch F-Atome ersetzt sein dürfen und eine oder mehrere CH₂-Gruppen durch O- oder NR44 ersetzt sein können;
R44 H oder C_{gg}H_{2gg+1};
gg 1, 2, 3, 4, 5, 6, 7 oder 8; wobei in der Gruppe C_{gg}H_{2gg+1} ein oder mehrere H-Atome durch F-Atome ersetzt sein können, oder
R40 und R41 mit dem N-Atom, an das sie gebunden sind, einen 5- oder 6-gliedrigen Ring;
R42 H oder CₕₕH₂ₕₕ₊₁;
hh 1, 2, 3, 4, 5, 6, 7 oder 8; wobei in der Gruppe CₕₕH₂ₕₕ₊₁ ein oder mehrere H-Atome durch F-Atome ersetzt sein können;
sowie von deren pharmazeutisch verträglichen Salzen.

Bevorzugt ist die Verwendung von Verbindungen der Formel I, worin bedeuten:
- R1, R2, R3 und R4: unabhängig voneinander H, F, Cl, Br, I, CN, NO₂, OH, NH₂, CₐH₂ₐ₊₁, Cycloalkyl mit 3, 4, 5 oder 6 C-Atomen, OC_{b}H_{2b+1}, COOR10;
a und b unabhängig voneinander 1, 2, 3 oder 4, wobei ein oder mehrere H-Atome durch F-Atome ersetzt sein können;
R10 H oder C_{c}H_{2c+1};
c 1, 2, 3 oder 4, wobei ein oder mehrere H-Atome durch F-Atome ersetzt sein dürfen;
oder
- R1, R2, R3 und R4: unabhängig voneinander 5- oder 6-gliedriges Heteroaryl, ausgewählt aus der Gruppe bestehend aus Pyridyl, Imidazolyl, Pyrazolyl, Pyrrolyl, Triazolyl, Tetrazolyl, Thiazolyl und Oxazolyl;
oder
- R1, R2, R3 und R4: unabhängig voneinander CONR11R12 oder NR11R12;
R11 und R12 unabhängig voneinander H, CₑH₂ₑ₊₁, CᵣᵣH₂ᵣᵣ₋₁;
e 1, 2, 3 oder 4,
rr 3, 4, 5 oder 6,
wobei in den Gruppen CₑH₂ₑ₊₁ und CᵣᵣH₂ᵣᵣ₋₁ ein oder mehrere H-Atome durch F-Atome ersetzt sein dürfen oder
R11 und R12 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl; oder
R11 und R12 gemeinsam mit dem N-Atom, an das sie gebunden sind, einen Pyrrolidin-, Piperidin-, N-Methylpiperazin-, Piperazin- oder Morpholinring; oder
R11 und R12 unabhängig voneinander COR14, CSR14, CONHR14, CSNHR14 oder SO₂R14;
R14 C_{g}H_{2g+1};
g 1, 2, 3 oder 4, wobei ein oder mehrere H-Atome durch F-Atome ersetzt sein;
oder
- R1, R2, R3 und R4: unabhängig voneinander OSO₃H, SO₃H, SO₂R₁₅, wobei
R15 CₖH₂ₖ₊₁, OCₗH₂ₗ₊₁ oder NR17R18;
k 1, 2, 3 oder 4, wobei ein oder mehrere H-Atome durch F-Atome ersetzt sein können;
l 1, 2, 3 oder 4, wobei ein oder mehrere H-Atome durch F-Atome ersetzt sein können;
R17 und R18 unabhängig voneinander H, CₘH₂ₘ₊₁, in welchem die an den Stickstoff gebundene erste CH₂-Gruppe durch CO und die zweite CH₂-Gruppe durch NR19 ersetzt ist;
m 1, 2, 3, 4 oder 5, wobei in der Gruppe CₘH₂ₘ₊₁ ein oder mehrere H-Atome durch F-Atome ersetzt sein können;
R19 H oder CₙH₂ₙ₊₁;
n 1, 2, 3 oder 4; wobei in CₙH₂ₙ₊₁ ein oder mehrere H-Atome durch F-Atome ersetzt sein können; oder
R17 und R18 gemeinsam mit dem N-Atom, an das sie gebunden sind, einen 5- oder, 6-gliedrigen Ring;
- R5: H, CₚH₂ₚ₊₁, CₛₛH₂ₛₛ₋₁;
p 1, 2, 3 oder 4;
ss 3, 4, 5, oder 6, wobei in CₚH₂ₚ₊₁ und CₛₛH₂ₛₛ₋₁ ein oder mehrere H-Atome durch F-Atome ersetzt sein können;
- R6: H, CₛH₂ₛ₊₁, OCₜH₂ₜ₊₁ oder OCOR22;
s und t unabhängig voneinander 1, 2, 3 oder 4;
wobei in CₛH₂ₛ₊₁ und OCₜH₂ₜ₊₁ ein oder mehrere H-Atome durch F-Atome ersetzt sein können;
R22 CᵤH₂ᵤ₊₁;
u 1, 2, 3 oder 4; wobei in CᵤH₂ᵤ₊₁ ein oder mehrere H-Atome durch F-Atome ersetzt sein können;
- R7, R8 und R9: unabhängig voneinander OSO₃H, SO₃H oder SO₂R23;
R23 CₙₙH₂ₙₙ₊₁, CₘₘH₂ₘₘ₋₁, OCₚₚH₂ₚₚ₊₁ oder NR25R26;
nn und pp unabhängig voneinander 1, 2, 3, 4 oder 5,
mm 3, 4, 5 oder 6, wobei in CₙₙH₂ₙₙ₊₁, CₘₘH₂ₘₘ₋₁ und OCₚₚH₂ₚₚ₊₁ ein oder mehrere H-Atome durch F-Atome ersetzt sein können;
R25 und R26 unabhängig voneinander H, CN, C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atome durch F-Atome ersetzt sein;
R27 H oder CₐₐH₂ₐₐ₊₁;
aa 1, 2, 3 oder 4; wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
- R7, R8 und R9: unabhängig voneinander NR32COR30, NR32CSR30 oder NR32SO₂R30;
R30 H, OH, C_{cc}H_{2cc+1} C_{yy}H_{2yy-1}, Pyrrolidinyl oder Piperidinyl, in welchen Ringen eine CH₂-Gruppe durch O oder NR33 ersetzt sein kann;
R32 und R33 unabhängig voneinander H oder CₕH₂ₕ₊₁;
cc 1, 2, 3, 4, 5, 6, 7 oder 8;
yy 3, 4, 5 oder 6;
h 1, 2, 3 oder 4; wobei in CₕH₂ₕ₊₁ ein oder mehrere H-Atome durch F-Atome ersetzt sein dürfen und in den Gruppen C_{cc}H_{2cc+1} und C_{yy}H_{2yy-1} ein oder mehrere H-Atome durch F-Atome ersetzt sein können und eine oder mehrere (CH₂)-Gruppen durch NR31 ersetzt sein können und eine (CH₂)-Gruppe durch O ersetzt sein kann;
R31 H, CₖₖH₂ₖₖ₊₁, COR65 oder SO₂R65;
kk 1, 2, 3, oder 4; wobei ein oder mehrere H-Atome durch F-Atome ersetzt sein können,
R65 H, CₓₓH₂ₓₓ₊₁;
xx 1, 2, 3 oder 4, wobei ein oder mehrere H-Atome durch F-Atome ersetzt sein dürfen;
oder
- R31: zusammen mit einer CH₂-Gruppe von R30 und dem N-Atom, an das sie gemeinsam gebunden sind einen 5- oder 6-gliedrigen Ring bilden;
oder
- R30: einen 5- oder 6-gliedrigen Heteroaromaten ausgewählt aus der Gruppe bestehend aus Pyridyl, Imidazolyl, Pyrazolyl, Pyrrolyl, Triazolyl, Tetrazolyl, Thienyl, Thiazolyl und Oxazolyl,
die unsubstituiert sind oder substituiert mit bis zu drei Substituenten ausgewählt aus der Gruppe bestehend aus F, Cl, Br, I, CₒₒH₂ₒₒ₊₁, NR70R71,
R70 und R71 unabhängig voneinander H, CᵤᵤH₂ᵤᵤ₊₁ oder COR72,;
R72 H, CᵥᵥH₂ᵥᵥ₊₁;
oo, uu und w unabhängig voneinander 1, 2, 3 oder 4; wobei in den Gruppen CₒₒH₂ₒₒ₊₁, CᵤᵤH₂ᵤᵤ₊₁ oder CᵥᵥH₂ᵥᵥ₊₁ ein oder mehrere H-Atome durch F-Atome ersetzt sein dürfen;
oder
- R7, R8 und R9: unabhängig voneinander H, F, Cl, Br, I, NO₂, CN, OH, NH₂, CₑₑH₂ₑₑ₊₁, C_{ww}H_{2ww-1}, OC_{ff}H_{2ff+1}, NR40R41, CONR40R41, COOR42, COR42 oder OCOR42;
ee und ff unabhängig voneinander 1, 2, 3 oder 4;
ww 3, 4, 5 oder 6, wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H, CₜₜH₂ₜₜ₊₁ oder C(NH)NH₂;
tt 1, 2, 3, 4, 5, 6, 7 oder 8; wobei in der Gruppe CₜₜH₂ₜₜ₊₁ ein oder mehrere H-Atome durch F-Atome ersetzt sein dürfen; oder
R40 und R41 unabhängig voneinander auszuwählen Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl; oder
R40 und R41 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin;
R42 H oder CₕₕH₂ₕₕ₊₁;
hh 1, 2, 3 oder 4; wobei in der Gruppe CₕₕH₂ₕₕ₊₁ ein oder mehrere H-Atome durch F-Atome ersetzt sein können;
sowie von deren pharmazeutisch verträglichen Salzen.

Besonders bevorzugt ist die Verwendung von Verbindungen der Formel I, worin bedeuten:
- R1, R2, R3 und R4: unabhängig voneinander H, F, Cl, Br, OH, NH₂, CₐH₂ₐ₊₁, Cycloalkyl mit 3, 4, 5 oder 6 C-Atomen, OC_{b}H_{2b+1};
a und b in den Gruppen CₐH₂ₐ₊₁ und OC_{b}H_{2b+1} unabhängig voneinander 1, 2, 3 oder 4, wobei ein oder mehrere H-Atome durch F-Atome ersetzt sein können;
oder
- R1, R2, R3 und R4: unabhängig voneinander NR11R12;
R11 und R12 unabhängig voneinander H, CₑH₂ₑ₊₁, CᵣᵣH₂ᵣᵣ₋₁;
e 1, 2, 3 oder 4,
rr 3, 4, 5 oder 6,
wobei in den Gruppen CₑH₂ₑ₊₁ und CᵣᵣH₂ᵣᵣ₋₁ ein oder mehrere H-Atome durch F-Atome ersetzt sein dürfen; oder
R11 und R12 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin; oder
R11 und R12 unabhängig voneinander COR14, CSR14, CONHR14, CSNHR14 oder SO₂R14;
R14 C_{g}H_{2g+1};
g 1, 2, 3 oder 4, wobei ein oder mehrere H-Atome durch F-Atome ersetzt sein können;
oder
- R1, R2, R3 und R4: unabhängig voneinander OSO₃H, SO₃H, SO₂R15;
R15 CₖH₂ₖ₊₁ oder NR17R18;
k 1, 2, 3 oder 4, wobei ein oder mehrere H-Atome durch F-Atome ersetzt sein können;
R17 und R18 unabhängig voneinander H oder CₘH₂ₘ₊₁;
m 1, 2, 3, 4 oder 5, wobei in der Gruppe CₘH₂ₘ₊₁ ein oder mehrere H-Atome durch F-Atome ersetzt sein können; oder
R17 und R18 gemeinsam mit dem N-Atom, an das sie gebunden sind, einen 5- oder, 6-gliedrigen Ring;
- R5: H, CₚH₂ₚ₊₁, CₛₛH₂ₛₛ₋₁;
p 1, 2, 3 oder 4;
ss 3, 4, 5, oder 6, wobei in CₚH₂ₚ₊₁ und CₛₛH₂ₛₛ₋₁ ein oder mehrere H-Atome durch F-Atome ersetzt sein können;
- R6: H, CH₃;
- R7, R8 und R9: unabhängig voneinander OSO₃H, SO₃H oder SO₂R23;
R23 CₙₙH₂ₙₙ₊₁ oder NR25R26;
nn 1, 2, 3, 4 oder 5,
wobei in CₙₙH₂ₙₙ₊₁ ein oder mehrere H-Atome durch F-Atome ersetzt sein könnten;
R25 und R26 unabhängig voneinander H, CN oder C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atome durch F-Atome ersetzt sein können;
R27 H oder CₐₐH₂ₐₐ₊₁;
aa 1, 2, 3 oder 4;
wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
- R7, R8 und R9: unabhängig voneinander NR32COR30, NR32CSR30 oder NR32SO₂R30;
R30 H, OH, C_{cc}H_{2cc+1}, C_{yy}H_{2yy-1}, Pyrrolidinyl oder Piperidinyl, in welchen Ringen eine CH₂-Gruppe durch O oder NR33 ersetzt sein kann;
R32 und R33 H, CH₃ oder CF₃;
cc 1, 2, 3, 4, 5, 6, 7 oder 8;
yy 3, 4, 5 oder 6; wobei in den Gruppen C_{cc}H_{2cc+1} und C_{yy}H_{2yy-1} ein oder mehrere H-Atome durch F-Atome ersetzt sein können und eine oder mehrere (CH₂)-Gruppen durch NR31 ersetzt sein können und eine (CH₂)- Gruppe durch O ersetzt sein kann;
R31 H, Methyl, Ethyl, CF₃, CH₂CF₃, Acetyl, Propionyl, Methansulfonyl oder Ethansulfonyl; oder
R31 zusammen mit einer CH₂-Gruppe von R30 und dem N-Atom, an das sie gemeinsam gebunden sind, einen 5- oder 6-gliedrigen Ring bilden;
oder
R30 Pyridyl, Imidazolyl, Pyrazolyl, Pyrrolyl, Triazolyl, Tetrazolyl, Thiazolyl oder Oxazolyl, die unsubstituiert sind oder substituiert mit maximal 3 Substituenten ausgewählt aus der Gruppe bestehend aus F, Cl, Methyl, Ethyl, Trifluormethyl, NH₂, NHAcetyl;
oder
- R7, R8 und R9: unabhängig voneinander H, F, Cl, OH, NH₂, CₑₑH₂ₑₑ₊₁, C_{ww}H_{2ww-1}, OC_{ff}H_{2ff+1}, NR40R41, CONR40R41, COOR42 oder OCOR42,
ee und ff unabhängig voneinander 1, 2, 3 oder 4;
ww 3, 4, 5 oder 6,
wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H, CₜₜH₂ₜₜ₊₁ oder C(NH)NH₂;
tt 1, 2, 3 oder 4; wobei in der Gruppe CₜₜH₂ₜₜ₊₁ ein oder mehreren H-Atome durch F-Atome ersetzt sein dürfen; oder
R40 und R41 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl; oder
R40 und R41 gemeinsam mit dem N-Atom, an das sie gebunden sind, einen Pyrrolidin-, Piperidin-, N-Methylpiperazin-, Piperazin- oder Morpholinring;
R42 H oder CₕₕH₂ₕₕ₊₁;
hh 1, 2, 3 oder 4; wobei in der Gruppe CₕₕH₂ₕₕ₊₁ ein oder mehrere H-Atome durch F-Atome ersetzt sein können;
sowie von deren pharmazeutisch verträglichen Salzen.

Ganz besonders bevorzugt ist die Verwendung von Verbindungen ausgewählt aus der Gruppe bestehend aus
1) N-[4-(6,8-Dichloro-2-methyl-1,2,3,4-tetrahydro-isochinolin-4-yl)-phenyl]-acetamid;
2) 4-(6,8-Dichloro-2-methyl-1,2,3,4-tetrahydro-isochinolin-4-yl)-benzolsulfonamid;
3) 3-(6,8-Dichloro-2-methyl-1,2,3,4-tetrahydro-isochinolin-4-yl)-benzolsulfonamid;
4) 4-(6,8-Dichloro-2-methyl-1,2,3,4-tetrahydro-isochinolin-4-yl)-N,N-dimethylbenzol-sulfonamid;
5) 4-(4-Bromo-phenyl)-6,8-dichloro-2-methyl-1,2,3,4-tetrahydroisochinolin;
6) 4-(6,8-Dichloro-2-methyl-1,2,3,4-tetrahydro-isochinolin-4-yl)-benzoesäure;
7) 4-(6,8-Dichloro-2-methyl-1,2,3,4-tetrahydroisochinolin-4-yl)-N-ethyl-benzamid;
8) 4-(6,8-Dichloro-2-methyl-1,2,3,4-tetrahydroisochinolin-4-yl)-N-propyl-benzamid;
9) 4-(6,8-Dichloro-2-methyl-1,2,3,4-tetrahydro-isochinolin-4-yl)-N-(2-dimethylamino-ethyl)-benzamid;
10) 6,8-Dichloro-2-methyl-4-(4-morpholin-4-yl-phenyl)-1,2,3,4-tetrahydroisochinolin;
11) [4-(6,8-Dichloro-2-methyl-1,2,3,4-tetrahydroisochinolin-4-yl)-phenyl]-diethyl-amin
12) 6,8-Dichioro-2-methyl-4-(4-piperidin-1-yl-phenyl)-1,2,3,4-tetrahydroisochinolin;
13) 6,8-Dichloro-2-methyl-4-(4-pyrrolidin-1-yl-phenyl)-1,2,3,4-tetrahydroisochinolin;
14) 6,8-Dichloro-2-methyl-4-[4-(4-methyl-piperazin-1-yl)-phenyl]-1,2,3,4-tetrahydroisochinolin;
15) 6,8-Dichloro-2-cyclopropyl-4-phenyl-1,2,3,4-tetrahydroisochinolin;
16) 4-(6,8-Dichloro-2-methyl-1,2,3,4-tetrahydro-isochinolin-4-yl)-phenylamin;
17) 1-[4-(6,8-Dichloro-2-methyl-1,2,3,4-tetrahydro-isochinolin-4-yl)-phenyl]-3-propyl-harnstoff;
18) 1-[4-(6,8-Dichloro-2-methyl-1,2,3,4-tetrahydro-isochinolin-4-yl)-phenyl]-3-methyl-thioharnstoff;
19) 1-[4-(6,8-Dichloro-2-methyl-1,2,3,4-tetrahydro-isochinolin-4-yl)-phenyl]-3-ethyl-harnstoff;
20) N-[4-(6-Methansulfonyl-2-methyl-1,2,3,4-tetrahydro-isochinolin-4-yl)-phenyl]-acetamid;
21) N-[4-(2,6,8-Trimethyl-1,2,3,4-tetrahydro-isochinolin-4-yl)-phenyl]-acetamid;
22) N-[4-(6-Bromo-8-chloro-2-methyl-1,2,3,4-tetrahydro-isochinolin-4-yl)-phenyl]-acetamid;
23) N-[4-(8-Chloro-2-methyl-6-pyrrolidin-1-yl-1,2,3,4-tetrahydro-isochinolin-4-yl)-phenyl]- acetamid;
24) N-[4-(8-Chloro-2-methyl-6-morpholin-4-yl-1,2,3,4-tetrahydro-isochinolin-4-yl)-phenyl]- acetamid;
25) N-{4-[8-Chloro-2-methyl-6-(4-methyl-piperazin-1-yl)-1,2,3,4-tetrahydroisochinolin-4- yl]-phenyl}-acetamid;
26) N-{4-[8-Chloro-6-(cyclopropylmethyl-amino)-2-methyl-1,2,3,4-tetrahydroisochinolin-4- yl]-phenyl}-acetamid;
27) 5-(6,8-Dichloro-2-methyl-1,2,3,4-tetrahydro-isochinolin-4-yl)-2-hydroxybenzoesäure;
28) 5-(6,8-Dichloro-2-methyl-1,2,3,4-tetrahydro-isochinolin-4-yl)-2-hydroxy-N-methyl-benzamid;
29) 5-(6,8-Dichloro-2-methyl-1,2,3,4-tetrahydro-isochinolin-4-yl)-N-ethyl-2-hydroxy-benzamid;
30) 5-(6,8-Dichloro-2-methyl-1,2,3,4-tetrahydro-isochinolin-4-yl)-N-(2-dimethylamino-ethyl)-2-hydroxy-benzamid;
31) N-[5-(6,8-Dichloro-2-methyl-1,2,3,4-tetrahydro-isochinolin-4-yl)-2-hydroxy-benzoyl]- guanidin;
32) N-[3-(6,8-Dichloro-2-methyl-1,2,3,4-tetrahydro-isochinolin-4-yl)-phenyl]-acetamid;
33) 3-(6,8-Dichloro-2-methyl-1,2,3,4-tetrahydro-isochinolin-4-yl)-phenylamin;
34) 2-(6,8-Dichloro-2-methyl-1,2,3,4-tetrahydro-isochinolin-4-yl)-phenylamin;
35) N-[4-(6,8-Dichloro-2-methyl-1,2,3,4-tetrahydro-isochinolin-4-yl)-phenyl]-propionamid;
36) N-[4-(6,8-Dichloro-2-methyl-1,2,3,4-tetrahydro-isochinolin-4-yl)-phenyl]-butyramid;
37) Pentansäure-[4-(6,8-dichloro-2-methyl-1,2,3,4-tetrahydro-isochinolin-4-yl)-phenyl]-amid;
38) N-[4-(6,8-Dichloro-2-methyl-1,2,3,4-tetrahydro-isochinolin-4-yl)-phenyl]-isobutyramid;
39) N-[4-(6,8-Dichloro-2-methyl-1,2,3,4-tetrahydro-isochinolin-4-yl)-phenyl]-2,2-dimethyl- propionamid;
40) Cyclopropancarbonsäure-[4-(6,8-dichloro-2-methyl-1,2,3,4-tetrahydro-isochinolin-4-yl)-phenyl]-amid;
41) Cyclobutancarbonsäure-[4-(6,8-dichloro-2-methyl-1,2,3,4-tetrahydro-isochinolin-4-yl)- phenyl]-amid;
42) Cyclopentancarbonsäure-[4-(6,8-dichloro-2-methyl-1,2,3,4-tetrahydro-isochinolin-4-yl)-phenyl]-amid;
43) N-[4-(6,8-Dichloro-2-methyl-1,2,3,4-tetrahydro-isochinolin-4-yl)-phenyl]-2,2,2-trifluoro-acetamid;
44) 1-Acetyl-piperidin-4-carbonsäure-[4-(6,8-dichloro-2-methyl-1,2,3,4-tetrahydroisochinolin-4-yl)-phenyl]-amid;
45) N-[4-(6,8-Dichloro-2-methyl-1,2,3,4-tetrahydro-isochinolin-4-yl)-phenyl]-nicotinamid;
46) N-[4-(6,8-Dichloro-2-methyl-1,2,3,4-tetrahydro-isochinolin-4-yl)-phenyl]-methansulfonamid;
47) Ethansulfonsäure-[4-(6,8-dichloro-2-methyl-1,2,3,4-tetrahydro-isochinolin-4-yl)-phenyl]-amid;
48) N',N'-Dimethylamino-N-[4-(6,8-Dichloro-2-methyl-1,2,3,4-tetrahydro-isochinolin-4-yl)-phenyl]-sulfamid;
49) N-[3-(6,8-Dichloro-2-methyl-1,2,3,4-tetrahydro-isochinolin-4-yl)-phenyl]-propionamid;
50) N-[3-(6,8-Dichloro-2-methyl-1,2,3,4-tetrahydro-isochinolin-4-yl)-phenyl]-butyramid;
51) Pentansäure-[3-(6,8-dichloro-2-methyl-1,2,3,4-tetrahydro-isochinolin-4-yl)-phenyl]-amid;
52) N-[3-(6,8-Dichloro-2-methyl-1,2,3,4-tetrahydro-isochinolin-4-yl)-phenyl]-isobutyramid;
53) N-[3-(6,8-Dichloro-2-methyl-1,2,3,4-tetrahydro-isochinolin-4-yl)-phenyl]-2,2-dimethyl-propionamid;
54) Cyclopropancarbonsäure-[3-(6,8-dichloro-2-methyl-1,2,3,4-tetrahydro-isochinolin-4-yl)-phenyl]-amid;
55) Cyclobutancarbonsäure-[3-(6,8-dichloro-2-methyl-1,2,3,4-tetrahydro-isochinolin-4-yl)- phenyl]-amid;
56) Cyclopentancarbonsäure-[3-(6,8-dichloro-2-methyl-1,2,3,4-tetrahydro-isochinolin-4-yl)-phenyl]-amid;
57) N-[3-(6,8-Dichloro-2-methyl-1,2,3,4-tetrahydro-isochinolin-4-yl)-phenyl]-2,2,2-trifluoro-acetamid;
58) 1-Acetyl-piperidin-4-carbonsäure-[3-(6,8-dichloro-2-methyl-1,2,3,4-tetrahydroisochinolin-4-yl)-phenyl]-amid;
59) N-[3-(6,8-Dichloro-2-methyl-1,2,3,4-tetrahydro-isochinolin-4-yl)-phenyl]-nicotinamid;
60) N-[3-(6,8-Dichloro-2-methyl-1,2,3,4-tetrahydro-isochinolin-4-yl)-phenyl]-methansulfonamid;
61) Ethansulfonsäure-[3-(6,8-dichloro-2-methyl-1,2,3,4-tetrahydro-isochinolin-4-yl)-phenyl]-amid;
62) N',N'-Dimethylamino-N-[3-(6,8-Dichloro-2-methyl-1,2,3,4-tetrahydro-isochinolin-4-yl)-phenyl]-sulfamid;
63) N-[2-(6,8-Dichloro-2-methyl-1,2,3,4-tetrahydro-isochinolin-4-yl)-phenyl]-propionamid;
64) N-[2-(6,8-Dichloro-2-methyl-1,2,3,4-tetrahydro-isochinolin-4-yl)-phenyl]-butyramid;
65) Pentansäure-[2-(6,8-dichloro-2-methyl-1,2,3,4-tetrahydro-isochinolin-4-yl)-phenyl]-amid;
66) N-[2-(6,8-Dichloro-2-methyl-1,2,3,4-tetrahydro-isochinolin-4-yl)-phenyl]-isobutyramid;
67) N-[2-(6,8-Dichloro-2-methyl-1,2,3,4-tetrahydro-isochinolin-4-yl)-phenyl]-2,2-dimethyl- propionamid;
68) Cyclopropancarbonsäure-[2-(6,8-dichloro-2-methyl-1,2,3,4-tetrahydro-isochinolin-4-yl)-phenyl]-amid;
69) Cyclobutancarbonsäure-[2-(6,8-dichloro-2-methyl-1,2,3,4-tetrahydro-isochinolin-4-yl)- phenyl]-amid;
70) Cyclopentancarbonsäure-[2-(6,8-dichloro-2-methyl-1,2,3,4-tetrahydro-isochinolin-4-yl)-phenyl]-amid;
71) N-[2-(6,8-Dichloro-2-methyl-1,2,3,4-tetrahydro-isochinolin-4-yl)-phenyl]-2,2,2-trifluoro-acetamid;
72) 1-Acetyl-piperidin-4-carbonsäure-[2-(6,8-dichloro-2-methyl-1,2,3,4-tetrahydroisochinolin-4-yl)-phenyl]-amid;
73) N-[2-(6,8-Dichloro-2-methyl-1,2,3,4-tetrahydro-isochinolin-4-yl)-phenyl]-methansulfonamid;
74) Ethansulfonsäure-[2-(6,8-dichloro-2-methyl-1,2,3,4-tetrahydro-isochinolin-4-yl)-phenyl]-amid;
75) N',N'-Dimethylamino-N-[2-(6,8-Dichloro-2-methyl-1,2,3,4-tetrahydro-isochinolin-4-yl)-phenyl]-sulfamid;
76) 1-[3-(6,8-Dichloro-2-methyl-1,2,3,4-tetrahydro-isochinolin-4-yl)-phenyl]-3-ethyl-harnstoff;
77) 1-[3-(6,8-Dichloro-2-methyl-1,2,3,4-tetrahydro-isochinolin-4-yl)-phenyl]-3-methyl-thioharnstoff;
78) 1-[2-(6,8-Dichloro-2-methyl-1,2,3,4-tetrahydro-isochinolin-4-yl)-phenyl]-3-ethyl-harnstoff;
79) 1-[2-(6,8-Dichloro-2-methyl-1,2,3,4-tetrahydro-isochinolin-4-yl)-phenyl]-3-methyl-thioharnstoff;
80) N-{5-[4-(6,8-Dichloro-2-methyl-1,2,3,4-tetrahydro-isochinolin-4-yl)-phenylsulfamoyl]-4-methyl-thiazol-2-yl}-acetamid;
81) N-{5-[3-(6,8-Dichloro-2-methyl-1,2,3,4-tetrahydro-isochinolin-4-yl)-phenylsulfamoyl]-4-methyl-thiazol-2-yl}-acetamid;
82) 1,2-Dimethyl-1 H-imidazole-4-sulfonsäure-[4-(6,8-dichloro-2-methyl-1,2,3,4-tetrahydro- isochinolin-4-yl)-phenyl]-amid;
83) 1,2-Dimethyl-1H-imidazole-4-sulfonsäure-[3-(6,8-dichloro-2-methyl-1,2,3,4-tetrahydro- isochinolin-4-yl)-phenyl]-amid;
84) 5-Chloro-1,3-dimethyl-1 H-pyrazol-4-sulfonsäure-[4-(6,8-dichloro-2-methyl-1,2,3,4-tetrahydro-isochinolin-4-yl)-phenyl]-amid;
85) 5-Chloro-1,3-dimethyl-1 H-pyrazol-4-sulfonsäure-[3-(6,8-dichloro-2-methyl-1,2,3,4-tetrahydro-isochinolin-4-yl)-phenyl]-amid;
86) 5-Bromo-thiophen-2-sulfonsäure-[4-(6,8-dichloro-2-methyl-1,2,3,4-tetrahydroisochinolin-4-yl)-phenyl]-amid;
87) 5-Bromo-thiophen-2-sulfonsäure-[3-(6,8-dichloro-2-methyl-1,2,3,4-tetrahydroisochinolin-4-yl)-phenyl]-amid;
88) N-[4-(6,8-Dichloro-2-methyl-1,2,3,4-tetrahydro-isochinolin-4-yl)-phenyl]-C,C,C-trifluoro-methanesulfonamid;
89) N-[3-(6,8-Dichloro-2-methyl-1,2,3,4-tetrahydro-isochinolin-4-yl)-phenyl]-C,C,C-trifluoro-methanesulfonamid;
90) 2,2,2-Trifluoro-ethansulfonsäure-4-(6,8-dichloro-2-methyl-1,2,3,4-tetrahydroisochinolin-4-yl)-phenyl]-amid;
91) 2,2,2-Trifluoro-ethansulfonsäure-3-(6,8-dichloro-2-methyl-1,2,3,4-tetrahydroisochinolin-4-yl)-phenyl]-amid;
92) N-Ethyl-N'-4-(6,8-Dichloro-2-methyl-1,2,3,4-tetrahydro-isochinolin-4-yl)-benzolsulfonyl-harnstoff;
93) 2-Chloro-5-(6,8-dichloro-2-methyl-1,2,3,4-tetrahydro-isochinolin-4-yl)-benzolsulfonamid;
94) 2-Methyl-4-phenyl-1,2,3,4-tetrahydro-isochinolin-8-ylamin;
95) 6,8-Dichloro-2-methyl-4-phenyl-1,2,3,4-tetrahydro-isochinolin;
96) 4-(8-Amino-2-methyl-1,2,3,4-tetrahydro-isochinolin-4-yl)-phenol;
97) 8-Methoxy-2-methyl-4-phenyl-1,2,3,4-tetrahydro-isochinolin;
98) 2-(8-Amino-2-ethyl-1,2,3,4-tetrahydro-isochinolin-4-yl)-phenol;
99) 2-(8-Amino-2-methyl-1,2,3,4-tetrahydro-isochinolin-4-yl)-phenol;
100) 5-(8-Amino-2-methyl-1,2,3,4-tetrahydro-isochinolin-4-yl)-2-methoxy-phenol;
101) 2-Methyl-8-nitro-4-phenyl-1,2,3,4-tetrahydro-isochinolin;
102) 4-(8-Amino-2-methyl-1,2,3,4-tetrahydro-isochinolin-4-yl)-benzol-1,2-diol;
103) 2,8-Dimethyl-4-phenyl-1,2,3,4-tetrahydro-isochinolin;
104) 4-(3,4-Dichloro-phenyl)-2-methyl-1,2,3,4-tetrahydro-isochinolin;
105) 4-(3,4-Dichloro-phenyl)-2-methyl-1,2,3,4-tetrahydro-isochinolin-8-ylamin;
106) 4-(2,4-Dichloro-phenyl)-2-methyl-1,2,3,4-tetrahydro-isochinolin-8-ylamin;
107) 4-(3-Chloro-phenyl)-2-methyl-1,2,3,4-tetrahydro-isochinolin-8-ylamin;
108) 2,4-Dimethyl-4-phenyl-1,2,3,4-tetrahydro-isochinolin;
109) 2-Butyl-4-phenyl-1,2,3,4-tetrahydro-isochinolin-8-ylamin;
110) N-(2-Methyl-4-phenyl-1,2,3,4-tetrahydro-isochinolin-8-yl)-acetamid;
111) 7-Chloro-2-methyl-4-phenyl-1,2,3,4-tetrahydro-isochinolin;
112) 8-Chloro-2-methyl-4-phenyl-1,2,3,4-tetrahydro-isochinolin;
113) 2,6-Dimethyl-4-phenyl-1,2,3,4-tetrahydro-isochinolin;
114) 6-Chloro-2-methyl-4-phenyl-1,2,3,4-tetrahydro-isochinolin;
115) 6-Methoxy-2-methyl-4-phenyl-1,2,3,4-tetrahydro-isochinolin;
116) 2-Ethyl-4-phenyl-1,2,3,4-tetrahydro-isochinolin;
117) 2-Methyl-4-phenyl-1,2,3,4-tetrahydro-isochinolin;
118) 6,8-Dichloro-2-ethyl-4-phenyl-1,2,3,4-tetrahydro-isochinolin;
119) 4-(4-Bromo-phenyl)-6,8-dichloro-2-methyl-1,2,3,4-tetrahydro-isochinolin;
120) 2-Methyl-4-phenyl-6,8-bis-trifluoromethyl-1,2,3,4-tetrahydro-isochinolin;
121) 6,8-Dichlöro-2-isopropyl-4-phenyl-1,2,3,4-tetrahydro-isochinolin;
122) 5,8-Dichloro-2-methyl-4-phenyl-1,2,3,4-tetrahydro-isochinolin;
123) 6,8-Dichloro-4-(4-fluoro-phenyl)-2-methyl-1,2,3,4-tetrahydro-isochinolin;
124) 6,8-Dichloro-2-methyl-4-p-tolyl-1,2,3,4-tetrahydro-isochinolin;
125) 5,6-Dichloro-2-methyl-4-phenyl-1,2,3,4-tetrahydro-isochinolin;
126) 6,7-Dichloro-2-methyl-4-phenyl-1,2,3,4-tetrahydro-isochinolin;
127) 8-Bromo-2-methyl-4-phenyl-1,2,3,4-tetrahydro-isochinolin;
128) 6,8-Dichloro-4-(4-chloro-phenyl)-2-methyl-1,2,3,4-tetrahydro-isochinolin;
129) 6,8-Dichloro-2-cyclopropyl-4-phenyl-1,2,3,4-tetrahydro-isochinolin;
130) 2-Amino-N-[4-(6,8-dichloro-2-methyl-1,2,3,4-tetrahydro- isochinolin -4-yl)-phenyl]-acetamid;
131) N-[4-(6,8-Dichloro-2-methyl-1,2,3,4-tetrahydro- isochinolin -4-yl)-phenyl]-2-methylamino- acetamid;
132) N-[4-(6,8-Dichloro-2-methyl-1,2,3,4-tetrahydro- isochinolin -4-yl)-phenyl]-2-dimethylamino-acetamid;.
133) 2-Amino-N-[4-(6,8-dichloro-2-methyl-1,2,3,4-tetrahydro- isochinolin -4-yl)-phenyl]-propionamid;
134) 2-Amino-N-[4-(6,8-dichloro-2-methyl-1,2,3,4-tetrahydro- isochinolin -4-yl)-phenyl]-butyramid;
135) 2,6-Diamino-hexansäure-[4-(6,8-dichloro-2-methyl-1,2,3,4-tetrahydro-isochinolin -4-yl)- phenyl]-amid;
136) Pyrrolidine-2-carbonsäure-[4-(6,8-dichloro-2-methyl-1,2,3,4-tetrahydroisochinolin -4- yl)-phenyl]-amid;
137) N-[4-(6,8-Dichloro-2-methyl-1,2,3,4-tetrahydro- isochinolin-4-yl)-phenyl]-isonicotinamid;
138) 1H-Pyrrole-3-carbonsäure-[4-(6,8-dichloro-2-methyl-1,2,3,4-tetrahydro-isochinolin-4- yl)-phenyl]-amid;
139) 1 H-Pyrrol-2- carbonsäure-[4-(6,8-dichloro-2-methyl-1,2,3,4-tetrahydroisochinolin-4- yl)-phenyl]-amid;
140) 1-Methyl-piperidin-4-carbonsäure-[4-(6,8-dichloro-2-methyl-1,2,3,4-tetrahydroisochinolin-4-yl)-phenyl]-amid;
141) 1,4-Dimethyl-1H-pyrrol-2-carbonsäure-[4-(6,8-dichloro-2-methyl-1,2,3,4-tetrahydro- isochinolin-4-yl)-phenyl]-amid;
142) 4-Nitro-1H-pyrrol-2-carbonsäure-[4-(6,8-dichloro-2-methyl-1,2,3,4-tetrahydroisochinolin-4-yl)-phenyl]-amid;
143) 2,5-Dimethyl-1H-pyrrol-3-carbonsäure-[4-(6,8-dichloro-2-methyl-1,2,3,4-tetrahydro- isochinolin-4-yl)-phenyl]-amid;
144) 1H-lmidazol-4-carbonsäure-[4-(6,8-dichloro-2-methyl-1,2,3,4-tetrahydroisochinolin-4-yl)-phenyl]-amid;
145) 1-Methansulfonyl-piperidin-4-carbonsäure-[4-(6,8-dichloro-2-methyl-1,2,3,4-tetrahydro- isochinolin-4-yl)-phenyl]-amid;
146) 3,5-Dimethyl-1H-pyrazol-4-carbonsäure-[4-(6,8-dichloro-2-methyl-1,2,3,4-tetrahydro- isochinolin-4-yl)-phenyl]-amid;
147) 1 H-Pyrazol-4- carbonsäure-[4-(6,8-dichloro-2-methyl-1,2,3,4-tetrahydroisochinolin- 4-yl)-phenyl]-amid;
148) 3-Trifluoromethyl-1 H-pyrazol-4- carbonsäure-[4-(6,8-dichloro-2-methyl-1,2,3,4-tetrahydro-isochinolin-4-yl)-phenyl]-amid;
149) N-[3-(6,8-Dichloro-2-methyl-1,2,3,4-tetrahydro-isochinolin-4-yl)-phenyl]-2-methylamino-acetamid;
150) N-[3-(6,8-Dichloro-2-methyl-1,2,3,4-tetrahydro-isochinolin-4-yl)-phenyl]-2-dimethylamino-acetamid;
151) 2-Amino-N-[3-(6,8-dichloro-2-methyl-1,2,3,4-tetrahydro-isochinolin-4-yl)-phenyl]-propionamid;
152) 2-Amino-N-[3-(6,8-dichloro-2-methyl-1,2,3,4-tetrahydro-isochinolin-4-yl)-phenyl]-butyramid;
153) 2,6-Diamino-hexansäure-[3-(6,8-dichloro-2-methyl-1,2,3,4-tetrahydro-isochinolin-4-yl)- phenyl]-amid;
154) Pyrrolidin-2- carbonsäure-[3-(6,8-dichloro-2-methyl-1,2,3,4-tetrahydro-isochinolin-4- yl)-phenyl]-amid;
155) N-[3-(6,8-Dichloro-2-methyl-1,2,3,4-tetrahydro-isochinolin-4-yl)-phenyl]-isonicotinamid;
156) 1 H-Pyrrol-3- carbonsäure-[3-(6,8-dichloro-2-methyl-1,2,3,4-tetrahydro-isochinolin-4- yl)-phenyl]-amid;
157) 1 H-Pyrrol-2- carbonsäure-[3-(6,8-dichloro-2-methyl-1,2,3,4-tetrahydro-isochinolin-4- yl)-phenyl]-amid;
158) 1-Methyl-piperidin-4- carbonsäure-[3-(6,8-dichloro-2-methyl-1,2,3,4-tetrahydroisochinolin-4-yl)-phenyl]-amid;
159) 1,4-Dimethyl-1 H-pyrrol-2- carbonsäure-[3-(6,8-dichloro-2-methyl-1,2,3,4-tetrahydrö- isochinolin-4-yl)-phenyl]-amid;
160) 4-Nitro-1 H-pyrrol-2- carbonsäure-[3-(6,8-dichloro-2-methyl-1,2,3,4-tetrahydroisochinolin-4-yl)-phenyl]-amid;
161) 2,5-Dimethyl-1 H-pyrrol-3- carbonsäure-[3-(6,8-dichloro-2-methyl-1,2,3,4-tetrahydro- isochuinolin-4-yl)-phenyl]-amid;
162) 1H-lmidazol-4- carbonsäure-[3-(6,8-dichloro-2-methyl-1,2,3,4-tetrahydro-isochuinolin- 4-yl)-phenyl]-amid;
163) 1-Methansulfonyl-piperidin-4- carbonsäure-[3-(6,8-dichloro-2-methyl-1,2,3,4-tetrahydro-isochinolin-4-yl)-phenyl]-amid;
164) 3,5-Dimethyl-1H-pyrazol-4- carbonsäure-[3-(6,8-dichloro-2-methyl-1,2,3,4-tetrahydro-isochinolin-4-yl)-phenyl]-amid;
165) 1 H-Pyrazol-4- carbonsäure-[3-(6,8-dichloro-2-methyl-1,2,3,4-tetrahydroisochinolin-4-yl)-phenyl]-amid;
166) 3-Trifluoromethyl-1H-pyrazol-4- carbonsäure-[3-(6,8-dichloro-2-methyl-1,2,3,4-tetrahydro-isochinolin-4-yl)-phenyl]-amid;
167) 1-[2-(6,8-Dichloro-2-methyl-1,2,3,4-tetrahydro- isochinolin-4-yl)-phenyl]-3-ethyl-thioharnstoff;
168) 1-[4-(6,8-Dichloro-2-methyl-1,2,3,4-tetrahydro-isochinolin-4-yl)-phenyl]-3-ethyl-thioharnstoff;
169) 1-[3-(6,8-Dichloro-2-methyl-1,2,3,4-tetrahydro-isochinolin-4-yl)-phenyl]-3-ethyl-thioharnstoff;
170) 3-[3-(6,8-Dichloro-2-methyl-1,2,3,4-tetrahydro-isochinolin-4-yl)-phenyl]-1,1-dimethyl-harnstoff;
171) 4-Methyl-piperazin-1-carbonsäure-[3-(6,8-dichloro-2-methyl-1,2,3,4-tetrahydroisochinolin-4-yl)-phenyl]-amid;
172) Piperidin-1-carbonsäure-[3-(6,8-dichloro-2-methyl-1,2,3,4-tetrahydro-isochinolin-4- yl)-phenyl]-amid;
173) Morpholin-4-carbonsäure-[3-(6,8-dichloro-2-methyl-1,2,3,4-tetrahydro-isochinolin-4- yl)-phenyl]-amid;
174) Pyrrolidin-1-carbonsäure-[3-(6,8-dichloro-2-methyl-1,2,3,4-tetrahydro-isochinolin-4- yl)-phenyl]-amid;
175) 3-[3-(6,8-Dichloro-2-methyl-1,2,3,4-tetrahydro-isochinolin-4-yl)-phenyl]-1,1-diethyl-harnstoff;
176) 1-[3-(6,8-Dichloro-2-methyl-1,2,3,4-tetrahydro-isochinolin-4-yl)-phenyl]-3-methyl-harnstoff;
177) 1-[3-(6,8-Dichloro-2-methyl-1,2,3,4-tetrahydro-isochinolin-4-yl)-phenyl]-3-(2-dimethylamino-ethyl)- harnstoff;
178) 1-[3-(6,8-Dichloro-2-methyl-1,2,3,4-tetrahydro-isochinolin-4-yl)-phenyl]-3-(tetrahydro-furan-3-yl)-harnstoff,
179) 1-[3-(6,8-Dichloro-2-methyl-1,2,3,4-tetrahydro-isochinolin-4-yl)-phenyl]-3-(tetrahydro-pyran-4-yl)-harnstoff;
180) 3-[3-(6,8-Dichloro-2-methyl-1,2,3,4-tetrahydro-isochinolin-4-yl)-phenyl]-1-methyl-1 -(1 -methyl-piperidin-4-yl)harnstoff;
181) 3-[3-(6,8-Dichloro-2-methyl-1,2,3,4-tetrahydro-isochinolin-4-yl)-phenyl]-1-(3-dimethylamino-propyl)-1-methyl-harnstoff;
182) 3-[3-(6,8-Dichloro-2-methyl-1,2,3,4-tetrahydro-isochinolin-4-yl)-phenyl]-1-(2-dimethylamino-ethyl)-1-methyl-harnstoff;
183) 1-[3-(6,8-Dichloro-2-methyl-1,2,3,4-tetrahydro-isochinolin-4-yl)-phenyl]-3-(3-dimethylamino-propyl)-harnstoff;
184) 1-[3-(6,8-Dichloro-2-methyl-1,2,3,4-tetrahydro-isochinolin-4-yl)-phenyl]-3-(2-methoxy-ethyl)-harnstoff;
185) 1-[3-(6,8-Dichloro-2-methyl-1,2,3,4-tetrahydro-isochinolin-4-yl)-phenyl]-3-pyridin-3-yl-harnstoff;
186) 1-[3-(6,8-Dichloro-2-methyl-1,2,3,4-tetrahydro-isochinolin-4-yl)-phenyl]-3-pyridin-4-yl-harnstoff;
187) 4-Methyl-piperazin-1-carbonsäure-[2-(6,8-dichloro-2-methyl-1,2,3,4-tetrahydro-isoquinolin-4-yl)-phenyl]-amid;
188) 1-[2-(6,8-Dichloro-2-methyl-1,2,3,4-tetrahydro-isochinolin-4-yl)-phenyl]-3-methyl-harnstoff;
189) 3-[2-(6,8-Dichloro-2-methyl-1,2,3,4-tetrahydro-isochinolin-4-yl)-phenyl]-1,1-dimethyl-harnstoff;
190) 3-[2-(6,8-Dichloro-2-methyl-1,2,3,4-tetrahydro-isochinolin-4-yl)-phenyl]-1,1-diethyl-harnstoff;
191) Piperidin-1-carbonsäure-[2-(6,8-dichloro-2-methyl-1,2,3,4-tetrahydro-isochinolin-4- yl)-phenyl]-amid;
192) Morpholin-4-carbonsäure-[2-(6,8-dichloro-2-methyl-1,2,3,4-tetrahydro-isochinolin-4- yl)-phenyl]-amid;
193) Pyrrolidin-1-carbonsäure-[2-(6,8-dichloro-2-methyl-1,2,3,4-tetrahydro-isochinolin-4- yl)-phenyl]-amid;
194) 1-[2-(6,8-Dichloro-2-methyl-1,2,3,4-tetrahydro-isochinolin-4-yl)-phenyl]-3-(2-dimethylamino-ethyl)-harnstoff;
195) 4-Methyl-piperazin-1-carbonsäure-[4-(6,8-dichloro-2-methyl-1,2,3,4-tetrahydroisochinolin-4-yl)-phenyl]-amid;
196) Pyrrolidin-1-carbonsäure-[4-(6,8-dichloro-2-methyl-1,2,3,4-tetrahydro-isochinolin-4- yl)-phenyl]-amid;
197) 1-[4-(6,8-Dichloro-2-methyl-1,2,3,4-tetrahydro-isochinolin-4-yl)-phenyl]-3-methyl-harnstoff;
198) 3-[4-(6,8-Dichloro-2-methyl-1,2,3,4-tetrahydro-isochinolin-4-yl)-phenyl]-1,1-dimethyl-harnstoff;
199) 3-[4-(6,8-Dichloro-2-methyl-1,2,3,4-tetrahydro-isochinolin-4-yl)-phenyl]-1,1-diethyl-harnstoff;
200) 1-[4-(6,8-Dichloro-2-methyl-1,2,3,4-tetrahydro-isochinolin-4-yl)-phenyl]-3-(2-dimethylamino-ethyl)-harnstoff;
201) Piperidin-1-carbonsäure-[4-(6,8-dichloro-2-methyl-1,2,3,4-tetrahydro-isochinolin-4- yl)-phenyl]-amid;
202) Morpholin-4-carbonsäure-[4-(6,8-dichloro-2-methyl-1,2,3,4-tetrahydro-isochinolin-4- yl)-phenyl]-amid;
203) N-[4-(6,8-Dichloro-2-methyl-1,2,3,4-tetrahydro-isochinolin-4-yl)-phenyl]-formamid;
204) [4-(6,8-Dichloro-2-methyl-1,2,3,4-tetrahydro-isochinolin-4-yl)-phenyl]-methyl-amin;
205) 1-[4-(6,8-Dichloro-2-methyl-1,2,3,4-tetrahydro-isochinolin-4-yl)-phenyl]-1,3-dimethyl-harnstoff;
206) 4-Methyl-piperazin-1-carbonsäure-[4-(6,8-dichloro-2-methyl-1,2,3,4-tetrahydroisochinolin-4-yl)-phenyl]-methyl-amid ;
207) 1-[4-(6,8-Dichloro-2-methyl-1,2,3,4-tetrahydro-isochinolin-4-yl)-phenyl]-1,3,3-trimethyl-harnstoff;
208) Piperidin-1-carbonsäure-[4-(6,8-dichloro-2-methyl-1,2,3,4-tetrahydro-isochinolin-4- yl)-phenyl]-methyl-amid;
209) Morpholin-4-carbonsäure-[4-(6,8-dichloro-2-methyl-1,2,3,4-tetrahydro-isochinolin-4- yl)-phenyl]-methyl-amid;
210) Pyrrolidin-1-carbonsäure-[4-(6,8-dichloro-2-methyl-1,2,3,4-tetrahydro-isochinolin-4- yl)-phenyl]-methyl-amid;
211) 1-[4-(6,8-Dichloro-2-methyl-1,2,3,4-tetrahydro-isochinolin-4-yl)-phenyl]-3-(2-dimethylamino-ethyl)-1-methyl-harnstoff;
212) 1-[4-(6,8-Dichloro-2-methyl-1,2,3,4-tetrahydro-isochinolin-4-yl)-phenyl]-3,3-diethyl-1- methyl-harnstoff;
213) N-[3-(6,8-Dichloro-2-methyl-1,2,3,4-tetrahydro-isochinolin-4-yl)-phenyl]-formamid;
214) [3-(6,8-Dichloro-2-methyl-1,2,3,4-tetrahydro-isochinolin-4-yl)-phenyl]-methyl-amin;
215) Pyrrolidin-1-carbonsäure-[3-(6,8-dichloro-2-methyl-1,2,3,4-tetrahydro-isochinolin-4- yl)-phenyl]-methyl-amid;
216) Piperidin-1-carbonsäure-[3-(6,8-dichloro-2-methyl-1,2,3,4-tetrahydro-isochinolin-4- yl)-phenyl]-methyl-amid;
217) 1-[3-(6,8-Dichloro-2-methyl-1,2,3,4-tetrahydro-isochinolin-4-yl)-phenyl]-1,3,3-trimethyl-harnstoff;
218) 1-[3-(6,8-Dichloro-2-methyl-1,2,3,4-tetrahydro-isochinolin-4-yl)-phenyl]-1,3-dimethyl-harnstoff;
219) Morpholin-4-carbonsäure-[3-(6,8-dichloro-2-methyl-1,2,3,4-tetrahydro-isochinolin-4- yl)-phenyl]-methyl-amid;
220) 4-Methyl-piperazin-1-carbonsäure-[3-(6,8-dichloro-2-methyl-1,2,3,4-tetrahydroisochinolin-4-yl)-phenyl]-methyl-amid;
221) 1-[3-(6,8-Dichloro-2-methyl-1,2,3,4-tetrahydro-isochinolin-4-yl)-phenyl]-3-(2-dimethylamino-ethyl)-1-methyl-harnstoff;
222) 1-[3-(6,8-Dichloro-2-methyl-1,2,3,4-tetrahydro-isochinolin-4-yl)-phenyl]-3,3-diethyl-1-methyl-harnstoff;
223) [3-(6,8-Dichloro-2-methyl-1,2,3,4-tetrahydro-isochinolin-4-yl)-phenyl]-carbaminsäure-2-dimethylamino-ethyl-ester;
224) [4-(6,8-Dichloro-2-methyl-1,2,3,4-tetrahydro-isochinolin-4-yl)-phenyl]-carbaminsäure-2-dimethylamino-ethyl-ester;
225) [2-(6,8-Dichloro-2-methyl-1,2,3,4-tetrahydro-isochinolin-4-yl)-phenyl]-carbaminsäure-2-dimethylamino-ethyl-ester;
226) [3-(6,8-Dichloro-2-methyl-1,2,3,4-tetrahydro-isochinolin-4-yl)-phenyl]-carbaminsäure-methyl-ester;
227) [3-(6,8-Dichloro-2-methyl-1,2,3,4-tetrahydro-isochinolin-4-yl)-phenyl]-carbaminsäu re-ethyl-ester;
228) [3-(6,8-Dichloro-2-methyl-1,2,3,4-tetrahydro-isochinolin-4-yl)-phenyl]-carbaminsäure-isopropyl-ester;
229) [3-(6,8-Dichloro-2-methyl-1,2,3,4-tetrahydro-isochinolin-4-yl)-phenyl]- carbaminsäure-2,2- dimethyl-propyl-ester;
230) [4-(6,8-Dichloro-2-methyl-1,2,3,4-tetrahydro-isochinolin-4-yl)-phenyl]- carbaminsäure-methyl-ester;
231) [4-(6,8-Dichloro-2-methyl-1,2,3,4-tetrahydro-isochinolin-4-yl)-phenyl]- carbaminsäure-isopropyl-ester;
232) [4-(6,8-Dichloro-2-methyl-1,2,3,4-tetrahydro-isochinolin-4-yl)-phenyl]- carbaminsäure-2,2- dimethyl-propyl-ester;
233) [4-(6,8-Dichloro-2-methyl-1,2,3,4-tetrahydro-isochinolin-4-yl)-phenyl]- carbaminsäure-ethyl-ester;
234) (R)-N-[3-(6,8-Dichloro-2-methyl-1,2,3,4-tetrahydro-isochinolin-4-yl)-phenyl]-methansulfonamid;
235) (S)-N-[3-(6,8-Dichloro-2-methyl-1,2,3,4-tetrahydro-isochinolin-4-yl)-phenyl]-methansulfonamid;
236) (R)-1-[2-(6,8-Dichloro-2-methyl-1,2,3,4-tetrahydro-isochinolin-4-yl)-phenyl]-3-ethyl-harnstoff;
237) (S)-1-[2-(6,8-Dichloro-2-methyl-1,2,3,4-tetrahydro-isochinolin-4-yl)-phenyl]-3-ethyl-harnstoff;
238) N-[3-(6,8-Difluoro-2-methyl-1,2,3,4-tetrahydro-isochinolin-4-yl)-phenyl]-acetamid;
239) 4-(3-Bromo-phenyl)-6,8-dichloro-2-methyl-1,2,3,4-tetrahydro-isochinolin;
240) 1-[3-(6,8-Dichloro-2-methyl-1,2,3,4-tetrahydro-isochinolin-4-yl)-phenyl]-3-(2-hydroxy-ethyl)-harnstoff;
241) 3-(6,8-Dichloro-2-methyl-1,2,3,4-tetrahydro-isochinolin-4-yl)-benzoesäure-ethylester;
242) 3-(6,8-Dichloro-2-methyl-1,2,3,4-tetrahydro-isochinolin-4-yl)-benzoesäure.
sowie aus deren pharmazeutisch verträglichen Salzen.

Außerordentlich ganz besonders bevorzugt ist die Verwendung von Verbindungen ausgewählt aus der Gruppe bestehend aus
1) N-[4-(6,8-Dichloro-2-methyl-1,2,3,4-tetrahydro-isochinolin-4-yl)-phenyl]-acetamid;
2) 4-(6,8-Dichloro-2-methyl-1,2,3,4-tetrahydro-isochinolin-4-yl)-benzolsulfonamid;
3) 3-(6,8-Dichloro-2-methyl-1,2,3,4-tetrahydro-isochinolin-4-yl)-benzolsulfonamid;
4) 4-(6,8-Dichloro-2-methyl-1,2,3,4-tetrahydro-isochiriolin-4-yl)-benzoesäure;
5) 4-(6,8-Dichloro-2-methyl-1,2,3,4-tetrahydroisochinolin-4-yl)-N-ethyl-benzamid;
6) 4-(6,8-Dichloro-2-methyl-1,2,3,4-tetrahydroisochinolin-4-yl)-N-propyl-benzamid;
7) 4-(6,8-Dichloro-2-methyl-1,2,3,4-tetrahydro-isochinolin-4-yl)-N-(2-dimethylamino-ethyl)-benzamid;
8) 6,8-Dichloro-2-methyl-4-(4-morpholin-4-yl-phenyl)-1,2,3,4-tetrahydroisochinolin;
9) 4-(6,8-Dichloro-2-methyl-1,2,3,4-tetrahydro-isochinolin-4-yl)-phenylamin;
10) 1-[4-(6,8-Dichloro-2-methyl-1,2,3,4-tetrahydro-isochinolin-4-yl)-phenyl]-3-methyl-thioharnstoff;
11) 1-[4-(6,8-Dichloro-2-methyl-1,2,3,4-tetrahydro-isochinolin-4-yl)-phenyl]-3-ethyl-harnstoff;
12) N-[4-(6-Bromo-8-chloro-2-methyl-1,2,3,4-tetrahydro-isochinolin-4-yl)-phenyl]-acetamid;
13) 5-(6,8-Dichloro-2-methyl-1,2,3,4-tetrahydro-isochinolin-4-yl)-2-hydroxybenzoesäure;
14) 5-(6,8-Dichloro-2-methyl-1,2,3,4-tetrahydro-isochinolin-4-yl)-N-(2-dimethylamino-ethyl)-2-hydroxy-benzamid;
15) N-[3-(6,8-Dichloro-2-methyl-1,2,3,4-tetrahydro-isochinolin-4-yl)-phenyl]-acetamid;
16) 3-(6,8-Dichloro-2-methyl-1,2,3,4-tetrahydro-isochinolin-4-yl)-phenylamin;
17) 2-(6,8-Dichloro-2-methyl-1,2,3,4-tetrahydrö-isochinolin-4-yl)-phenylamin;
18) N-[4-(6,8-Dichloro-2-methyl-1,2,3,4-tetrahydro-isochinolin-4-yl)-phenyl]-propionamid;
19) 1-Acetyl-piperidin-4-carbonsäure-[4-(6,8-dichloro-2-methyl-1,2,3,4-tetrahydro-isochinolin-4-yl)-phenyl]-amid;
20) N-[4-(6,8-Dichloro-2-methyl-1,2,3,4-tetrahydro-isochinolin-4-yl)-phenyl]-methansulfonamid;
21) Ethansulfonsäure-[4-(6,8-dichloro-2-methyl-1,2,3,4-tetrahydro-isochinolin-4-yl)-phenyl]-amid;
22) N',N'-Dimethylamino-N-[4-(6,8-Dichloro-2-methyl-1,2,3,4-tetrahydro-isochinolin-4-yl)-phenyl]-sulfamid;
23) N-[3-(6,8-Dichloro-2-methyl-1,2,3,4-tetrahydro-isochinolin-4-yl)-phenyl]-propionamid;
24) N-[3-(6,8-Dichloro-2-methyl-1,2,3,4-tetrahydro-isochinolin-4-yl)-phenyl]-butyramid;
25) N-[3-(6,8-Dichloro-2-methyl-1,2,3,4-tetrahydro-isochinolin-4-yl)-phenyl]-isobutyramid;
26) Cyclopropancarbonsäure-[3-(6,8-dichloro-2-methyl-1,2,3,4-tetrahydro-isochinolin-4-yl)-phenyl]-amid;
27) Cyclobutancarbonsäure-[3-(6,8-dichloro-2-methyl-1,2,3,4-tetrahydro-isochinolin-4-yl)- phenyl]-amid;
28) N-[3-(6,8-Dichloro-2-methyl-1,2,3,4-tetrahydro-isochinolin-4-yl)-phenyl]-2,2,2-trifluoro-acetamid;
29) 1-Acetyl-piperidin-4-carbonsäure-[3-(6,8-dichloro-2-methyl-1,2,3,4-tetrahydroisochinolin-4-yl)-phenyl]-amid;
30) N-[3-(6,8-Dichloro-2-methyl-1,2,3,4-tetrahydro-isochinolin-4-yl)-phenyl]-nicotinamid;
31) N-[3-(6,8-Dichloro-2-methyl-1,2,3,4-tetrahydro-isochinolin-4-yl)-phenyl]-methansulfonamid;
32) Ethansulfonsäure-[3-(6,8-dichloro-2-methyl-1,2,3,4-tetrahydro-isochinolin-4-yl)-phenyl]-amid;
33) N',N'-Dimethylamino-N-[3-(6,8-Dichloro-2-methyl-1,2,3,4-tetrahydro-isochinolin-4-yl)-phenyl]-sulfamid;
34) Cyclopropancarbonsäure-[2-(6,8-dichloro-2-methyl-1,2,3,4-tetrahydro-isochinolin-4-yl)-phenyl]-amid;
35) 1-Acetyl-piperidin-4-carbonsäure-[2-(6,8-dichloro-2-methyl-1,2,3,4-tetrahydroisochinolin-4-yl)-phenyl]-amid ;
36) 1-[3-(6,8-Dichloro-2-methyl-1,2,3,4-tetrahydro-isochinolin-4-yl)-phenyl]-3-ethyl-harnstoff;
37) 1-[3-(6,8-Dichloro-2-methyl-1,2,3,4-tetrahydro-isochinolin-4-yl)-phenyl]-3-methyl-thioharnstoff;
38) 1-[2-(6,8-Dichloro-2-methyl-1,2,3,4-tetrahydro-isochinolin-4-yl)-phenyl]-3-ethyl-harnstoff;
39) 1-[2-(6,8-Dichloro-2-methyl-1,2,3,4-tetrahydro-isochinolin-4-yl)-phenyl]-3-methyl-thioharnstoff;
40) N-{5-[3-(6,8-Dichloro-2-methyl-1,2,3,4-tetrahydro-isochinolin-4-yl)-phenylsulfamoyl]-4-methyl-thiazol-2-yl}-acetamid;
41) 1,2-Dimethyl-1 H-imidazole-4-sulfonsäure-[3-(6,8-dichloro-2-methyl-1,2,3,4-tetrahydro- isochinolin-4-yl)-phenyl]-amid;
42) N-[4-(6,8-Dichloro-2-methyl-1,2,3,4-tetrahydro-isochinolin-4-yl)-phenyl]-C,C,C-trifluoro-methanesulfonamid;
43) N-[3-(6,8-Dichloro-2-methyl-1,2,3,4-tetrahydro-isochinolin-4-yl)-phenyl]-C,C,C-trifluoro-methanesulfonamid;
44) N-Ethyl-N'-4-(6,8-Dichloro-2-methyl-1,2,3,4-tetrahydro-isochinolin-4-yl)-benzolsulfonyl-harnstoff;
45) N-[4-(6,8-Dichloro-2-methyl-1,2,3,4-tetrahydro- isochinolin -4-yl)-phenyl]-2-dimethylamino-acetamid;
46) 2,6-Diamino-hexansäure-[4-(6,8-dichloro-2-methyl-1,2,3,4-tetrahydro- isochinolin -4-yl)- phenyl]-amid;
47) 1H-Pyrrole-3-carbonsäure-[4-(6,8-dichloro-2-methyl-1,2,3,4-tetrahydro-isochinolin-4- yl)-phenyl]-amid;
48) 1-Methyl-piperidin-4-carbonsäure-[4-(6,8-dichloro-2-methyl-1,2,3,4-tetrahydroisochinolin-4-yl)-phenyl]-amid;
49) 1-Methansulfonyl-piperidin-4-carbonsäure-[4-(6,8-dichloro-2-methyl-1,2,3,4-tetrahydro- isochinolin-4-yl)-phenyl]-amid;
50) 1 H-Pyrazol-4- carbonsäure-[4-(6,8-dichloro-2-methyl-1,2,3,4-tetrahydroisochinolin- 4-yl)-phenyl]-amid;
51) N-[3-(6,8-Dichloro-2-methyl-1,2,3,4-tetrahydro-isochinolin-4-yl)-phenyl]-2-methylamino-acetamid;
52) N-[3-(6,8-Dich(oro-2-methyl-1,2,3,4-tetrahydro-isochinolin-4-yl)-phenyl]-2-dimethylamino-acetamid;
53) 2-Amino-N-[3-(6,8-dichloro-2-methyl-1,2,3,4-tetrahydro-isochinolin-4-yl)-phenyl]-propionamid;
54) 2-Amino-N-[3-(6,8-dichloro-2-methyl-1,2,3,4-tetrahydro-isochinolin-4-yl)-phenyl]-butyramid;
55) 2,6-Diamino-hexansäure-[3-(6,8-dichloro-2-methyl-1,2,3,4-tetrahydro-isochinolin-4-yl)- phenyl]-amid;
56) 1-Methyl-piperidin-4- carbonsäure-[3-(6,8-dichloro-2-methyl-1,2,3,4-tetrahydroisochinolin-4-yl)-phenyl]-amid;
57) 1H-Imidazol-4- carbonsäure-[3-(6,8-dichloro-2-methyl-1,2,3,4-tetrahydro-isochuinolin- 4-yl)-phenyl]-amid;
58) 1-Methansulfonyl-piperidin-4- carbonsäure-[3-(6,8-dichloro-2-methyl-1,2,3,4-tetrahydro-isochinolin-4-yl)-phenyl]-amid;
59) 3,5-Dimethyl-1 H-pyrazol-4- carbonsäure-[3-(6,8-dichloro-2-methyl-1,2,3,4-tetrahydro-isochinolin-4-yl)-phenyl]-amid;
60) 1H-Pyrazol-4- carbonsäure-[3-(6,8-dichloro-2-methyl-1,2,3,4-tetrahydroisochinolin- 4-yl)-phenyl]-amid;
61) 3-[3-(6,8-Dichloro-2-methyl-1,2,3,4-tetrahydro-isochinolin-4-yl)-phenyl]-1,1-dimethyl-harnstoff;
62) 4-Methyl-piperazin-1-carbonsäure-[3-(6,8-dichloro-2-methyl-1,2,3,4-tetrahydroisochinolin-4-yl)-phenyl]-amid;
63) Piperidin-1-carbonsäure-[3-(6,8-dichloro-2-methyl-1,2,3,4-tetrahydro-isochinolin-4- yl)-phenyl]-amid;
64) Morpholin-4-carbonsäure-[3-(6,8-dichloro-2-methyl-1,2,3,4-tetrahydro-isochinolin-4- yl)-phenyl]-amid;
65) Pyrrolidin-1-carbonsäure-[3-(6,8-dichloro-2-methyl-1,2,3,4-tetrahydro-isochinolin-4- yl)-phenyl]-amid;
66) 3-[3-(6,8-Dichloro-2-methyl-1,2,3,4-tetrahydro-isochinolin-4-yl)-phenyl]-1,1-diethyl-harnstoff;
67) 1-[3-(6,8-Dichloro-2-methyl-1,2,3,4-tetrahydro-isochinolin-4-yl)-phenyl]-3-methyl-harnstoff;
68) 1-[3-(6,8-Dichloro-2-methyl-1,2,3,4-tetrahydro-isochinolin-4-yl)-phenyl]-3-(2-dimethylamino-ethyl)- harnstoff;
69) 1-[3-(6,8-Dichloro-2-methyl-1,2,3,4-tetrahydro-isochinolin-4-yl)-phenyl]-3-(tetrahydro-furan-3-yl)-harnstoff;
70) 1-[3-(6,8-Dichloro-2-methyl-1,2,3,4-tetrahydro-isochinolin-4-yl)-phenyl]-3-(tetrahydro-pyran-4-yl)-harnstoff;
71) 3-[3-(6,8-Dichloro-2-methyl-1,2,3,4-tetrahydro-isochinolin-4-yl)-phenyl]-1-methyl-1-(1-methyl-piperidin-4-yl)-harnstoff;
72) 3-[3-(6,8-Dichloro-2-methyl-1,2,3,4-tetrahydro-isochinolin-4-yl)-phenyl]-1-(3-dimethylamino-propyl)-1-methyl-harnstoff;
73) 3-[3-(6,8-Dichloro-2-methyl-1,2,3,4-tetrahydro-isochinolin-4-yl)-phenyl]-1-(2-dimethylamino-ethyl)-1-methyl-harnstoff;
74) 1-[3-(6,8-Dichloro-2-methyl-1,2,3,4-tetrahydro-isochinolin-4-yl)-phenyl]-3-(3-dimethylamino-propyl)-harnstoff;
75) 1-[3-(6,8-Dichloro-2-methyl-1,2,3,4-tetrahydro-isochinolin-4-yl)-phenyl]-3-(2-methoxy-ethyl)-harnstoff;
76) 1-[3-(6,8-Dichloro-2-methyl-1,2,3,4-tetrahydro-isochinolin-4-yl)-phenyl]-3-pyridin-3-yl-harnstoff;
77) 1-[3-(6,8-Dichloro-2-methyl-1,2,3,4-tetrahydro-isochinolin-4-yl)-phenyl]-3-pyridin-4-yl-harnstoff;
78) 4-Methyl-piperazin-1-carbonsäure-[2-(6,8-dichloro-2-methyl-1,2,3,4-tetrahydro-isoquinolin-4-yl)-phenyl]-amid;
79) 1-[2-(6,8-Dichloro-2-methyl-1,2,3,4-tetrahydro-isochinolin-4-yl)-phenyl]-3-methyl-harnstoff;
80) 1-[2-(6,8-Dichloro-2-methyl-1,2,3,4-tetrahydro-isochinolin-4-yl)-phenyl]-3-(2-dimethylamino-ethyl)-harnstoff;
81) 4-Methyl-piperazin-1-carbonsäure-[4-(6,8-dichloro-2-methyl-1,2,3,4-tetrahydroisochinolin-4-yl)-phenyl]-amid;
82) 1-[4-(6,8-Dichloro-2-methyl-1,2,3,4-tetrahydro-isochinolin-4-yl)-phenyl]-3-methyl-harnstoff;
83) 3-[4-(6,8-Dichloro-2-methyl-1,2,3,4-tetrahydro-isochinolin-4-yl)-phenyl]-1,1-dimethyl-harnstoff;
84) 3-[4-(6,8-Dichloro-2-methyl-1,2,3,4-tetrahydro-isochinolin-4-yl)-phenyl]-1,1-diethyl-harnstoff;
85) 1-[4-(6,8-Dichloro-2-methyl-1,2,3,4-tetrahydro-isochinolin-4-yl)-phenyl]-3-(2-dimethylamino-ethyl)-harnstoff;
86) Morpholin-4-carbonsäure-[4-(6,8-dichloro-2-methyl-1,2,3,4-tetrahydro-isochinolin-4- yl)-phenyl]-amid;
87) N-[4-(6,8-Dichloro-2-methyl-1,2,3,4-tetrahydro-isochinolin-4-yl)-phenyl]-formamid;
88) [4-(6,8-Dichloro-2-methyl-1,2,3,4-tetrahydro-isochinolin-4-yl)-phenyl]-methyl-amin;
89) N-[3-(6,8-Dichloro-2-methyl-1,2,3,4-tetrahydro-isochinolin-4-yl)-phenyl]-formamid;
90) [3-(6,8-Dichloro-2-methyl-1,2,3,4-tetrahydro-isochinolin-4-yl)-phenyl]-methyl-amin;

Enthalten die Verbindungen der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste, bzw. teilweise oder vollständig fluorierten Alkylreste können sowohl geradkettig als auch verzweigt vorliegen. Gruppen CₐH₂ₐ₋₁ bzw. deren Analoga bis C_{yy}H_{2yy-1} bedeuten entweder die entsprechenden Alkenyle, Cycloalkyle, Cycloalkyl-alkyle oder Alkyl-cycloalkyle.

Als Pyrrolyl gelten insbesondere 1-, 2- oder 3- Pyrrolyl, als Imidazolyl gelten insbesondere 1-, 2-, 4- oder 5-lmidazolyl, als Pyrazolyl gelten insbesondere 1-, 3-, 4- oder 5-Pyrazolyl, als Triazolyl gelten insbesondere 1,2,3-Triazol-1-, -4- oder 5-yl, 1,2,4-Triazol-1-, -3- oder -5-yl, als Tetrazolyl gelten insbesondere 1- oder 5-Tetrazolyl, als Oxazolyl gelten insbesondere 2-, 4- oder 5-Oxazolyl, als Thiazolyl gelten insbesondere 2-, 4- oder 5-Thiazolyl, als Pyridyl gelten insbesondere 2-, 3- oder 4-Pyridyl. Umfasst sind weiterhin die entsprechenden N-Oxide dieser Verbindungen, also z.B. 1-Oxy-2-, 3- oder 4-pyridyl.

Insbesondere bevorzugt sind die Heterocyclen Imidazolyl, Pyrazolyl, Pyrrolyl, Triazolyl, Tetrazolyl, Thiazolyl und Oxazolyl

Als CH₂-Einheiten gelten auch die in einer Alkylkette terminalen CH₃-Gruppen, die in diesem Zusammenhang als CH₂-H Gruppierungen aufgefasst werden.

Beschrieben werden auch Methoden zur Herstellung der verwendeten Verbindungen.

So lassen sich die hier beschriebenen Substanzen ausgehend von den Benzylaminvorläufern IV herstellen, Diese wiederum können, falles nicht käuflich erhältlich, nach Standartverfahren aus den entsprechenden Benzylchloriden oder -bromiden III synthetisiert werden.

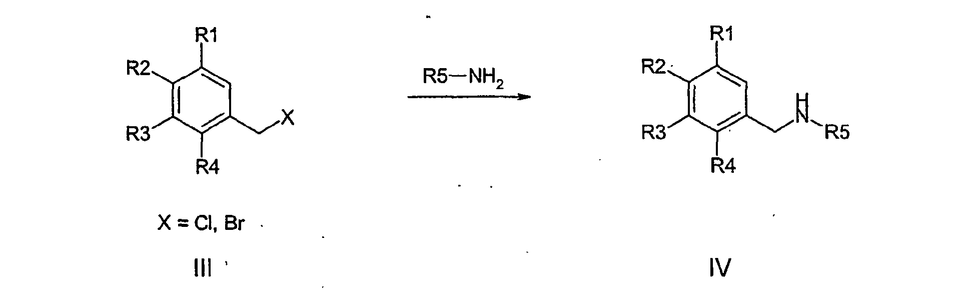

Die so erhaltenen Benzylamine IV, werden in dem Fachmann bekannter Weise mit den entsprechend substituierten alpha-Bromacetophenonverbindungen V alkyliert.

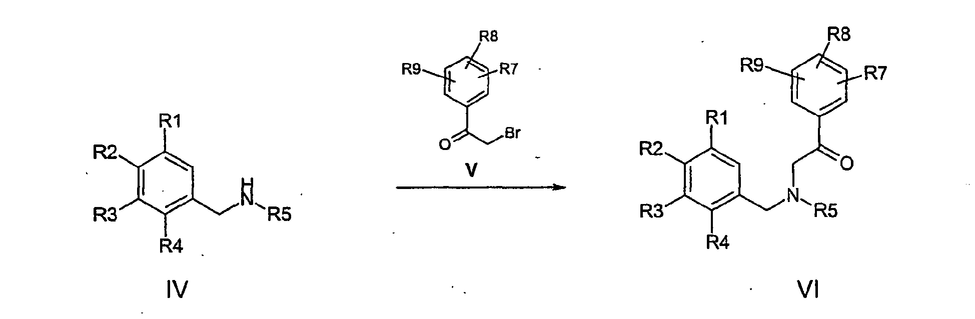

Die alpha-Bromacetophenonverbindungen V lassen sich in literaturbekannten Verfahren aus den entsprechenden Acetophenonvorläufern durch Bromierung gewinnen. Durch Reduktion der Carbonylgruppierung in VI und anschließender säurekatalysierter Cyclisierung der entsprechenden Alkohole VII (vgl. Tetrahedron Lett.; 1989, 30, 5837; Org. Prep. Proced. Int.; 1995, 27, 513) können die gewünschten Tetrahydroisochinoline I nach bekannten Verfahren gewonnen werden.

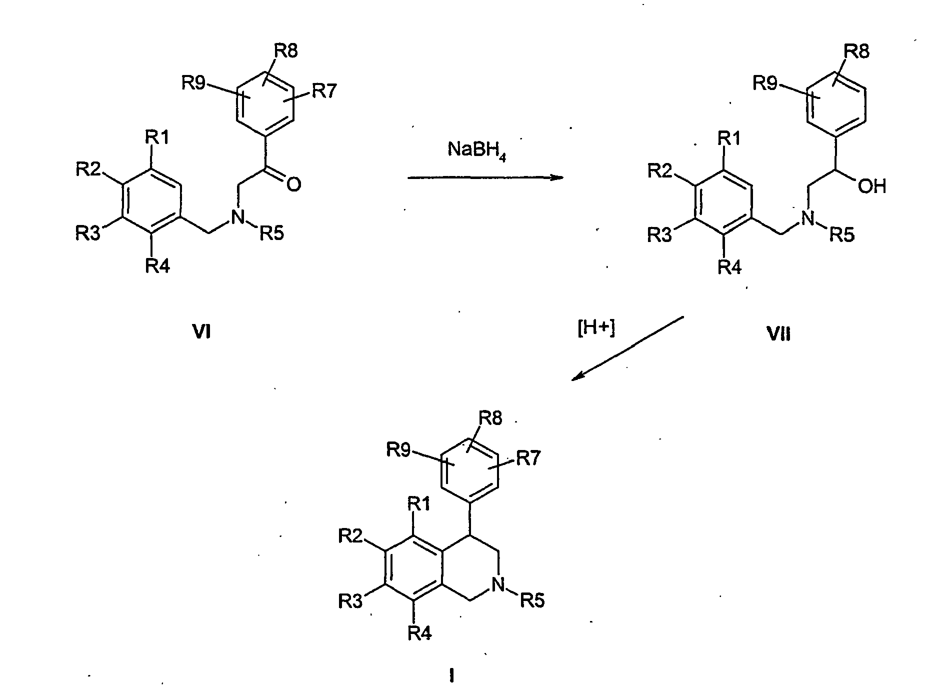

Für R6 ungleich H lassen sich die gewünschten Verbindungen der Formel I z. B. aus den Iodiden VIII durch Halogen-Metall-Austausch und anschließendem nucleophilen Angriff der intermediären lithiumorganischen Spezies an der Carbonylgruppe herstellen (vgl. Chem. Pharm. Bull.; 1995, 43, 1543).

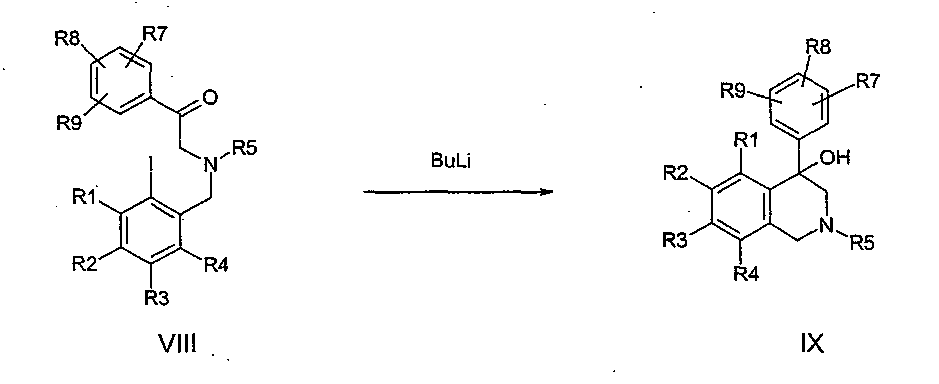

Die dabei synthetisierten tertiären Alkohole lassen sich durch bekannte Methoden in weitere Derivate überführen.

Zur Herstellung alkylverzweigter Analoge (I) werden die entsprechenden Diphenylessigsäureester X in alpha-Stellung nach bekannten Methoden alkyliert. Die gewünschten Produkt XI können durch Standardverfahren in die entsprechenden Amide XII überführt werden, welche in einer Pictet-Spengler-analogen Reaktion in die gewünschten Tetrahydroisochinoline I überführt werden (vgl. Tetrahedron; 1987, 43, 439; Chem. Pharm. Bull.; 1985, 33, 340).

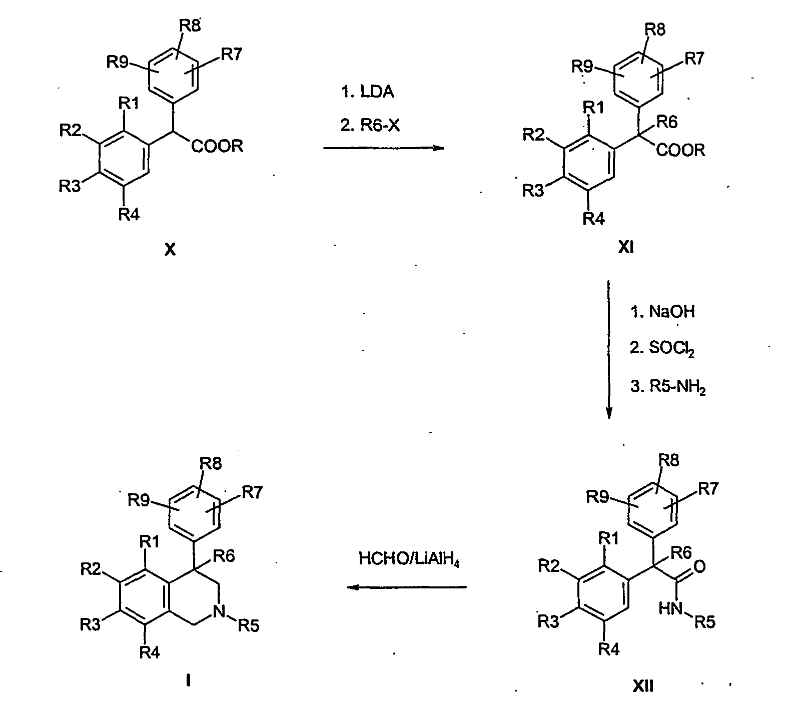

In den Offenlegungsschriften WO 01 32 624 und WO 01 32 625 sind Verbindungen des Typs I als Reuptake-Inhibitoren von Norepinephrin, Dopamin und Serotonin beschrieben. Allerdings sind in diesen Patentanmeldungen ausschließlich Verbindungen geschützt, bei denen R1 und R2 ausschließlich H sein dürfen. Bei den erfindungsgemäßen Verbindungen hat sich allerdings gezeigt, dass zumindest für R2 gelten muss, dass R2 nicht gleich H ist. Des weiteren konnte anhand einer Beispielverbindung der erfindungsgemäßen Verbindungen keine inhibitorischen Eigenschaften an den beschriebenen Rezeptoren nachgewiesen werden, so dass sich die beschriebenen Verbindungen sowohl strukturell als auch in ihren pharmakologischen Eigenschaften von den in den erwähnten Patentanmeldungen beschriebenen Verbindungen deutlich unterscheiden.

Des weiteren sind Verbindungen des Typs I in der Offenlegungsschrift EP 11 13 007 als Östrogenagonisten und -antagonisten beschrieben. Es konnte gezeigt werden, dass die erfindungsgemäßen Verbindungen keine Aktivität an den genannten Rezeptoren zeigen, so dass auch hier die strukturellen Unterschiede der erfindungsgemäßen Verbindungen in deutlich anderen pharmakologischen Eigenschaften resultieren.

Es konnte gezeigt werden, dass Verbindungen der Formel I hervorragende Inhibitoren des Natrium-Wasserstoffaustauschers (NHE) - insbesondere des Natrium-Wasserstoffaustauschers vom Subtyp 3 (NHE3) - darstellen.

Aufgrund dieser Eigenschaften eignen sich die Verbindungen zur Behandlung von Krankheiten, die durch Sauerstoffmangel hervorgerufen werden. Die Verbindungen sind infolge ihrer pharmakologischen Eigenschaften als antiarrhythmische Arzneimittel mit cardioprotektiver Komponente zur Infärktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäß verwendeten Verbindungen der Formel I ebenfalls zur Behandlung von Formen des Schocks, wie beispielweise des allergischen, cardiogenen, hypovolämischen und des bakteriellen Schocks.

Weiterhin induzieren die Verbindungen eine Verbesserung des Atemantriebes und werden deshalb zur Behandlung von Atmungszuständen bei folgenden klinischen Zuständen und Krankheiten herangezogen: Gestörter zentraler Atemantrieb (z. B. zentrale Schlafapnoen, plötzlicher Kindstod, postoperative Hypoxie), muskulärbedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.
Zusätzlich erhöhen die Verbindungen den Muskeltonus der oberen Atemwege, so dass das Schnarchen unterdrückt wird.

Eine Kombination eines NHE-Inhibitors mit einem Carboanhydrase-Hemmer (z. B. Acetazolamid), wobei letzterer eine metabolische Azidose herbeiführt und dadurch bereits die Atmungstätigkeit steigert, erweist sich als vorteilhaft durch verstärkte Wirkung und verminderten Wirkstoffeinsatz.

Es hat sich gezeigt, dass die erfindungsgemäß verwendeten Verbindungen eine milde abführende Wirkung besitzen und demzufolge vorteilhaft als Abführmittel oder bei drohender Darmverstopfung verwendet werden können, wobei die Verhinderung der mit Verstopfungen im Darmbereich einhergehenden ischämischen Schäden besonders vorteilhaft ist.
Weiterhin besteht die Möglichkeit, der Gallenstein-Bildung vorzubeugen.

Darüber hinaus zeichnen sich die erfindungsgemäß verwendeten Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäß verwendeten Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Es wurde außerdem gefunden, dass NHE-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Die erfindungsgemäß verwendeten Verbindungen können daher zur Prophylaxe und zur Regression von atherosklerotischen Veränderungen herangezogen werden, indem sie einen kausalen Risikofaktor ausschalten. Mit diesem Schutz der Gefäße gegen das Syndrom der endothelialen Dysfunktion sind Verbindungen der Formel I wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Prävention und Behandlung von Schlafapnoen und muskulär bedingter Atemstörungen; zur Herstellung eines Medikaments zur Prävention und Behandlung des Schnarchens; zur Herstellung eines Medikaments zur Blutdrucksenkung; zur Herstellung eines Medikaments zur Prävention und Behandlung von Erkrankungen, die durch Ischämie und Reperfusion von zentralen und peripheren Organen ausgelöst werden wie das akute Nierenversagen, der Schlaganfall, endogene Schockzustände, Darmerkrankungen etc.; zur Herstellung eines Medikaments zur Behandlung diabetischer Spätschäden und chronischer Nierenerkrankungen, insbesondere von allen Nierenentzündungen (Nephritiden), die mit einer vermehrten Protein-/ Albuminausscheidung verbunden sind; zur Herstellung eines Medikaments zur Behandlung des Befalls durch Ektoparasiten in der Human- und Veterinärmedizin; zur Herstellung eines Medikaments zur Behandlung der genannten Leiden in Kombinationen mit blutdrucksenkenden Stoffen, bevorzugt mit Angiotensin Converting Enzyme (ACE)-Hemmem, mit Diuretika und Saluretika wie Furosemid, Hydrochlorothiazid, Pseudoaldosteronantagonisten und Aldosteron-Antagonisten; mit Adenosinrezeptor-Modulatoren, insbesondere mit Adenosinrezeptor-Aktivatoren (A2-Agonisten); und mit Angiotensin-Rezeptorantagonisten.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Arzneimittel, die eine Verbindung I enthalten, können dabei oral, parenteral, intravenös, rektal, transdermal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.
Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Versuchsbeschreibungen und Beispiele:

### Liste der verwendeten Abkürzungen:

- Rₜ: Retentionszeit
- TFA: Trifluoressigsäure
- HPLC: High Performance Liquid Chromatography
- eq: Äquivalente
- LCMS: Liquid Chromatography Mass Spectroscopy
- MS: Mass Spectroscopy
- Cl: Chemical lonization
- RT: Raumtemperatur
- THF: Tetrahydrofuran
- TOTU: O-[(Ethoxycarbonyl)-cyanomethylenamino]-N,N,N',N'-tetramethyluronium-tetrafluoroborat
- DMSO: Dimethylsulfoxid
- abs.: absolut(es)
- Zers.: Zersetzung
- DMF: Dimethylformamid

### Allgemeines:

Die im Folgenden angegebenen Retentionszeiten (Rₜ) beziehen sich auf LCMS-Messungen mit den folgenden Parametern:
Methode A:
- stationäre Phase:: Merck Purospher 3µ2 x 55 mm
- mobile Phase:: 95% H₂O (0.05% TFA)→ 95% Acetonitril; 4 min; 95% Acetonitril; 1.5 min → 5% Acetonitril; 1 min; 0.5 ml/min, 30 °C.

Methode B:
- stationäre Phase:: Merck Purospher 3µ 2 x 55 mm
- mobile Phase:: 0 min 90% H₂O (0.05% TFA) 2,5 min-95 % Acetonitril; 95 % Acetonitril bis 3,3 min; 10 % Acetonitril 3,4 min; 1 ml/min.

Methode B1:
- stationäre Phase:: YMC, J'sphere ODS H80 4µ 2 x 20 mm
- mobile Phase:: 0 min 90% H₂O (0.05% TFA) 1,9 min-95 % Acetonitril; 95 % Acetonitril bis 2,4 min; 10 % Acetonitril 2,45 min; 1 ml/min.

Methode C:
stationäre Phase: Merck LiChroCart 55-2 Purospher STAR RP 18e
Solvent: Solvent A: Acetonitril/Wasser 90:10 + 0,5% HCOOH
Solvent B: Acetonitril/Wasser 10:90 + 0,5% HCOOH
Fluß: 0,75 ml/min

| Time[min] | Solvent B[%] |
|---|---|
| 0,00 | 95,0 |
| 0,50 | 95,0 |
| 1,75 | 5,0 |
| 4,25 | 5,0 |
| 4,50 | 95,0 |
| 5,00 | 95,0 |

Stoptime: 6,20 min
Temperatur: 40 °C
Methode D:
stationäre Phase: Merck RP18 Purospher Star, 55 x 2 mm, 3 µ Korngröße
Solvent: Solvent A: Acetonitril + 0,08% HCOOH
Solvent B: Wasser + 0,1% HCOOH
Fluß: 0,45 ml/min

| Time [min] | Solvent B[%] |
|---|---|
| 0 | 95 |
| 5 | 5 |
| 7 | 5 |
| 8 | 95 |
| 9 | 5 |

Temperatur: Raumtemperatur

### Beispiel 1: N-[4-(6,8-Dichloro-2-methyl-1,2,3,4-tetrahydro-isochinolin-4-yl)-phenyl]-acetamid

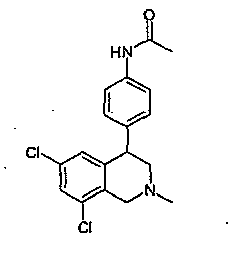

Zwischenprodukt 1: 2,4-Dichlorbenzyl-methyl-amin
wird nach literaturbekannten Methoden hergestellt (J. Med. Chem.; 1984, 27, 1111).

Zwischenprodukt 2: N-[4-(2-Bromo-acetyl)-phenyl]-acetamid
wird in dem Fachmann bekannter Weise durch Bromierung von N-(4-Acetyl-phenyl)-acetamid synthetisiert.
Die Ausgangsverbindung (0,256 mol) wird in 300 ml Essigsäure vorgelegt und bei 60 °C eine Lösung von 39,9 g Brom (1,0 eq) in 60 ml Essigsäure zugetropft. Nach 1,5 h wird auf Raumtemperatur abkühlen lassen und die Reaktionsmischung auf 1 l Eiswasser gegeben. Der Niederschlag wird abgesaugt, mit Wasser gewaschen und getrocknet, wobei 60 g der Titelverbindung isoliert werden (Schmp.: 192°C ).

Zwischenprodukt 3: N-{4-[2-(2,4-Dichloro-benzylamino)-acetyl]-phenyl}-acetamid; 37,1 g (0,195 mol) des Zwischenprodukts 1 werden in 400 ml Dioxan vorgelegt und mit einer Lösung aus 60 g (0,234 mol) des Zwischenprodukts 2 in 600 ml Dioxan versetzt. Es werden 134 ml Triethylamin zugegeben und 4 h bei Raumtemperatur gerührt. Nach Stehen über Nacht wird der Niederschlag abfiltriert und das Filtrat i. Vak. eingeengt. Der Rückstand wird in Essigester aufgenommen, mit NaHCO₃ und H₂O gewaschen, mit MgSO₄ getrocknet und eingeengt. Der hierbei anfallende ölige Rückstand wird mit einem Essigester/Ether-Gemisch verrieben, wobei 36 g des Zwischenprodukt 3 in Form eines kristallinen Feststoffs anfallen (Schmp.: 115-117°C).

### Zwischenprodukt 4:

### N-{4-[2-(2,4-Dichloro-benzylamino)-1-hydroxy-ethyl]-phenyl}-acetamid;

36 g (0,099 mol) des Zwischenprodukts 3 werden in 500 ml Methanol gelöst und bei 0°C mit 7,8 g (2 eq) Natriumborhydrid versetzt. Es wird noch 30 min bei 0°C und eine weitere Stunde bei Raumtemperatur nachgerührt. Zur Aufarbeitung wird der Reaktionsansatz eingeengt und der Rückstand zwischen 1 N HCl und Essigester verteilt. Die wässrige Phase wird abgetrennt, auf pH 9 eingestellt und zweimal mit Essigester extrahiert. Die vereinigten organischen Phasen werden mit MgSO₄ getrocknet und eingeengt. Das so erhaltene Rohprodukt kann ohne weitere Reinigung weiter umgesetzt werden.

### N-[4-(6,8-Dichloro-2-methyl-1,2,3,4-tetrahydro-isochinolin-4-yl)-phenyl]-acetamid;

20 g (0,054 mol) der Zwischenverbindung 4 werden in 250 ml Dichlormethan gelöst und bei 0°C mit 250 ml konz. H₂SO₄ tropfenweise versetzt. Es wird 2 h bei 0°C und 1 h bei Raumtemperatur gerührt. Zur Aufarbeitung wird die Reaktionsmischung auf Eiswasser gegeben und der Niederschlag abgesaugt. Der Niederschlag wird in 300 ml 1 N NaOH aufgenommen und dreimal mit Essigester extrahiert. Trocknen der organischen Phasen und Einengen liefert ein Rohprodukt, das mit Diisopropylether verrieben wird, wobei 11,7 g der Beispielverbindung als kristalliner Feststoff isoliert werden (Schmp.: 205-206°C).

### 1a: N-[4-(6,8-Dichloro-2-methyl-1,2,3,4-tetrahydro-isochinolin-4-yl)-phenyl]-acetamid-Hydrochlorid;

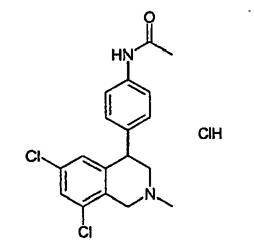

Eine analytische Probe (100 mg) der Titelverbindung aus Beispiel 1 wird in 10 ml 2 N HCl suspendiert und mit soviel THF versetzt, dass eine klare Lösung entsteht. Es wird i. Vak. eingeengt, der Rückstand mit Ether verrieben und abgesaugt, worauf die Titelverbindung als kristalliner Feststoff erhalten wird (Rₜ = 3,807 min (Methode A); Schmp.: 125°C unter Zersetzung).

### Beispiel 2:

2a: (+)-4-(6,8-Dichloro-2-methyl-1,2,3,4-tetrahydro-isochinolin-4-yl)-benzol-sulfonamid-Hydrochlorid;
2b: (+)-3-(6,8-Dichloro-2-methyl-1,2,3,4-tetrahydro-isochinolin-4-yl)-benzol-sulfonamid-Hydrochlorid;
2c: (-)-4-(6,8-Dichloro-2-methyl-1,2,3,4-tetrahydro-isochinolin-4-yl)-benzol-sulfonamid-Acetat;
2d: (-)-3-(6,8-Dichloro-2-methyl-1,2,3,4-tetrahydro-isochinolin-4-yl)-benzol-sulfonamid-Acetat;

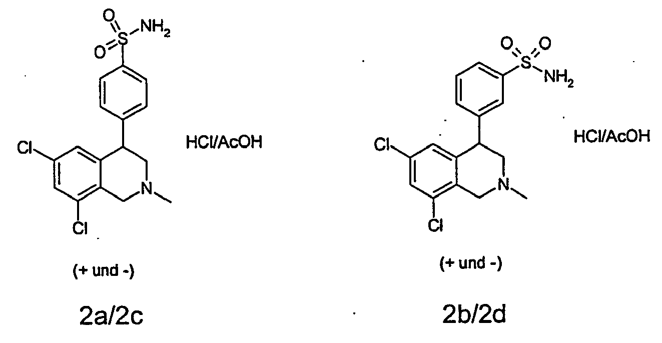

Zwischenprodukt 1: 2,4-Dichlorbenzyl-methyl-amin
wird nach literaturbekannten Methoden hergestellt (J. Med. Chem.; 1984, 27, 1111).

### Zwischenprodukt 2: 2-[(2,4-Dichloro-benzyl)-methyl-amino]-1-phenyl-ethanon;

Zwischenprodukt 1 wird mit 2-Bromo-1-phenyl-ethanon in der unter Beispiel 1, Zwischenprodukt 3 beschriebenen Weise umgesetzt. Aufarbeitung in analoger Weise und Reinigung an Kieselgel liefert das gewünschte Alkylierungsprodukt in guter Ausbeute als gelbliches Öl (Rt = 4,188 min(Methode A); MS(Cl⁺) = 308,2/310,2).

### Zwischenprodukt 3: 2-[(2,4-Dichloro-benzyl)-methyl-amino]-1-phenyl-ethanol;

Zwischenprodukt 2 wird in der in Beispiel 1, Zwischenprodukt 4 beschriebenen Weise mit Natriumborhydrid reduziert. Nachdem die Reaktionskontrolle vollständigen Umsatz anzeigt, wird eingeengt und der Rückstand in Essigester aufgenommen. Es wird zweimal mit H₂O gewaschen, mit MgSO₄ getrocknet und vom Lösungsmittel befreit. Das in quantitativer Ausbeute erhaltene Rohprodukt kann ohne weitere Reinigung weiter umgesetzt werden (Rₜ = 4,149 min(Methode A); MS(Cl⁺) = 310,2/312,2).

### Zwischenprodukt 4: 6,8-Dichloro-2-methyl-4-phenyl-1,2,3,4-tetrahydro-isochinolin;

20 g (64,5 mmol) des Zwischenprodukts 3 werden in 55 ml Dichlormethan gelöst und auf 0°C gekühlt. Diese Lösung wird zu 55 ml einer vorgekühlten konz. H₂SO₄ getropft und im Anschluss zwei Stunden bei Raumtemperatur gerührt. Zur Aufarbeitung gibt man auf Eis und stellt mit 6 N NaOH stark alkalisch. Es wird dreimal mit Dichlormethan extrahiert. Die vereinigten organischen Phasen werden mit MgSO₄ getrocknet und eingeengt. Das ölige Rohprodukt wird an Kieselgel gereinigt, wobei Zwischenprodukt 4 in 53 % Ausbeute erhalten wird (Rt = 4,444 min(Methode A); MS(Cl⁺) = 292,2/294,2).
4a: (-)-6,8-Dichloro-2-methyl-4-phenyl-1,2,3,4-tetrahydro-isochinolin-Trifluoracetat;
4b: (+)-6,8-Dichloro-2-methyl-4-phenyl-1,2,3,4-tetrahydro-isochinolin-Trifluoracetat;

Zwischenprodukt 4 wird an einer chiralen Phase mittels HPLC in die beiden Enantiomere getrennt.
chirale Säule: Chiralpak OD 250 x 4,6 cm;
Lösungsmittel: n-Heptan/iso-Propanol 7:3 + 0,1 % TFA;
Flussrate: 1 ml/min;
Rₜ((-)-Enantiomer/4a) = 9,340 min;
Rₜ((+)-Enantiomer/4b) = 20,327 min.
2a: (+)-4-(6,8-Dichloro-2-methyl-1,2,3,4-tetrahydro-isochinolin-4-yl)-benzol-sulfonamid-Hydrochlorid;
2b: (+)-3-(6,8-Dichloro-2-methyl-1,2,3,4-tetrahydro-isochinolin-4-yl)-benzol-sulfonamid-Hydrochlorid;

Eine Suspension von 500 mg (1,7 mmol) des Zwischenprodukts 4a in 10 ml Dichlormethan wird bei 0°C in 1,2 ml Chlorsulfonsäure eingetragen. Es wird eine Stunde bei 0°C und eine weitere bei Raumtemperatur gerührt. Es werden weitere 5 ml Chlorsulfonsäure zugegeben und noch eine Stunde bei Raumtemperatur gerührt. Zur Aufarbeitung gibt man auf Eis und stellt mit NaHCO₃ auf einen pH-Wert von 8. Es wird dreimal mit Essigester extrahiert. Die vereinigten organischen Phasen werden mit Na₂SO₄ getrocknet und vom Lösungsmittel befreit. Das so erhaltene Rohprodukt wird in 20 ml konz. NH₃-Lösung drei Stunden auf 90°C erhitzt. Nach vollständigem Umsatz wird die Reaktionslösung eingeengt und der Rückstand zwischen H₂O und Essigester verteilt. Die organische Phase wird abgetrennt und die wässrige noch einmal mit Essigester extrahiert. Die vereinigten organischen Phasen werden mit Na₂SO₄ getrocknet und das Lösungsmittel i. Vak. entfernt. Anschließende Chromatographie an Kieselgel liefert 335 mg einer Mischung aus Beispiel 2a und 2b in Form eines gelben amorphen Feststoffs. Weitere Reinigung an einer präparativen HPLC liefert 212 mg der para-substituierten Titelverbindung 2a, sowie 58 mg des meta-Isomeren 2b. Bedingungen der präparativen HPLC.
chirale Säule: Chiralpak AS 250 x 4,6 mm;
Lösungsmittel: n-Heptan/Ethanol/Methanol/Acetonitril 20:1,5:0,5:0,5
Flussrate: 1 ml/min;
Rt(Hauptfraktion)= 14,145 min (→2a);
Rt(Nebenfraktion) = 11,623 min (→2b).

Beide Fraktionen wurden in Methanol/2 N HCl -Gemischen gelöst und gefriergetrocknet, wobei die Titelverbindungen 2a und 2b in Form kristalliner Feststoffe erhalten werden konnten.
(Rₜ(2a) = 3,630 min (Methode A); MS(2a),(ES⁺) =371,3/373,3 (M⁺+H)/ 412,3/ 414,3 (M⁺+CH₃CN); Rt (2b) = 3,668 min (Methode A); MS(2b),(ES⁺) =371,3/373,3 (M⁺+H)/412,3/ 414,3 (M⁺+CH₃CN).
2c: (-)-4-(6,8-Dichloro-2-methyl-1,2,3,4-tetrahydro-isochinolin-4-yl)-benzol-sulfonamid-Acetat;
2d: (-)-3-(6,8-Dichloro-2-methyl-1,2,3,4-tetrahydro-isochinolin-4-yl)-benzol-sulfonamid-Acetat;

Die Synthese der Titelverbindung erfolgt nach der unter 2a/2b beschriebenen Methode, wobei als Ausgangsverbindung das Zwischenprodukt 4b verwendet wird. Die Reinigung und Abtrennung von dem zu erwartenden meta-Isomeren erfolgt unter den folgenden Bedingungen:
chirale Säule: Chiralpak AS 250 x 4,6/12 mm;
Lösungsmittel: Acetonitril
Flussrate: 1 ml/min;
Rt(Hauptfraktion)= 4,394 min (→2c);
Rt(Nebenfraktion) = 4,130 min (→2d).
Die gereinigten Produkte werden jeweils in einer 10 % Essigsäure-Lösung aufgenommen und gefriergetrocknet, wobei die gewünschten Acetate als leicht gelbliche Feststoffe erhalten werden. (Rt(2c) = 3,656 min (Methode A); MS(ES⁺) =371,1/373,1 (M⁺+H)/ 412,1/ 414,1 (M⁺+CH₃CN)); (Rt(2d) = 1,562 min (Methode B); MS(ES⁺) =371,1/373,1 (M⁺+H)/ 412,1/ 414,1 (M⁺+CH₃CN)).

### Beispiel 3: 4-(6,8-Dichloro-2-methyl-1,2,3,4-tetrahydro-isochinolin-4-yl)-N,N-dimethyl-benzolsulfonamid, Hydrochlorid;

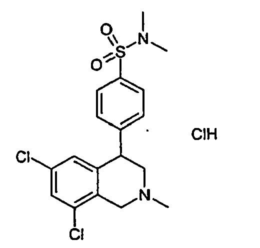

Chlorsulfonsäure (6,6 ml) wurde vorgelegt und portionsweise 6,8-Dichloro-2-methyl-4-phenyl-1,2,3,4-tetrahydro-isochinolin (Zwischenprodukt 4, Beispiel 2) eingetragen.
Anschließend wurde eine Stunde bei 40°C gerührt. Dann wurde das Reaktionsgemisch auf Raumtemperatur abgekühlt und mit einem Eis-Wasser-Gemisch versetzt. Der dabei ausgefallene Niederschlag wurde abgesaugt und in Essigester aufgenommen, der nach dem Waschen mit gesättigter Kochsalzlösung über Magnesiumsulfat getrocknet wurde. Das anschließende Einengen lieferte 4-(6,8-Dichlor-2-methyl-1,2,3,4-tetrahydro-isochinolin-4-yl)-benzolsulfonyl-chlorid als festes Rohprodukt, von dem ein Teil (150 mg) direkt in auf 10°C gekühlte Dimethylamin-Lösung (5 ml, ca. 40% in Wasser) portionsweise eingetragen wurde. Die entstandene Suspension wurde anschließend bei dieser Temperatur 1,5 h gerührt. Dann wurde mit Eiswasser versetzt, dreimal mit Essigester extrahiert, die vereinigten Essigesterphasen mit gesättigter Kochsalzlösung gewaschen und über Magnesiumsulfat getrocknet. Der Rückstand wurde in Wasser aufgenommen, mit 2 N HCl versetzt und gefriergetrocknet. Das so erhaltene Rohprodukt wurde dann mittels präparativer HPLC aufgereinigt.

### Bedingungen:

stationäre Phase: Merck Purospher RP18 (10µM) 250 x 25 mm
mobile Phase: 90% H₂O (0,05% TFA)→ 90% Acetonitril; 40 min; Fluss: 25 ml/min

Die das Produkt enthaltenden Fraktionen wurden vereinigt, das Acetonitril am Rotationsverdampfer abgezogen, die wässrige Phase mit gesättigter Kaliumcarbonat-Lösung gewaschen und dann dreimal mit Essigester extrahiert. Die vereinigten Essigesterphasen wurden mit gesättigter Kochsalzlösung gewaschen, über Magnesiumsulfat getrocknet und eingeengt. Der Rückstand wurde in Wasser aufgenommen, mit 2 N HCl versetzt und gefriergetrocknet. Es wurden 80 mg eines hellen Feststoffs erhalten. Dieser bestand zu -80% aus der gewünschten Verbindung, neben -20% eines Regioisomeren (Rₜ = 4,000 min (Methode A); MS(Cl⁺) = 399,1).

### Beispiel 4:

### 4a: 4-(6,8-Dichloro-2-methyl-1,2,3,4-tetrahydro-isochinolin-4-yl)-benzol-sulfonamid, Hydrochlorid;

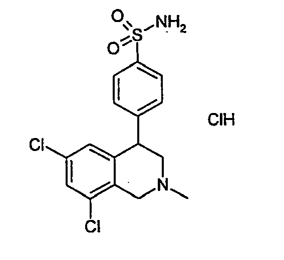

### Zwischenprodukt 1: 4-(6,8-Dichloro-2-methyl-1,2,3,4-tetrahydroisochinolin-4-yl)-benzolsulfonylchlorid

Bei 0 °C wird 1 mmol 6,8-Dichloro-2-methyl-4-phenyl-1,2,3,4-tetrahydro-isochinolin (Zwischenprodukt 4, Beispiel 2) in 1 ml Chlorsulfonsäure eingetragen und 3 Stunden bei Raumtemperatur gerührt. Zur Aufarbeitung gibt man das Reaktionsgemisch auf Eis, stellt mit 1 N NaOH auf einen pH von 7 bis 8 und extrahiert zweimal mit Essigester. Die vereinigten Essigesterphasen werden mit Na₂SO₄ getrocknet und einrotiert. Das so erhaltene Rohprodukt wird ohne weitere Reinigung weiter umgesetzt.

### Zwischenprodukt 2: 4-(6,8-Dichloro-2-methyl-1,2,3,4-tetrahydro-isochinolin-4-yl)-benzol-sulfonamid,

319 mg des Zwischenprodukts 1 werden in 6 ml 25% igem Ammoniak suspendiert und auf 90 °C erhitzt. Nach 3 h wird mit H₂O verdünnt und mit Essigester extrahiert. Die organische Phase wird abgetrennt und mit Na₂SO₄ getrocknet, wobei 165 mg der Titelverbindung erhalten werden.

### 4-(6,8-Dichloro-2-methyl-1,2,3,4-tetrahydro-isochinolin-4-yl)-benzol-sulfonamid, Hydrochlorid;

145 mg des Zwischenprodukts 2 werden in 15 ml Diethylether suspendiert und mit 1 ml etherischer HCl versetzt. Nachdem 30 Minuten bei Raumtemperatur gerührt wurde, wird der Niederschlag abgesaugt und getrocknet, wobei 136 mg des Hydrochlorids in Form eines gelblichen Feststoffs erhalten werden.

### 4b: 4-(6,8-Dichloro-2-methyl-1,2,3,4-tetrahydro-isochinolin-4-yl)-benzol-sulfonamid, Acetat;

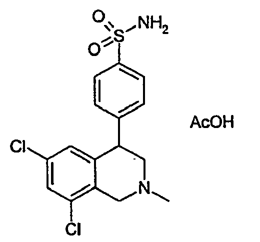

255 mg des Zwischenprodukts 2, Beispiel 8 werden mit 5 ml Eisessig versetzt und 50 ml H₂O zugegeben. Nach Filtration von schwer löslichen Bestandteilen wird gefriergetrocknet, wobei 250 mg der Titelverbindung erhalten werden.

### Beispiel 5: 4-(4-Bromo-phenyl)-6,8-dichloro-2-methyl-1,2,3,4-tetrahydroisochinolin, Hydrochlorid;

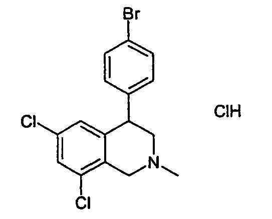

### Zwischenprodukt 1:

### 1-(4-Bromo-phenyl)-2-[(2,4-dichloro-benzyl)-methyl-amino]-ethanon;

(2,4-Dichloro-benzyl)-methyl-amin (s. Beispiel 1, Zwischenprodukt 1) und 2-Bromo-1-(4-bromo-phenyl)-ethanon werden analog der in Beispiel 1, Zwischenprodukt 3 beschriebenen Methode zur Reaktion gebracht. Nach analoger Aufarbeitung und Chromatographie an Kieselgel kann das Alkylierungsprodukt in 69 %iger Ausbeute isoliert werden.

### Zwischenprodukt 2: 1-(4-Bromo-phenyl)-2-[(2,4-dichloro-benzyl)-methyl-amino]-ethanol;

Zwischenprodukt 1 wird mit 2 Äquivalenten NaBH₄, analog zu der in Zwischenprodukt 4, Beispiel 1 beschriebenen Weise zu dem entsprechenden Alkohol reduziert, der in einer Ausbeute von 86 % isoliert werden kann.

### Zwischenprodukt 3: 4-(4-Bromo-phenyl)-6,8-dichloro-2-methyl-1,2,3,4-tetrahydroisochinolin;

5,45 g (14,0 mmol) des 1-(4-Bromo-phenyl)-2-[(2,4-dichloro-benzyl)-methyl-amino]-ethanol werden in 15 ml Dichlormethan vorgelegt und bei 0 °C mit 15 ml konz. H₂SO₄ versetzt. Nach 2 Stunden Rühren bei Raumtemperatur wird das Reaktionsgemisch auf Eis gegeben und mit 6 N NaOH alkalisch gestellt. Es wird dreimal mit Dichlormethan extrahiert. Die vereinigten organischen Phasen werden mit MgSO₄ getrocknet und eingeengt. Zur weiteren Reinigung wird der Rückstand an Kieselgel chromatographiert, wobei 2,6 g der Titelverbindung als gelbliches Öl erhalten werden.

### 4-(4-Bromo-phenyl)-6,8-dichloro-2-methyl-1,2,3,4-tetrahydroisochinolin, Hydrochlorid;

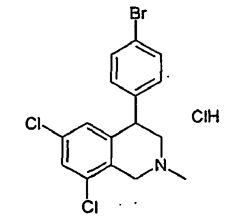

300 mg 4-(4-Bromo-phenyl)-6,8-dichloro-2-methyl-1,2,3,4-tetrahydroisochinolin werden in 2 N HCl bei Raumtemperatur gerührt. Der entstandene Niederschlag wird abgesaugt und getrocknet.

### Beispiel 6: 4-(6,8-Dichloro-2-methyl-1,2,3,4-tetrahydro-isochinolin-4-yl)-benzoesäure;

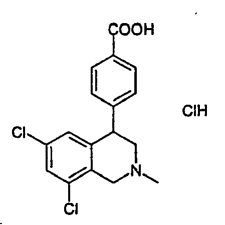

### 4-(6,8-Dichloro-2-methyl-1,2,3,4-tetrahydro-isochinolin-4-yl)-benzoesäure;

5,57 g (15 mmol) des 4-(4-Bromo-phenyl)-6,8-dichloro-2-methyl-1,2,3,4-tetrahydroisochinolin (Beispiel 5, Zwischenprodukt 3) werden in 150 ml abs. DMF/Benzol (1:1) gelöst. Nach dem Entgasen der Lösung werden unter Argon 1,18 g (4,5 mmol) Triphenylphosphin und 1,17 g (9 mmol) Ca(HCO₂)₂ zugegeben. Nach erneutem Spülen mit Argon werden 867 mg (0,75 mmol) Pd(PPh₃)₄ zugegeben und Kohlenmonoxid in die Lösung eingeleitet. Es wird bei 120 °C gerührt. Nach sechs Stunden bei 120 °C und Stehen über Nacht unter Argon wurden nochmals 867 mg (0,75 mmol) Pd(PPh₃)₄ zugegeben und weitere acht Stunden bei 120 °C gerührt und Kohlenmonoxid in die Lösung eingeleitet. Nach erneutem Stehen über Nacht wurden 135 mg PdCl₂ zugegeben und unter gleichen Bedingungen reagieren lassen. Zur Aufarbeitung wird das Lösungsmittel i. Vak. entfernt und der Rückstand in Essigester aufgenommen. Es wird dreimal mit 2 N NaOH extrahiert. Die vereinigten wässrigen Phasen werden mit 6 N HCl auf einen pH-Wert von 6 gestellt und dreimal mit Essigester extrahiert. Die organischen Phasen werden mit MgSO₄ getrocknet und vom Lösungsmittel befreit. Der Rückstand wird an Kieselgel mit einem Dichlormethan/Methanol- Gemisch gereinigt, wobei 420 mg der Titelverbindung erhalten werden (Rₜ = 4,025 min (Methode A); MS(Cl⁺) = 336,1/338,1).

### Beispiel 7: 4-(6,8-Dichloro-2-methyl-1,2,3,4-tetrahydroisochinolin-4-yl)-N-ethyl-benzamid, Trifluoracetat;

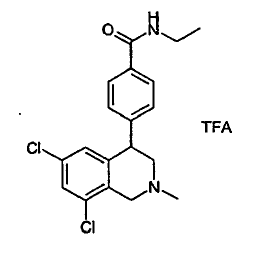

146 mg (0,43 mmol) 4-(6,8-Dichloro-2-methyl-1,2,3,4-tetrahydro-isochinolin-4-yl)-benzoesäure (s. Beispiel 6) werden in 5 ml DMF gelöst und mit 1,0 Äquivalent Triethylamin versetzt. Bei 0 °C wird eine Lösung aus 141 mg (0,43 mmol) TOTU in 3 ml DMF zugegeben. Es wird 30 min bei 0 °C, sowie 30 min bei Raumtemperatur gerührt. Diese Lösung wird dann bei 0 °C zu einer Lösung aus 0,28 ml 2 M Ethylamin-Lösung und 0,06 ml (0,043 mmol) Triethylamin in 5 ml DMF gegeben und das Reaktionsgemisch drei Stunden bei Raumtemperatur gerührt. Zur Aufarbeitung wird das Lösungsmittel i. Vak. abdestilliert, der Rückstand in Essigester aufgenommen und zweimal mit 1 N KOH, sowie einmal mit H₂O gewaschen. Die organische Phase wird mit Na₂SO₄ getrocknet und eingeengt. Zur weiteren Reinigung wird an Kieselgel chromatographiert (Dichlormethan/Methanol 95:5). Eine weitere Reinigung an einer präp. HPLC (Acetonitril/H₂O/Trifluoressigsäure) liefert das gewünschte Carbonsäureamid als Trifluoracetat. (Rt = 4,169 min (Methode A); MS(Cl⁺) = 363,3/365,3).

### Beispiel 8: 4-(6,8-Dichloro-2-methyl-1,2,3,4-tetrahydroisochinolin-4-yl)-N-propyl-benzamid, Trifluoracetat;

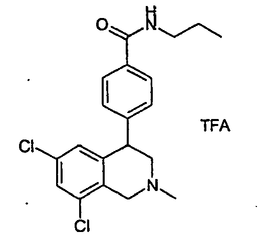

Ausgehend von n-Propylamin und 4-(6,8-Dichloro-2-methyl-1,2,3,4-tetrahydroisochinolin-4-yl)-benzoesäure (s. Beispiel 6) kann die Titelverbindung nach der in Beispiel 7 beschriebenen Methode hergestellt werden.
(Rt = 1,881 min (Methode B); MS(Cl⁺) = 377,3/379,3).

### Beispiel 9: 4-(6,8-Dichloro-2-methyl-1,2,3,4-tetrahydro-isochinolin-4-yl)-N-(2-dimethylamino-ethyl)-benzamid, Trifluoracetat;

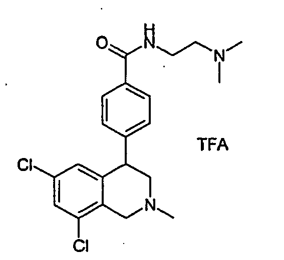

wurde analog Beispiel 7 ausgehend von Beispiel 6 und N1,N1-Dimethylethan-1,2-diamin durch eine TOTU vermittelte Kupplungsreaktion hergestellt (Rt = 1,449 min (Methode B); MS(Cl⁺) = 406,3/408,3).

### Beispiel 10: 6,8-Dichloro-2-methyl-4-(4-morpholin-4-yl-phenyl)-1,2,3,4-tetrahydroisochinolin, Trifluoracetat;

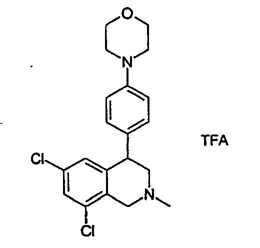

456 mg (1,4 mmol) Cs₂CO₃, 6,75 mg (0,03 mmol) Palladiumacetat, sowie 28 mg (0,045 mmol) 2,2-Bis-(diphenylphosphino)-1,1 binaphthyl werden in 5 ml abs. Toluol vorgelegt. Unter Argon wird eine Lösung aus 0,104 ml (1,2 mmol) Morpholin in 2,5 ml abs. DMF, sowie ein Lösung aus 371 mg (1,0 mmol) 4-(4-Bromo-phenyl)-6,8-dichloro-2-methyl-1,2,3,4-tetrahydroisochinolin in 2,5 ml abs. Toluol zugegeben und insgesamt 9 Stunden bei 100 °C gerührt. Zur Aufarbeitung wird vom Lösungsmittel befreit, der Rückstand in Dichlormethan aufgenommen und von unlöslichen Bestandteilen abfiltriert. Nach Einengen des Filtrats wird der Rückstand an Kieselgel chromatographiert (CH₂Cl₂ / Methanol 95:5), wobei 350 mg des gewünschten Morpholinderivats erhalten werden. Nach einer weiteren Reinigung an einer präp. HPLC können 160 mg des entsprechenden Trifluoracetats in Form eines farblosen Feststoffs isoliert werden.

### Beispiel 11: [4-(6,8-Dichloro-2-methyl-1,2,3,4-tetrahydroisochinolin-4-yl)-phenyl]-diethyl-amin, Trifluoracetat;

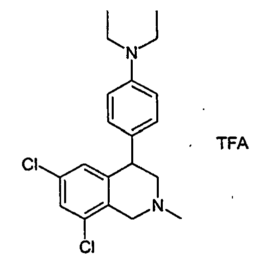

Ausgehend von Diethylamin und 4-(4-Bromo-phenyl)-6,8-dichloro-2-methyl-1,2,3,4-tetrahydroisochinolin (Beispiel 5, Zwischenprodukt 3) wird analog nach der in Beispiel 10 beschriebenen Methode verfahren. Reaktionsdauer: 2 Tage bei 100 °C; dreifache Menge an Pd-Katalysator und Phosphinligand. Nach präp. HPLC kann das gewünschte Trifluoracetat als farbloser Feststoff isoliert werden.

### Beispiel 12: 6,8-Dichloro-2-methyl-4-(4-piperidin-1-yl-phenyl)-1,2,3,4-tetrahydroisochinolin, Trifluoracetat;

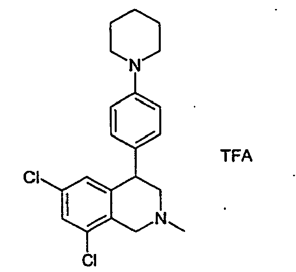

Analog zu der in Beispiel 10 beschriebenen Methode kann das gewünschte Piperidinderivat ausgehend von Piperidin und 4-(4-Bromo-phenyl)-6,8-dichloro-2-methyl-1,2,3,4-tetrahydroisochinolin (Beispiel 5, Zwischenprodukt 3) erhalten werden.

### Beispiel 13: 6,8-Dichloro-2-methyl-4-(4-pyrrolidin-1-yl-phenyl)-1,2,3,4-tetrahydroisochinolin, Hydrochlorid;

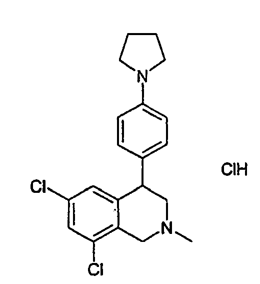

Die Reaktionsführung geschieht in Analogie zu der in Beispiel 10 beschriebenen Methode, ausgehend von Pyrrolidin und 4-(4-Bromo-phenyl)-6,8-dichloro-2-methyl-1,2,3,4-tetrahydroisochinolin (Beispiel 5, Zwischenprodukt 3). Nach chromatographischer Reinigung wird das erhaltene Produkt in einem DMSO/Acetonitril-Gemisch aufgenommen, wobei ein Niederschlag ausfällt. Dieser wird abfiltriert in 2 N HCl gelöst und gefriergetrocknet, wobei die Titelverbindung als farbloser Feststoff erhalten wird.

### Beispiel 14: 6,8-Dichloro-2-methyl-4-[4-(4-methyl-piperazin-1-yl)-phenyl]-1,2,3,4-tetrahydro- isochinolin, Trifluoracetat;

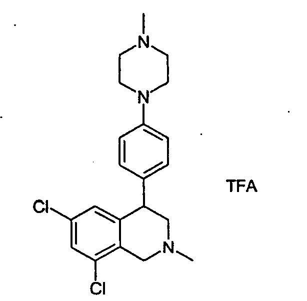

Umsetzung von N-Methyl-piperazin und 4-(4-Bromo-phenyl)-6,8-dichloro-2-methyl-1,2,3,4-tetrahydroisochinolin (Beispiel 5, Zwischenprodukt 3) nach der in Beispiel 10 beschriebenen Methode liefert die Titelverbindung in Form eines farblosen Feststoffs.

### Beispiel 15: 6,8-Dichloro-2-cyclopropyl-4-phenyl-1,2,3,4-tetrahydroisochinolin, Trifluoracetat;

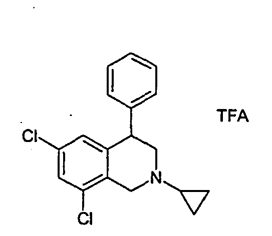

### Zwischenprodukt 1:

### Cyclopropyl-(2,4-dichloro-benzyl)-amin;

5,25 g (30 mmol) 2,4-Dichlorbenzaldehyd werden in 140 ml Methanol vorgelegt und bei Raumtemperatur eine Lösung aus 1,71 g (30 mmol) Cyclopropylamin zugegeben. Es wird 40 min bei Raumtemperatur gerührt und im Anschluss portionsweise mit 1,42 g (37,5 mmol) NaBH₄ versetzt. Nach Stehen über Nacht wird vom Lösungsmittel befreit und der Rückstand in 2 N HCl aufgenommen. Es wird zweimal mit Essigester extrahiert. Die wässrige Phase wird mit NaOH alkalisch gestellt und wiederum zweimal mit Essigester extrahiert. Die organischen Phasen werden mit MgSO₄ getrocknet und eingeengt. Das so erhaltene Rohprodukt in Form eines leicht gelblichen Öls kann ohne weitere Reinigung weiter umgesetzt werden.

Zwischenprodukt 2: 2-[Cyclopropyl-(2,4-dichloro-benzyl)-amino]-1-phenyl-ethanon; Zwischenprodukt 1 wird in Gegenwart von Triethylamin in Dioxan mit alpha-Bromaceto-phenon nach der in Beispiel 1, Zwischenprodukt 3 beschriebenen Methode zur Reaktion gebracht. Zur Aufarbeitung wird das Lösungsmittel abdestilliert und der Rückstand in Essigester aufgenommen. Es wird zweimal mit H₂O und zweimal mit 2 N HCl gewaschen, mit MgSO₄ getrocknet und eingeengt. Das so erhaltene Rohprodukt kann ohne weitere Reinigung weiter ungesetzt werden.

Zwischenprodukt 3: 2-[Cyclopropyl-(2,4-dichloro-benzyl)-amino]-1-phenyl-ethanol; Zwischenprodukt 2 wird analog zu der in Beispiel 1, Zwischenprodukt 4 beschriebenen Methode mit NaBH₄ reduziert. Zur Aufarbeitung wird eingeengt, und der Rückstand zwischen 1 N HCl und Essigester verteilt. Die wässrige Phase wird abgetrennt und noch einmal mit Essigester extrahiert. Die vereinigten organischen Phasen werden mit MgSO₄ getrocknet und das Lösungsmittel i. Vak. entfernt.

6,8-Dichloro-2-cyclopropyl-4-phenyl-1,2,3,4-tetrahydroisochinolin, Trifluoracetat; Zwischenprodukt 3 (1,9 g) wird ohne weitere Reinigung in 10 ml Dichlormethan gelöst und nach der in Beispiel 1 beschriebenen Methode mit konz. H₂SO₄ cyclisiert. Zur Aufarbeitung wird die Reaktionsmischung auf Eis gegeben. Die organische Phase wird abgetrennt und die wässrige noch einmal mit Dichlormethan extrahiert. Die vereinigten organischen Phasen werden mit MgSO₄ getrocknet und vom Lösungsmittel befreit. Chromatographie an Kieselgel (n-Heptan/Essigester 5:1 → 3:1) liefert 200 mg eines gelblichen Öls das einer weiteren Reinigung an einer präp. HPLC unterworfen wird. Hierbei werden 184 mg der Titelverbindung als Trifluoracetat erhalten.

### Beispiel 16: 16a: (-)-N-[4-(6,8-Dichloro-2-methyl-1,2,3,4-tetrahydro-isochinolin-4-yl)-phenyl]-acetamid;

### 16b: (+)-N-[4-(6,8-Dichloro-2-methyl-1,2,3,4-tetrahydro-isochinolin-4-yl)-phenyl]-acetamid;

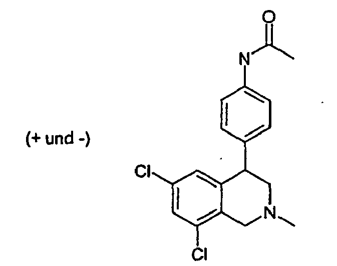

500 mg der Titelverbindung aus Beispiel 1 werden an einer chiralen Phase getrennt,
wobei ca. 250 mg der beiden enantiomeren Acetamide 16a und 16b erhalten werden. chirale Säule: Chiralpak OD 250 x 4,6 mm;
Lösungsmittel: Acetonitril;
Flussrate: 1 ml/min;
Rₜ((-)-Enantiomer/16a) = 5,856 min;
Rₜ((+)-Enantiomer/16b) = 8,613 min.

### Beispiel 17: 4-(6,8-Dichloro-2-methyl-1,2,3,4-tetrahydro-isochinolin-4-yl)-phenylamin, Hydrochlorid;

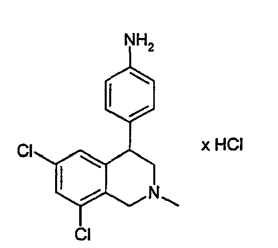

### Zwischenprodukt 1:

### 4-(6,8-Dichloro-2-methyl-1,2,3,4-tetrahydro-isochinolin-4-yl)-phenylamin;

3,0 g (8,6 mmol) N-[4-(6,8-Dichloro-2-methyl-1,2,3,4-tetrahydro-isochinolin-4-yl)-phenyl]-acetamid (Beispiel 1) werden in 100 ml 20%iger Natriumethanolat-Lösung gelöst und vier Stunden zum Rückfluss erhitzt. Es werden weitere 2,0 g (29,4 mmol) festes Natriumethanolat zugegeben und noch drei Stunden unter Rückfluss erhitzt. Zur Aufarbeitung wird das Lösungsmittel i. Vak. entfernt, der Rückstand in 200 ml H₂O aufgenommen und zweimal mit Dichlormethan extrahiert. Die vereinigten organischen Phasen werden mit MgSO₄ getrocknet und eingeengt. Zur weiteren Reinigung erfolgt eine Chromatographie an Kieselgel (Essigester/Heptan 1:1), wobei das Anilin in quantitativer Ausbeute als gelbliches Öl erhalten wird.

4-(6,8-Dichloro-2-methyl-1,2,3,4-tetrahydro-isochinolin-4-yl)-phenylamin, Hydrochlorid; 200 mg (0,65 mmol) 4-(6,8-Dichloro-2-methyl-1,2,3,4-tetrahydro-isochinolin-4-yl)-phenylamin werden in 30 ml ethanolischer HCl gelöst. Die klare Lösung wird i. Vak. eingeengt. Der Rückstand wird in Ether verrieben, abgesaugt und getrocknet, wobei 208 mg des gewünschten Hydrochlorids isoliert werden können.

### Beispiel 18: N-Ethyl-N'-4-(6,8-Dichloro-2-methyl-1,2,3,4-tetrahydro-isochinolin-4-yl)-benzolsulfonyl-harnstoff, Hydrochlorid

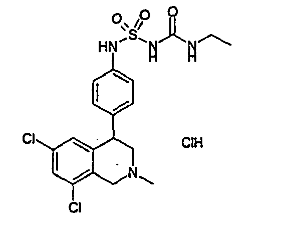

1,0 mmol 4-(6,8-Dichloro-2-methyl-1,2,3,4-tetrahydro-isochinolin-4-yl)-benzolsulfonamid (Beispiel 4, Zwischenprodukt 2) wird in 15 ml trockenem Aceton mit 350 mg (2,5 eq.) K₂CO₃ versetzt und 1,5 Stunden bei Raumtemperatur gerührt. Bei Raumtemperatur wird eine Lösung aus 2,5 eq Ethylisocyanat in Aceton zugetropft und die Lösung zum Rückfluss erhitzt. Zur Aufarbeitung wird i. Vak. eingeengt, der Rückstand in H₂O aufgenommen und zweimal mit Essigester extrahiert. Die wässrige Phase wird mit 6 N HCl angesäuert und der entstanden Niederschlag abgesaugt. Waschen mit Essigester und Trocknen i. Vak. liefert die Titelverbindung in guter Ausbeute.

### Beispiel 19: 1-[4-(6,8-Dichloro-2-methyl-1,2,3,4-tetrahydro-isochinolin-4-yl)-phenyl]-3-propyl-harnstoff;

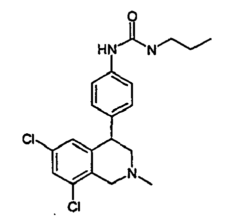

Zu einer Lösung aus 500 mg (1,63 mmol) 4-(6,8-Dichloro-2-methyl-1,2,3,4-tetrahydroisochinolin-4-yl)-phenylamin (s. Beispiel 17) in 15 ml Toluol wird unter Rühren eine Lösung aus 0,17 g (2,0 mmol) n-Propylisocyanat in Toluol zugetropft. Nach einer Stunde bei 40 °C werden weitere 0,17 g n-Propylisocyanat zugegeben und eine Stunde bei 80 °C gerührt. Zur Aufarbeitung wird vom Lösungsmittel befreit und der Rückstand mit H₂O und Ether verrieben. Trocknen liefert 503 mg des gewünschten n-Propyl-Harnstoffs.

### 19a: 1-[4-(6,8-Dichloro-2-methyl-1,2,3,4-tetrahydro-isochinolin-4-yl)-phenyl]-3-propyl-harnstoff, Hydrochlorid;

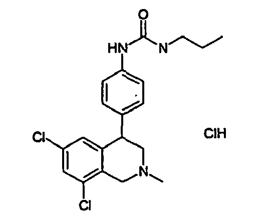

450 mg 1-[4-(6,8-Dichloro-2-methyl-1,2,3,4-tetrahydro-isochinolin-4-yl)-phenyl]-3-propyl-harnstoff werden in einem Gemisch aus 2 N HCl und THF gelöst. Die klare Lösung wird i. Vak. eingeengt und der Rückstand mit Ether verrieben und abgesaugt. Trocknen liefert 473 mg des gewünschten Hydrochlorids.

### Beispiel 20: 1-[4-(6,8-Dichloro-2-methyl-1,2,3,4-tetrahydro-isochinolin-4-yl)-phenyl]-3-methyl- thioharnstoff;

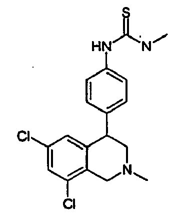

Ausgehend von 500 mg (1,63 mmol) 4-(6,8-Dichloro-2-methyl-1,2,3,4-tetrahydroisochinolin-4-yl)-phenylamin (s. Beispiel 17) und 220 mg (3,0 mmol) Methylisothiocyanat wird analog der in Beispiel 19 beschriebenen Methode verfahren,
wobei 245 mg des gewünschten Thioharnstoffs isoliert werden können.

### Beispiel 21: 1-[4-(6,8-Dichloro-2-methyl-1,2,3,4-tetrahydro-isochinolin-4-yl)-phenyl]-3-ethyl- harnstoff;

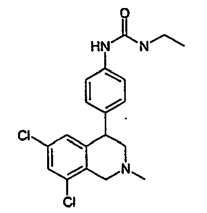

Die Herstellung erfolgt analog zu der in Beispiel 19 beschriebenen Methode, ausgehend von 4-(6,8-Dichloro-2-methyl-1,2,3,4-tetrahydro-isochinolin-4-yl)-phenylamin (500 mg; 1,63 mmol) und Ethylisocyanat (284 mg/ 4 mmol).

### 21 a: 1-[4-(6,8-Dichloro-2-methyl-1,2,3,4-tetrahydro-isochinolin-4-yl)-phenyl]-3-ethyl-harnstoff, Hydrochlorid;

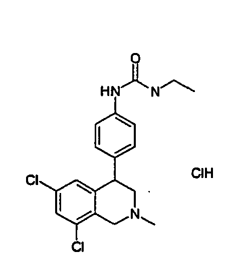

Die Überführung in das entsprechende Hydrochlorid erfolgt analog zu der in Beispiel 19a beschriebenen Methode.

### Beispiel 22: N-[4-(6-Methanesulfonyl-2-methyl-1,2,3,4-tetrahydro-isochinolin-4-yl)-phenyl]- acetamid;

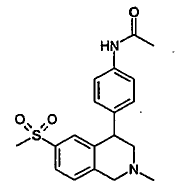

Zwischenprodukt 1: (4-Methanesulfonyl-benzyl)-methyl-amin wird ausgehend von 1-Bromomethyl-4-methanesulfonyl-benzol und Methylamin in dem Fachmann bekannter Weise synthetisiert.

Die Herstellung der Titelverbindung geschieht analog zu der in Beispiel 1 angegebenen Syntheseroute ausgehend von (4-Methanesulfonyl-benzyl)-methyl-amin (Zwischenprodukt 1) und N-[4-(2-Bromo-acetyl)-phenyl]-acetamid (Beispiel 1, Zwischenprodukt 2).

### Beispiel 23: N-[4-(2,6,8-Trimethyl-1,2,3,4-tetrahydro-isochinolin-4-yl)-phenyl]-acetamid;

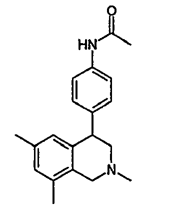

Ausgehend von (2,4-Dimethyl-benzyl)-methyl-amin, welches aus 1-Bromomethyl-2,4-dimethyl-benzol und Methylamin in dem Fachmann bekannter,Weise hergestellt werden kann, und N-[4-(2-Bromo-acetyl)-phenyl]-acetamid (Beispiel 1, Zwischenprodukt 2) wird nach der in Beispiel 1 angeführten Syntheseroute verfahren.

### Beispiel 24: N-[4-(6-Bromo-8-chloro-2-methyl-1,2,3,4-tetrahydro-isochinolin-4-yl)-phenyl]- acetamid;

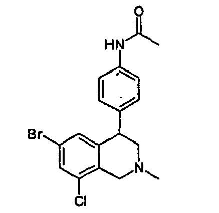

Ausgehend von (4-Bromo-2-chloro-benzyl)-methyl-amin, welches aus 4-Bromo-1-bromomethyl-2-chloro-benzol und Methylamin in dem Fachmann bekannter Weise hergestellt werden kann, und N-[4-(2-Bromo-acetyl)-phenyl]-acetamid (Beispiel 1, Zwischenprodukt 2) wird nach der in Beispiel 1 angeführten Syntheseroute verfahren.

### Beispiel 25: N-[4-(8-Chloro-2-methyl-6-pyrrolidin-1-yl-1,2,3,4-tetrahydro-isochinolin-4-yl)-phenyl]- acetamid;

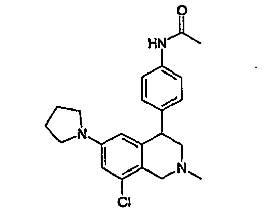

1,02 g (3,12 mmol) Cs₂CO₃, 8,8 mg (0,04 mmol) Palladiumacetat sowie 36,1 mg (0,06 mmol) 2,2-Bis-diphenylphosphino-1,1-binaphthyl werden unter Argon in 6,5 ml abs. Toluol vorgelegt. Bei Raumtemperatur wird eine Lösung aus 512 mg (1,3 mmol) N-[4-(6-Bromo-8-chloro-2-methyl-1,2,3,4-tetrahydro-isochinolin-4-yl)-phenyl]- acetamid (Beispiel 24) in 4 ml abs. DMF, sowie eine Lösung aus 111 mg (1,56 mmol) Pyrrolidin in 4 ml DMF zugegeben und 7 Stunden auf 100 °C erhitzt. Zur Aufarbeitung wird das Lösungsmittel i. Vak. entfernt und der Rückstand in Dichlormethan aufgenommen. Es wird von unlöslichen Bestandteilen abfiltriert und das Filtrat eingeengt. Der Rückstand wird an Kieselgel mit einem Dichlormethan/Methanol-Gemisch chromatographiert, wobei 360 mg der Beispielverbindung isoliert werden können.

### 25a: N-[4-(8-Chloro-2-methyl-6-pyrrolidin-1-yl-1,2,3,4-tetrahydro-isochinolin-4-yl)-phenyl]-acetamid, Hydrochlorid;

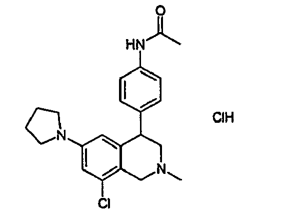

320 mg N-[4-(8-Chloro-2-methyl-6-pyrrolidin-1-yl-1,2,3,4-tetrahydro-isochinolin-4-yl)-phenyl]-acetamid werden in 20 ml ethanolischer HCl gelöst, 30 min bei Raumtemperatur gerührt und eingeengt. Der Rückstand wird in H₂O aufgenommen und gefriergetrocknet.

### Beispiel 26: N-[4-(8-Chloro-2-methyl-6-morpholin-4-yl-1,2,3,4-tetrahydro-isochinolin-4-yl)-phenyl]-acetamid, Trifluoracetat;

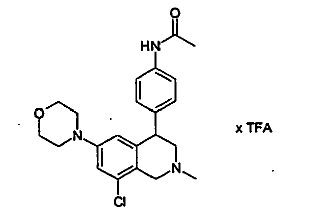

Der Herstellung erfolgt analog zu der in Beispiel 25 beschriebenen Methode ausgehend von N-[4-(6-Bromo-8-chloro-2-methyl-1,2,3,4-tetrahydro-isochinolin-4-yl)-phenyl]-acetamid (Beispiel 24) und Morpholin. An die chromatographische Trennung an Kieselgel schloss sich eine weitere Reinigung an einer präp. HPLC an.

### Beispiel 27: N-{4-[8-Chloro-2-methyl-6-(4-methyl-piperazin-1-yl)-1,2,3,4-tetrahydroisochinolin-4- yl]-phenyl}-acetamid;

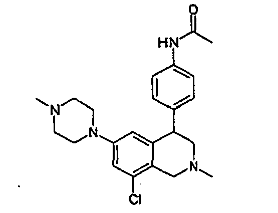

Der Herstellung erfolgt analog zu der in Beispiel 25 beschriebenen Methode ausgehend von N-[4-(6-Bromo-8-chloro-2-methyl-1,2,3,4-tetrahydro-isochinolin-4-yl)-phenyl]-acetamid (Beispiel 24) und N-Methyl-piperazin.

### 27a: N-{4-[8-Chloro-2-methyl-6-(4-methyl-piperazin-1-yl)-1,2,3,4-tetrahydro-isochinolin-4- yl]-phenyl}-acetamid, Hydrochlorid;

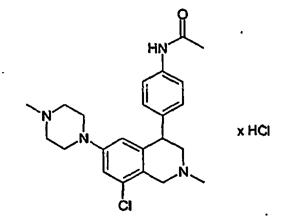

220 mg N-{4-[8-Chloro-2-methyl-6-(4-methyl-piperazin-1-yl)-1,2,3,4-tetrahydroisochinolin-4- yl]-phenyl}-acetamid werden in wenig Methanol gelöst, mit 2 N HCl verdünnt und gefriergetrocknet, wobei 226 mg des gewünschten Hydrochlorids erhalten wurden.

### Beispiel 28: N-{4-[8-Chloro-6-(cyclopropylmethyl-amino)-2-methyl-1,2,3,4-tetrahydroisochinolin-4- yl]-phenyl}-acetamid, Hydrochlorid;

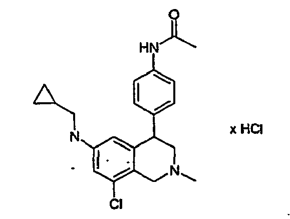

Der Herstellung erfolgt analog zu der in Beispiel 25 beschriebenen Methode ausgehend von N-[4-(6-Bromo-8-chloro-2-methyl-1,2,3,4-tetrahydro-isochinolin-4-yl)-phenyl]- acetamid (Beispiel 24) und C-Cyclopropyl-methylamin. An die chromatographische Trennung an Kieselgel schloss sich eine weitere Reinigung an einer präp. HPLC an. Die gereinigte Verbindung wurde in 1 N HCl gelöst, mit H₂O verdünnt und gefriergetrocknet.

### Beispiel 29: 5-(6,8-Dichloro-2-methyl-1,2,3,4-tetrahydro-isochinolin-4-yl)-2-hydroxybenzoesäure;

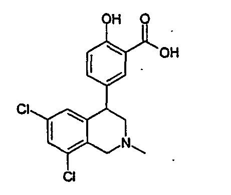

Zwischenprodukt 1: 5-Acetyl-2-hydroxy-benzoesäure-ethylester wird in dem Fachmann bekannter Weise aus 5-Acetyl-2-hydroxy-benzoesäure durch säurekatalysierte Veresterung hergestellt.

Zwischenprodukt 2: 5-(2-Bromo-acetyl)-2-hydroxy-benzoesäure-ethylester wird nach bekannten Methoden aus 5-Acetyl-2-hydroxy-benzoesäure-ethylester analog dem in Beispiel 1, Zwischenprodukt 2 beschriebenen Verfahren hergestellt.

### Zwischenprodukt 3: 5-(6,8-Dichloro-2-methyl-1,2,3,4-tetrahydro-isochinolin-4-yl)-2-hydroxy-benzoesäure-ethylester

Ausgehend von 5-(2-Bromo-acetyl)-2-hydroxy-benzoesäure-ethylester und 2,4-Dichlorbenzyl-methyl-amin (s. Beispiel 1, Zwischenprodukt 1) wird die Titelverbindung nach der in Beispiel 1 dargelegten Syntheseroute synthetisiert.

### 5-(6,8-Dichloro-2-methyl-1,2,3,4-tetrahydro-isochinolin-4-yl)-2-hydroxy-benzoesäure;

6,8 g (18 mmol) 5-(6,8-Dichloro-2-methyl-1,2,3,4-tetrahydro-isochinolin-4-yl)-2-hydroxy-benzoesäure-ethylester werden in dem Fachmann bekannter Weise in einem Ethanol / 2 N KOH-Gemisch verseift, wobei 5,4 g der freien Säure erhalten werden.

### 29a: 5-(6,8-Dichloro-2-methyl-1,2,3,4-tetrahydro-isochinolin-4-yl)-2-hydroxybenzoesäure, Natriumsalz;

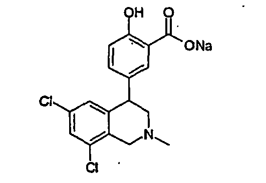

352 mg (1 mmol) der freien Säure 5-(6,8-Dichloro-2-methyl-1,2,3,4-tetrahydroisochinolin-4-yl)-2-hydroxy-benzoesäure werden in 10 ml 0,1 M NaOH gelöst, mit H₂O verdünnt und gefriergetrocknet, wobei 375 mg der Titelverbindung erhalten werden.

### Beispiel 30: 5-(6,8-Dichloro-2-methyl-1,2,3,4-tetrahydro-isochinolin-4-yl)-2-hydroxy-N-methyl- benzamid, Trifluoracetat;

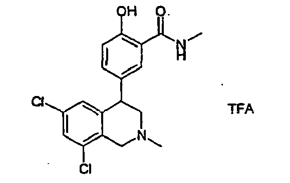

Ausgehend von 5-(6,8-Dichloro-2-methyl-1,2,3,4-tetrahydro-isochinolin-4-yl)-2-hydroxy-benzoesäure kann die Titelverbindung in einer TOTU-vermittelten Reaktion mit Methylamin analog der in Beispiel 7 beschriebenen Methode hergestellt werden.

### Beispiel 31: 5-(6,8-Dichloro-2-methyl-1,2,3,4-tetrahydro-isochinolin-4-yl)-N-ethyl-2-hydroxy- benzamid, Trifluoracetat;

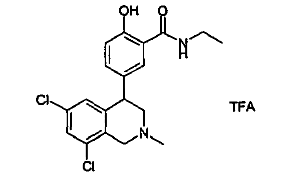

Ausgehend von 5-(6,8-Dichloro-2-methyl-1,2,3,4-tetrahydro-isochinolin-4-yl)-2-hydroxy-benzoesäure kann die Titelverbindung in einer TOTU-vermittelten Reaktion mit Ethylamin analog der in Beispiel 7 beschriebenen Methode hergestellt werden.

### Beispiel 32: 5-(6,8-Dichloro-2-methyl-1,2,3,4-tetrahydro-isochinolin-4-yl)-N-(2-dimethylamino-ethyl)- 2-hydroxy-benzamid, Trifluoracetat;

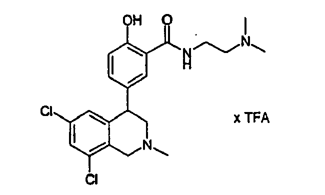

Ausgehend von 5-(6,8-Dichloro-2-methyl-1,2,3,4-tetrahydro-isochinolin-4-yl)-2-hydroxy-benzoesäure kann die Titelverbindung in einer TOTU-vermittelten Reaktion mit N1,N1-Dimethyl-ethan-1,2-diamin analog der in Beispiel 7 beschriebenen Methode hergestellt werden.

### Beispiel 33: N-[5-(6,8-Dichloro-2-methyl-1,2,3,4-tetrahydro-isochinolin-4-yl)-2-hydroxy-benzoyl]- guanidin;

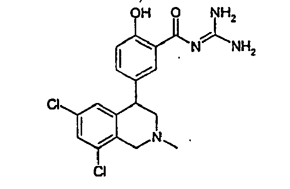

Zu einer Lösung von 2,39 g (25 mmol) Guanidin-Hydrochlorid in 15 ml abs. DMF werden 2,52 g Kalium-tert-butylat gegeben und 45 min bei Raumtemperatur gerührt. Es wird eine Lösung von 950 mg (2,5 mmol) 5-(6,8-Dichloro-2-methyl-1,2,3,4-tetrahydro-isochinolin-4-yl)-2-hydroxy-benzoesäure-ethylester (Beispiel 29, Zwischenprodukt 3) in 10 ml abs. DMF zugegeben und vier Stunden bei Raumtemperatur gerührt. Nachdem keine Umsatzsteigerung mehr festgestellt werden kann, wird vom Niederschlag abgesaugt und das Lösungsmittel i. Vak. entfernt. Der Rückstand wird in 2 N HCl aufgenommen und zweimal mit Dichlormethan extrahiert. Die wässrige Phase wird mit KOH auf einen pH von ca. 10 gestellt, wobei das gewünschte Acylguanidin als farbloser Niederschlag ausfällt. Absaugen und Trocknen liefert 793 mg der Titelverbindung.

### Beispiel 34: N-[3-(6,8-Dichloro-2-methyl-1,2,3,4-tetrahydro-isochinolin-4-yl)-phenyl]-acetamid;

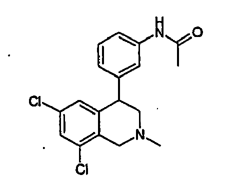

### N-[3-(6,8-Dichloro-2-methyl-1,2,3,4-tetrahydro-isochinolin-4-yl)-phenyl]-acetamid;

Die Herstellung des gewünschten meta-Acetanilids erfolgt analog der für Beispiel 1 angegebenen Syntheseroute, ausgehend von N-(3-Acetyl-phenyl)-acetamid und 2,4-Dichlorbenzyl-methyl-amin (Beispiel 1, Zwischenprodukt 1) in vier analogen Stufen.

### Beispiel 35: 3-(6,8-Dichloro-2-methyl-1,2,3,4-tetrahydro-isochinolin-4-yl)-phenylamin;

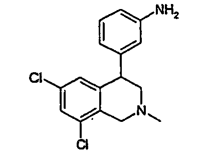

Die Acetylabspaltung aus N-[3-(6,8-Dichloro-2-methyl-1,2,3,4-tetrahydro-isochinolin-4-yl)-phenyl]-acetamid (Beispiel 34) gelingt nach der in Beispiel 17, Zwischenprodukt 1 beschriebenen Methode in Gegenwart von Natriumethanolat.

### Beispiel 36: 2-(6,8-Dichloro-2-methyl-1,2,3,4-tetrahydro-isochinolin-4-yl)-phenylamin;

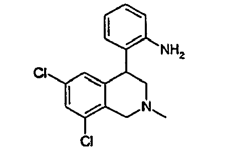

### Zwischenprodukt 1: N-[2-(2-Bromo-acetyl)-phenyl]-acetamid;

31 g (0.175 mol) N-(2-Acetylphenyl)-acetamid (hergestellt durch Acylieren von 2-Aminoacetophenon mit Acetylchlorid nach Fuerstner, Alois; Jumbam, Denis N.; Tetrahedron; 48; 29; 5991-6010, (1992)) werden in 200 ml Eisessig gelöst. Man gibt 127 ml 33%-iger HBr in Eisessig zu und lässt dann bei Raumtemperatur 8,75 ml (0,175 mol) Brom langsam zulaufen. Der Ansatz wird über Nacht bei RaumTemperatur gerührt. Der Ansatz wird in 1,5 L Eiswasser eingerührt, das ausgefallene Produkt wird abgesaugt, gut mit Eiswasser nachgewaschen und im Vakuum getrocknet. Das Rohprodukt enthält laut HPLC und NMR etwas Edukt und doppelt bromiertes Produkt, ist aber für die weitere Umsetzung sauber genug (ca. 85%ig). Ausbeute: 43 g

### Zwischenprodukt 2: N-(2-{2-[(2,4-Dichloro-benzyl)-methyl-amino]-acetyl}-phenyl)-acetamid;

12,4 g (65,24 mmol) 2,4-Dichlor-N-methylbenzylamin (Beispiel 1, Zwischenprodukt 1) werden in 200 ml Dioxan gelöst. Dazu gibt man 19,96 g des Rohproduktes der vorstehenden Bromierung, ebenfalls in 200 ml Dioxan gelöst, und 45 ml Triethylamin. Der Ansatz wird über Nacht bei Raumtemperatur gerührt und dann filtriert. Das Filtrat wird evaporiert, der Rückstand in Essigester aufgenommen und mit gesättigter Natriumhydrogencarbonat- und Kochsalzlösung gewaschen, über Natriumsulfat getrocknet und einrotiert. Das Rohprodukt (20,4 g) ist laut NMR für die weitere Umsetzung sauber genug.

### Zwischenprodukt 3: N-(2-{2-[(2,4-Dichloro-benzyl)-methyl-amino]-1-hydroxy-ethyl}-phenyl)-acetamid;

20 g des Rohproduktes der vorhergehenden Stufe (ca. 50 mMol) werden in 200 ml Methanol gelöst und im Eisbad auf < 5 °C gekühlt. Dazu gibt man unter gutem Rühren portionsweise 4,3 g (109 mMol) Natriumborhydrid, so dass die Innentemperatur 10 °C nicht überschreitet. Anschließend wird noch 30 Min. im Eisbad und 1h bei RT nachgerührt. Nach Stehen über Nacht wird der Ansatz evaporiert, der Rückstand in Essigester aufgenommen, 3x mit Wasser und 1x mit Kochsalzlösung gewaschen, über Natriumsulfat getrocknet und einrotiert. Das Rohprodukt (19,4 g) wird ohne Reinigung weiter umgesetzt.

### Zwischenprodukt 4: 1-(2-Amino-phenyl)-2-[(2,4-dichloro-benzyl)-methyl-amino]-ethanol;

10g des Rohproduktes aus der vorhergehenden Stufe werden in 300 ml Methanol gelöst. Man gibt 200 ml conc. Salzsäure zu und rührt 10h bei 50 °C. Man lässt abkühlen, gießt den Ansatz in Wasser ein und stellt den pH-Wert mit 20%-iger NaOH auf 10-12 ein. Das Produkt wird mit Essigester extrahiert, die vereinigten Extrakte mit Kochsalzlösung gewaschen, über Natriumsulfat getrocknet und evaporiert. Das Rohprodukt (9,9 g) enthält etwas Kochsalz, was jedoch für die weitere Umsetzung nicht stört.

### 2-(6,8-Dichloro-2-methyl-1,2,3,4-tetrahydro-isochinolin-4-yl)-phenylamin;

9,9 g des Rohproduktes aus der vorhergehenden Stufe werden in 350 ml Chloroform gelöst. Unter Kühlung im Eisbad lässt man 123 ml conc. Schwefelsäure zutropfen. Man rührt 2h im Eisbad nach, lässt dann langsam auf RT kommen und erhitzt schließlich über Nacht auf 50 °C. Der abgekühlte Ansatz wird auf Eis gegossen und mit Natronlauge alkalisch gestellt (pH > 10). Die organische Phase wird abgetrennt, die wässrige Phase 2x mit Methylenchlorid nachextrahiert, die vereinigten organischen Phasen werden mit Wasser und NaCl gewaschen, über Natriumsulfat getrocknet und evaporiert.

### Allgemeine Vorschrift für die Herstellung der Beispielverbindungen 37 bis 77:

154 mg (0,5 mmol) der Titelverbindungen aus Beispiel 35, Beispiel 36 oder Beispiel 17, Zwischenprodukt 1 werden in 5 ml Dichlormethan vorgelegt und mit 0,076 ml (0,55 mmol) Triethylamin versetzt. Bei 0 °C wird eine Lösung von 1,1 Äquivalenten (0,55 mmol) eines Säurechlorids in 5 ml Dichlormethan zugegeben und über Nacht auftauend gerührt. Zur Aufarbeitung wird filtriert und vom Lösungsmittel befreit. Der Rückstand wird in 20 ml Essigester gelöst und je einmal mit 5 %iger NaHCO₃-Lösung, sowie 5 %iger NaCl-Lösung gewaschen und getrocknet. Nach Einengen des Lösungsmittels schließt sich eine Endreinigung an einer präp. HPLC an.

**Tabelle 1:**

| Beispiel | Edukt 1 / Anilin | Edukt 2 / Säurechlorid | Produkt |
|---|---|---|---|
| 37 | 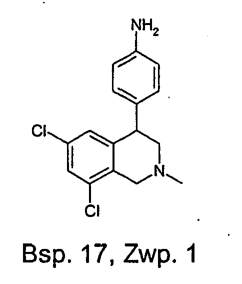 | 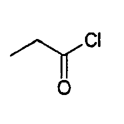 | 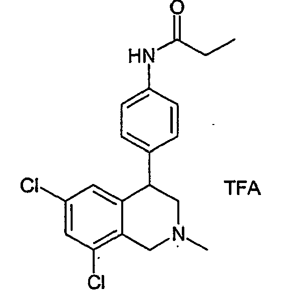 |
| 38 | 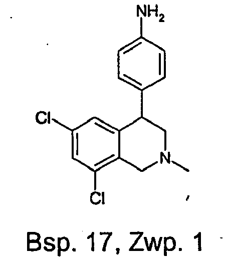 | 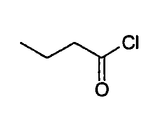 | 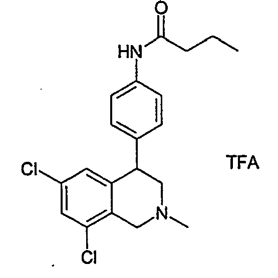 |
| 39 | 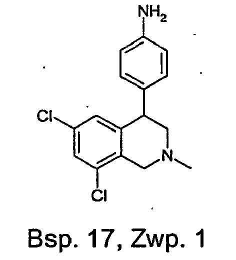 | 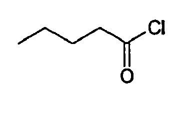 | 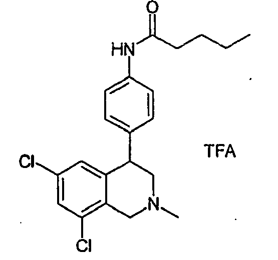 |
| 40 | 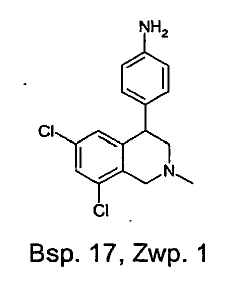 | 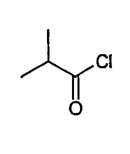 | 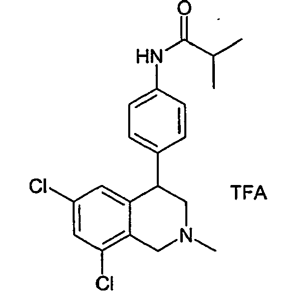 |
| 41 | 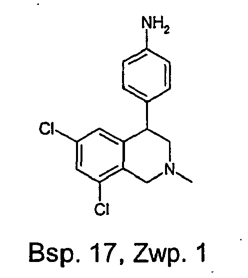 | 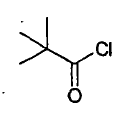 | 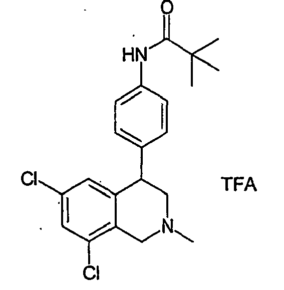 |
| 42 | 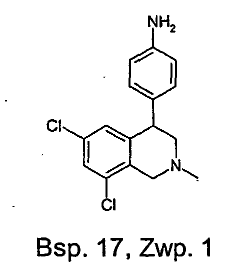 | 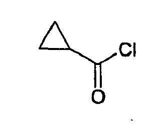 | 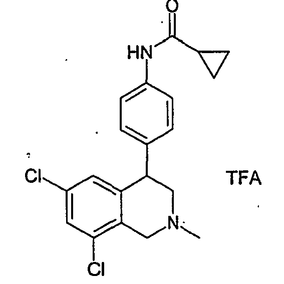 |
| 43 | 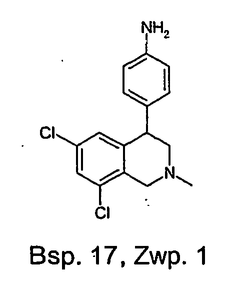 | 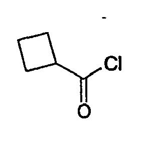 | 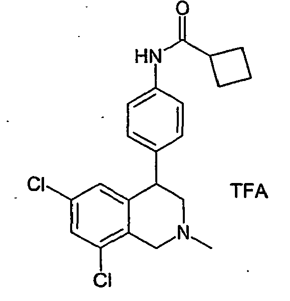 |
| 44 | 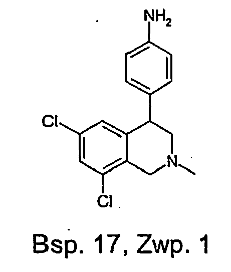 | 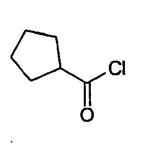 | 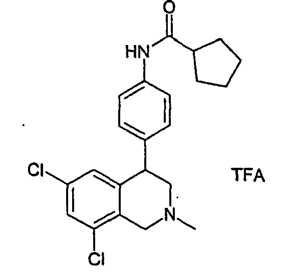 |
| 45 | 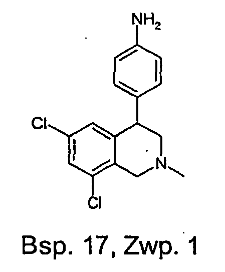 | 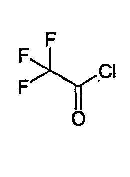 | 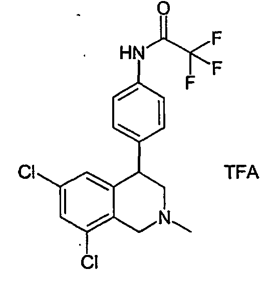 |
| 46 * | 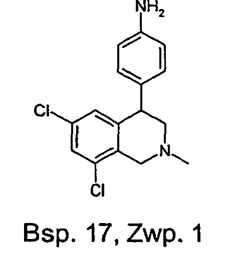 | 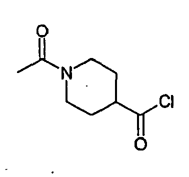 | 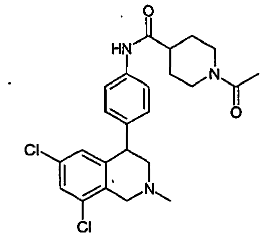 |
| 47 | 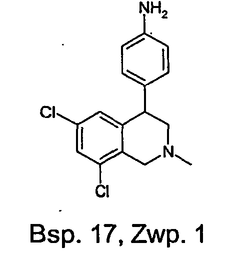 | 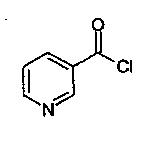 | 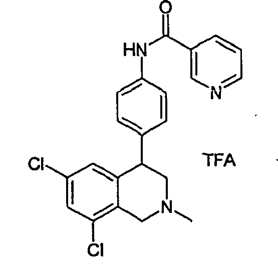 |
| 48 | 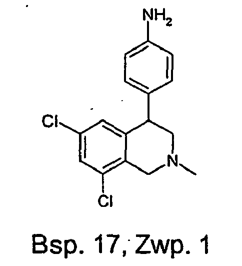 | 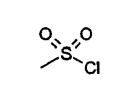 | 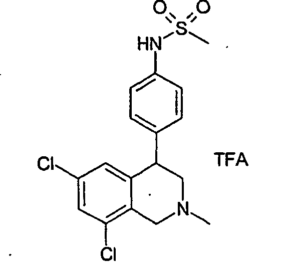 |
| 49 | 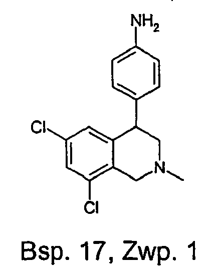 | 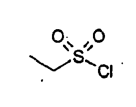 | 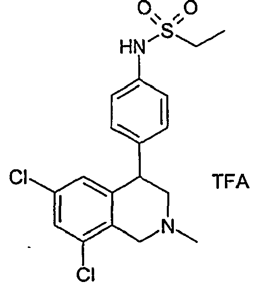 |
| 50 | 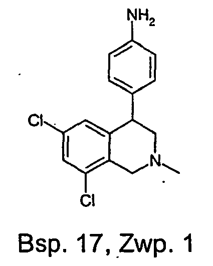 | 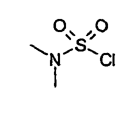 | 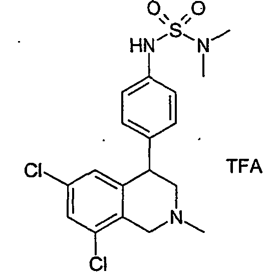 |
| 51 | 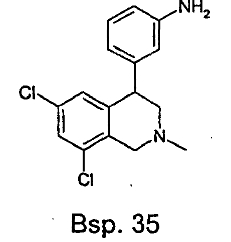 | 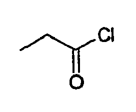 | 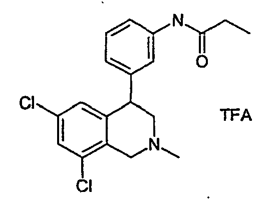 |
| 52 | 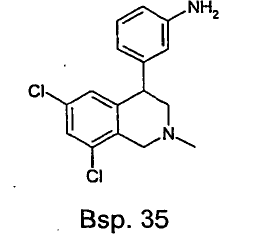 | 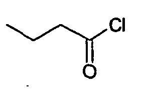 | 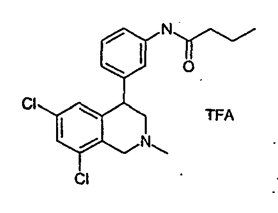 |
| 53 | 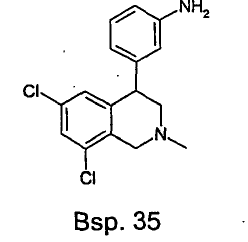 | 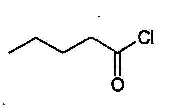 | 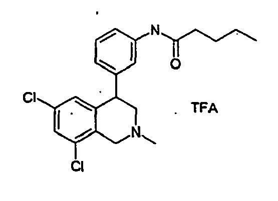 |
| 54 | 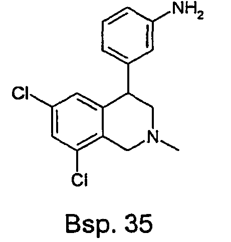 | 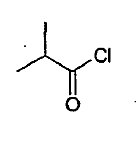 | 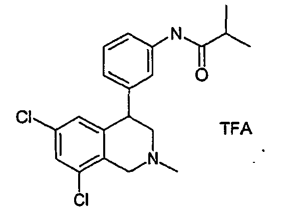 |
| 55 | 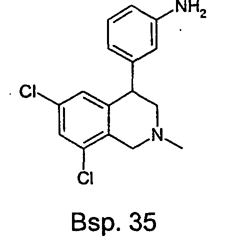 | 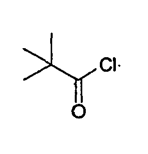 | 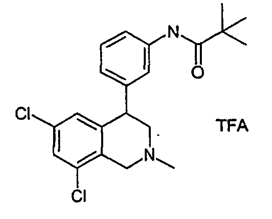 |
| 56 | 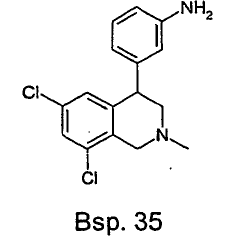 | 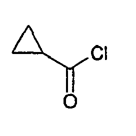 | 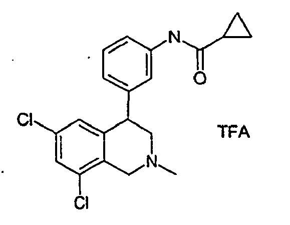 |
| 57 | 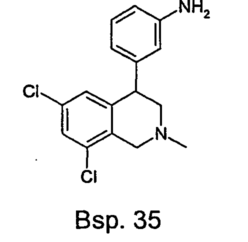 | 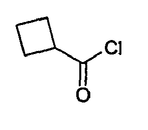 | 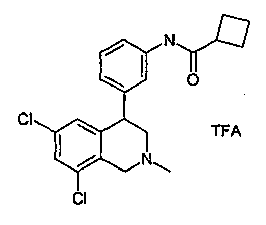 |
| 58 | 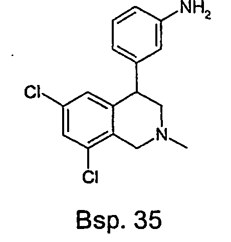 | 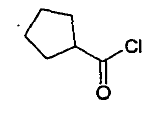 | 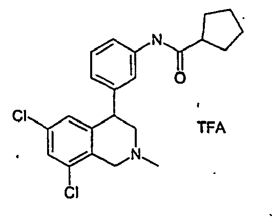 |
| 59 | 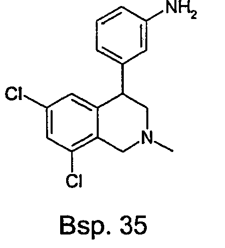 | 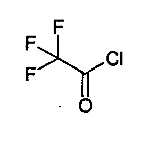 | 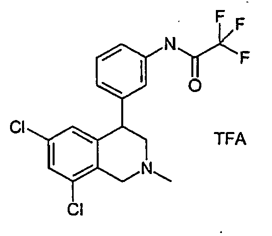 |
| 60 | 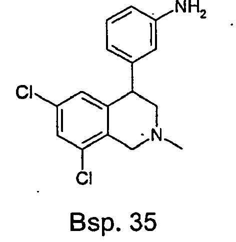 | 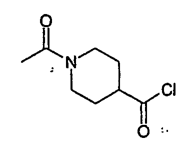 | 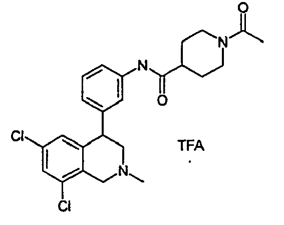 |
| 61 | 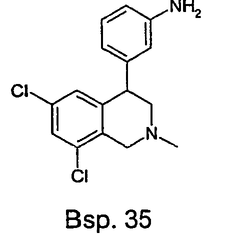 | 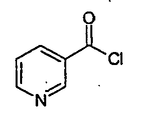 | 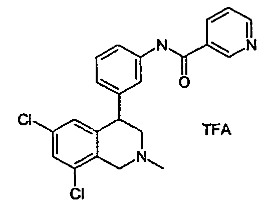 |
| 62 | 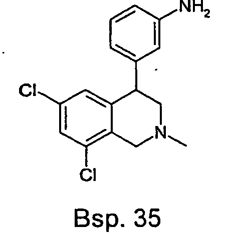 | 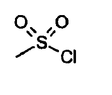 | 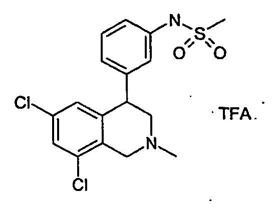 |
| 63 | 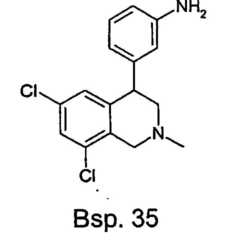 | 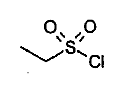 | 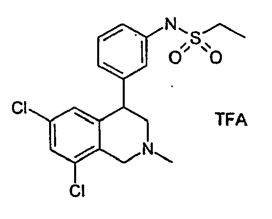 |
| 64 | 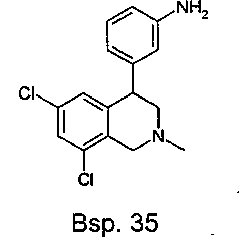 | 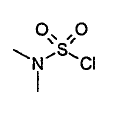 | 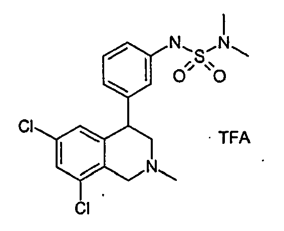 |
| 65 | 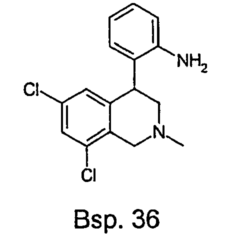 | 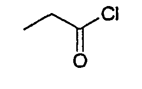 | 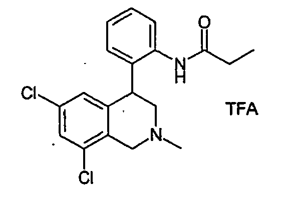 |
| 66 | 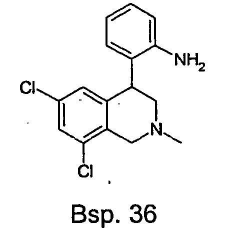 | 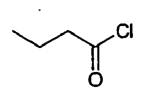 | 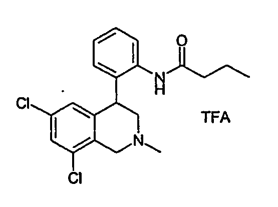 |
| 67 | 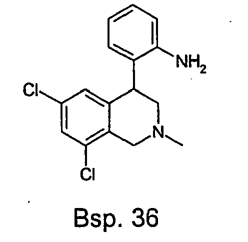 | 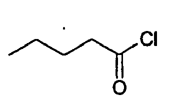 | 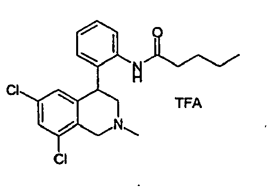 |
| 68 | 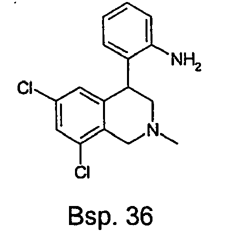 | 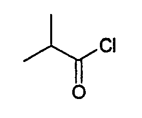 | 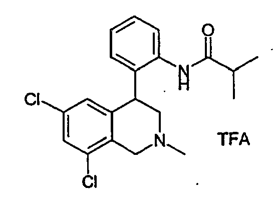 |
| 69 | 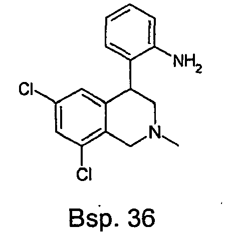 | 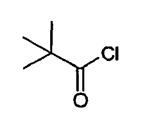 | 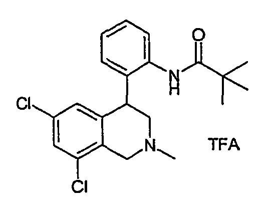 |
| 70 | 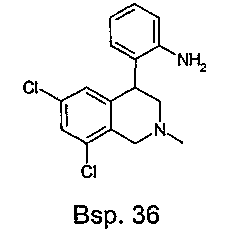 | 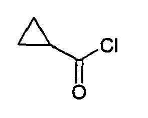 | 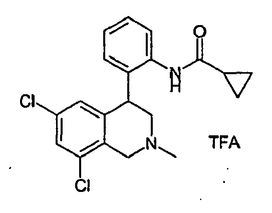 |
| 71 | 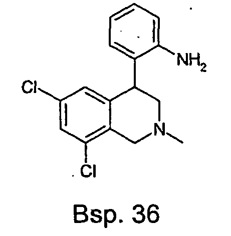 | 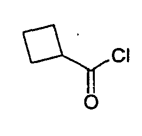 | 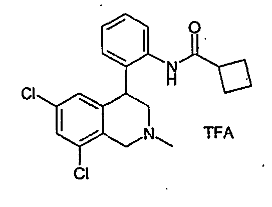 |
| 72 | 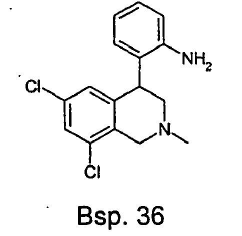 | 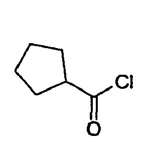 | 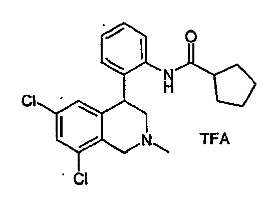 |
| 73 | 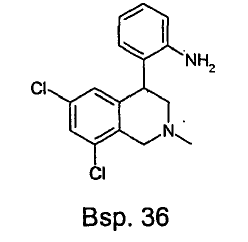 | 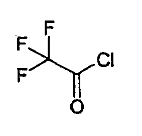 | 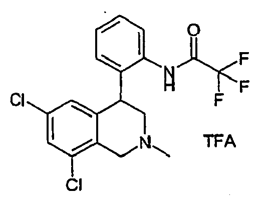 |
| 74 | 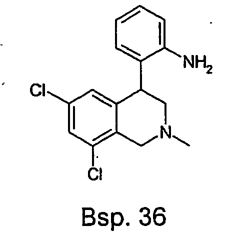 | 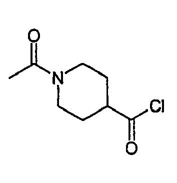 | 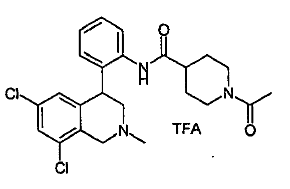 |
| 75 | 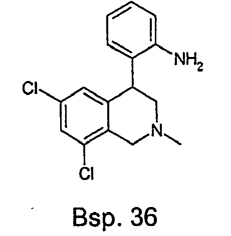 | 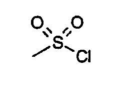 | 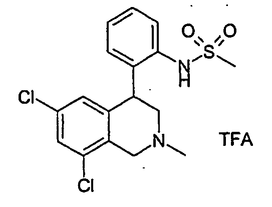 |
| 76 | 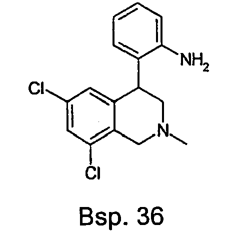 | 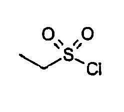 | 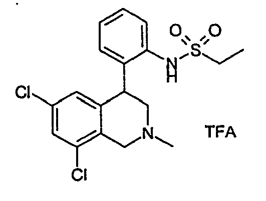 |
| 77 | 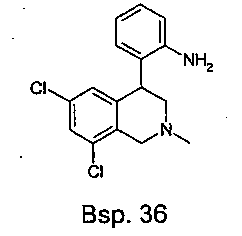 | 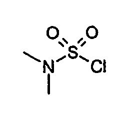 | 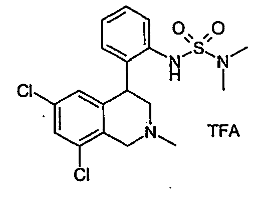 |

| | | | |
|---|---|---|---|
| *) Produkt fällt aus der Reaktionslösung aus und bedart keiner weiteren Reinigung | | | |

### Beispiel 78: 1-[3-(6,8-Dichloro-2-methyl-1,2,3,4-tetrahydro-isochinolin-4-yl)-phenyl]-3-ethyl-harnstoff, Trifluoracetat;

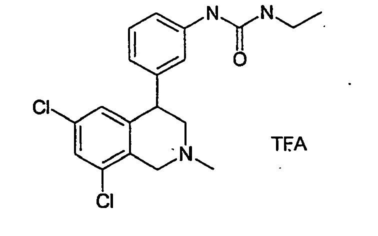

0,355 mmol der Beispielverbindung 35 werden in 5 ml trockenem Acetonitril gelöst und mit 0,39 mmol Ethylisocyanat versetzt. Nach Stehen über Nacht unter Feuchtigkeitsausschluss wird vom Lösungsmittel befreit und das Rohprodukt an einer präparativen HPLC gereinigt, wobei die Titelverbindung als farbloser Feststoff erhalten wird.

### Beispiel 79: 1-[3-(6,8-Dichloro-2-methyl-1,2,3,4-tetrahydro-isochinolin-4-yl)-phenyl]-3-methyl- thioharnstoff, Trifluoracetat;

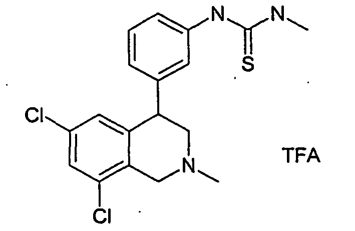

Ausgehend von Beispielverbindung 35 und Methylisothiocyanat wird die Titelverbindung nach der in Beispiel 78 beschriebenen Methode synthetisiert.

### Beispiel 80: 1-[2-(6,8-Dichloro-2-methyl-1,2,3,4-tetrahydro-isochinolin-4-yl)-phenyl]-3-ethyl-harnstoff;

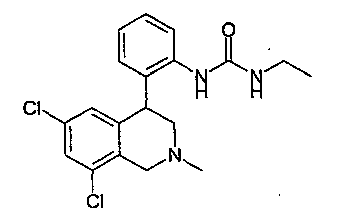

Ausgehend von Beispielverbindung 36 und Ethylisocyanat wird analog Beispiel 78 verfahren. Zur Aufarbeitung wird der entstandene Niederschlag abgesaugt und mit Acetonitril gewaschen, wobei der gewünschte Ethylharnstoff als farbloser Feststoff erhalten wird.

### Beispiel 81: 1-[2-(6,8-Dichloro-2-methyl-1,2,3,4-tetrahydro-isochinolin-4-yl)-phenyl]-3-methyl- thioharnstoff, Trifluoracetat;

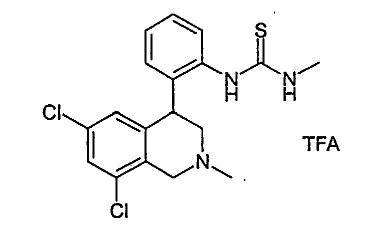

Beispielverbindung 36 und Methylisothiocyanat werden analog der in Beispiel 78 beschriebenen Methode umgesetzt.

### Beispiel 82: Ethansulfonsäure-[3-(6,8-dichloro-2-methyl-1,2,3,4-tetrahydro-isochinolin-4-yl)- phenyl]-amid, Hydrochlorid;

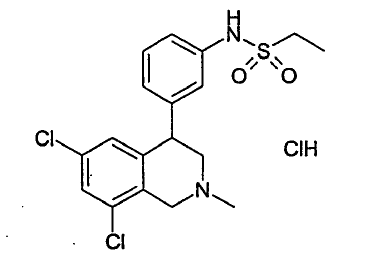

307,1 mg (1 mmol) der Beispielverbindung 35 werden in 10 ml Pyridin gelöst und bei 0 °C mit 0,19 g (1,5 mmol) Ethansulfonylchlorid, sowie einer katalytischen Menge DMAP versetzt. Es wird bei Raumtemperatur gerührt. Zur Aufarbeitung wird das Lösungsmittel i. Vak. entfernt, der Rückstand in Essigester aufgenommen und mit H₂O gewaschen. Die organische Phase wird mit MgSO₄ getrocknet und eingeengt. Das Rohprodukt wird an Kieselgel chromatographiert. Das so erhaltene Sulfonamid wird in einem THF/2N HCl-Gemisch gelöst und wiederum i. Vak. eingeengt, wobei 208 mg des gewünschten Hydrochlorids erhalten werden.

### Beispiel 83: N-[3-(6,8-Dichloro-2-methyl-1,2,3,4-tetrahydro-isochinolin-4-yl)-phenyl]-methansulfonamid, Hydrochlorid;

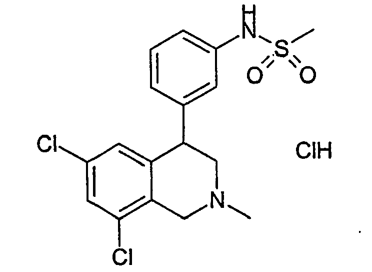

Ausgehend von Beispielverbindung 35 und Methansulfonylchlorid wird analog der in Beispiel 82 beschriebenen Methode verfahren.

### Beispiel 84: Ethansulfonsäure-[4-(6,8-dichloro-2-methyl-1,2,3,4-tetrahydro-isochinolin-4-yl)- phenyl]-amid, Hydrochlorid;

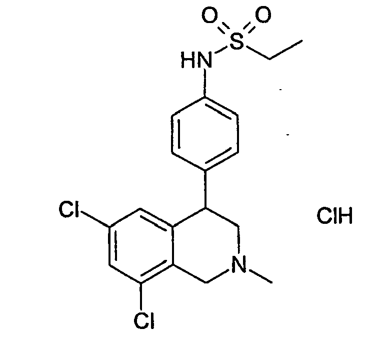

Ausgehend von Beispielverbindung 17, Zwischenprodukt 1 und Ethansulfonyl-chlorid wird analog der in Beispiel 82 beschriebenen Methode verfahren.

### Beispiel 85: N-[4-(6,8-Dichloro-2-methyl-1,2,3,4-tetrahydro-isochinolin-4-yl)-phenyl]-methansulfonamid, Hydrochlorid;

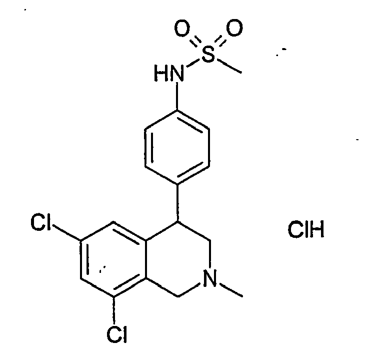

Ausgehend von Beispielverbindung 17, Zwischenprodukt 1 und Methansulfonyl-chlorid wird analog der in Beispiel 82 beschriebenen Methode verfahren.

### Beispiel 86: 86a: (-)-N-[3-(6,8-Dichloro-2-methyl-1,2,3,4-tetrahydro-isochinolin-4-yl)-phenyl]-acetamid;

### 86b: (+)-N-[3-(6,8-Dichloro-2-methyl-1,2,3,4-tetrahydro-isochinolin-4-yl)-phenyl]-acetamid;

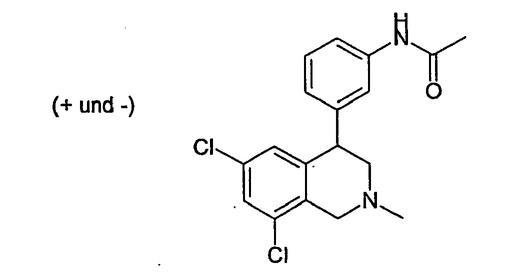

2,0 g der Titelverbindung aus Beispiel 34 werden an einer chiralen Phase getrennt, wobei ca. 1,0 g der beiden enantiomeren Acetamide 86a und 86b erhalten werden. chirale Säule: Chiralpak ADH/31 250 x 4,6 mm;
Lösungsmittel: Acetonitril;
Flussrate: 1 ml/min;
Rₜ((-)-Enantiomer/86a) = 5,541 min;
Rₜ((+)-Enantiomer/86b) = 7,033 min.

Allgemeine Vorschrift für die Herstellung der Beispielverbindungen 87 bis 98 1,0 mmol 4-(6,8-Dichloro-2-methyl-1,2,3,4-tetrahydro-isochinolin-4-yl)-phenylamin (Beispiel 17, Zwischenprodukt 1), bzw. 3-(6,8-Dichloro-2-methyl-1,2,3,4-tetrahydroisochinolin-4-yl)-phenylamin (Beispiel 35) wird in 10 ml Pyridin vorgelegt und bei 0 °C eine Lösung von 1,2 Äquivalenten des entsprechenden Sulfonsäurechlorids (s. Tabelle 2) in 5 ml Dichlormethan zugetropft. Es wird bei Raumtemperatur gerührt. Je nach Reaktionsfortschritt wird eine katalytische Menge DMAP zugegeben und gegebenenfalls die Reaktionstemperatur auf 50 °C erhöht bis keine Umsatzsteigerung mehr festgestellt werden kann. Zur Aufarbeitung wird eingeengt und der Rückstand zwischen Essigester und ges. NaHCO₃-Isg. verteilt. Die organische Phase wird abgetrennt und noch einmal mit ges. NaHCO₃-Isg. und H₂O gewaschen, mit Na₂SO₄ getrocknet und eingeengt. Zur weiteren Reinigung wird das so erhaltene Rohprodukt an Kieselgel chromatographiert. Zur Überführung der so erhaltenen Produkte in die entsprechenden Hydrochloride werden die Substanzen in 2 N HCl oder ethanolischer HCl gelöst und vom Lösungsmittel befreit, wobei die gewünschten HCl-Salze erhalten werden.
Bei einer Reinigung an einer präparativen HPLC-Anlage werden die entsprechenden Produkte als Trifluoracetate erhalten.

**Tabelle 2:**

| Beispiel | Edukt 1 / Anilin | Edukt 2 / Säurechlorid | Produkt |
|---|---|---|---|
| 87 | 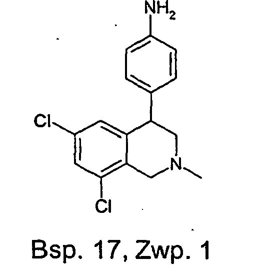 | 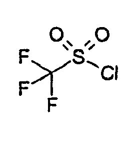 | 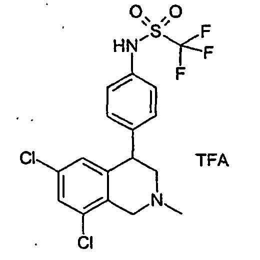 |
| 88 | 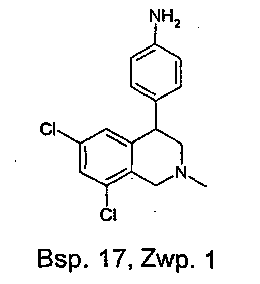 | 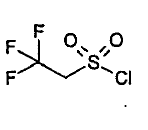 | 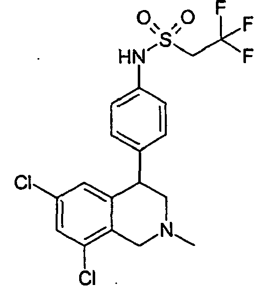 |
| 88a | 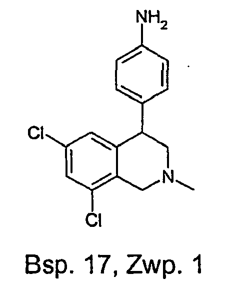 | 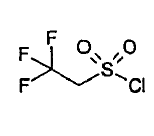 | 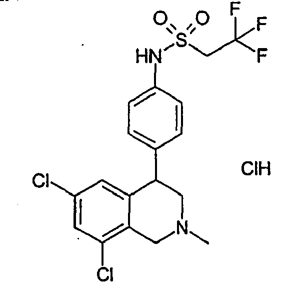 |
| 89 | 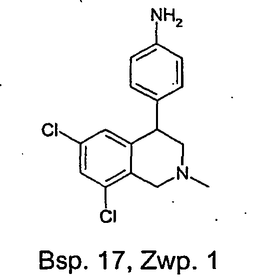 | 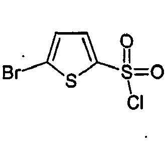 | 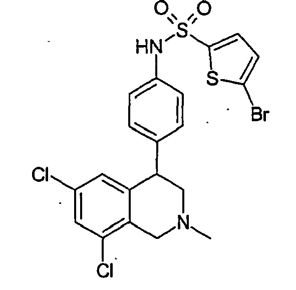 |
| 89a | 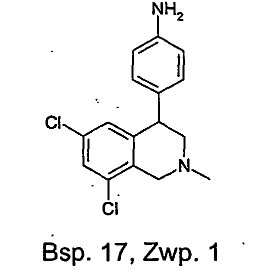 | 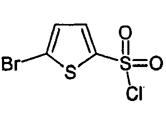 | 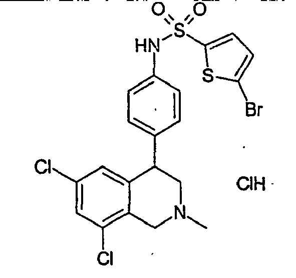 |
| 90 | 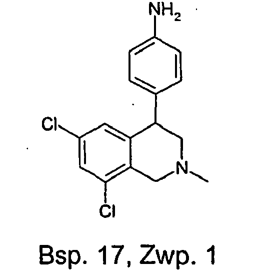 | 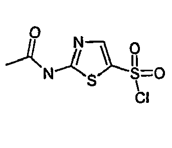 | 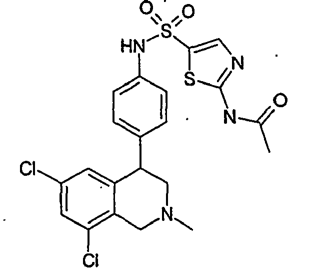 |
| 90a | 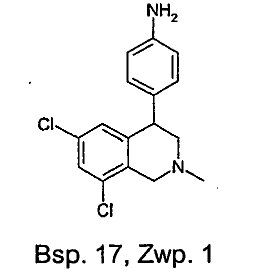 | 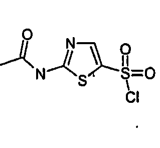 | 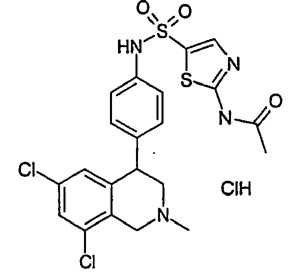 |
| 91 | 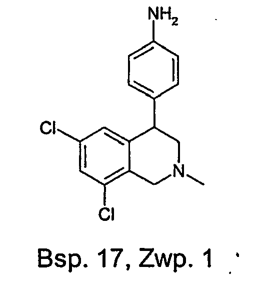 | 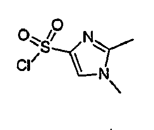 | 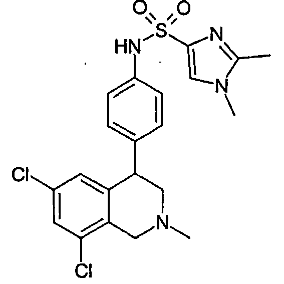 |
| 91a | 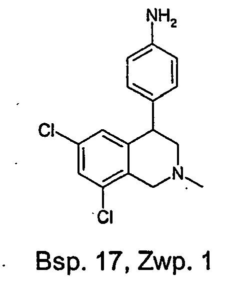 | 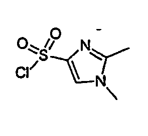 | 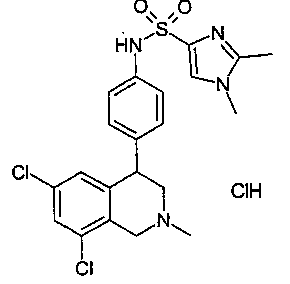 |
| 92 | 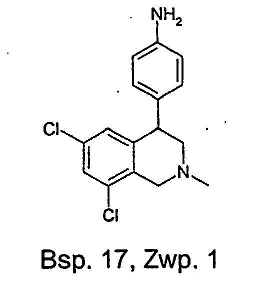 | 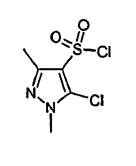 | 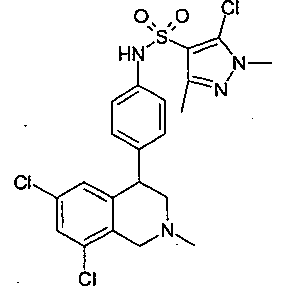 |
| 92a | 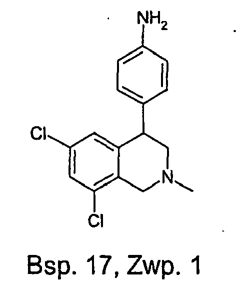 | 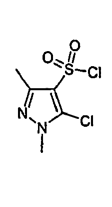 | 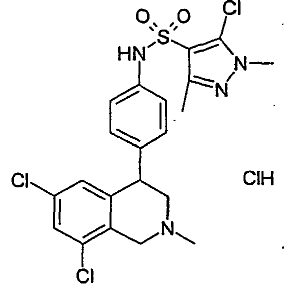 |
| 93 | 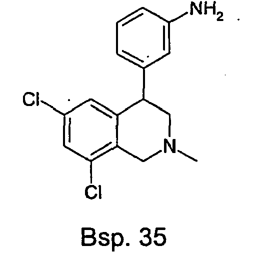 | 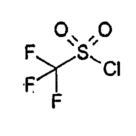 | 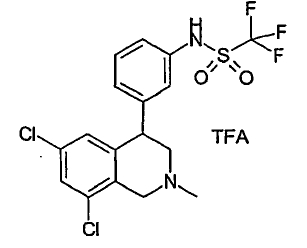 |
| 94 | 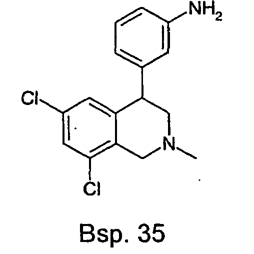 | 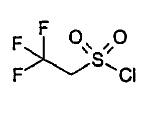 | 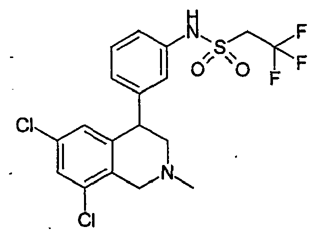 |
| 94a | 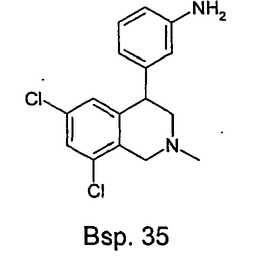 | 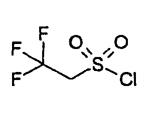 | 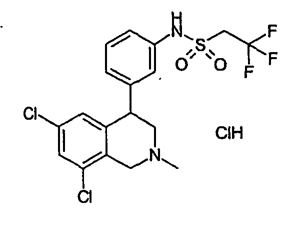 |
| 95 | 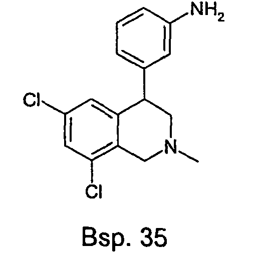 | 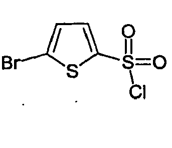 | 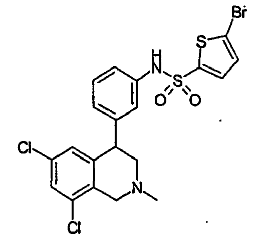 |
| 96 | 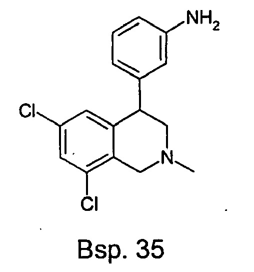 | 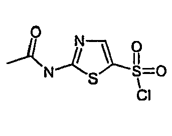 | 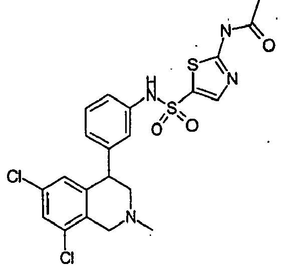 |
| 96a | 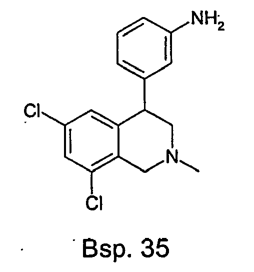 | 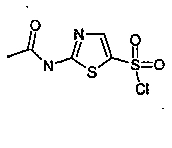 | 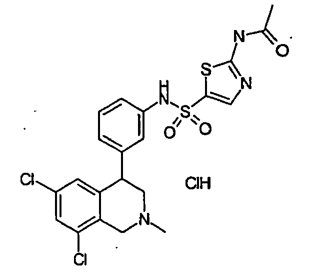 |
| 97 | 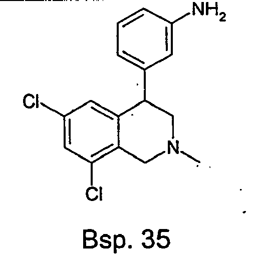 | 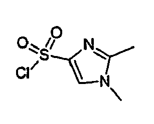 | 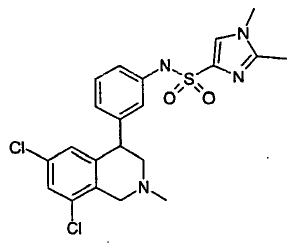 |
| 97a | 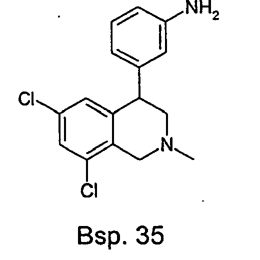 | 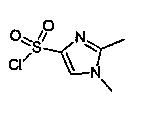 | 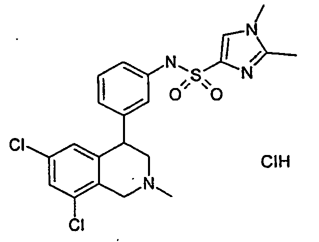 |
| 98 | 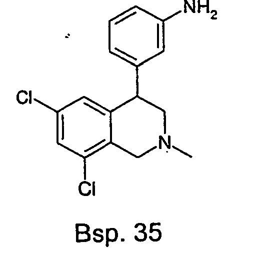 | 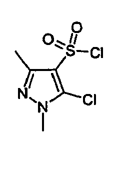 | 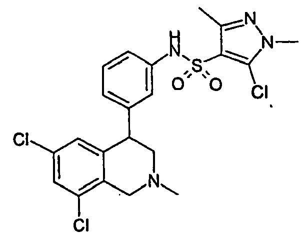 |

### Allgemeine Arbeitsvorschrift zur Synthese der Beispielverbindungen 99 bis 110 Herstellung der Aminkomponente

4,0 mmol des aromatischen Aldehyds (s. Tabelle 3) werden mit 8,0 mmol des aliphatischen Amins (s. Tabelle 3) in Methanol 2 Stunden bei Raumtemperatur gerührt und im Anschluss, je nach Reaktionsfortschritt, portionsweise mit 0,67 bis 2,0 eq. NaBH₄ versetzt. Nach Stehen über Nacht bei Raumtemperatur wird vom Lösungsmittel befreit und der Rückstand in 1 N HCl aufgenommen. Es wird mit Dichlormethan extrahiert. Die wässrige Phase wird mit NaOH auf einen pH-Wert von 11 bis 12 eingestellt und wiederum mit Dichlormethan extrahiert. Die organischen Phasen werden mit MgSO₄ getrocknet und einrotiert. Die weitere Reinigung geschieht durch Chromatographie an Kieselgel oder an einer präp. HPLC.

### Herstellung der Bromketonkomponente

Die Bromketonbausteine werden nach literaturbekannten Methoden ausgehend von käuflichen Acetophenonen durch Behandeln mit Brom in Eisessig, analog Beispiel 1, Zwischenprodukt 2, synthetisiert.

Ausgehend von den in Tabelle 3 gezeigten Amin- und Bromketonkomponenten lassen sich die Beispielverbindungen 100 bis 111 in Analogie zu der in Beispiel 1 gezeigten Syntheseroute (Alkylierung der Aminkomponente durch die Bromketon-komponente, anschließende Reduktion mit NaBH₄ und abschließende H₂SO₄-vermittelte Cyclisierung) darstellen.
Die erhaltenen Tetrahydroisochinoline lassen sich in dem Fachmann bekannter Weise in die entsprechenden Salze überführen.

**Tabelle 3:**

| Beispiel-Nr. | aromatischer Aldehyd | aliphatisches Amin | Aminkomponente | Bromketonkomponente | Beispielverbindung |
|---|---|---|---|---|---|
| 99 | 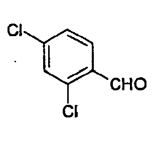 | -NH₂ | 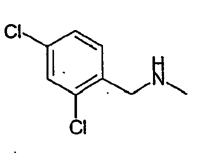 | 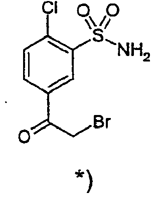 | 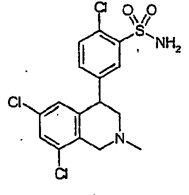 |
| 100 | 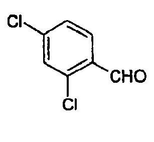 | -NH₂ | 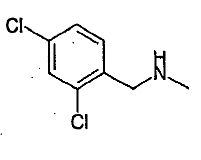 | 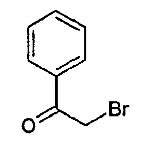 | 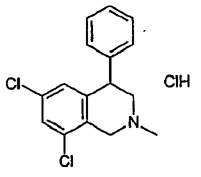 |
| 101 | 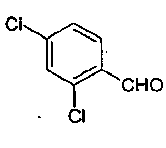 | -NH₂ | 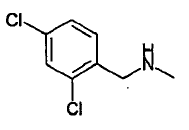 | 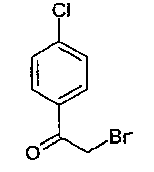 | 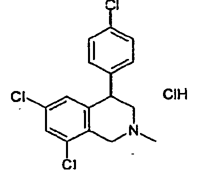 |
| 102 | 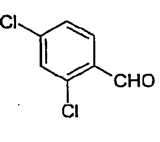 | 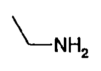 | 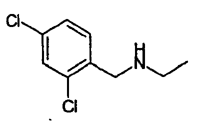 | 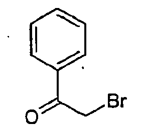 | 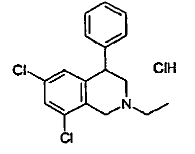 |
| 103 | 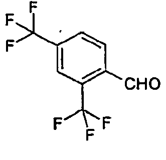 | -NH₂ | 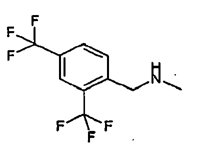 | 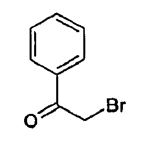 | 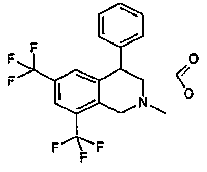 |
| 104 | 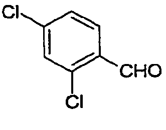 | 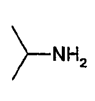 | 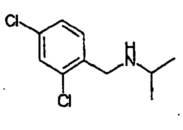 | 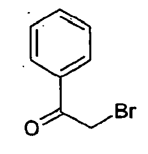 | 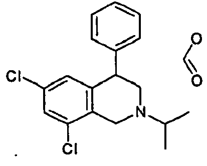 |
| 105 | 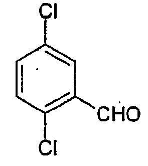 | -NH₂ | 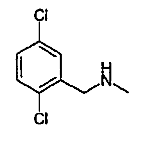 | 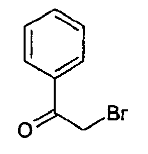 | 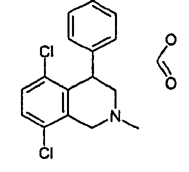 |
| 106 | 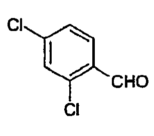 | -NH₂ | 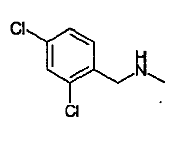 | 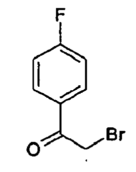 | 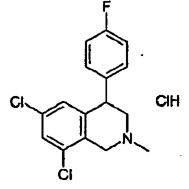 |
| 107 | 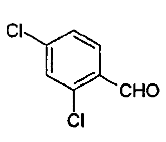 | -NH₂ | 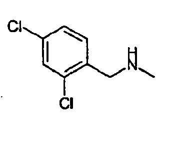 | 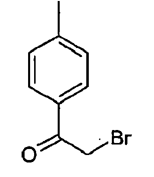 | 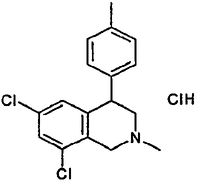 |
| 108 | 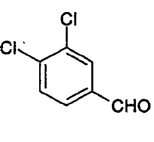 | -NH₂ | 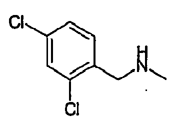 | 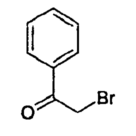 | 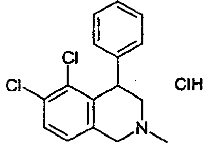 |
| 109 | 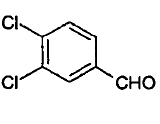 | -NH₂ | 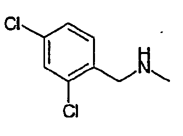 | 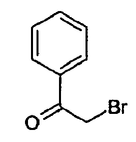 | 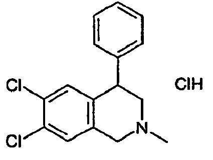 |
| 110 | 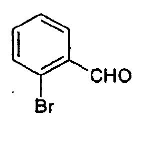 | -NH₂ | 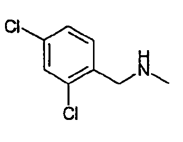 | 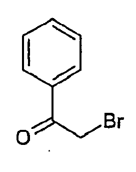 | 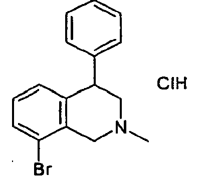 |

| | | | | | |
|---|---|---|---|---|---|
| *) Synthese beschrieben in: Lang et al., DOS 24 36 263 | | | | | |

Allgemeine Arbeitsverschrift zur Herstellung der Beispielverbindungen 111 bis 124 0,358 mmol der in Tabelle 4 angegebenen Säuren werden in 1 ml DMF gelöst und 0,221 ml (1,30 mmol) Diisopropylethylamin zugegeben. Bei 0 °C wird eine Lösung aus 128 mg (0,390 mmol) TOTU in 1 ml DMF zugetropft. Nachdem mit einer Lösung aus 100 mg (0,325 mmol) der in Tabelle 4 angegebenen Aminkomponente in 2 ml DMF versetzt wurde, wird bei Raumtemperatur über Nacht gerührt. Zur Aufarbeitung wird von ungelösten Bestandteilen abfiltriert und mit 20 ml Essigester nachgewaschen. Das Filtrat wird zweimal mit ges. NaHCO₃-lsg., sowie einmal mit 5 %iger NaCl-lsg. gewaschen, getrocknet und eingeengt.

Diejenigen Rohprodukte, die noch Boc-Schutzgruppen enthalten, werden ohne weitere Reinigung entschützt (s.u.: allgemeine Arbeitsverschrift zur Abspaltung der Boc-Schutzgruppen). Bei nicht Boc-geschützten Bausteinen wird nach der Aufarbeitung an einer präparativen HPLC gereinigt, wobei die gewünschten Beispielverbindungen als Trifluoracetate erhalten werden.

Allgemeine Arbeitsverschrift zur Herstellung der Beispielverbindungen 124 bis 147 0,358 mmol der in Tabelle 4 angegebenen Säuren werden in 1 ml DMF gelöst und 0,221 ml (1,30 mmol) Diisopropylethylamin zugegeben. Bei 0 °C wird mit 151 mg (0,975 mmol) Diethylcarbodiimid, einer Lösung aus 132 mg (0,975 mmol) HOBt in 1 ml DMF, sowie 20 mg ( 0,162 mmol) DMAP versetzt. Nachdem eine Lösung der in Tabelle 4 angegebenen Aminkomponente in 2 ml DMF zugetropft wurde, wird bei Raumtemtperatur über Nacht gerührt. Zur Aufarbeitung wird von ungelösten Bestandteilen abfiltriert und mit 20 ml Essigester nachgewaschen. Das Filtrat wird zweimal mit ges. NaHCO₃-lsg., sowie einmal mit 5 %iger NaCl-lsg. gewaschen, getrocknet und eingeengt.

Diejenigen Rohprodukte, die noch Boc-Schutzgruppen enthalten, werden ohne weitere Reinigung entschützt (s.u.: allgemeine Arbeitsverschrift zur Abspaltung der Boc-Schutzgruppen). Bei nicht Boc-geschützten Bausteinen wird nach der Aufarbeitung an einer präparativen HPLC gereinigt, wobei die gewünschten Beispielverbindungen als Trifluoracetate erhalten werden.

### Allgemeine Arbeitsvorschrift zur Abspaltumg der Boc-Schutzgruppen

Die erhaltenen Rohprodukte werden in 5 ml einer 10% igen Lösung von Trifluoressigsäure in Dichlormethan eine Stunde bei Raumtemperatur gerührt. Anschließend wird i. Vak. eingeengt und der Rückstand an einer präparativen HPLC gereinigt, wobei die gewünschten Beispielverbindungen als Trifluoracetate erhalten werden.

**Tabelle 4:**

| Beispiel-Nr. | Säurekomponente | Aminkomponente | Beispielverbindung |
|---|---|---|---|
| 111 | 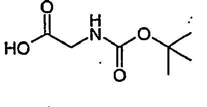 | 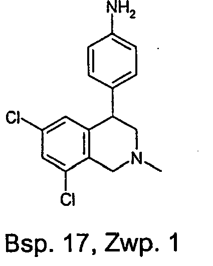 | 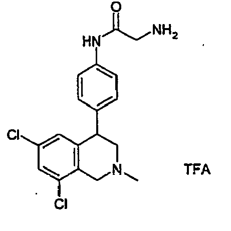 |
| 112 | 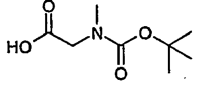 | 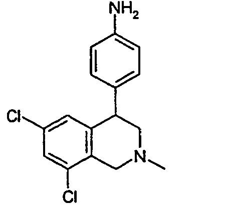 | 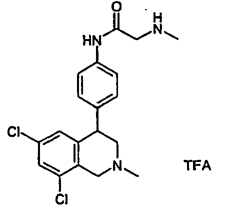 |
| 113 | 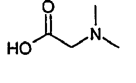 | 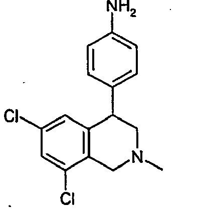 | 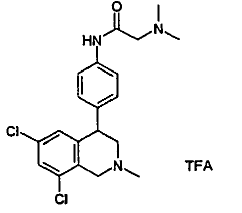 |
| 114 | 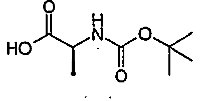 | 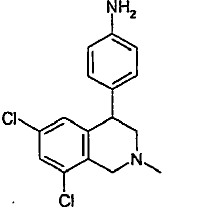 | 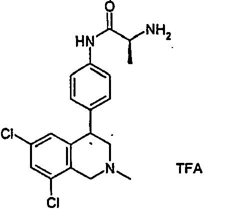 |
| 115 | 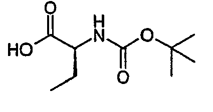 | 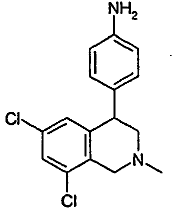 | 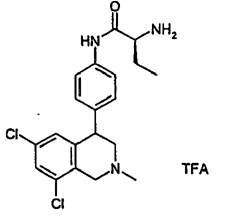 |
| 116 | 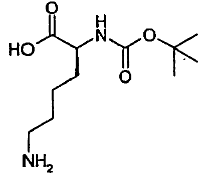 | 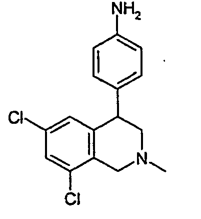 | 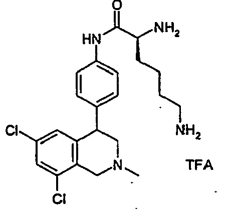 |
| 117 | 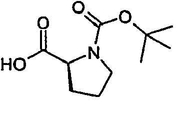 | 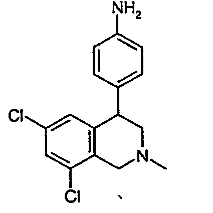 | 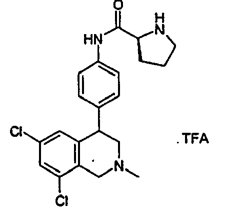 |
| 118 | 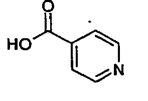 | 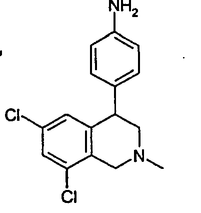 | 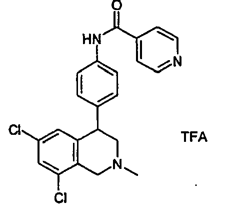 |
| 119 | 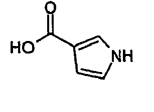 | 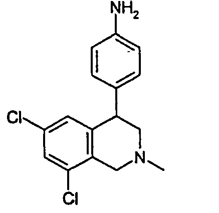 | 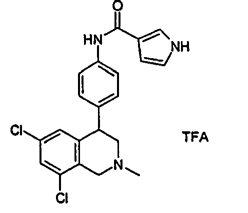 |
| 120 | 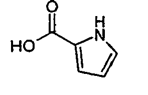 | 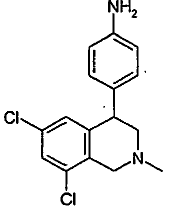 | 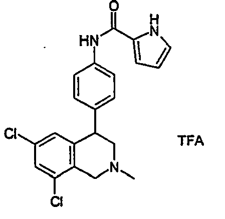 |
| 121 | 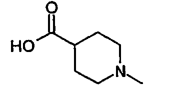 | 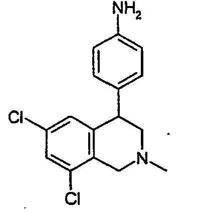 | 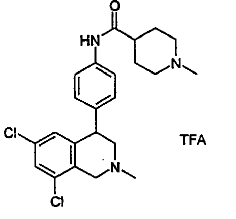 |
| 122 | 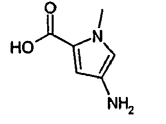 | 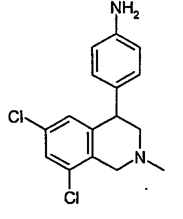 | 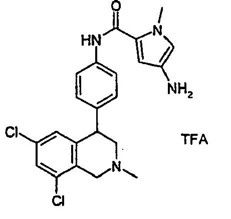 |
| 123 | 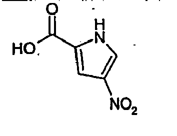 | 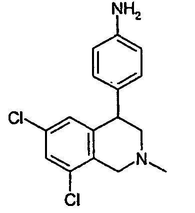 | 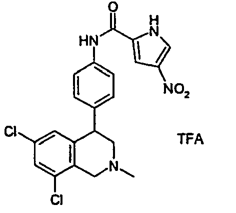 |
| 124 | 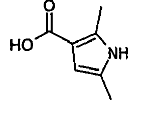 | 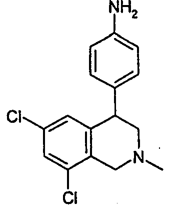 | 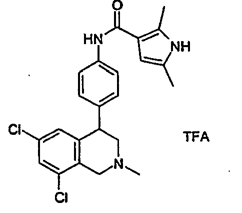 |
| 125 | 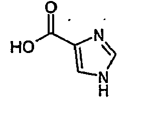 | 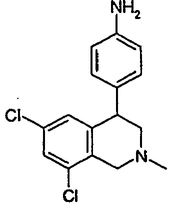 | 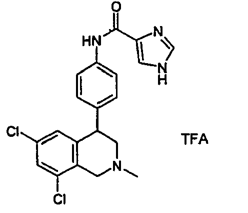 |
| 126 | 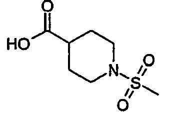 | 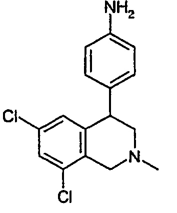 | 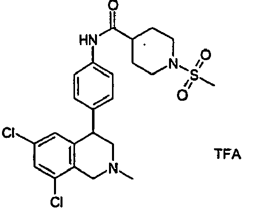 |
| 127 | 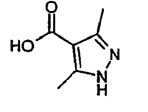 | 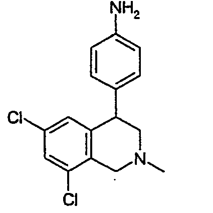 | 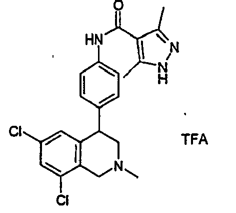 |
| 128 | 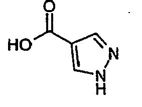 | 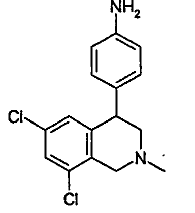 | 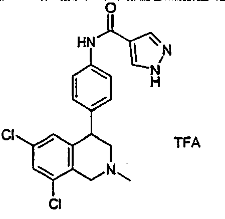 |
| 129 | 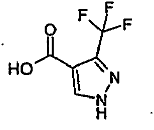 | 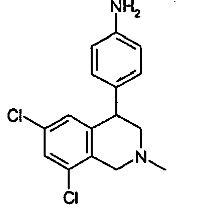 | 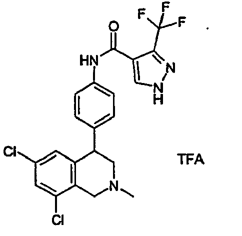 |
| 130 | 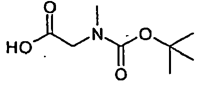 | 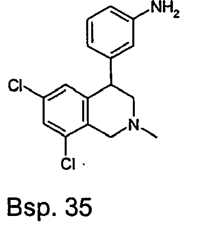 | 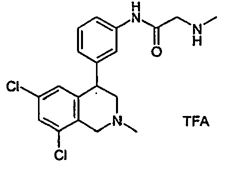 |
| 131 | 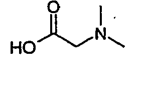 | 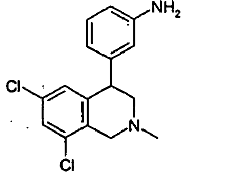 | 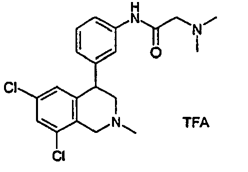 |
| 132 | 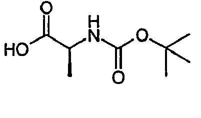 | 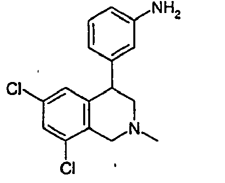 | 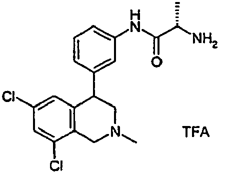 |
| 133 | 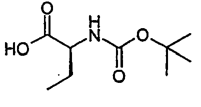 | 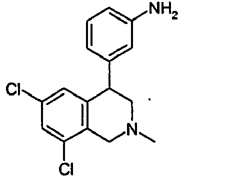 | 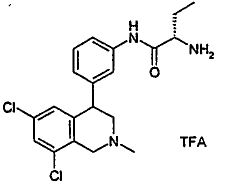 |
| 134 | 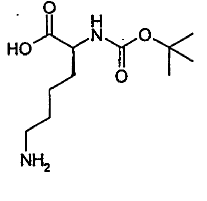 | 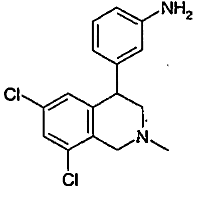 | 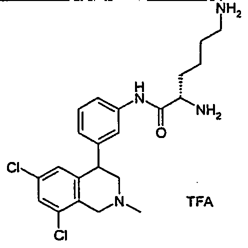 |
| 135 | 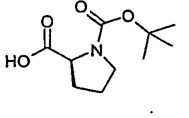 | 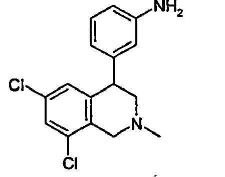 | 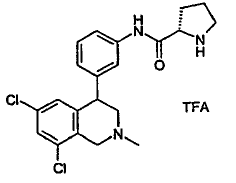 |
| 136 | 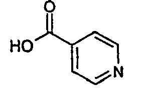 | 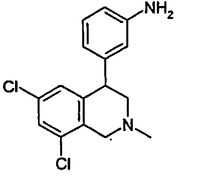 | 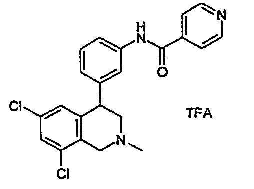 |
| 137 | 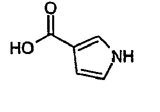 | 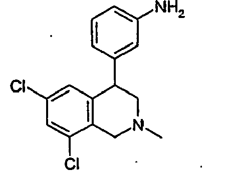 | 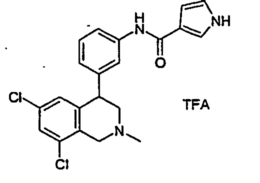 |
| 138 | 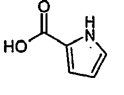 | 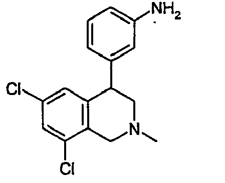 | 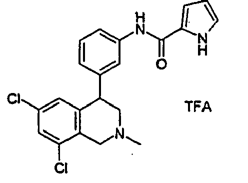 |
| 139 | 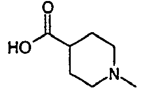 | 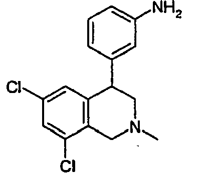 | 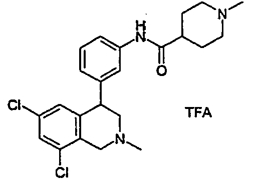 |
| 140 | 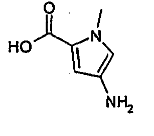 | 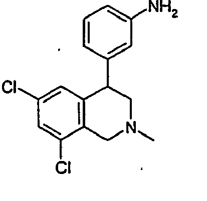 | 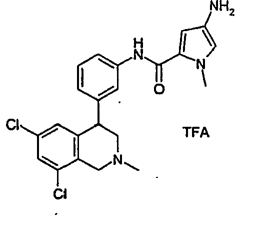 |
| 141 | 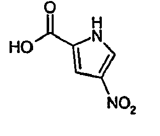 | 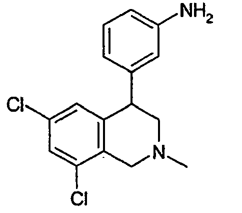 | 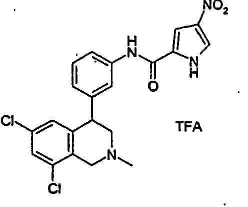 |
| 142 | 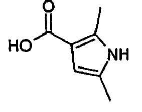 | 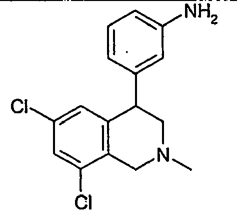 | 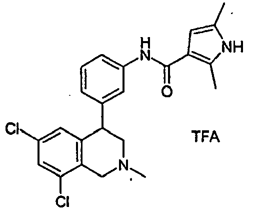 |
| 143 | 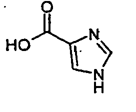 | 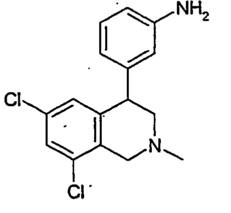 | 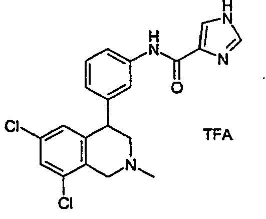 |
| 144 | 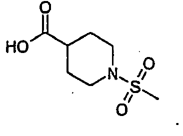 | 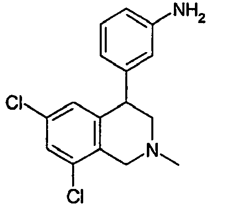 | 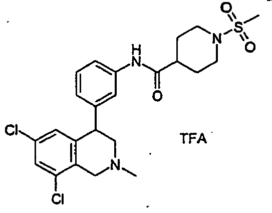 |
| 145 | 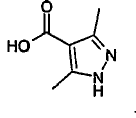 | 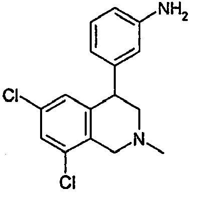 | 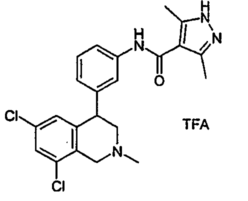 |
| 146 | 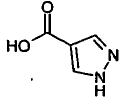 | 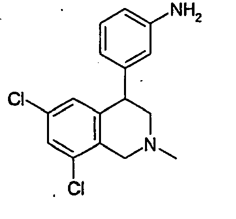 | 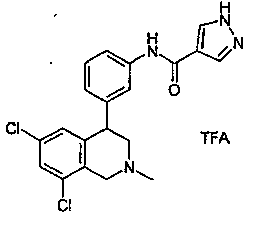 |
| 147 | 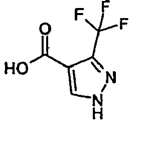 | 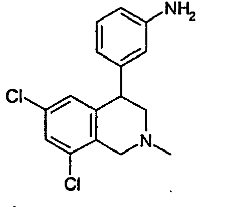 | 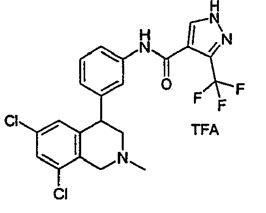 |

### Beispiel 148: 1-[2-(6,8-Dichloro-2-methyl-1,2,3,4-tetrahydro-isochinolin-4-yl)-phenyl]-3-ethyl- thioharnstoff, Hydrochloride;

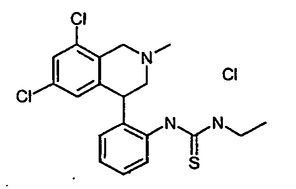

2-(6,8-Dichloro-2-methyl-1,2,3,4-tetrahydro-isochinolin-4-yl)-phenylamin (95mg, Beispielverbindung 36) wird in 4 ml Acetonitril vorgelegt und unter Rühren mit 27 mg Ethylisothiocyanat zugegeben. Nach Stehen für 15 Stunden bei Raumtemperatur wird das Lösungsmittel i. Vak. entfernt und der Rückstand an einer präp. HPLC gereinigt.

Das so erhaltene Trifluoracetat wird in Wasser aufgenommen und mit K₂CO₃ alkalisch gestellt. Die wässrige Phase wird mit Essigester extrahiert. Die organische Phase wird abgetrennt, mit MgSO₄ getrocknet und eingeengt. Der Rückstand wird in verdünnter HCl aufgenommen und gefriergetrocknet, wobei 36 mg der Titelverbindung erhalten werden.

### Beispiel 149: 1-[4-(6,8-Dichloro-2-methyl-1,2,3,4-tetrahydro-isochinolin-4-yl)-phenyl]-3-ethyl-thioharnstoff, Hydrochloride;

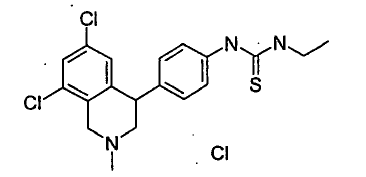

4-(6,8-Dichloro-2-methyl-1,2,3,4-tetrahydro-isochinolin-4-yl)-phenylamin (50 mg, Beispielverbindung 17, Zwischenprodukt 1) werden in 4 ml THF vorgelegt und mit Ethylisothiocyanat (14 mg) versetzt. Nachdem 2 Stunden zum Rückfluß erhitzt wurde, wird die Reaktionslösung aufkonzentriert und 2 Stunden auf 85 °C erhitzt. Das so erhaltene Rohprodukt wird an einer präp. HPLC gereinigt. Weitere Behandlung des so erhaltenen Trifluoracetats wie in Beispiel 148 beschrieben liefert nach Gefriertrocknung 33 mg des gewünschten Hydrochlorids.

### Beispiel 150: 1-[3-(6,8-Dichloro-2-methyl-1,2,3,4-tetrahydro-isochinolin-4-yl)-phenyl]-3-ethyl-thioharnstoff, Trifluoracetat;

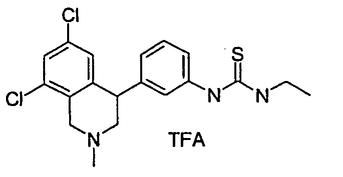

3-(6,8-Dichloro-2-methyl-1,2,3,4-tetrahydro-isochinolin-4-yl)-phenylamin (50 mg, Beispielverbindung 35) werden in 3 ml THF gelöst und unter Rühren mit 14 mg Ethylisothiocyanat verstezt. Nachdem 2 Stunden zum Rückfluß erhitzt wurde, wird die Reaktionslösung aufkonzentriert und 2 Stunden auf 85 °C erhitzt. Das so erhaltene Rohprodukt wird an einer präp. HPLC gereinigt, wobei 66 mg der Titelverbindung werden.

### Beispiel 151: 3-[3-(6,8-Dichloro-2-methyl-1,2,3,4-tetrahydro-isochinolin-4-yl)-phenyl]-1,1-dimethyl-harnstoff, Hydrochlorid;

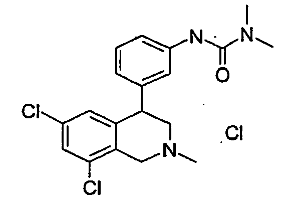

Zwischenprodukt 1: [3-(6,8-Dichloro-2-methyl-1,2,3,4-tetrahydro-isochinolin-4-yl)-phenyl]-carbaminsäure-4-nitro-phenyl ester, Hydrochloride; 3-(6,8-Dichloro-2-methyl-1,2,3,4-tetrahydro-isochinolin-4-yl)-phenylamin (350 mg, Beispielverbindung 35) werden in 17,5 ml Dichlormethan gelöst und unter Rühren mit 230 mg Chlorameisensäure-4-nitrophenylester versetzt. Nach 4,5 Stunden wurden weitere 0,1 Äquivalente (23 mg) Chlorameisensäure-4-nitrophenylester zugegeben und die Lösung über Nacht gerührt. Zur Aufarbeitung wird der entstandene Niederschlag abfiltriert und mit Dichlormethan gewaschen. Die so erhaltene Titelverbindung kann ohne weitere Reinigung weiter umgesetzt werden.

### 3-[3-(6,8-Dichloro-2-methyl-1,2,3,4-tetrahydro-isochinolin-4-yl)-phenyl]-1,1-dimethylharnstoff, Hydrochlorid;

35 mg [3-(6,8-Dichloro-2-methyl-1,2,3,4-tetrahydro-isochinolin-4-yl)-phenyl]-carbaminsäure-4-nitro-phenyl-ester-Hydrochloride (Zwischenprodukt 1) werden in 3,5 ml Dichlormethan suspendiert und unter Rühren eine Lösung von 3,7 mg Dimethylamin in 1 ml Dichlormethan zugetropft. Nach 1 Stunden wird mit Dichlormethan verdünnt und mit wässriger K₂CO₃ -lösung gewaschen. Die organische Phase wird getrennt und zweimal mit ges. K₂CO₃ -lsg. gewaschen, mit MgSO₄ getrocknet und eingeengt. Der Rückstand wird in verdünnter HCl aufgenommen und gefriergetrocknet, wobei 29 mg der Titelverbindung erhalten werden.

Die folgenden Beispiele werden analog zu der in Beispiel 151 beschriebenen Methode ausgehend von Zwischenprodukt 1 und den entsprechenden Aminkomponenten hergestellt:

**Tabelle 5:**

| Beispiel-Nr.: | Struktur |
|---|---|
| 152 | 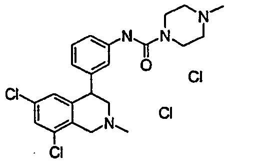 |
| 153 | 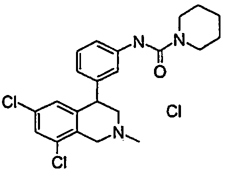 |
| 154 | 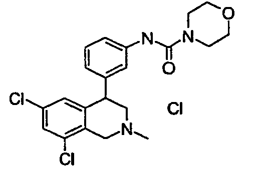 |
| 155 | 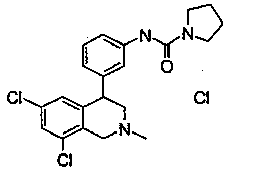 |
| 156 | 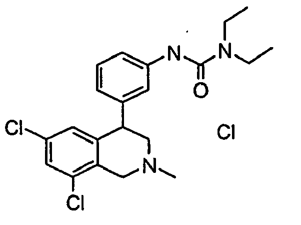 |
| 157 | 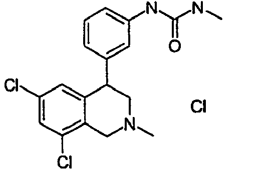 |
| 158 | 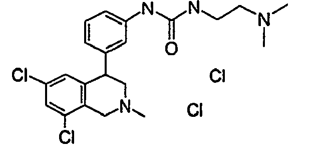 |

Die folgenden Beispiele wurden analog zu der in Beispiel 151 beschriebenen Methode hergestellt. Als Lösungsmittel diente THF, die Reaktionen wurden im geschlossenen Reaktionsgefäß durchgeführt. Bei den Beispielen 159 bis 166 waren Reaktionstemperaturen von 85 °C notwendig. Beispeilverbindung 167 wurde durch präp. HPLC gereinigt.

**Tabelle 6:**

| Beispiel-Nr.: | Struktur |
|---|---|
| 159 | 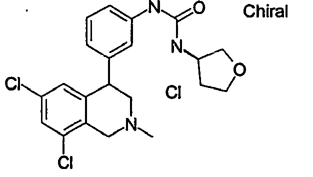 |
| 160 | 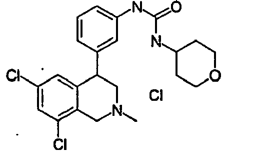 |
| 161 | 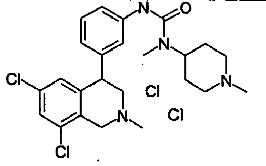 |
| 162 | 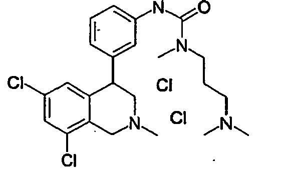 |
| 163 | 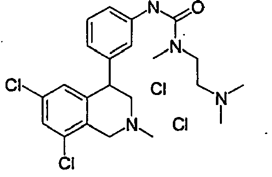 |
| 164 | 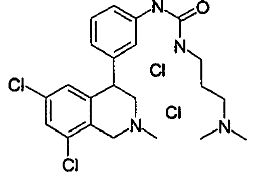 |
| 165 | 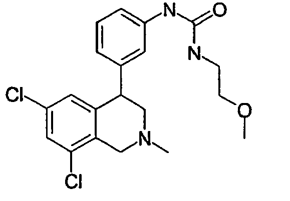 |
| 166 | 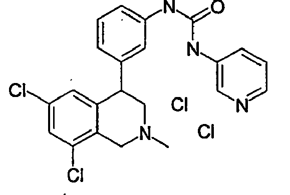 |
| 167 | 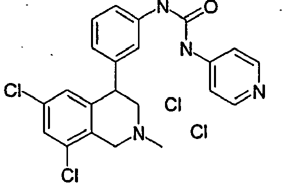 |

### Beispiel 168: 4-Methyl-piperazin-1-carbonsäure-[2-(6,8-dichloro-2-methyl-1,2,3,4-tetrahydro- isochinolin-4-yl)-phenyl]-amid, Hydrochlorid;

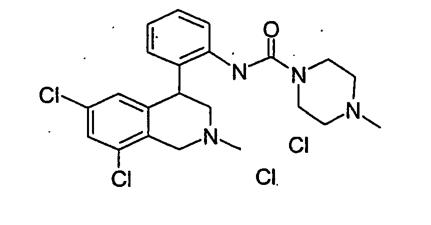

Zwischenprodukt 1: [2-(6,8-Dichloro-2-methyl-1,2,3,4-tetrahydro-isochinolin-4-yl)-phenyl]-carbaminsäure-4-nitro-phenyl-ester, Hydrochloride; 2-(6,8-Dichloro-2-methyl-1,2,3,4-tetrahydro-isochinolin-4-yl)-phenylamin (200 mg, Beispielverbindung 36) werden in 10 ml Dichlormethan gelöst und unter Rühren mit 131 mg Chlorameisensäure-4-nitrophenylester versetzt. Nach 3,5 Stunden wird der entstandene Niederschlag abgesaugt und mit Dichlormethan gewaschen. Das so erhaltene Rohprodukt wird aus Dichlormethan umkristallisiert, wobei 159 mg der Titelverbindung erhalten werden.

### 4-Methyl-piperazin-1-carbonsäure-[2-(6,8-dichloro-2-methyl-1,2,3,4-tetrahydroisochinolin-4-yl)-phenyl]-amid, Hydrochlorid;

15 mg [2-(6,8-Dichloro-2-methyl-1,2,3,4-tetrahydro-isochinolin-4-yl)-phenyl]-carbaminsäure-4-nitro-phenyl-ester-Hydrochloride weren in 2 ml Dichlormethan suspendiert und mit einer Lösung aus 3,2 mg 1-Methyl-piperazin in 1 ml Dichlormethan versetzt. Nach 1 Stunden wird mit Dichlormethan verdünnt und mit wässriger K₂CO₃ - lösung gewaschen. Die organische Phase wird getrennt und zweimal mit ges. K₂CO₃ - lsg. gewaschen, mit MgSO₄ getrocknet und eingeengt. Der Rückstand wird in verdünnter HCl aufgenommen und gefriergetrocknet, wobei 13 mg der Titelverbindung erhalten werden.

Die nachfolgenden Beispiele werden analog der unter Beispiel 168 beschrieben Methode hergestellt.

**Tabelle 7:**

| Beispiel-Nr.: | Struktur |
|---|---|
| 169 | 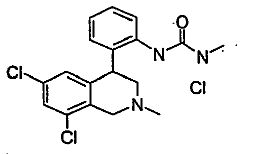 |
| 170 | 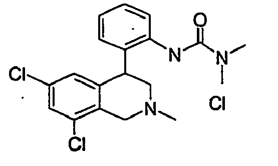 |
| 171 | 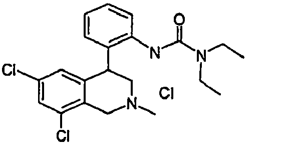 |
| 172 | 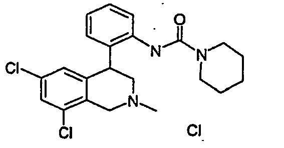 |
| 173 | 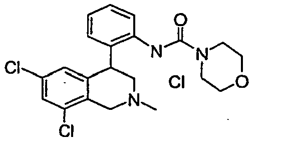 |
| 174 | 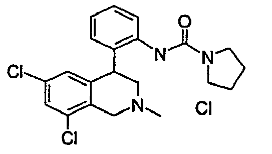 |
| 175 | 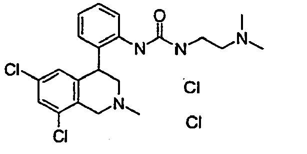 |

### Beispiel 176: 4-Methyl-piperazin-1-carbonsäure-[4-(6,8-dichloro-2-methyl-1,2,3,4-tetrahydro- isochinolin-4-yl)-phenyl]-amid, Hydrochloride;

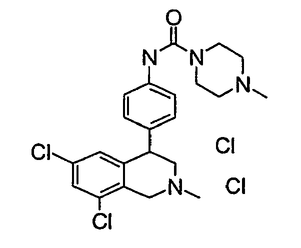

Zwischenprodukt 1: [4-(6,8-Dichloro-2-methyl-1,2,3,4-tetrahydro-isochinolin-4-yl)-phenyl]-carbaminsäure-4-nitro-phenyl-ester, Hydrochloride; 4-(6,8-Dichloro-2-methyl-1,2,3,4-tetrahydro-isochinolin-4-yl)-phenylamin (200 mg, Beispielverbindung 17, Zwischenprodukt 1) werden in 10 ml Dichlormethan gelöst und unter Rühren mit 131 mg Chlorameisensäure-4-nitrophenylester versetzt. Nach 4,5 Stunden wird der entstandene Niederschlag abgesaugt und mit Dichlormethan gewaschen. Das so erhaltene Rohprodukt wird zweimal aus Dichlormethan umkristallisiert, wobei 254 mg der Titelverbindung erhalten werden.

### 4-Methyl-piperazin-1-carbonsäure-[4-(6,8-dichloro-2-methyl-1,2,3,4-tetrahydroisochinolin-4-yl)-phenyl]-amid, Hydrochloride;

15 mg [4-(6,8-Dichloro-2-methyl-1,2,3,4-tetrahydro-isochinolin-4-yl)-phenyl]-carbaminsäure-4-nitro-phenyl-ester-Hydrochlorid werden in 2 ml Dichlormethan suspendiert und mit einer Lösung aus 3,2 mg 1-Methyl-piperazin in 1 ml Dichlormethan versetzt. Nach 5 Stunden rühren und Stehen über Nacht wird mit Dichlormethan verdünnt und mit wässriger K₂CO₃ -lösung gewaschen. Die organische Phase wird getrennt und zweimal mit ges. K₂CO₃ -lsg. gewaschen, mit MgSO₄ getrocknet und eingeengt. Der Rückstand wird in verdünnter HCl aufgenommen und gefriergetrocknet, wobei 13 mg der Titelverbindung erhalten werden.

Die nachfolgenden Beispiele werden analog der unter Beispiel 176 beschrieben Methode hergestellt.

**Tabelle 8**

| Beispiel-Nr.: | Struktur |
|---|---|
| 177 | 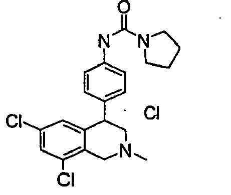 |
| 178 | 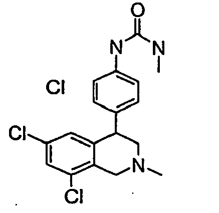 |
| 179 | 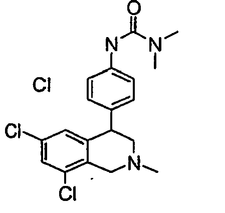 |
| 180 | 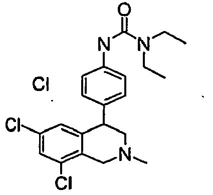 |
| 181 | 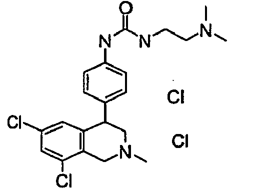 |
| 182 | 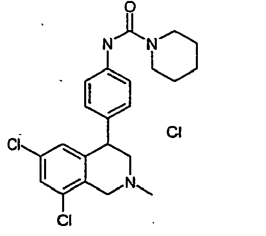 |
| 183 | 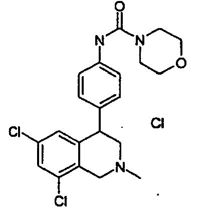 |

### Beispiel 184: N-[4-(6,8-Dichloro-2-methyl-1,2,3,4-tetrahydro-isochinolin-4-yl)-phenyl]formamid, Hydrochlorid;

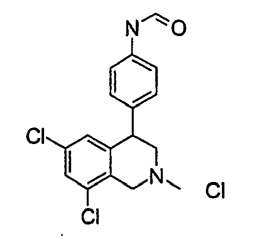

4-(6,8-Dichloro-2-methyl-1,2,3,4-tetrahydro-isochinolin-4-yl)-phenylamin (200 mg, Beispielverbindung 17, Zwischenprodukt 1) wird in 1 ml Ameisensäure gelöst und 15 min zum Rückfluß erhitzt. Nach Stehen über Nacht wird der Ansatz auf ein Eis/Wasser-Gemisch gegeben und zweimal mit Essigester extrahiert. Die vereinigten organischen Phasen werden mit MgSO₄ getrocknet und eingeengt. Der Rückstand wird in Dichlormethan aufgenommen und mit ges. NaHCO₃-lsg. gewaschen. Die Phasen werden getrennt und die wässrigen noch dreimal mit Dichlormethan extrahiert. Trocknen der organischen Phasen (MgSO₄) und Abdestillieren des Lösungsmittels ergibt 167 mg Rohprodukt. 10 mg werden in verdünnter HCl gelöst und gefriergetrocknet wobei 11 mg der Titelverbindung erhalten werden.

### Beispiel 185: [4-(6,8-Dichloro-2-methyl-1,2,3,4-tetrahydro-isochinolin-4-yl)-phenyl]-methyl-amin, Hydrochlorid

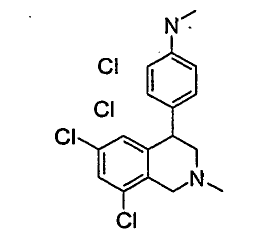

N-[4-(6,8-Dichloro-2-methyl-1,2,3,4-tetrahydro-isochinolin-4-yl)-phenyl]formamid (150 mg, Beispielverbindung 184) werden in 2,5 ml THF gelöst und bei 50 °C unter Argon zu einer Lösung aus 0,45 ml einer 1 M Lösung von Lithiumaluminiumhydrid/THF in 2,5 ml THF getropft. Es wird 1 Stunde zum Rückfluß erhitzt. Nach Stehen über Nacht werden bei 50 °C weitere 0,22 ml einer 1 M Lithiumaluminiumhydrid-Lösung zugegeben und eine weitere Stunde zum Rückfluß erhitzt. Zur Aufarbeitung wird das Lösungsmittel entfernt und der Rückstand zwischen Dichlormethan und wässriger HCl verteilt. Die Phasen werden getrennt und die wässrigen dreimal mit Dichlormethan extrahiert, Die vereinigten organischen Phasen werden mit MgSO₄ getrocknet und eingeengt. Die weitere Reinigung geschieht an einer präp. HPLC. Das so erhaltene Produkt wird in NaHCO₃-Lösung aufgenommen und mit Dichlormethan extrahiert. Trocknen der organischen Phase mit MgSO₄ liefert 80 mg der freien Base. 10 mg werden in verdünnter HCl aufgenommen und gefriertgetrocknet wobei 10 mg der Titelverbindung erhalten werden.

### Beispiel 186: 1-[4-(6,8-Dichloro-2-methyl-1,2,3,4-tetrahydro-isochinolin-4-yl)-phenyl]-1,3-dimethyl- harnstoff, Hydrochloride;

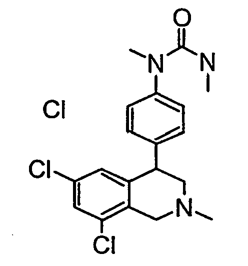

Die Titelverbindung wird nach der unter Beispiel 151 beschriebenen Methode ausgehend von [4-(6,8-dichloro-2-methyl-1,2,3,4-tetrahydro-isochinolin-4-yl)-phenyl]-methyl-amin (Beispiel 185), 4-Nitrophenylchloroformat und Methylamin (20 µl, 2 M in THF) hergestellt, wobei 9 mg des gewünschten Hydrochlorids erhalten werden.

Analog zu Beispiel 186 wurden folgende Verbindungen ausgehend von [4-(6,8-dichloro-2-methyl-1,2,3,4-tetrahydro-isochinolin-4-yl)-phenyl]-methyl-amin (Beispiel 185), 4-Nitrophenylchloroformat und den entsprechenden Aminkomponenten hergestellt:

**Tabelle 9**

| Beispiel-Nr. | Struktur |
|---|---|
| 187 | 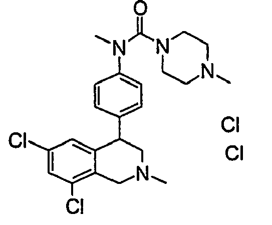 |
| 188 | 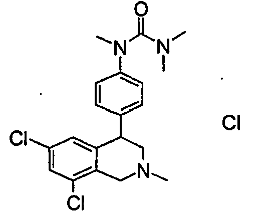 |
| 189 | 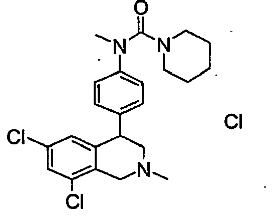 |
| 190 | 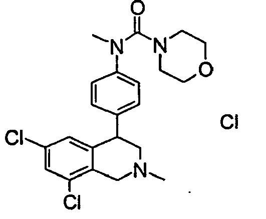 |
| 191 | 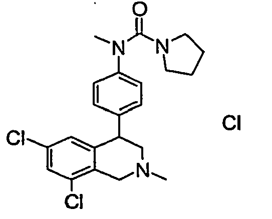 |
| 192 | 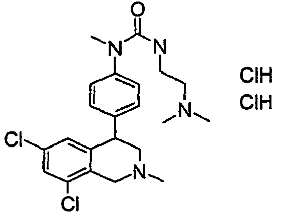 |
| 193 | 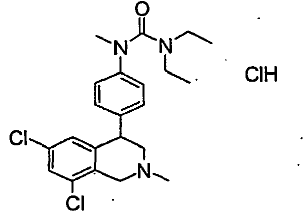 |

### Beispiel 194: N-[3-(6,8-Dichloro-2-methyl-1,2,3,4-tetrahydro-isochinolin-4-yl)-phenyl]-formamid

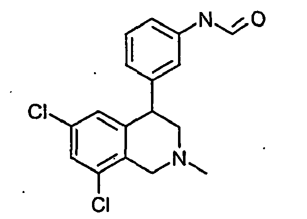

3-(6,8-Dichloro-2-methyl-1,2,3,4-tetrahydro-isochinolin-4-yl)-phenylamin (600 mg, Beispielverbindung 35) wird in 2,4 ml Ameisensäure gelöst und 15 min zum Rückfluß erhitzt. Nach Stehen über Nacht wird wird der Ansatz auf ein Gemisch aus Eis/Wasser und ges. NaHCO₃-Lsg. gegeben und dreimal mit Dichlormethan extrahiert. Die vereingten organischen Phasen werden mit MgSO₄ getrocknet und eingeengt, wobei 588 mg der Titelverbindung erhalten werden.

### Beispiel 195: [3-(6,8-Dichloro-2-methyl-1,2,3,4-tetrahydro-isochinolin-4-yl)-phenyl]-methyl-amin, Hydrochlorid;

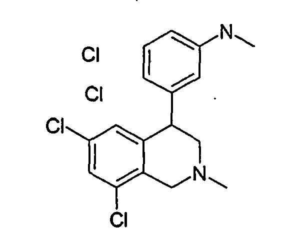

N-[3-(6,8-Dichloro-2-methyl-1,2,3,4-tetrahydro-isochinolin-4-yl)-phenyl]formamid (588 mg, Beispielverbindung 194) wird in 10 ml THF gelöst und bei 50 °C unter Argon zu einer Lösung aus 1,8 ml einer 1 M Lösung von Lithiumaluminiumhydrid in THF gegeben. Es wird 1 Stunde zum Rückfluß erhitzt. Nach Stehen über Nacht werden bei 50 °C weitere 2 ml einer 1 M Lithiumaluminiumhydrid-Lösung zugegeben und weitere 30 min zum Rückfluß erhitzt. Zur Aufarbeitung wird mit Eis versetzt und die wässrige Phase viermal mit Essigester extrahiert. Die vereinigten organischen Phasen werden mit MgSO₄ getrocknet und eingeengt. Die weitere Reinigung geschieht an einer präp. HPLC. Das so erhaltene Produkt wird in NaHCO₃-Lösung aufgenommen und mit Essigester extrahiert. Trocknen der organischen Phase mit MgSO₄ liefert 270 mg der freien Base. 45 mg werden in verdünnter HCl aufgenommen und gefriertgetrocknet wobei 45 mg der Titelverbindung erhalten werden.

Nach der unter Beispiel 151 beschriebenen Methode können die folgenden Beispielverbindungen, ausgehen von [3-(6,8-Dichloro-2-methyl-1,2,3,4-tetrahydroisochinolin-4-yl)-phenyl]-methyl-amin (Beispiel 195), 4-Nitrochloroformat und den entsprechenden Aminkomponenten hergestellt werden:

**Tabelle 10:**

| Beispiel-Nr. | Struktur |
|---|---|
| 196 | 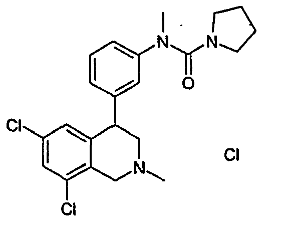 |
| 197 | 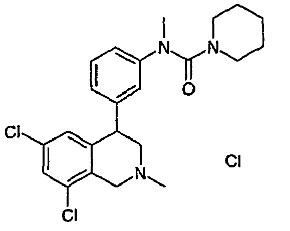 |
| 198 | 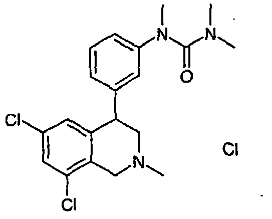 |
| 199 | 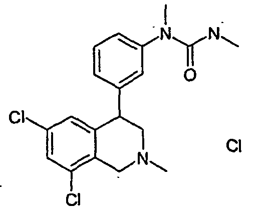 |
| 200 | 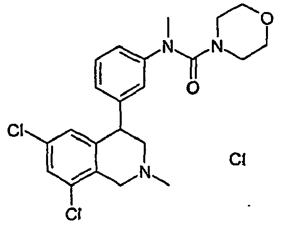 |
| 201 | 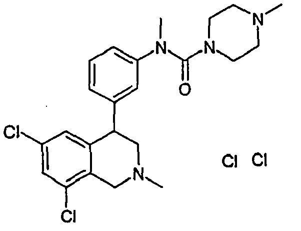 |
| 202 | 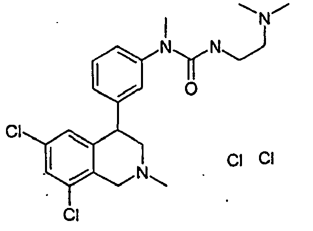 |
| 203 | 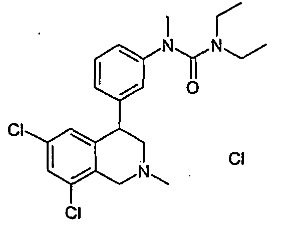 |

### Beispiel 204: [3-(6,8-Dichloro-2-methyl-1,2,3,4-tetrahydro-isochinolin-4-yl)-phenyl]-carbaminsäure-2-dimethylamino-ethyl-ester, Hydrochlorid;

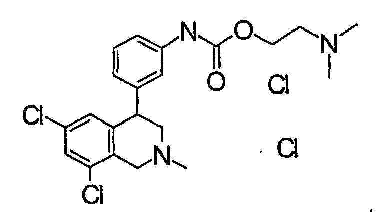

Unter Rühren und Argonatmosphäre werden 15 mg [3-(6,8-dichloro-2-methyl-1,2,3,4-tetrahydro-isochinolin-4-yl)-phenyl]-carbaminsäure-4-nitro-phenyl-ester-hydrochlorid (s. Beispiel 151, Zwischenprodukt 1) in 1,5 ml Dichlormethan suspendiert und mit einer Lösung von 3 mg 2-Dimethylaminoethanol in 0,5 ml Dichlormethan versetzt und 6 Stunden gerührt. Nach Stehen über Nacht werden Wasser, Dichlormethan und ges. NaHCO₃-Lösung zugefügt und die organische Phase getrennt. Die Dichlormethanphase wird dreimal mit ges. NaHCO₃-Lösung gewaschen, mit MgSO₄ getrocknet und das Lösungsmittel i. Vak. entfernt. Das so erhaltene Rohprodukt wird an einer präp. HPLC gereinigt. Die Produktfraktionen werden eingeengt und zwischen Essigester und ges. NaHCO₃-Lösung verteilt. Die organische Phase wird abgetrennt, mit MgSO₄ getrocknet und eingeengt. Der Rückstand wird in verd. HCl aufgenommen und gefriergetrocknet, wobei 5 mg der Titelverbindung erhalten werden.

In analoger Weise werden die entsprechenden Isomere [4-(6,8-Dichloro-2-methyl-1,2,3,4-tetrahydro-isochinolin-4-yl)-phenyl]-carbaminsäure-2-dimethylamino-ethylester-Hydrochlorid und [2-(6,8-Dichloro-2-methyl-1,2,3,4-tetrahydro-isochinolin-4-yl)-phenyl]-carbaminsäure-2-dimethylamino-ethyl-ester-Hydrochlorid ausgehend von [4-(6,8-dichloro-2-methyl-1,2,3,4-tetrahydro-isochinolin-4-yl)-phenyl]-carbaminsäure-4-nitro-phenyl-ester-hydrochlorid und [2-(6,8-dichloro-2-methyl-1,2,3,4-tetrahydroisochinolin-4-yl)-phenyl]-carbaminsäure-4-nitro-phenyl-ester-hydrochlorid hergestellt.

**Tabelle 11:**

| Beispiel-Nr. | Struktur |
|---|---|
| 205 | 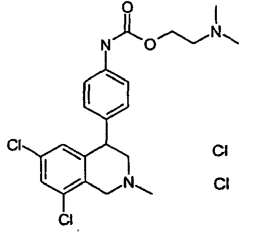 |
| 206 | 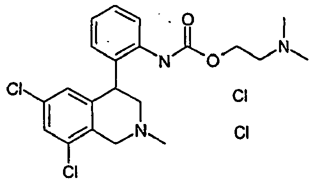 |

### Beispiel 207: [3-(6,8-Dichloro-2-methyl-1,2,3,4-tetrahydro-isochinolin-4-yl)-phenyl]-carbamin-säure-methyl-ester, Hydrochlorid;

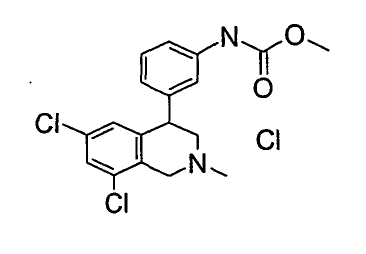

Unter Argonatmosphäre werden unter Rühren 15 mg 3-(6,8-dichloro-2-methyl-1,2,3,4-tetrahydro-isochinolin-4-yl)-phenylamin (Beispiel 35) in 1,5 ml Dichlormethan vorgelegt und mit einer Lösung 4,6 mg Methylchloroformat in 0,5 ml Dichlormethan versetzt. Nach 6 Stunden Rühren und Stehen über Nacht werden weitere 2,3 mg Methylchloroformat zugegeben und für 5 Stunden gerührt. Zur Aufarbeitung wird vom Lösungsmittel befreit, der Rückstand in verdünnter HCl aufgenommen und gefriergetrocknet, wobei 20 mg der Titelverbindung erhalten werden.

In analoger Weise können die folgenden Carbamate ausgehend von den entsprechenden Anilinen 3-(6,8-dichloro-2-methyl-1,2,3,4-tetrahydro-isochinolin-4-yl)-phenylamin, bzw. 4-(6,8-dichloro-2-methyl-1,2,3,4-tetrahydro-isochinolin-4-yl)-phenylamin hergestellt werden.

**Tabelle 12:**

| Beispiel-Nr. | Struktur |
|---|---|
| 208 | 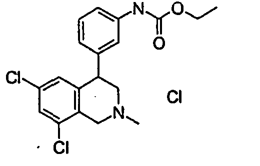 |
| 209 | 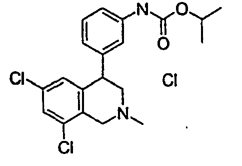 |
| 210 | 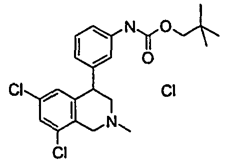 |
| 211 | 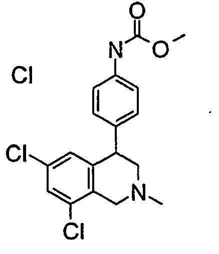 |
| 212 | 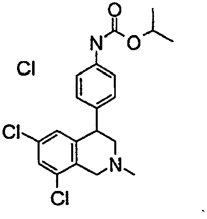 |
| 213 | 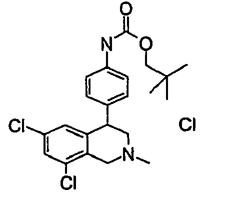 |
| 214 | 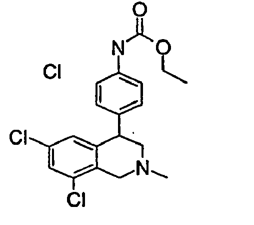 |

### Beispiel

### 215a: (+)-N-[3-(6,8-Dichloro-2-methyl-1,2,3,4-tetrahydro-isochinolin-4-yl)-phenyl]-methanesulfonamid, Hydrochlorid;

### 215b: (-)-N-[3-(6,8-Dichloro-2-methyl-1,2,3,4-tetrahydro-isochinolin-4-yl)-phenyl]-methanesulfonamid, Hydrochlorid;

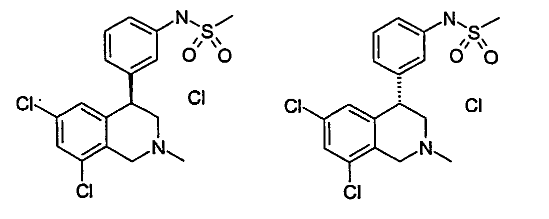

96 mg racemisches N-[3-(6,8-Dichloro-2-methyl-1,2,3,4-tetrahydro-isochinolin-4-yl)-phenyl]- methanesulfonamid (s. Beispiel 83) werden an einer chiralen präp. HPLC in die Enantiomere getrennt.
chirale Säule: Chiralpak AD 250 x 50 mm, 20 µ;
Lösungsmittel: Heptan:Ethanol:Methanol: 10:1:1;
Flussrate: 50 ml/min;

Die erhaltenen Enantiomere werden in verdünnter HCl gelöst und gefriergetrocknet, wobei jeweils 37 mg der Titelverbindungen 215a und 215b erhalten werden. Die Enantiomerenreinheit wird an einer chiralen HPLC bestimmt.
chirale Säule: Chiralpak AD-H/31 250 x 4,6 mm;
Lösungsmittel: Heptan:Ethanol:Methanol 10:1:1;
Flussrate: 1 ml/min;
Rt(erstes eluierendes Enantiomer) = 6,84 min, 100% ee;
Rt (zweites eluierendes Enantiomer) = 8,02 min, 100% ee.

### Beispiel

### 216a: (+)-1-[2-(6,8-Dichloro-2-methyl-1,2,3,4-tetrahydro-isochinolin-4-yl)-phenyl]-3-ethyl-harnstoff, Hydrochlorid;

### 216b: (-)-1-[2-(6,8-Dichloro-2-methyl-1,2,3,4-tetrahydro-isochinolin-4-yl)-phenyl]-3-ethyl-harnstoff, Hydrochlorid;

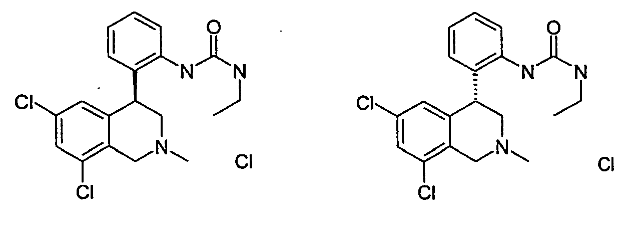

316 mg racemischer 1-[2-(6,8-Dichloro-2-methyl-1,2,3,4-tetrahydro-isochinolin-4-yl)-phenyl]-3-ethyl-harnstoff (Beispielverbindung 80) werden an einer chiralen präp. HPLC in die Enantiomere getrennt.
chirale Säule: Chiralpak OD 250 x 50 mm, 20 µ;
Lösungsmittel: Heptan:Ethanol:iso-Propanol: 50:2:1; 0,3% Diethylammin
Flussrate: 50 ml/min;

Die Enantiomere werden separat einer weiteren Reinigung an einer präp. HPLC unterzogen. Die erhaltenen Produkte werden zwischen ges. NaHCO₃-Lösung und Essigester verteilt, die organsiche Phase wird abgetrennt, mit MgSO₄ getrocknet und vom Lösungsmittel befreit. Lösen der Rückstände in verd. HCl und Gefriertrocknung liefert 37 mg des ersteluierenden und 58 mg des zweiteluierenden Enantiomers. Die Enantiomerenreinheit wird durch analytische HPLC bestimmt.
chirale Säule: Chiralpak OD-20, 250 x 4,6 mm;
Lösungsmittel: Heptan:Ethanol:Isopropanol 50:2:1 (0,3% Diethylamin);
Flussrate: 1 ml/min;
Rₜ(erstes eluierendes Enantiomer) =9,22 min, 100% ee;
Rt (zweites eluierendes Enantiomer) = 9,96 min, 98% ee.

### Beispiel 217: N-[3-(6,8-Difluoro-2-methyl-1,2,3,4-tetrahydro-isochinolin-4-yl)-phenyl]-acetamid, Hydrochlorid;

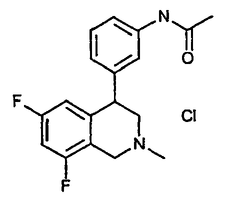

Zwischenprodukt 1: 2,4-Difluorbenzyl-methyl-amin
Ausgehend von 2,4-Difluorbenzaldehyd kann 2,4-Difluorbenzyl-methyl-amin in dem Fachmann bekannter Weise hergestellt (vgl. Beispiel1, Zwischenprodukt 1) werden.

### N-[3-(6,8-Difluoro-2-methyl-1,2,3,4-tetrahydro-isochinolin-4-yl)-phenyl]-acetamid, Hydrochlorid;

Ausgehend von N-(3-Acetyl-phenyl)-acetamid und 2,4-Difluorbenzyl-methyl-amin (Zwischenprodukt 1) läßt sich die Titelverbindung nach der in Beispiel 1 beschriebenen Syntheseroute herstellen.

### Beispiel 218: 4-(3-Bromo-phenyl)-6,8-dichloro-2-methyl-1,2,3,4-tetrahydro-isochinolin, Hydrochlorid;

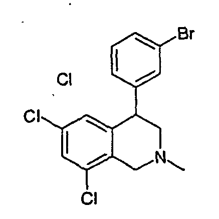

Ausgehend von 2,4-Dichlorbenzyl-methyl-amin (s. Beispiel 1) und 2-Brom-1-(3-bromphenyl)-ethanon als Alkylierungsmittel kann die Titelverbindung nach der in Beispiel 1 beschriebenen Syntheseroute hergestellt werden.

### Beispiel 219: 1-[3-(6,8-Dichloro-2-methyl-1,2,3,4-tetrahydro-isochinolin-4-yl)-phenyl]-3-(2-hydroxy-ethyl)-harnstoff

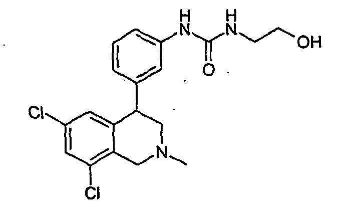

509 mg (1 mmol) [3-(6,8-Dichloro-2-methyl-1,2,3,4-tetrahydro-isochinolin-4-yl)-phenyl]-carbaminsäure-4-nitro-phenyl ester-Hydrochloride (Beispielverbindung 151, Zwischenprodukt 1) werden in 15 ml abs. DMF gelöst und bei 0 °C mit einer Lösung aus 67,2 mg (1,1 mmol) 2-Aminoethanol in 10 ml DMF versetzt. Es wird drei Stunden bei Raumtemperatur gerührt und dann vom Lösungsmittel i. Vak. befreit. Der Rückstand wird zwischen Essigester und ges. NaHCO₃-Lösung verteilt. Die organische Phase wird abgetrennt und die wässrige noch zweimal mit Essigester extrahiert. Die vereinten organischen Phasen werden mit ges. NaCl-Lösung gewaschen, mit MgSO₄ getrocknet ein eingeengt. Chromatographie an Kieselgel (Dichlormethan/Methanol) liefert 265 mg der Titelverbindung.

### Beipsiel 220: 3-(6,8-Dichloro-2-methyl-1,2,3,4-tetrahydro-isochinolin-4-yl)-benzoesäure-ethylester;

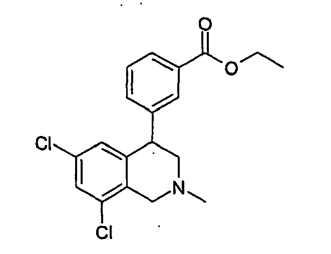

Zwischenprodukt 1: 3-Acetyl-benzoesäure wird in dem Fachmann bekannter Weise aus 3-Acetylbenzonitril durch Verseifung der Nitrilgruppe hergestellt.

Zwischenprodukt 2: 3-Acetyl-benzoesäure-ethylester wird aus Zwischenprodukt 1 in dem Fachmann bekannter Weise hergestellt.

Zwischenprodukt 3: 3-(2-Brom-acetyl)-benzoesäure-ethylester wird analog zu der in Beispiel 1, Zwischenprodukt 2 beschriebenen Methode aus 3-Acetyl-benzoesäure-ethylester (Zwischenprodukt 2) synthetisiert.

3-(6,8-Dichloro-2-methyl-1,2,3,4-tetrahydro-isochinolin-4-yl)-benzoesäure-ethylester, Analog zu der unter Beispiel 1 beschriebenen Syntheseroute kann ausgehend von 3-(2-Brom-acetyl)-benzoesäure-ethylester (Zwischenprodukt 3) und 2,4-Dichlorbenzyl-methyl-amin (Beispiel 1, Zwischenprodukt 1) weiterverfahren werden, wobei nach Alkylierungsreaktion, Reduktion und Ringschlußreaktion der 3-(6,8-Dichloro-2-methyl-1,2,3,4-tetrahydro-isochinolin-4-yl)-benzoesäure-ethylester erhalten wird.

### Beispiel 221: 3-(6,8-Dichloro-2-methyl-1,2,3,4-tetrahydro-isochinolin-4-yl)-benzoesäure;

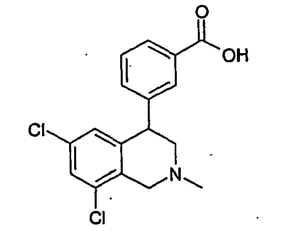

500 mg 3-(6,8-Dichloro-2-methyl-1,2,3,4-tetrahydro-isochinolin-4-yl)-benzoesäure-ethylester (Beispielverbindung 220) werden in 15 ml Methanol gelöst und mit 10 ml 2 N KOH versetzt. Nach einer Stunde bei 50 °C wird i. Vak. eingeengt und der Rückstand zwischen Wasser und Ether verteilt. Die Wasserphase wird mit 2 N HCl auf einen pH-Wert von ca. 6 eingestellt und der entstandene Niederschlag abgesaugt. Trocknen liefert 304 mg der Titelverbindung als farblosen Feststoff.

### Analytische Daten zu den Beispielverbindungen 1 bis 221:

**Tabelle 13:**

| Bsp. | Struktur | Rₜ[mi n] | Metho de | MS, [M+H⁺] | MS-Meth ode | Bemerkung |
|---|---|---|---|---|---|---|
| 1 | 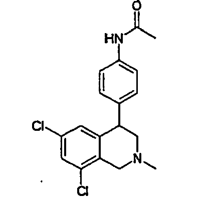 | 1,60 | B | 349,1/350,1/ 351,0 | ESI | Schmp.: 205-206 °C |
| 1a | 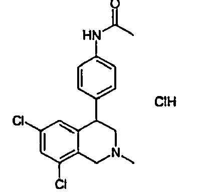 | 1,60 | B | 349,2/351,2 | ESI | Schmp.: 125 °C (Zers.) |
| 2a | 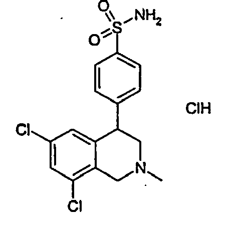 | 3,63 | A | 371,3/373,3 412,3/414,3 | ESI | (+)-Enantiomer |
| 2b | 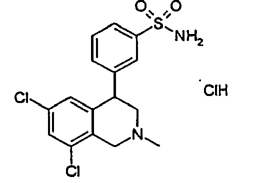 | 3,67 | A | 371,3/373,3 412,3/414,3 | ESI | (+)-Enantiomer |
| 2c | 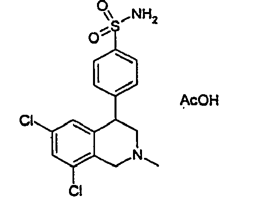 | 3,66 | A | 371,1/373,1 412,1/414,1 | ESI | (-)-Enantiomer |
| 2d | 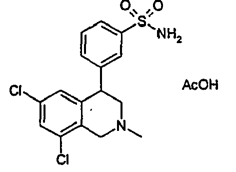 | 1,56 | B | 371,1/373,1 412,1/414,1 | ESI | (-)-Enantiomer |
| 3 | 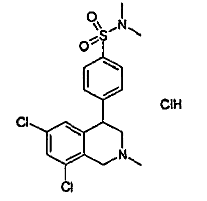 | 4,00 | A | 399,1/401,1 | Cl | |
| 4a | 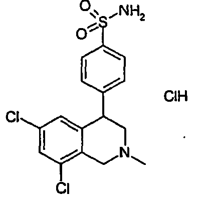 | 3,59 | A | 371,2/373,2 412,2/414,2 | ESI | |
| 4b | 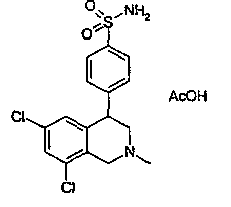 | 1,58 | B | 371,0/372,0/ 373,0/373,9 | ESI | |
| 5 | 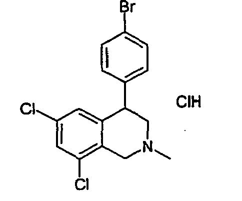 | 4,57 | A | 369,9/371,9/ 373,9 | Cl | |
| 6 | 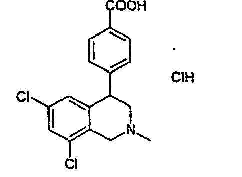 | 4,03 | A | 336,1/338,1 | Cl | |
| 7 | 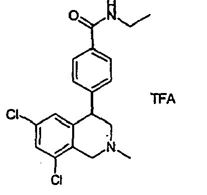 | 4,17 | A | 363,3/365,3 | Cl | |
| 8 | 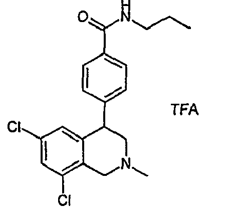 | 1,88 | B | 377,3/379,3 | Cl | |
| 9 | 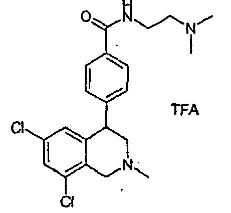 | 1,45 | B | 406,3/408,3 | Cl | |
| 10 | 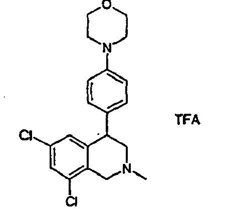 | 4,37 | A | 377.1/379,1 | Cl | |
| 11 | 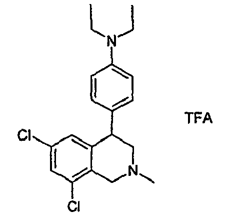 | 4,05 | A | 363,2/365,2 | ESI | |
| 12 | 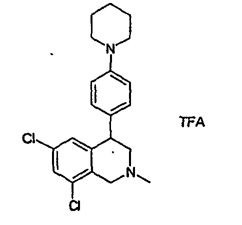 | 3,79 | A | 375,2/377,2 | ESI | |
| 13 | 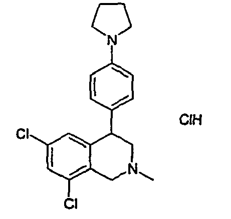 | 4,92 | A | 361,2/363,2 | ESI | |
| 14 | 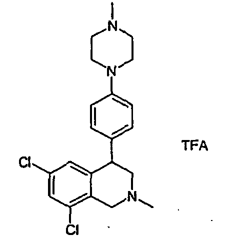 | 4,08 | A | 390,2/392,2 | ESI | |
| 15 | 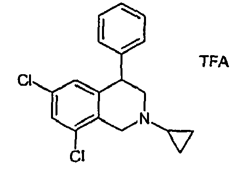 | 4,80 | A | 318,2/320,2 | Cl | |
| 16a | 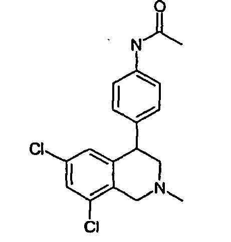 | 1,61 | B | 349,1/350,1/ 351,1 | ESI | (-)-Enantiomer |
| 16b | 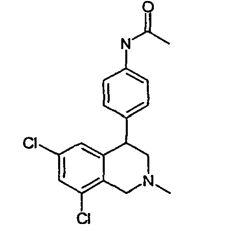 | 1,61 | B | 349,1/350,1/ 351,1 | ESI | (+)-Enantiomer |
| 17 | 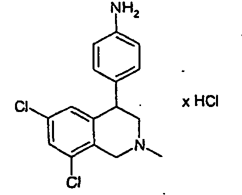 | 0,91 | B | 307,1/309,0 | ESI | |
| 18 | 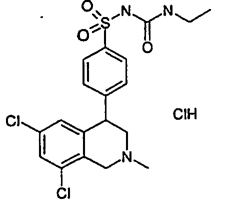 | 1,63 | B | 442,0/444,0 | ESI | |
| 19 | 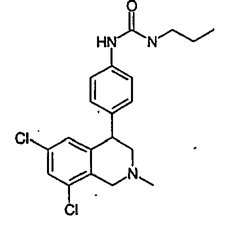 | 4,00 | A | 392,2/394,2 | ESI | |
| 19a | 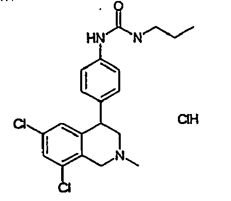 | 1,80 | B | 392,1/394,1 | ESI | |
| 20 | 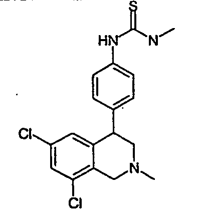 | 1,67 | B | 380,1/382,2 | ESI | |
| 21 | 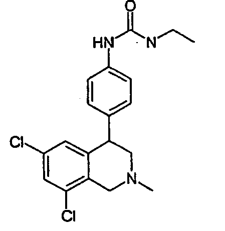 | 1,68 | B | 378,3/380,2 | ESI | Schmp.: 218-220 °C |
| 21a | 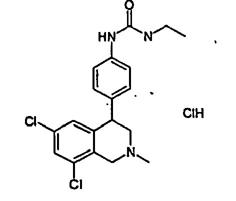 | 1,68 | B | 378,1/379,1/ 380,1 | ESI | |
| 22 | 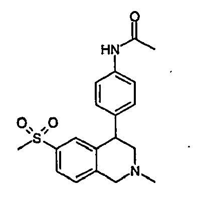 | 0,32 | B | 359,1 717,3/718,3/ 719,3 | ESI | |
| 23 | 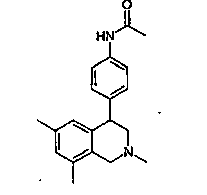 | 1,60 | B | 309,2/310,1 | ESI | |
| 24 | 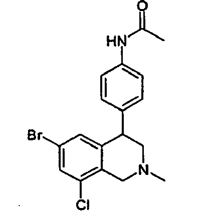 | 1,67 | B | 393,0/394,0/3 96,0/397,0 | ESI | |
| 25 | 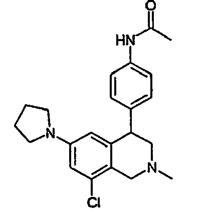 | 1,85 | B | 384,1/386,1 | ESI | |
| 25a | 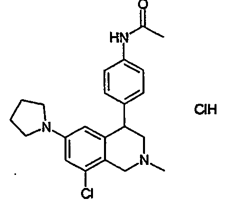 | 1,78 | B | 384,1/385,1/ 386,2 | ESI | |
| 26 | 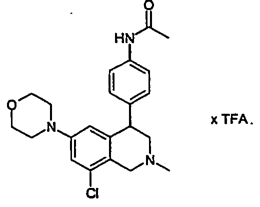 | 1,46 | B | 400,1/401,2/ 401,2 | ESI | |
| 27 | 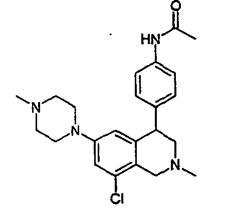 | 0.27 | B | 413,2/414,2/ 415,2 | ESI | |
| 27a | 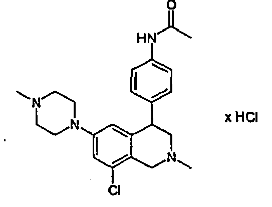 | 0,25 | B | 413,2/414,2/ 415,2 | ESI | |
| 28 | 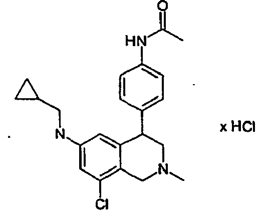 | 1,65 | B | 384,2/385,2 386,2 | ESI | |
| 29 | 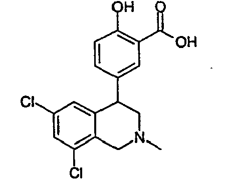 | 1,67 | B | 352,0/353,0/ 354,0 | ESI | |
| 29a | 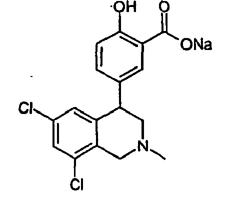 | 1,68 | B | 352,0/353,0/ 354,0 | ESI | |
| 30 | 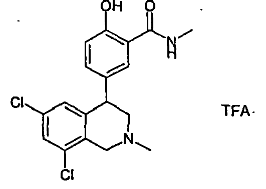 | 1,68 | B | 365,1/366,1/ 367,0/368,0 | ESI | |
| 31 | 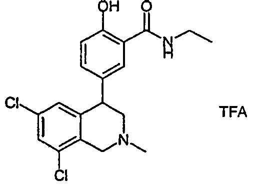 | 1,79 | B | 379,1/380,1/ 381,1 | ESI | |
| 32 | 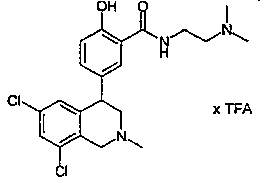 | 1,47 | B | 422,1/423,1/ 424,1/425,1 | ESI | |
| 33 | 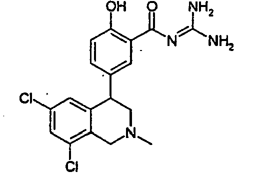 | 1,46 | B | 393,1/394,1/ 395,1 | ESI | |
| 34 | 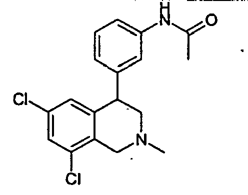 | 1,63 | B | 349,0/350,1/ 351,0 | ESI | |
| 35 | 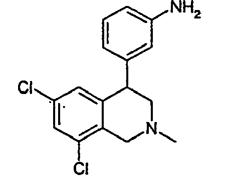 | 1,17 | B | 307,0/308,1/ 309,1 | ESI | |
| 36 | 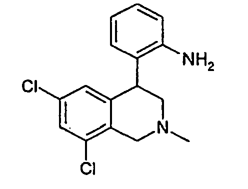 | 1,66 | B | 307,0/308,0/ 309,1 | ESI | |
| 37 | 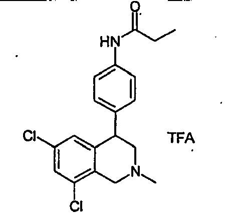 | 2,35 | C | 363,3/365,3 | ESI | |
| 38 | 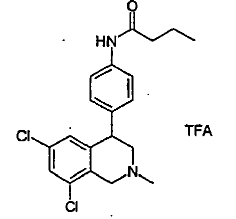 | 2,43 | C | 377,3/379,3 | ESI | |
| 39 | 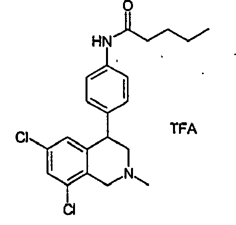 | 2,49 | C | 391,3/393,3 | ESI | |
| 40 | 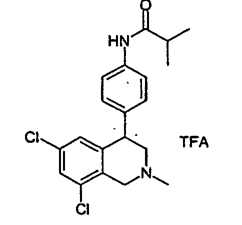 | 2,43 | C | 377,3/379,3 | ESI | |
| 41 | 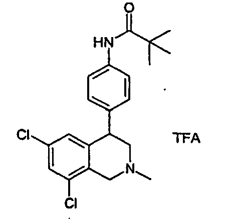 | 2,48 | C | 391,3/393,3 | ESI | |
| 42 | 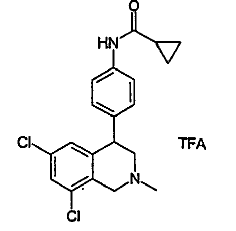 | 2,40 | C | 375,3/377,3 | ESI | |
| 43 | 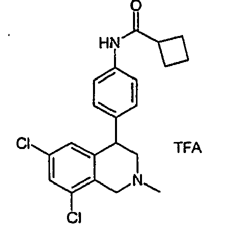 | 2,45 | C | 389,3/391,3 | ESI | |
| 44 | 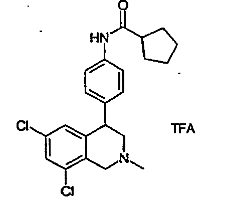 | 2,52 | C | 403,4/405,4 | ESI | |
| 45 | 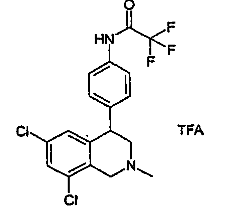 | 2,49 | C | 403,2/404,2 | ESI | |
| 46 | 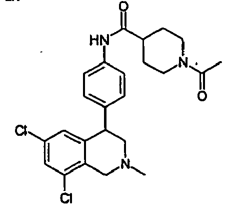 | 2,36 | C | 460,4/462,4 | ESI | |
| 47 | 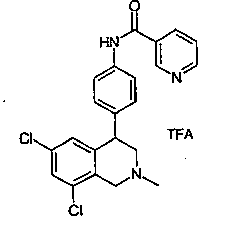 | 2,35 | C | 412,2/414,3 | ESI | |
| 48 | 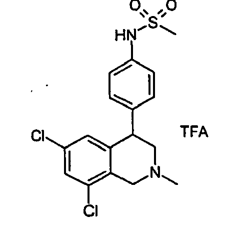 | 2,29 | C | 385,3/387,3 | ESI | |
| 49 | 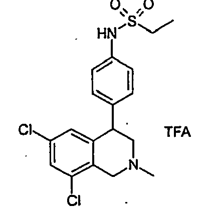 | 2,37 | C | 399,3/401,3 | ESI | |
| 50 | 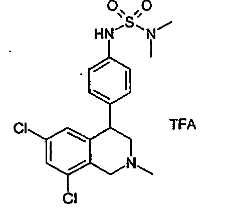 | 2,42 | C | 414,4/416,4 | ESI | |
| 51 | 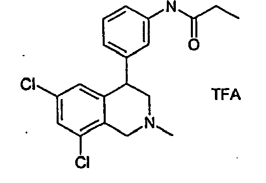 | 2,37 | C | 363,3/365,3 | ESI | |
| 52 | 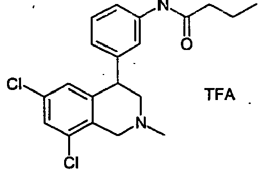 | 2,44 | C | 377,3/379,3 | ESI | |
| 53 | 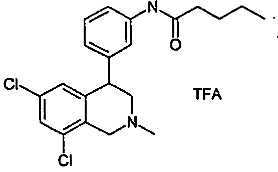 | 2,51 | C | 391,3/393,3 | ESI | |
| 54 | 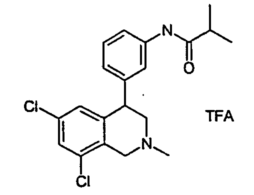 | 2,44 | C | 377,3/379,3 | ESI | |
| 55 | 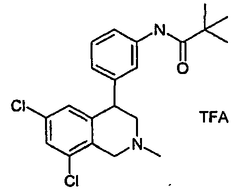 | 2,49 | C | 391,3/393,3 | ESI | |
| 56 | 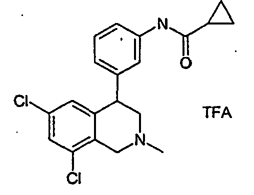 | 2,41 | C | 375,3/377,3 | ESI | |
| 57 | 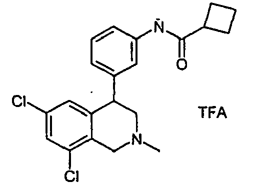 | 2,47 | C | 389,3/391,3 | ESI | |
| 58 | 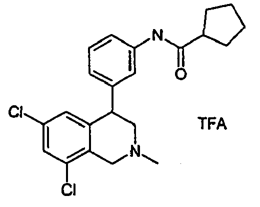 | 2,52 | C | 403,4/405,4 | ESI | |
| 59 | 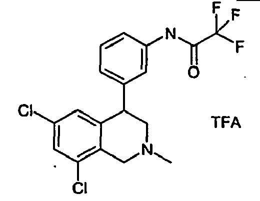 | 2,48 | C | 403,2/404,2 | ESI | |
| 60 | 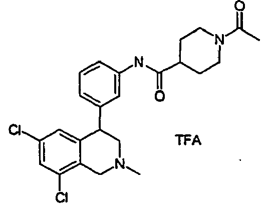 | 2,34 | C | 460,4/462,4 | ESI | |
| 61 | 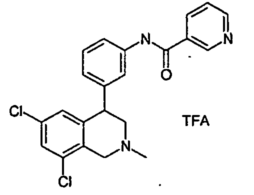 | 2,36 | C | 412,2/414,3 | ESI | |
| 62 | 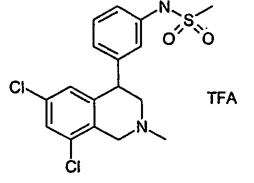 | 2,32 | C | 385,3/387,3 | ESI | |
| 63 | 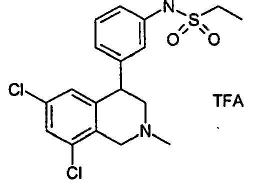 | 2,38 | C | 399,3/401,3 | ESI | |
| 64 | 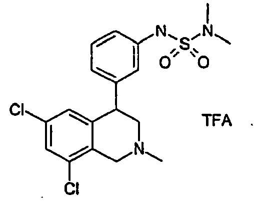 | 2,41 | C | 414,4/416,4 | ESI | |
| 65 | 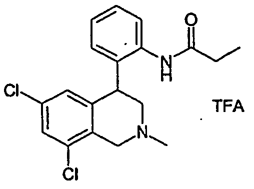 | 2,30 | C | 363,3/365,3 | ESI | |
| 66 | 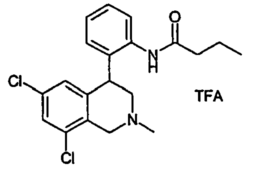 | 2,41 | C | 377,3/379,3 | ESI | |
| 67 | 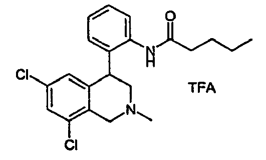 | 2,52 | C | 391,3/393,3 | ESI | |
| 68 | 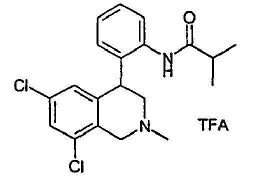 | 2,41 | C | 377,3/379,3 | ESI | |
| 69 | 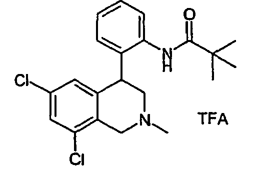 | 2,45 | C | 391,3/393,3 | ESI | |
| 70 | 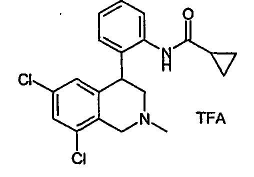 | 2,36 | C | 375,3/377,3 | ESI | |
| 71 | 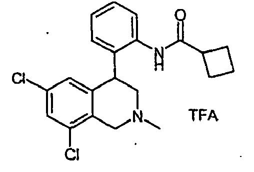 | 2,44 | C | 389,3/391,3 | ESI | |
| 72 | 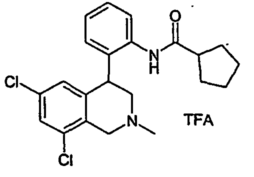 | 2,51 | C | 403,4/405,4 | ESI | |
| 73 | 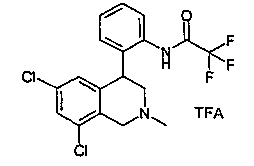 | 2,70 | C | 403,2/404,2 | ESI | |
| 74 | 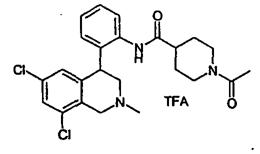 | 2,30 | C | 460,4/462,4 | ESI | |
| 75 | 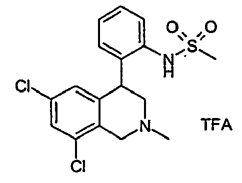 | 2,41 | C | 385,3/387,3 | ESI | |
| 76 | 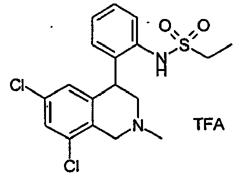 | 2,49 | C | 399,3/401,3 | ESI | |
| 77 | 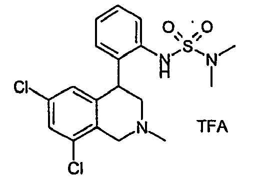 | 2,55 | C | 414,4/416,4 | ESI | |
| 78 | 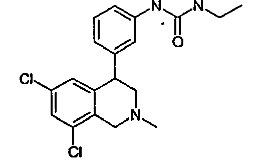 | 1,72 | B | 378,3/380,3 | ESI | |
| 79 | 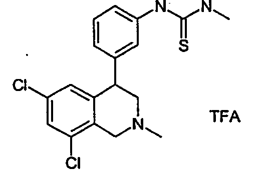 | 1,74 | B | 380,3/382,3 | ESI | |
| 80 | 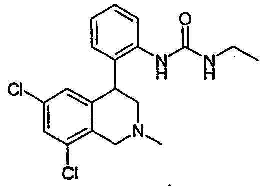 | 1,75 | B | 378,3/380,3 | ESI | |
| 81 | 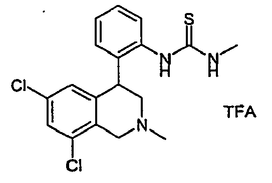 | 1,68 | B | 380,3/382,3 | ESI | |
| 82 | 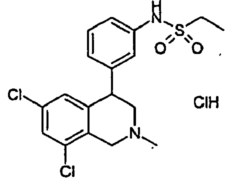 | 1,71 | B | 399,0/400,0/ 401,0/402,0/ 403,0 | ESI | |
| 83 | 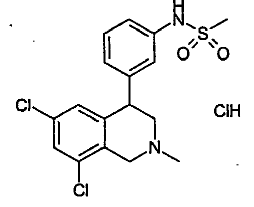 | 1,66 | B | 385,0/386,0/ 387,0 | ESI | |
| 84 | 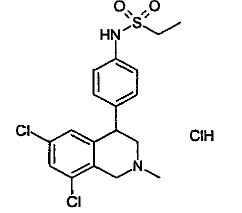 | 1,69 | B | 399,0/400,0/ 401,0/402,0 | ESI | |
| 85 | 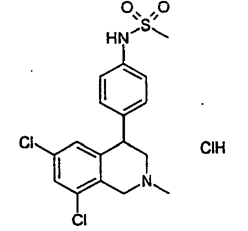 | 1,64 | B | 385,0/386,0/ 387,0/388,0 | ESI | |
| 86a | 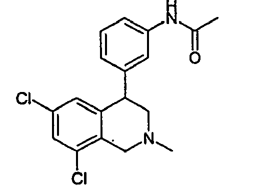 | 1,64 | B | 349,3/351,3 | ESI | (-) Enantiomer |
| 86b | 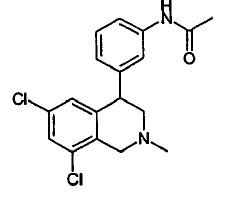 | 1,63 | B | 349,3/351,3 | ESI | (+)-Enantiomer |
| 87 | 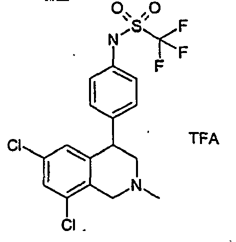 | 1,97 | B | 439,0/441,1 | ESI | |
| 88 | 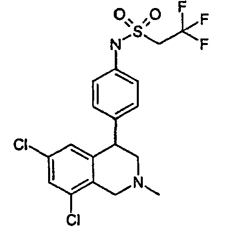 | 1,83 | B | 453,0/455,0 | ESI | |
| 88a | 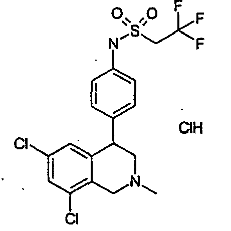 | 1,83 | B | 453,0/455,0 | ESI | |
| 89 | 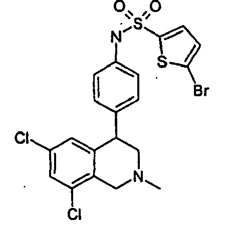 | 2,01 | B | 531,0/533,0/ 534,9 | ESI | |
| 89a | 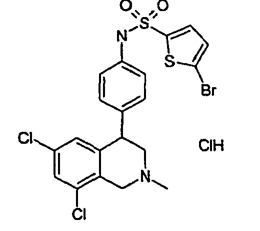 | 2,02 | B | 531,0/533,0/ 534,9 | ESI | |
| 90 | 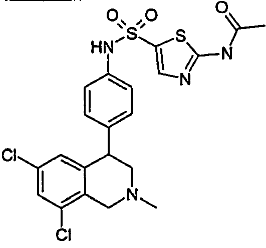 | 1,78 | B | 525,1/527,1 | ESI | |
| 90a | 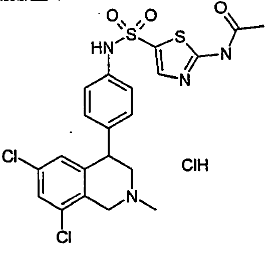 | 1,79 | B | 525,1/527,1 | ESI | |
| 91 | 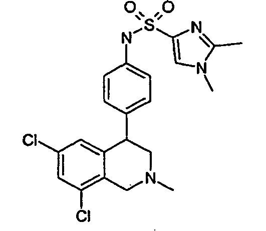 | 1,63 | B | 465,1/467,1 | ESI | |
| 91a | 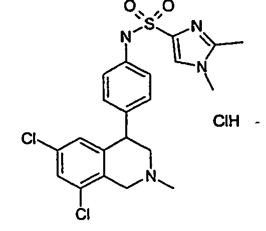 | 1,64 | B | 465,1/467,1 | ESI | |
| 92 | 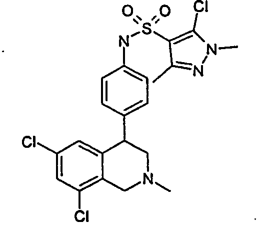 | 1,81 | B | 499,1/501,1/ 503,1 | ESI | |
| 92a | 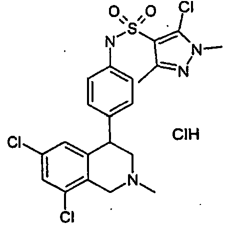 | 1,82 | B | 499,1/501,/ 503,1 | ESI | |
| 93 | 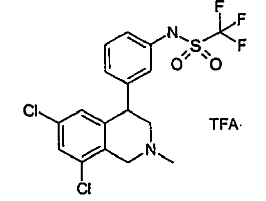 | 1,99 | B | 439,0/441,1 | ESI | |
| 94 | 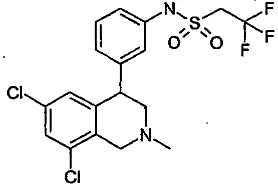 | 1,87 | B | 453,0/455,0 | ESI | |
| 94a | 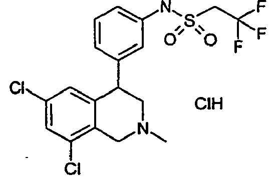 | 1,87 | B | 453,0/455,0 | ESI | |
| 95 | 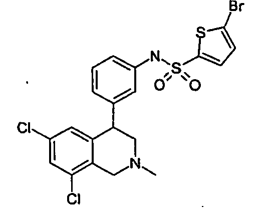 | 2,01 | B | 531,0/533,0/ 535,0 | ESI | |
| 96 | 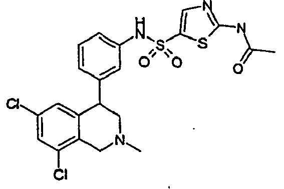 | 1,75 | B | 525,0/527,0 | ESI | |
| 96a | 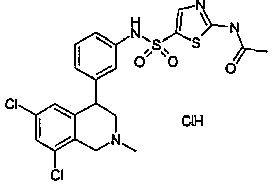 | 1,75 | B | 525,0/527,0 | ESI | |
| 97 | 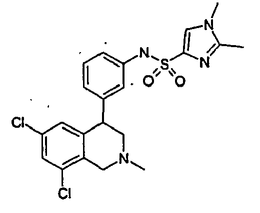 | 1,66 | B | 465,0/467,0 | ESI | |
| 97a | 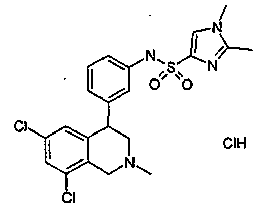 | 1,66 | B | 465,0/467,0 | ESI | |
| 98 | 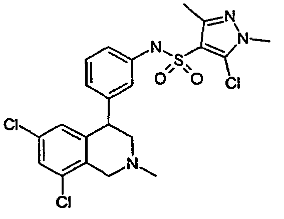 | 1,81 | B | 499,1/501,1/ 503,1 | ESI | |
| 99 | 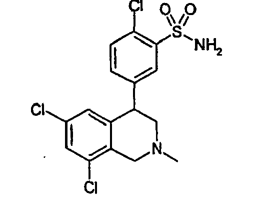 | | | 405,1/407,1 | ESI | Schmp.: 122 °C |
| 100 | 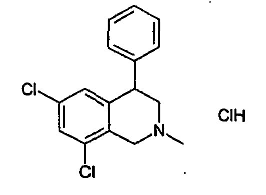 | 4,44 | A | 292,2/294,2 | Cl | s. Bsp.2; Zwischenprodukt 4 |
| 100a | 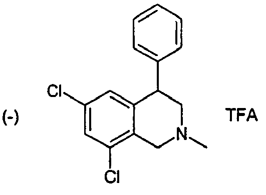 | | | | | s. Bsp.2; Zwischenprodukt 4a |
| 100b | 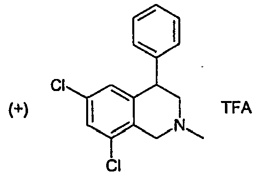 | | | | | s. Bsp.2; Zwischenprodukt 4b |
| 101 | 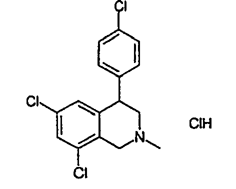 | 4,43 | A | 326,0/328,0 | ESI | |
| 102 | 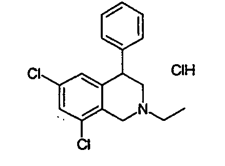 | 4,23 | A | 306,1/308,0 | ESI | |
| 103 | 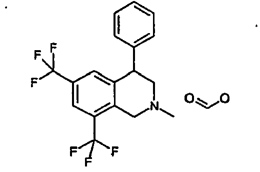 | 2,84 | C | 360,0 | ESI | |
| 104 | 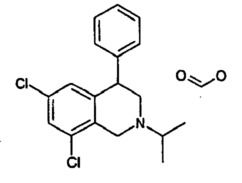 | 2,79 | C | 320,0/322,0 | ESI | |
| 105 | 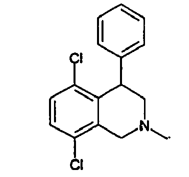 | 2,64 | C | 291,9/293,9 | ESI | |
| 106 | 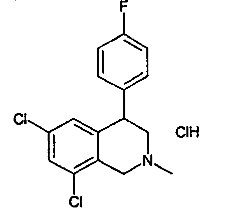 | 4,26 | A | 310,0/312,0 | ESI | |
| 107 | 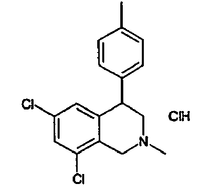 | 4,43 | A | 306,1/308,1 | ESI | |
| 108 | 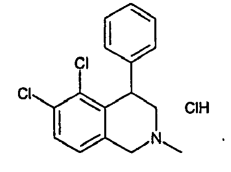 | 4,11 | A | 292,0/294,0 | ESI | |
| 109 | 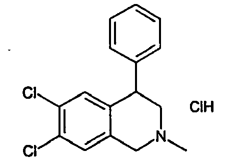 | 4,28 | A | 292,0/294,0 | ESI | |
| 110 | 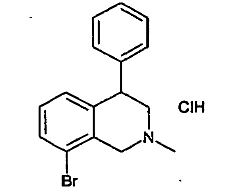 | 4,05 | A | 302,0/304 | ESI | |
| 111 | 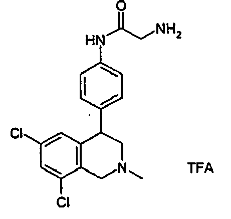 | 1,37 | D | 364,4/366,4 | ESI | |
| 112 | 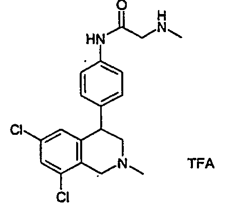 | 1,44 | D | 378,4/380,4 | ESI | |
| 113 | 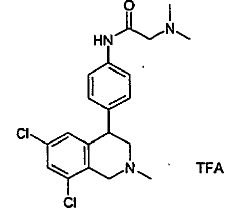 | 1,51 | D | 392,4/394,4 | ESI | |
| 114 | 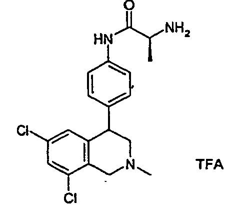 | 1,51 | D | 378,3/380,3 | ESI | |
| 115 | 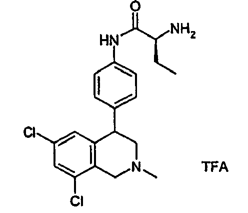 | 1,58 | D | 392,4/394,4 | ESI | |
| 116 | 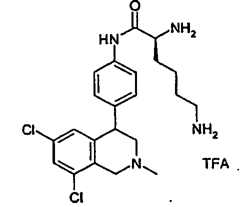 | 1,04 | D | 435,5/437,5 | ESI | |
| 117 | 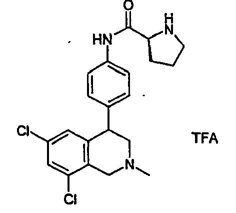 | 1,67 | D | 404,4/406,4 | ESI | |
| 118 | 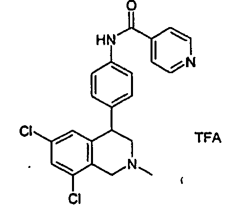 | 2,08 | D | 412,3/414,3 | ESI | |
| 119 | 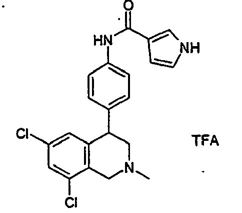 | 2,27 | D | 400,4/402,4 | ESI | |
| 120 | 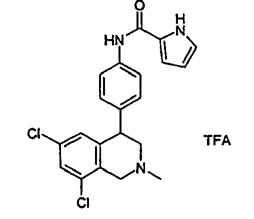 | 2,37 | D | 400,4/402,4 | ESI | |
| 121 | 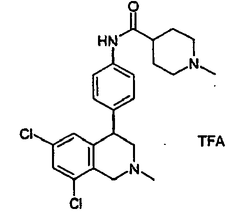 | 1,54 | D | 432,5/434,5 | ESI | |
| 122 | 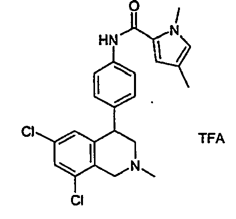 | 1,70 | D | 428,5/430,5 | ESI | |
| 123 | 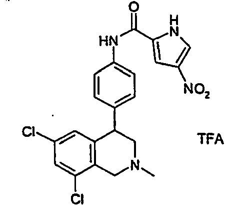 | 2,55 | D | 445,4/447,4 | ESI | |
| 124 | 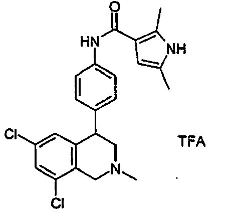 | 2,43 | D | 428,5/430,5 | ESI | |
| 125 | 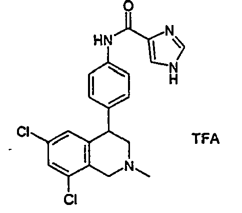 | 1,88 | D | 401,4/403,4 | ESI | |
| 126 | 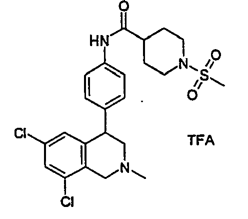 | 2,31 | D | 496,5/498,5 | ESI | |
| 127 | 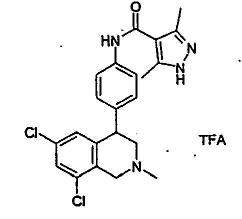 | 2,14 | D | 429,4/431,4 | ESI | |
| 128 | 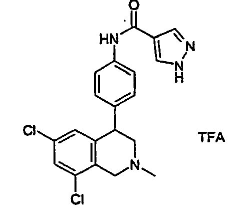 | 2,07 | D | 401,4/403,4 | ESI | |
| 129 | 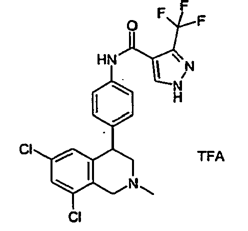 | 2,44 | D | 469,4/471,4 | ESI | |
| 130 | 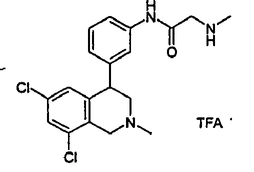 | 1,55 | D | 378,4/380,4 | ESI | |
| 131 | 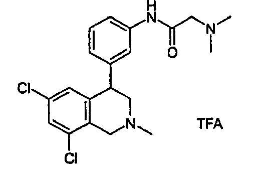 | 1.52 | D | 392,4/394,4 | ESI | |
| 132 | 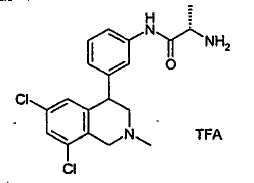 | 1,63 | D | 378,3/380,3 | ESI | |
| 133 | 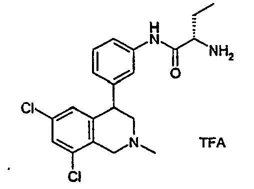 | 1,64 | D | 392,4/394,4 | ESI | |
| 134 | 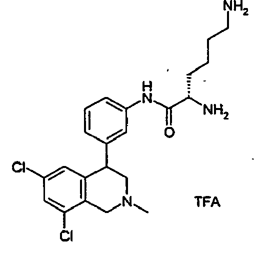 | 1,14 | D | 435,5/437,5 | ESI | |
| 135 | 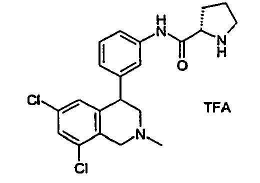 | 1,62 | D | 404,4/406,4 | ESI | |
| 136 | 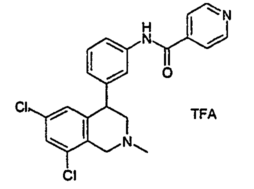 | 2,16 | D | 412,3/414,3 | ESI | |
| 137 | 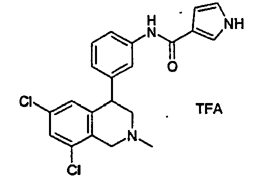 | 2,31 | D | 400,4/402,4 | ESI | |
| 138 | 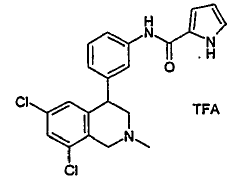 | 2,41 | D | 400,4/402,4 | ESI | |
| 139 | 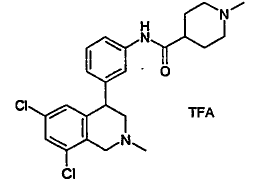 | 1,62 | D | 432,5/434,5 | ESI | |
| 140 | 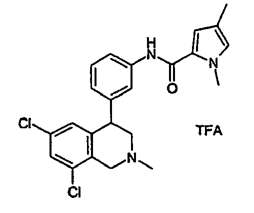 | 1,75 | D | 428,5/430,5 | ESI | |
| 141 | 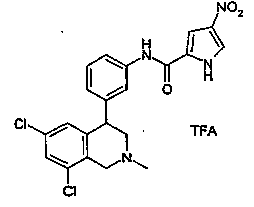 | 2,54 | D | 445,4/447,4 | ESI | |
| 142 | 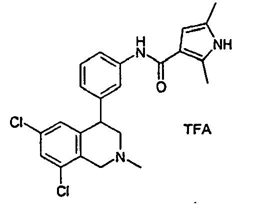 | 2,50 | D | 428,5/430,5 | ESI | |
| 143 | 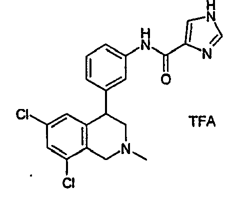 | 1,95 | D | 401,4/403,4 | ESI | |
| 144 | 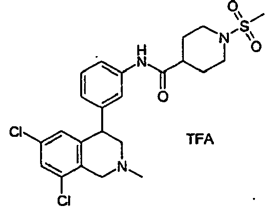 | 2,34 | D | 496,5/498,5 | ESI | |
| 145 | 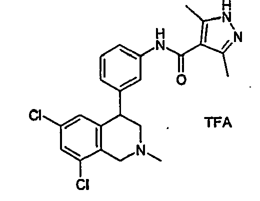 | 2,31 | D | 429,4/431,4 | ESI | |
| 146 | 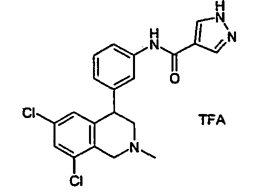 | 2,11 | D | 401,4/403,4 | ESI | |
| 147 | 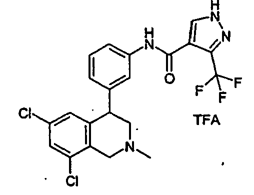 | 2,48 | D | 469,4/471,4 | ESI | |
| 148 | 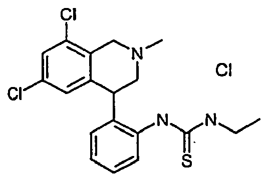 | 2,36 | B | 394,2 | ESI | |
| 149 | 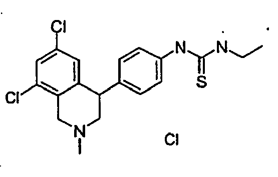 | 2,35 | B | 394,2 | ESI | |
| 150 | 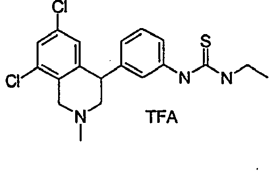 | 2,35 | B | 394,2 | ESI | |
| 151 | 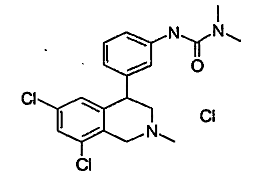 | 2,15 | B | 378,2 | ESI | |
| 152 | 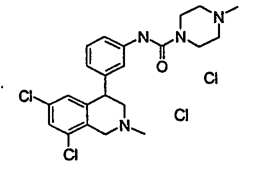 | 1,64 | B | 433,3 | ESI | |
| 153 | 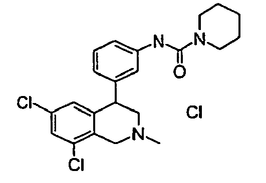 | 2,56 | B | 418,3 | ESI | |
| 154 | 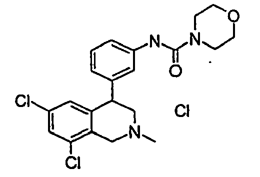 | 2,16 | B | 420,2 | ESI | |
| 155 | 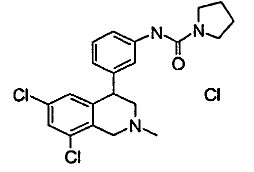 | 2,34 | B | 404,2 | ESI | |
| 156 | 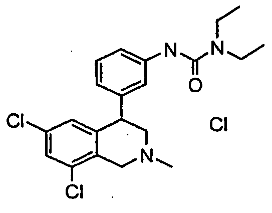 | 2,43 | B | 406,2 | ESI | |
| 157 | 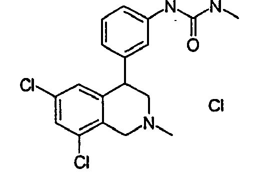 | 2,12 | B | 364,2 | ESI | |
| 158 | 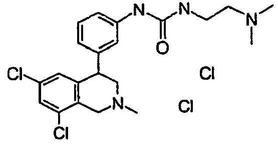 | 1,65 | B | 421,2 | ESI | |
| 159 | 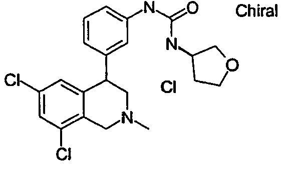 | 2,23 | B | 420,3 | ESI | |
| 160 | 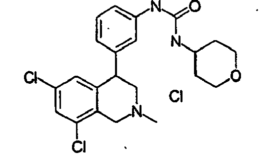 | 2,25 | B | 434,3 | ESI | |
| 161 | 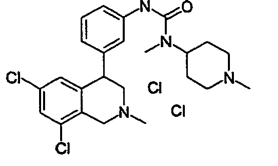 | 1,72 | B | 461,4 | ESI | |
| 162 | 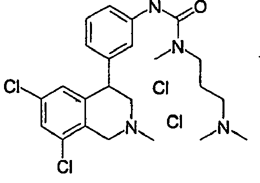 | 1,68 | B | 449,4 | ESI | |
| 163 | 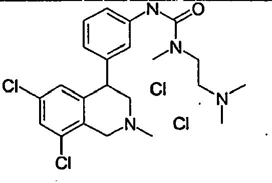 | 1,62 | B | 435,3 | ESI | |
| 164 | 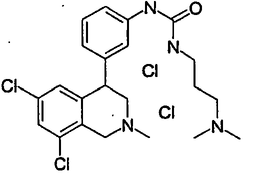 | 1,71 | B | 435,3 | ESI | |
| 165 | 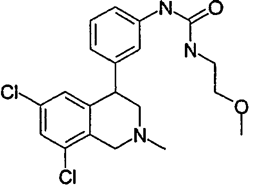 | 2,21 | B | 408,3 | ESI | |
| 166 | 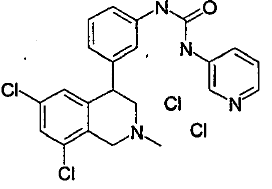 | 1,88 | B | 427,3 | ESI | |
| 167 | 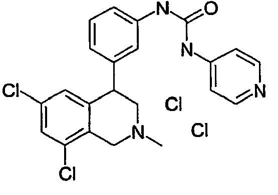 | 1,80 | B | 427,3 | ESI | |
| 168 | 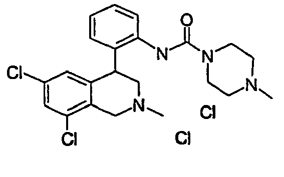 | 1,59 | B | 433,3 | ESI | |
| 169 | 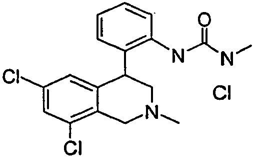 | 2,12 | B | 364,2 | ESI | |
| 170 | 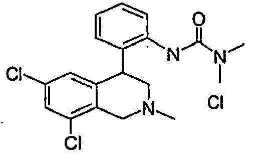 | 2,12 | B | 378,2 | ESI | |
| 171 | 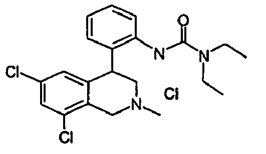 | 2,34 | B | 406,3 | ESI | |
| 172 | 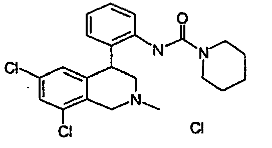 | 2,44 | B | 418,3 | ESI | |
| 173 | 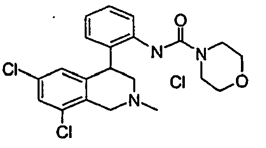 | 2,11 | B | 420,3 | ESI | |
| 174 | 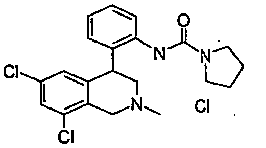 | 2,25 | B | 404,3 | ESI | |
| 175 | 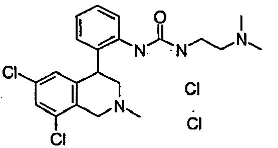 | 0,90 | B | 421,5 | ESI | |
| 176 | 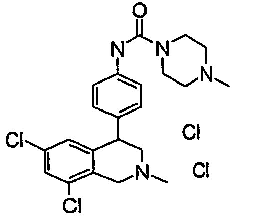 | 1,52 | B | 433,3 | ESI | |
| 177 | 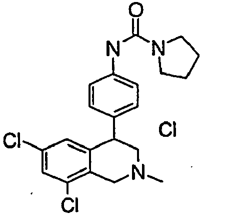 | 2,34 | B | 404,3 | ESI | |
| 178 | 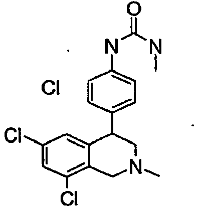 | 2,11 | B | 364,2 | ESI | |
| 179 | 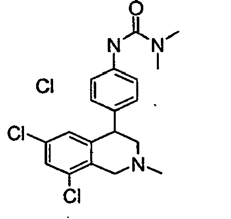 | 2,17 | B | 378,3 | ESI | |
| 180 | 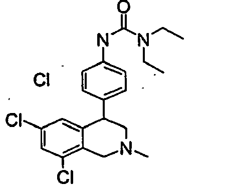 | 2,51 | B | 406,3 | ESI | |
| 181 | 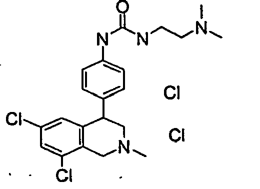 | 1,59 | B | 421,2 | ESI | |
| 182 | 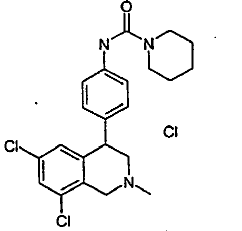 | 2,47 | B | 418,2 | ESI | |
| 183 | 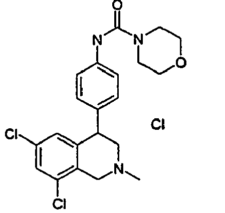 | 2,16 | B | 420,2 | ESI | |
| 184 | 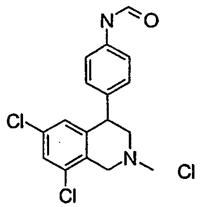 | 2,02 | B | 335,2 | ESI | |
| 185 | 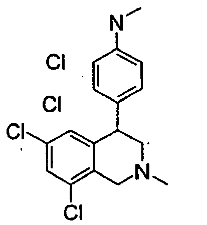 | 1,92 | B | 321,2 | ESI | |
| 186 | 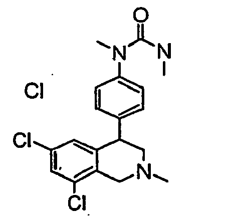 | 1,05 | C | 378,4 | ESI | |
| 187 | 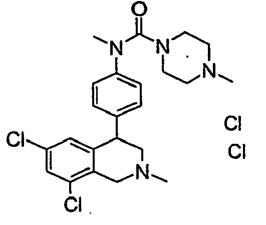 | 0,92 | C | 447,5 | ESI | |
| 188 | 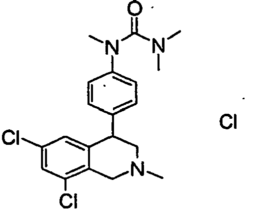 | 1,10 | C | 392,5 | ESI | |
| 189 | 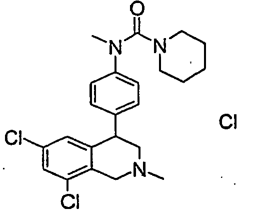 | 1,24 | C | 432,5 | ESI | |
| 190 | 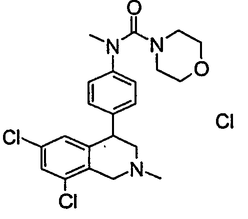 | 1,10 | C | 434,5 | ESI | |
| 191 | 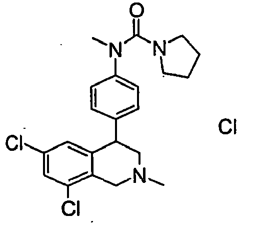 | 1,15 | C | 418,4 | ESI | |
| 192 | 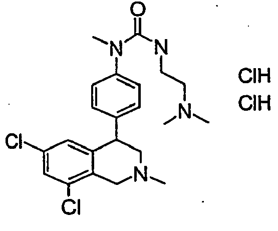 | 0,93 | C | 435,4 | ESI | |
| 193 | 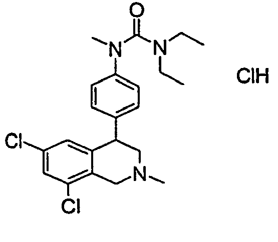 | 1,22 | C | 420,5 | ESI | |
| 194 | 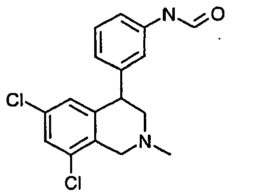 | 2,04 | C | 335,4 | ESI | |
| 195 | 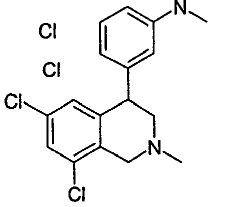 | 0,90 | C | 321,3 | ESI | |
| 196 | 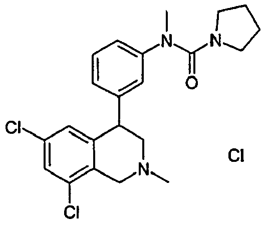 | 2,48 | B | 418,3 | ESI | |
| 197 | 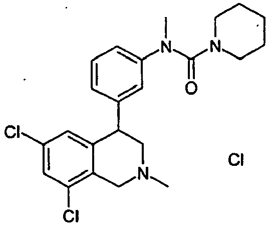 | 2,62 | B | 432,3 | ESI | |
| 198 | 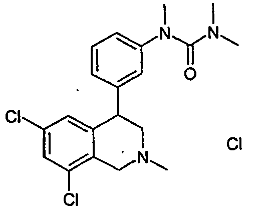 | 2,32 | B | 392,3 | ESI | |
| 199 | 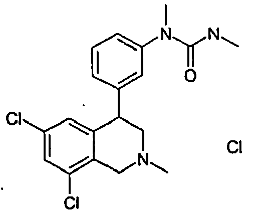 | 2,19 | B | 378,2 | ESI | |
| 200 | 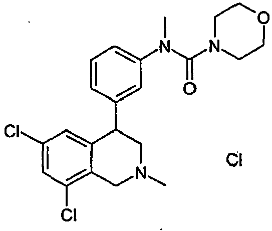 | 2,16 | B | 434,3 | ESI | |
| 201 | 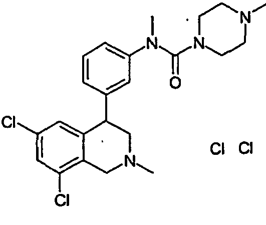 | 1,61 | B | 447,4 | ESI | |
| 202 | 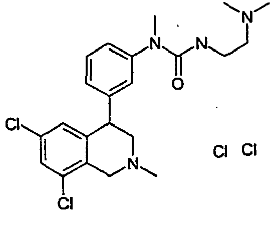 | 1,59 | B | 435,3 | ESI | |
| 203 | 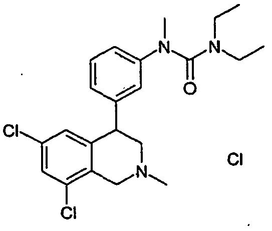 | 2,57 | B | 420,3 | ESI | |
| 204 | 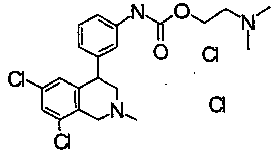 | 1,71 | B | 422,2 | ESI | |
| 205 | 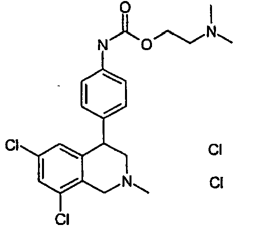 | 1,70 | B | 422,3 | ESI | |
| 206 | 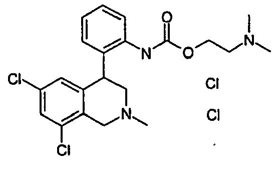 | 1,68 | B | 422,3 | ESI | |
| 207 | 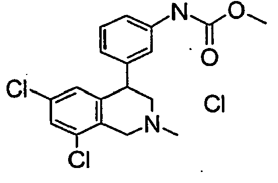 | 2,34 | B | 365,1 | ESI | |
| 208 | 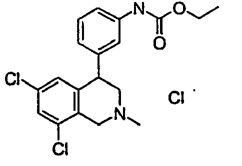 | 1,18 | C | 379,4 | ESI | |
| 209 | 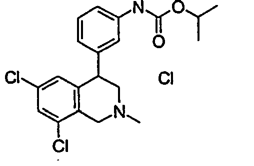 | 1,24 | C | 393,4 | ESI | |
| 210 | 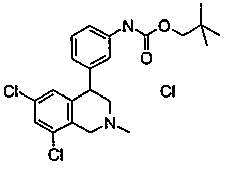 | 1,38 | C | 421,5 | ESI | |
| 211 | 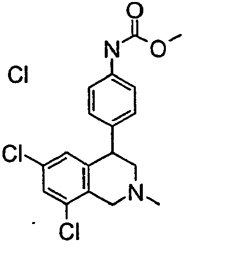 | 1,13 | C | 365,4 | ESI | |
| 212 | 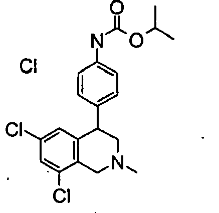 | 1,26 | C | 393,4 | ESI | |
| 213 | 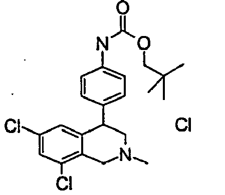 | 1,40 | C | 421,5 | ESI | |
| 214 | 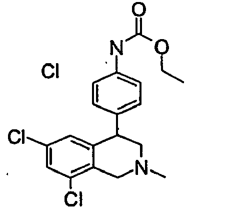 | 1,20 | C | 379,4 | ESI | |
| 215a 215b | 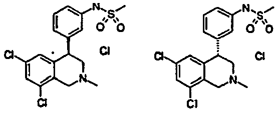 | | | 385,2 | ESI | |
| 216a 216b | 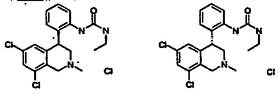 | | | 378,1 | ESI | |
| 217 | 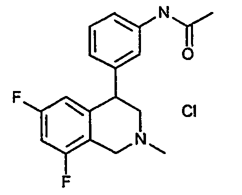 | 1,86 | B | 317,2 | ESI | |
| 218 | 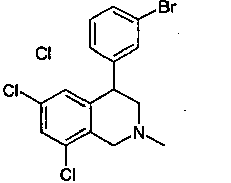 | 1,27 | C | 370,2 | ESI | |
| 219 | 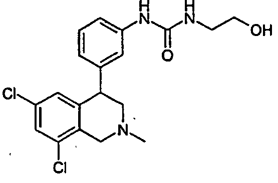 | 0,99 | B1 | 394,1/396,2 | ESI | |
| 220 | 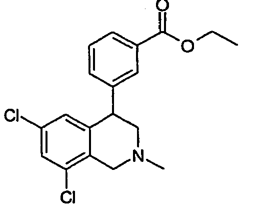 | 1,24 | B1 | 364,1/366,1 | ESI | |
| 221 | 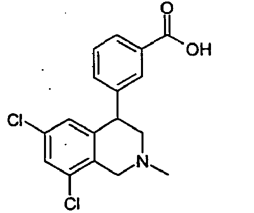 | 1,02 | B1 | 336,1/338,1 | ESI | |

### Pharmakologische Daten:

### Testbeschreibung:

In diesem Test wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHi mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHi umgerechnet. Die Zellen wurden bereits bei der BCECF-Beladung in NH₄Cl-Puffer (pH 7,4) inkubiert (NH₄Cl -Puffer: 115 mM NaCl, 20 mM NH₄Cl, 5 mM KCI, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl - Puffer inkubierten Zellen induziert. Die nachfolgende Geschwindigkeit der pH-Erholung wurde bei NHE1 zwei Minuten, bei NHE2 fünf Minuten und bei NHE3 drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4;7 mM KCl, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCI, 1,25 mM CaCl₂, 1,25 mM MgCl₂, 0,97 mM K₂HPO₄, 0,23 mM KH₂PO₄, 5 mM Hepes, 5 mM Glucose, mit 1 M NaOH wird ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC₅₀-Wert der jeweiligen Substanz für die einzelnen NHE-Subtypen berechnet.

### Ergebnisse:

**Tabelle 14:**

| Beispiel | IC₅₀ [µM], (NHE3) | Beispiel | IC₅₀ [µM], (NHE3) |
|---|---|---|---|
| 1a | 0,075 | 119 | 0,682 |
| 2a | 0,082 | 144 | 0,695 |
| 2b | 0,026 | 146 | 0,024 |
| 6 | 0,670 | 153 | 0,602 |
| 7 | 0,250 | 183 | 0,597 |
| 10 | 1,000 | 199 | 0,252 |
| 17 | 0,049 | 207 | 0,186 |
| 21 | 0,814 | | |
| 23 | 1,507 | | |
| 24 | 0,340 | | |
| 29 | 0,318 | | |
| 36 | 0,274 | | |
| 48 | 0,349 | | |
| 51 | 0,215 | | |
| 60 | 0,202 | | |
| 64 | 0,507 | | |
| 81 | 0,730 | | |
| 87 | 0,418 | | |
| 97 | 0,308 | | |
| 113 | 0,279 | | |

## Patentansprüche

1. 4-Phenyltetrahydroisochinoline der Formel 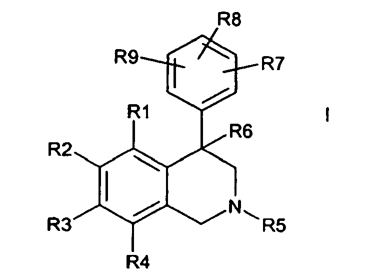 worin bedeuten:
R1, R2, R3 und R4 unabhängig voneinander H, F, Cl, Br, OH, NH₂, CₐH₂ₐ₊₁, Cycloalkyl mit 3, 4, 5 oder 6 C-Atomen, OC_{b}H_{2b+1};
a und b in den Gruppen CₐH₂ₐ₊₁ und OC_{b}H_{2b+1} unabhängig voneinander 1, 2, 3 oder 4, wobei ein oder mehrere H-Atome durch F-Atome ersetzt sein können;
oder
R1, R2, R3 und R4 unabhängig voneinander NR11R12;
R11 und R12 unabhängig voneinander H, CₑH₂ₑ₊₁, CᵣᵣH₂ᵣᵣ₋₁;
e 1, 2, 3 oder 4,
rr 3, 4, 5 oder 6,
wobei in den Gruppen CₑH₂ₑ₊₁ und CᵣᵣH₂ᵣᵣ₋₁ ein oder mehrere H-Atome durch F-Atome ersetzt sein dürfen;
oder
R11 und R12 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin;
oder
R11 und R12 unabhängig voneinander COR14, CSR14, CONHR14, CSNHR14 oder SO₂R14;
R14 C_{g}H_{2g+1};
g 1, 2, 3 oder 4, wobei ein oder mehrere H-Atome durch F-Atome ersetzt sein können;
oder
R1, R2, R3 und R4 unabhängig voneinander OSO₃H, SO₃H, SO₂R15;
R15 CₖH₂ₖ₊₁ oder NR17R18;
k 1, 2, 3 oder 4, wobei ein oder mehrere H-Atome durch F-Atome ersetzt sein können;
R17 und R18 unabhängig voneinander H oder CₘH₂ₘ₊₁;
m 1, 2, 3, 4 oder 5, wobei in der Gruppe CₘH₂ₘ₊₁ ein oder mehrere H-Atome durch F-Atome ersetzt sein können;
oder
R17 und R18 gemeinsam mit dem N-Atom, an das sie gebunden sind, einen 5- oder, 6-gliedrigen Ring;
wobei jedoch immer R2 ungleich H sein muss;
R5 Methyl oder Trifluormethyl;
R6 H;
R7, R8 und R9 unabhängig voneinander OSO₃H, SO₃H oder SO₂R23;
R23 CₙₙH₂ₙₙ₊₁ oder NR25R26;
nn 1, 2, 3, 4 oder 5,
wobei in CₙₙH₂ₙₙ₊₁ ein oder mehrere H-Atome durch F-Atome ersetzt sein können;
R25 und R26 unabhängig voneinander H, CN oder C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atome durch F-Atome ersetzt sein können;
R27 H oder CₐₐH₂ₐₐ₊₁;
aa 1, 2, 3 oder 4;
wobei in CₐₐH₂ₐₐ₊₁ ein oder mehrere H-Atome durch F-Atome ersetzt sein können;
oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
R7, R8 und R9 unabhängig voneinander NR32COR30, NR32CSR30 oder NR32SO₂R30;
R30 H, OH, C_{cc}H_{2cc+1}, C_{yy}H_{2yy-1}, Pyrrolidinyl oder Piperidinyl, in welchen Ringen eine CH₂-Gruppe durch O oder NR33 ersetzt sein kann;
R32 und R33 H, Methyl oder CF₃;
cc 1, 2, 3, 4, 5, 6, 7 oder 8;
yy 3, 4, 5 oder 6;
wobei in den Gruppen C_{cc}H_{2cc+1} und C_{yy}H_{2yy-1} ein oder mehrere H-Atome durch F-Atome ersetzt sein können und eine oder mehrere CH₂-Gruppen durch NR31 ersetzt sein können und eine CH₂-Gruppe durch O ersetzt sein kann;
R31 H, Methyl, Ethyl, CF₃, CH₂CF₃, Acetyl, Propionyl, Methansulfonyl oder Ethansulfonyl;
oder
R31 zusammen mit einer CH₂-Gruppe von R30 und dem N-Atom, an das sie gemeinsam gebunden sind, einen 5- oder 6-gliedrigen Ring bilden;
oder
R30 Pyridyl, Imidazolyl, Pyrazolyl, Pyrrolyl, Triazolyl, Tetrazolyl, Thiazolyl oder Oxazolyl, die unsubstituiert sind oder substituiert mit maximal 3 Substituenten ausgewählt aus der Gruppe bestehend aus F, Cl, Methyl, Ethyl, Trifluormethyl, NH₂, NHAcetyl;
oder
R7, R8 und R9 unabhängig voneinander H, F, Cl, OH, NH₂, CₑₑH₂ₑₑ₊₁, C_{ww}H_{2ww-1}, OC_{ff}H_{2ff+1}, NR40R41, CONR40R41, COOR42 oder OCOR42,
ee und ff unabhängig voneinander 1, 2, 3 oder 4;
ww 3, 4, 5 oder 6,
wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H, CₜₜH₂ₜₜ₊₁ oder C(NH)NH₂;
tt 1, 2, 3 oder 4;
wobei in der Gruppe CₜₜH₂ₜₜ₊₁ ein oder mehrere H-Atome durch F-Atome ersetzt sein dürfen;
oder
R40 und R41 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl;
oder
R40 und R41 gemeinsam mit dem N-Atom, an das sie gebunden sind, einen Pyrrolidin-, Piperidin-, N-Methylpiperazin-, Piperazin- oder Morpholinring;
R42 H oder CₕₕH₂ₕₕ₊₁;
hh 1, 2, 3 oder 4;
wobei in der Gruppe CₕₕH₂ₕₕ₊₁ ein oder mehrere H-Atome durch F-Atome ersetzt sein können;
wobei jedoch nicht zwei Substituenten aus der Gruppe R7, R8 und R9 gleichzeitig OH oder OCH₃ sein dürfen
und wobei mindestens einer der Reste R7, R8 oder R9 ausgewählt sein muss aus der Gruppe bestehend aus -OᵥSO_{w}R23, NR32COR30, NR32CSR30 und NR32SO_{bb}R30; sowie deren pharmazeutisch verträgliche Salze und Trifluoracetate.

2. Verbindung der Formel I, **dadurch gekennzeichnet, dass** sie ausgewählt ist aus der Gruppe bestehend aus:
1) N-[4-(6,8-Dichloro-2-methyl-1,2,3,4-tetrahydro-isochinolin-4-yl)-phenyl]-acetamid;
2) 4-(6,8-Dichloro-2-methyl-1,2,3,4-tetrahydro-isochinolin-4-yl)-benzolsulfonamid;
3) 3-(6,8-Dichloro-2-methyl-1,2,3,4-tetrahydro-isochinolin-4-yl)-benzolsulfonamid;
4) 4-(6,8-Dichloro-2-methyl-1,2,3,4-tetrahydro-isochinolin-4-yl)-N,N-dimethylbenzol-sulfonamid;
5) 4-(4-Bromo-phenyl)-6,8-dichloro-2-methyl-1,2,3,4-tetrahydroisochinolin;
6) 4-(6,8-Dichloro-2-methyl-1,2,3,4-tetrahydro-isochinolin-4-yl)-benzoesäure;
7) 4-(6,8-Dichloro-2-methyl-1,2,3,4-tetrahydroisochinolin-4-yl)-N-ethyl-benzamid;
8) 4-(6,8-Dichloro-2-methyl-1,2,3,4-tetrahydroisochinolin-4-yl)-N-propyl-benzamid;
9) 4-(6,8-Dichloro-2-methyl-1,2,3,4-tetrahydro-isochinolin-4-yl)-N-(2-dimethylamino-ethyl)-benzamid;
10) 6,8-Dichloro-2-methyl-4-(4-morpholin-4-yl-phenyl)-1,2,3,4-tetrahydroisochinolin;
11) [4-(6,8-Dichloro-2-methyl-1,2,3,4-tetrahydroisochinolin-4-yl)-phenyl]-diethyl-amin
12) 6,8-Dichloro-2-methyl-4-(4-piperidin-1-yl-phenyl)-1,2,3,4-tetrahydroisochinolin;
13) 6,8-Dichloro-2-methyl-4-(4-pyrrolidin-1-yl-phenyl)-1,2,3,4-tetrahydroisochinolin;
14) 6,8-Dichloro-2-methyl-4-[4-(4-methyl-piperazin-1-yl)-phenyl]-1,2,3,4-tetrahydroisochinolin;
15) 6,8-Dichloro-2-cyclopropyl-4-phenyl-1,2,3,4-tetrahydroisochinolin;
16) 4-(6,8-Dichloro-2-methyl-1,2,3,4-tetrahydro-isochinolin-4-yl)-phenylamin;
17) 1-[4-(6,8-Dichloro-2-methyl-1,2,3,4-tetrahydro-isochinolin-4-yl)-phenyl]-3-propyl-harnstoff;
18) 1-[4-(6,8-Dichloro-2-methyl-1,2,3,4-tetrahydro-isochinolin-4-yl)-phenyl]-3-methyl-thioharnstoff;
19) 1-[4-(6,8-Dichloro-2-methyl-1,2,3,4-tetrahydro-isochinolin-4-yl)-phenyl]-3-ethyl-harnstoff;
20) N-[4-(6-Methansulfonyl-2-methyl-1,2,3,4-tetrahydro-isochinolin-4-yl)-phenyl]-acetamid;
21) N-[4-(2,6,8-Trimethyl-1,2,3,4-tetrahydro-isochinolin-4-yl)-phenyl]-acetamid;
22) N-[4-(6-Bromo-8-chloro-2-methyl-1,2,3,4-tetrahydro-isochinolin-4-yl)-phenyl]-acetamid;
23) N-[4-(8-Chloro-2-methyl-6-pyrrolidin-1-yl-1,2,3,4-tetrahydro-isochinolin-4-yl)-phenyl]- acetamid;
24) N-[4-(8-Chloro-2-methyl-6-morpholin-4-yl-1,2,3,4-tetrahydro-isochinolin-4-yl)-phenyl]- acetamid;
25) N-{4-[8-Chloro-2-methyl-6-(4-methyl-piperazin-1-yl)-1,2,3,4-tetrahydroisochinolin-4- yl]-phenyl}-acetamid;
26) N-{4-[8-Chloro-6-(cyclopropylmethyl-amino)-2-methyl-1,2,3,4-tetrahydroisochinolin-4- yl]-phenyl}-acetamid;
27) 5-(6,8-Dichloro-2-methyl-1,2,3,4-tetrahydro-isochinolin-4-yl)-2-hydroxybenzoesäure;
28) 5-(6,8-Dichloro-2-methyl-1,2,3,4-tetrahydro-isochinolin-4-yl)-2-hydroxy-N-methyl-benzamid;
29) 5-(6,8-Dichloro-2-methyl-1,2,3,4-tetrahydro-isochinolin-4-yl)-N-ethyl-2-hydroxy-benzamid;
30) 5-(6,8-Dichloro-2-methyl-1,2,3,4-tetrahydro-isochinolin-4-yl)-N-(2-dimethylamino-ethyl)-2-hydroxy-benzamid;
31) N-[5-(6,8-Dichloro-2-methyl-1,2,3,4-tetrahydro-isochinolin-4-yl)-2-hydroxy-benzoyl]- guanidin;
32) N-[3-(6,8-Dichloro-2-methyl-1,2,3,4-tetrahydro-isochinolin-4-yl)-phenyl]-acetamid;
33) 3-(6,8-Dichloro-2-methyl-1,2,3,4-tetrahydro-isochinolin-4-yl)-phenylamin;
34) 2-(6,8-Dichloro-2-methyl-1,2,3,4-tetrahydro-isochinolin-4-yl)-phenylamin;
35) N-[4-(6,8-Dichloro-2-methyl-1,2,3,4-tetrahydro-isochinolin-4-yl)-phenyl]-propionamid;
36) N-[4-(6,8-Dichloro-2-methyl-1,2,3,4-tetrahydro-isochinolin-4-yl)-phenyl]-butyramid;
37) Pentansäure-[4-(6,8-dichloro-2-methyl-1,2,3,4-tetrahydro-isochinolin-4-yl)-phenyl]-amid;
38) N-[4-(6,8-Dichloro-2-methyl-1,2,3,4-tetrahydro-isochinolin-4-yl)-phenyl]-isobutyramid;
39) N-[4-(6,8-Dichloro-2-methyl-1,2,3,4-tetrahydro-isochinolin-4-yl)-phenyl]-2,2-dimethyl- propionamid;
40) Cyclopropancarbonsäure-[4-(6,8-dichloro-2-methyl-1,2,3,4-tetrahydro-isochinolin-4-yl)-phenyl]-amid;
41) Cyclobutancarbonsäure-[4-(6,8-dichloro-2-methyl-1,2,3,4-tetrahydro-isochinolin-4-yl)- phenyl]-amid;
42) Cyclopentancarbonsäure-[4-(6,8-dichloro-2-methyl-1,2,3,4-tetrahydro-isochinolin-4-yl)-phenyl]-amid;
43) N-[4-(6,8-Dichloro-2-methyl-1,2,3,4-tetrahydro-isochinolin-4-yl)-phenyl]-2,2,2-trifluoro-acetamid;
44) 1-Acetyl-piperidin-4-carbonsäure-[4-(6,8-dichloro-2-methyl-1,2,3,4-tetrahydroisochinolin-4-yl)-phenyl]-amid;
45) N-[4-(6,8-Dichloro-2-methyl-1,2,3,4-tetrahydro-isochinolin-4-yl)-phenyl]-nicotinamid;
46) N-[4-(6,8-Dichloro-2-methyl-1,2,3,4-tetrahydro-isochinolin-4-yl)-phenyl]-methansulfonamid;
47) Ethansulfonsäure-[4-(6,8-dichloro-2-methyl-1,2,3,4-tetrahydro-isochinolin-4-yl)-phenyl]-amid;
48) N',N'-Dimethylamino-N-[4-(6,8-Dichloro-2-methyl-1,2,3,4-tetrahydro-isochinolin-4-yl)-phenyl]-sulfamid;
49) N-[3-(6,8-Dichloro-2-methyl-1,2,3,4-tetrahydro-isochinolin-4-yl)-phenyl]-propionamid;
50) N-[3-(6,8-Dichloro-2-methyl-1,2,3,4-tetrahydro-isochinolin-4-yl)-phenyl]-butyramid;
51) Pentansäure-[3-(6,8-dichloro-2-methyl-1,2,3,4-tetrahydro-isochinolin-4-yl)-phenyl]-amid;
52) N-[3-(6,8-Dichloro-2-methyl-1,2,3,4-tetrahydro-isochinolin-4-yl)-phenyl]-isobutyramid;
53) N-[3-(6,8-Dichloro-2-methyl-1,2,3,4-tetrahydro-isochinolin-4-yl)-phenyl]-2,2-dimethyl-propionamid;
54) Cyclopropancarbonsäure-[3-(6,8-dichloro-2-methyl-1,2,3,4-tetrahydro-isochinolin-4-yl)-phenyl]-amid;
55) Cyclobutancarbonsäure-[3-(6,8-dichloro-2-methyl-1,2,3,4-tetrahydro-isochinolin-4-yl)- phenyl]-amid;
56) Cyclopentancarbonsäure-[3-(6,8-dichloro-2-methyl-1,2,3,4-tetrahydro-isochinolin-4-yl)-phenyl]-amid;
57) N-[3-(6,8-Dichloro-2-methyl-1,2,3,4-tetrahydro-isochinolin-4-yl)-phenyl]-2,2,2-trifluoro-acetamid;
58) 1-Acetyl-piperidin-4-carbonsäure-[3-(6,8-dichloro-2-methyl-1,2,3,4-tetrahydroisochinolin-4-yl)-phenyl]-amid;
59) N-[3-(6,8-Dichloro-2-methyl-1,2,3,4-tetrahydro-isochinolin-4-yl)-phenyl]-nicotinamid;
60) N-[3-(6,8-Dichloro-2-methyl-1,2,3,4-tetrahydro-isochinolin-4-yl)-phenyl]-methansulfonamid;
61) Ethansulfonsäure-[3-(6,8-dichloro-2-methyl-1,2,3,4-tetrahydro-isochinolin-4-yl)-phenyl]-amid;
62) N',N'-Dimethylamino-N-[3-(6,8-Dichloro-2-methyl-1,2,3,4-tetrahydro-isochinolin-4-yl)-phenyl]-sulfamid;
63) N-[2-(6,8-Dichloro-2-methyl-1,2,3,4-tetrahydro-isochinolin-4-yl)-phenyl]-propionamid;
64) N-[2-(6,8-Dichloro-2-methyl-1,2,3,4-tetrahydro-isochinolin-4-yl)-phenyl]-butyramid;
65) Pentansäure-[2-(6,8-dichloro-2-methyl-1,2,3,4-tetrahydro-isochinolin-4-yl)-phenyl]-amid;
66) N-[2-(6,8-Dichloro-2-methyl-1,2,3,4-tetrahydro-isochinolin-4-yl)-phenyl]-isobutyramid;
67) N-[2-(6,8-Dichloro-2-methyl-1,2,3,4-tetrahydro-isochinolin-4-yl)-phenyl]-2,2-dimethyl- propionamid;
68) Cyclopropancarbonsäure-[2-(6,8-dichloro-2-methyl-1,2,3,4-tetrahydro-isochinolin-4-yl)-phenyl]-amid;
69) Cyclobutancarbonsäure-[2-(6,8-dichloro-2-methyl-1,2,3,4-tetrahydro-isochinolin-4-yl)-phenyl]-amid;
70) Cyclopentancarbonsäure-[2-(6,8-dichloro-2-methyl-1,2,3,4-tetrahydro-isochinolin-4-yl)-phenyl]-amid;
71) N-[2-(6,8-Dichloro-2-methyl-1,2,3,4-tetrahydro-isochinolin-4-yl)-phenyl]-2,2,2-trifluoro-acetamid;
72) 1-Acetyl-piperidin-4-carbonsäure-[2-(6,8-dichloro-2-methyl-1,2,3,4-tetrahydroisochinolin-4-yl)-phenyl]-amid;
73) N-[2-(6,8-Dichloro-2-methyl-1,2,3,4-tetrahydro-isochinolin-4-yl)-phenyl]-methansulfonamid;
74) Ethansulfonsäure-[2-(6,8-dichloro-2-methyl-1,2,3,4-tetrahydro-isochinolin-4-yl)-phenyl]-amid;
75) N',N'-Dimethylamino-N-[2-(6,8-Dichloro-2-methyl-1,2,3,4-tetrahydro-isochinolin-4-yl)-phenyl]-sulfamid;
76) 1-[3-(6,8-Dichloro-2-methyl-1,2,3,4-tetrahydro-isochinolin-4-yl)-phenyl]-3-ethyl-harnstoff;
77) 1-[3-(6,8-Dichloro-2-methyl-1,2,3,4-tetrahydro-isochinolin-4-yl)-phenyl]-3-methyl-thioharnstoff;
78) 1-[2-(6,8-Dichloro-2-methyl-1,2,3,4-tetrahydro-isochinolin-4-yl)-phenyl]-3-ethyl-harnstoff;
79) 1-[2-(6,8-Dichloro-2-methyl-1,2,3,4-tetrahydro-isochinolin-4-yl)-phenyl]-3-methyl-thioharnstoff;
80) N-{5-[4-(6,8-Dichloro-2-methyl-1,2,3,4-tetrahydro-isochinolin-4-yl)-phenylsulfamoyl]-4-methyl-thiazol-2-yl}-acetamid;
81) N-{5-[3-(6,8-Dichloro-2-methyl-1,2,3,4-tetrahydro-isochinolin-4-yl)-phenylsulfamoyl]-4-methyl-thiazol-2-yl}-acetamid;
82) 1,2-Dimethyl-1H-imidazole-4-sulfonsäure-[4-(6,8-dichloro-2-methyl-1,2,3,4-tetrahydro- isochinolin-4-yl)-phenyl]-amid;
83) 1,2-Dimethyl-1H-imidazole-4-sulfonsäure-[3-(6,8-dichloro-2-methyl-1,2,3,4-tetrahydro- isochinolin-4-yl)-phenyl]-amid;
84) 5-Chloro-1,3-dimethyl-1H-pyrazol-4-sulfonsäure-[4-(6,8-dichloro-2-methyl-1,2,3,4-tetrahydro-isochinolin-4-yl)-phenyl]-amid;
85) 5-Chloro-1,3-dimethyl-1H-pyrazol-4-sulfonsäure-[3-(6,8-dichloro-2-methyl-1,2,3,4-tetrahydro-isochinolin-4-yl)-phenyl]-amid;
86) 5-Bromo-thiophen-2-sulfonsäure-[4-(6,8-dichloro-2-methyl-1,2,3,4-tetrahydroisochinolin-4-yl)-phenyl]-amid;
87) 5-Bromo-thiophen-2-sulfonsäure-[3-(6,8-dichloro-2-methyl-1,2,3,4-tetrahydroisochinolin-4-yl)-phenyl]-amid;
88) N-[4-(6,8-Dichloro-2-methyl-1,2,3,4-tetrahydro-isochinolin-4-yl)-phenyl]-C,C,C-trifluoro-methanesulfonamid;
89) N-[3-(6,8-Dichloro-2-methyl-1,2,3,4-tetrahydro-isochinolin-4-yl)-phenyl]-C,C,C-trifluoro-methanesulfonamid;
90) 2,2,2-Trifluoro-ethansulfonsäure-4-(6,8-dichloro-2-methyl-1,2,3,4-tetrahydroisochinolin-4-yl)-phenyl]-amid;
91) 2,2,2-Trifluoro-ethansulfonsäure-3-(6,8-dichloro-2-methyl-1,2,3,4-tetrahydroisochinolin-4-yl)-phenyl]-amid;
92) N-Ethyl-N'-4-(6,8-Dichloro-2-methyl-1,2,3,4-tetrahydro-isochinolin-4-yl)-benzolsulfonyl-harnstoff;
93) 2-Chloro-5-(6,8-dichloro-2-methyl-1,2,3,4-tetrahydro-isochinolin-4-yl)-benzolsulfonamid;
94) 2-Methyl-4-phenyl-6,8-bis-trifluoromethyl-1,2,3,4-tetrahydro-isochinolin;
95) 2-Amino-N-[4-(6,8-dichloro-2-methyl-1,2,3,4-tetrahydro-isochinolin-4-yl)-phenyl]-acetamid;
96) N-[4-(6,8-Dichloro-2-methyl-1,2,3,4-tetrahydro-isochinolin-4-yl)-phenyl]-2-methylamino- acetamid;
97) N-[4-(6,8-Dichloro-2-methyl-1,2,3,4-tetrahydro- isochinolin-4-yl)-phenyl]-2-dimethylamino-acetamid;
98) 2-Amino-N-[4-(6,8-dichloro-2-methyl-1,2,3,4-tetrahydro-isochinolin-4-yl)-phenyl]-propionamid;
99) 2-Amino-N-[4-(6,8-dichloro-2-methyl-1,2,3,4-tetrahydro-isochinolin-4-yl)-phenyl]-butyramid;
100) 2,6-Diamino-hexansäure-[4-(6,8-dichloro-2-methyl-1,2,3,4-tetrahydro-isochinolin -4-yl)- phenyl]-amid;
101) Pyrrolidine-2-carbonsäure-[4-(6,8-dichloro-2-methyl-1,2,3,4-tetrahydroisochinolin -4- yl)-phenyl]-amid;
102) N-[4-(6,8-Dichloro-2-methyl-1,2,3,4-tetrahydro-isochinolin-4-yl)-phenyl]-isonicotinamid;
103) 1H-Pyrrole-3-carbonsäure-[4-(6,8-dichloro-2-methyl-1,2,3,4-tetrahydroisochinolin-4- yl)-phenyl]-amid;
104) 1H-Pyrrol-2- carbonsäure-[4-(6,8-dichloro-2-methyl-1,2,3,4-tetrahydroisochinolin-4- yl)-phenyl]-amid;
105) 1-Methyl-piperidin-4-carbonsäure-[4-(6,8-dichloro-2-methyl-1,2,3,4-tetrahydroisochinolin-4-yl)-phenyl]-amid;
106) 1,4-Dimethyl-1H-pyrrol-2-carbonsäure-[4-(6,8-dichloro-2-methyl-1,2,3,4-tetrahydro- isochinolin-4-yl)-phenyl]-amid;
107) 4-Nitro-1H-pyrrol-2-carbonsäure-[4-(6,8-dichloro-2-methyl-1,2,3,4-tetrahydroisochinolin-4-yl)-phenyl]-amid;
108) 2,5-Dimethyl-1H-pyrrol-3-carbonsäure-[4-(6,8-dichloro-2-methyl-1,2,3,4-tetrahydro- isochinolin-4-yl)-phenyl]-amid;
109) 1H-Imidazol-4-carbonsäure-[4-(6,8-dichloro-2-methyl-1,2,3,4-tetrahydroisochinolin-4-yl)-phenyl]-amid;
110) 1-Methansulfonyl-piperidin-4-carbonsäure-[4-(6,8-dichloro-2-methyl-1,2,3,4-tetrahydro- isochinolin-4-yl)-phenyl]-amid;
111) 3,5-Dimethyl-1 H-pyrazol-4-carbonsäure-[4-(6,8-dichloro-2-methyl-1,2,3,4-tetrahydro- isochinolin-4-yl)-phenyl]-amid;
112) 1H-Pyrazol-4- carbonsäure-[4-(6,8-dichloro-2-methyl-1,2,3,4-tetrahydroisochinolin-4-yl)-phenyl]-amid;
113) 3-Trifluoromethyl-1H-pyrazol-4- carbonsäure-[4-(6,8-dichloro-2-methyl-1,2,3,4-tetrahydro-isochinolin-4-yl)-phenyl]-amid;
114) N-[3-(6,8-Dichloro-2-methyl-1,2,3,4-tetrahydro-isochinolin-4-yl)-phenyl]-2-methylamino-acetamid;
115) N-[3-(6,8-Dichloro-2-methyl-1,2,3,4-tetrahydro-isochinolin-4-yl)-phenyl]-2-dimethylamino-acetamid;
116) 2-Amino-N-[3-(6,8-dichloro-2-methyl-1,2,3,4-tetrahydro-isochinolin-4-yl)-phenyl]-propionamid;
117) 2-Amino-N-[3-(6,8-dichloro-2-methyl-1,2,3,4-tetrahydro-isochinolin-4-yl)-phenyl]-butyramid;
118) 2,6-Diamino-hexansäure-[3-(6,8-dichloro-2-methyl-1,2,3,4-tetrahydro-isochinolin-4-yl)- phenyl]-amid;
119) Pyrrolidin-2- carbonsäure-[3-(6,8-dichloro-2-methyl-1,2,3,4-tetrahydro-isochinolin-4- yl)-phenyl]-amid;
120) N-[3-(6,8-Dichloro-2-methyl-1,2,3,4-tetrahydro-isochinolin-4-yl)-phenyl]-isonicotinamid;
121) 1H-Pyrrol-3-carbonsäure-[3-(6,8-dichloro-2-methyl-1,2,3,4-tetrahydro-isochinolin-4- yl)-phenyl]-amid;
122) 1H-Pyrrol-2- carbonsäure-[3-(6,8-dichloro-2-methyl-1,2,3,4-tetrahydro-isochinolin-4- yl)-phenyl]-amid;
123) 1-Methyl-piperidin-4-carbonsäure-[3-(6,8-dichloro-2-methyl-1,2,3,4-tetrahydroisochinolin-4-yl)-phenyl]-amid;
124) 1,4-Dimethyl-1H-pyrrol-2- carbonsäure-[3-(6,8-dichloro-2-methyl-1,2,3,4-tetrahydro- isochinolin-4-yl)-phenyl]-amid;
125) 4-Nitro-1H-pyrrol-2- carbonsäure-[3-(6,8-dichloro-2-methyl-1,2,3,4-tetrahydroisochinolin-4-yl)-phenyl]-amid;
126) 2,5-Dimethyl-1H-pyrrol-3- carbonsäure-[3-(6,8-dichloro-2-methyl-1,2,3,4-tetrahydro- isochuinolin-4-yl)-phenyl]-amid;
127) 1 H-Imidazol-4- carbonsäure-[3-(6,8-dichloro-2-methyl-1,2,3,4-tetrahydro-isochuinolin- 4-yl)-phenyl]-amid;
128) 1-Methansulfonyl-piperidin-4- carbonsäure-[3-(6,8-dichloro-2-methyl-1,2,3,4-tetrahydro-isochinolin-4-yl)-phenyl]-amid;
129) 3,5-Dimethyl-1 H-pyrazol-4- carbonsäure-[3-(6,8-dichloro-2-methyl-1,2,3,4-tetrahydro-isochinolin-4-yl)-phenyl]-amid;
130) 1 H-Pyrazol-4- carbonsäure-[3-(6,8-dichloro-2-methyl-1,2,3,4-tetrahydroisochinolin- 4-yl)-phenyl]-amid;
131) 3-Trifluoromethyl-1H-pyrazol-4- carbonsäure-[3-(6,8-dichloro-2-methyl-1,2,3,4-tetrahydro-isochinolin-4-yl)-phenyl]-amid;
132) 1-[2-(6,8-Dichloro-2-methyl-1,2,3,4-tetrahydro- isochinolin-4-yl)-phenyl]-3-ethyl-thioharnstoff;
133) 1-[4-(6,8-Dichloro-2-methyl-1,2,3,4-tetrahydro-isochinolin-4-yl)-phenyl]-3-ethyl-thioharnstoff;
134) 1-[3-(6,8-Dichloro-2-methyl-1,2,3,4-tetrahydro-isochinolin-4-yl)-phenyl]-3-ethyl-thioharnstoff;
135) 3-[3-(6,8-Dichloro-2-methyl-1,2,3,4-tetrahydro-isochinolin-4-yl)-phenyl]-1,1-dimethyl-harnstoff;
136) 4-Methyl-piperazin-1-carbonsäure-[3-(6,8-dichloro-2-methyl-1,2,3,4-tetrahydroisochinolin-4-yl)-phenyl]-amid;
137) Piperidin-1-carbonsäure-[3-(6,8-dichloro-2-methyl-1,2,3,4-tetrahydro-isochinolin-4- yl)-phenyl]-amid;
138) Morpholin-4-carbonsäure-[3-(6,8-dichloro-2-methyl-1,2,3,4-tetrahydro-isochinolin-4- yl)-phenyl]-amid;
139) Pyrrolidin-1-carbonsäure-[3-(6,8-dichloro-2-methyl-1,2,3,4-tetrahydro-isochinolin-4- yl)-phenyl]-amid;
140) 3-[3-(6,8-Dichloro-2-methyl-1,2,3,4-tetrahydro-isochinolin-4-yl)-phenyl]-1,1-diethyl-harnstoff;
141) 1-[3-(6,8-Dichloro-2-methyl-1,2,3,4-tetrahydro-isochinolin-4-yl)-phenyl]-3-methyl-harnstoff;
142) 1-[3-(6,8-Dichloro-2-methyl-1,2,3,4-tetrahydro-isochinolin-4-yl)-phenyl]-3-(2-dimethylamino-ethyl)- harnstoff;
143) 1-[3-(6,8-Dichloro-2-methyl-1,2,3,4-tetrahydro-isochinolin-4-yl)-phenyl]-3-(tetrahydro-furan-3-yl)-harnstoff;
144) 1-[3-(6,8-Dichloro-2-methyl-1,2,3,4-tetrahydro-isochinolin-4-yl)-phenyl]-3-(tetrahydro-pyran-4-yl)-harnstoff;
145) 3-[3-(6,8-Dichloro-2-methyl-1,2,3,4-tetrahydro-isochinolin-4-yl)-phenyl]-1-methyl-1-(1-methyl-piperidin-4-yl)-harnstoff;
146) 3-[3-(6,8-Dichloro-2-methyl-1,2,3,4-tetrahydro-isochinolin-4-yl)-phenyl]-1-(3-dimethylamino-propyl)-1-methyl-harnstoff;
147) 3-[3-(6,8-Dichloro-2-methyl-1,2,3,4-tetrahydro-isochinolin-4-yl)-phenyl]-1-(2-dimethylamino-ethyl)-1-methyl-harnstoff;
148) 1-[3-(6,8-Dichloro-2-methyl-1,2,3,4-tetrahydro-isochinolin-4-yl)-phenyl]-3-(3-dimethylamino-propyl)-harnstoff;
149) 1-[3-(6,8-Dichloro-2-methyl-1,2,3,4-tetrahydro-isochinolin-4-yl)-phenyl]-3-(2-methoxy-ethyl)-harnstoff;
150) 1-[3-(6,8-Dichloro-2-methyl-1,2,3,4-tetrahydro-isochinolin-4-yl)-phenyl]-3-pyridin-3-yl-harnstoff;
151) 1-[3-(6,8-Dichloro-2-methyl-1,2,3,4-tetrahydro-isochinolin-4-yl)-phenyl]-3-pyridin-4-yl-harnstoff;
152) 4-Methyl-piperazin-1-carbonsäure-[2-(6,8-dichloro-2-methyl-1,2,3,4-tetrahydro-isoquinolin-4-yl)-phenyl]-amid;
153) 1-[2-(6,8-Dichloro-2-methyl-1,2,3,4-tetrahydro-isochinolin-4-yl)-phenyl]-3-methyl-harnstoff;
154) 3-[2-(6,8-Dichloro-2-methyl-1,2,3,4-tetrahydro-isochinolin-4-yl)-phenyl]-1,1-dimethyl-harnstoff;
155) 3-[2-(6,8-Dichloro-2-methyl-1,2,3,4-tetrahydro-isochinolin-4-yl)-phenyl]-1,1-diethyl-harnstoff;
156) Piperidin-1-carbonsäure-[2-(6,8-dichloro-2-methyl-1,2,3,4-tetrahydro-isochinolin-4- yl)-phenyl]-amid;
157) Morpholin-4-carbonsäure-[2-(6,8-dichloro-2-methyl-1,2,3,4-tetrahydro-isochinolin-4- yl)-phenyl]-amid;
158) Pyrrolidin-1-carbonsäure-[2-(6,8-dichloro-2-methyl-1,2,3,4-tetrahydro-isochinolin-4- yl)-phenyl]-amid;
159) 1-[2-(6,8-Dichloro-2-methyl-1,2,3,4-tetrahydro-isochinolin-4-yl)-phenyl]-3-(2-dimethylamino-ethyl)-harnstoff;
160) 4-Methyl-piperazin-1-carbonsäure-[4-(6,8-dichloro-2-methyl-1,2,3,4-tetrahydroisochinolin-4-yl)-phenyl]-amid;
161) Pyrrolidin-1-carbonsäure-[4-(6,8-dichloro-2-methyl-1,2,3,4-tetrahydro-isochinolin-4- yl)-phenyl]-amid;
162) 1-[4-(6,8-Dichloro-2-methyl-1,2,3,4-tetrahydro-isochinolin-4-yl)-phenyl]-3-methyl-harnstoff;
163) 3-[4-(6,8-Dichloro-2-methyl-1,2,3,4-tetrahydro-isochinolin-4-yl)-phenyl]-1,1-dimethyl-harnstoff;
164) 3-[4-(6,8-Dichloro-2-methyl-1,2,3,4-tetrahydro-isochinolin-4-yl)-phenyl]-1,1-diethyl-harnstoff;
165) 1-[4-(6,8-Dichloro-2-methyl-1,2,3,4-tetrahydro-isochinolin-4-yl)-phenyl]-3-(2-dimethylamino-ethyl)-harnstoff;
166) Piperidin-1-carbonsäure-[4-(6,8-dichloro-2-methyl-1,2,3,4-tetrahydro-isochinolin-4- yl)-phenyl]-amid;
167) Morpholin-4-carbonsäure-[4-(6,8-dichloro-2-methyl-1,2,3,4-tetrahydro-isochinolin-4- yl)-phenyl]-amid;
168) N-[4-(6,8-Dichloro-2-methyl-1,2,3,4-tetrahydro-isochinolin-4-yl)-phenyl]-formamid;
169) [4-(6,8-Dichloro-2-methyl-1,2,3,4-tetrahydro-isochinolin-4-yl)-phenyl]-methyl-amin;
170) 1-[4-(6,8-Dichloro-2-methyl-1,2,3,4-tetrahydro-isochinolin-4-yl)-phenyl]-1,3-dimethyl-harnstoff;
171) 4-Methyl-piperazin-1-carbonsäure-[4-(6,8-dichloro-2-methyl-1,2,3,4-tetrahydroisochinolin-4-yl)-phenyl]-methyl-amid;
172) 1-[4-(6,8-Dichloro-2-methyl-1,2,3,4-tetrahydro-isochinolin-4-yl)-phenyl]-1,3,3-trimethyl-harnstoff;
173) Piperidin-1-carbonsäure-[4-(6,8-dichloro-2-methyl-1,2,3,4-tetrahydro-isochinolin-4- yl)-phenyl]-methyl-amid;
174) Morpholin-4-carbonsäure-[4-(6,8-dichloro-2-methyl-1,2,3,4-tetrahydro-isochinolin-4- yl)-phenyl]-methyl-amid;
175) Pyrrolidin-1-carbonsäure-[4-(6,8-dichloro-2-methyl-1,2,3,4-tetrahydro-isochinolin-4- yl)-phenyl]-methyl-amid;
176) 1-[4-(6,8-Dichloro-2-methyl-1,2,3,4-tetrahydro-isochinolin-4-yl)-phenyl]-3-(2-dimethylamino-ethyl)-1-methyl-harnstoff;
177) 1-[4-(6,8-Dichloro-2-methyl-1,2,3,4-tetrahydro-isochinolin-4-yl)-phenyl]-3,3-diethyl-1- methyl-harnstoff;
178) N-[3-(6,8-Dichloro-2-methyl-1,2,3,4-tetrahydro-isochinolin-4-yl)-phenyl]-formamid;
179) [3-(6,8-Dichloro-2-methyl-1,2,3,4-tetrahydro-isochinolin-4-yl)-phenyl]-methyl-amin;
180) Pyrrolidin-1-carbonsäure-[3-(6,8-dichloro-2-methyl-1,2,3,4-tetrahydro-isochinolin-4- yl)-phenyl]-methyl-amid;
181) Piperidin-1-carbonsäure-[3-(6,8-dichloro-2-methyl-1,2,3,4-tetrahydro-isochinolin-4- yl)-phenyl]-methyl-amid;
182) 1-[3-(6,8-Dichloro-2-methyl-1,2,3,4-tetrahydro-isochinolin-4-yl)-phenyl]-1,3,3-trimethyl-harnstoff;
183) 1-[3-(6,8-Dichloro-2-methyl-1,2,3,4-tetrahydro-isochinolin-4-yl)-phenyl]-1,3-dimethyl-harnstoff;
184) Morpholin-4-carbonsäure-[3-(6,8-dichloro-2-methyl-1,2,3,4-tetrahydro-isochinolin-4- yl)-phenyl]-methyl-amid;
185) 4-Methyl-piperazin-1-carbonsäure-[3-(6,8-dichloro-2-methyl-1,2,3,4-tetrahydroisochinolin-4-yl)-phenyl]-methyl-amid;
186) 1-[3-(6,8-Dichloro-2-methyl-1,2,3,4-tetrahydro-isochinolin-4-yl)-phenyl]-3-(2-dimethylamino-ethyl)-1-methyl-harnstoff;
187) 1-[3-(6,8-Dichloro-2-methyl-1,2,3,4-tetrahydro-isochinolin-4-yl)-phenyl]-3,3-diethyl-1-methyl-harnstoff;
188) [3-(6,8-Dichloro-2-methyl-1,2,3,4-tetrahydro-isochinolin-4-yl)-phenyl]-carbaminsäure-2-dimethylamino-ethyl-ester;
189) [4-(6,8-Dichloro-2-methyl-1,2,3,4-tetrahydro-isochinolin-4-yl)-phenyl]-carbaminsäure-2-dimethylamino-ethyl-ester;
190) [2-(6,8-Dichloro-2-methyl-1,2,3,4-tetrahydro-isochinolin-4-yl)-phenyl]-carbaminsäure-2-dimethylamino-ethyl-ester;
191) [3-(6,8-Dichloro-2-methyl-1,2,3,4-tetrahydro-isochinolin-4-yl)-phenyl]-carbaminsäure-methyl-ester;
192) [3-(6,8-Dichloro-2-methyl-1,2,3,4-tetrahydro-isochinolin-4-yl)-phenyl]-carbaminsäure-ethyl-ester;
193) [3-(6,8-Dichloro-2-methyl-1,2,3,4-tetrahydro-isochinolin-4-yl)-phenyl]-carbaminsäure-isopropyl-ester;
194) [3-(6,8-Dichloro-2-methyl-1,2,3,4-tetrahydro-isochinolin-4-yl)-phenyl]- carbaminsäure-2,2- dimethyl-propyl-ester;
195) [4-(6,8-Dichloro-2-methyl-1,2,3,4-tetrahydro-isochinolin-4-yl)-phenyl]- carbaminsäure-methyl-ester;
196) [4-(6,8-Dichloro-2-methyl-1,2,3,4-tetrahydro-isochinolin-4-yl)-phenyl]- carbaminsäure-isopropyl-ester;
197) [4-(6,8-Dichloro-2-methyl-1,2,3,4-tetrahydro-isochinolin-4-yl)-phenyl]- carbaminsäure-2,2- dimethyl-propyl-ester;
198) [4-(6,8-Dichloro-2-methyl-1,2,3,4-tetrahydro-isochinolin-4-yl)-phenyl]- carbaminsäure-ethyl-ester;
199) (R)-N-[3-(6,8-Dichloro-2-methyl-1,2,3,4-tetrahydro-isochinolin-4-yl)-phenyl]-methansulfonamid;
200) (S)-N-[3-(6,8-Dichloro-2-methyl-1,2,3,4-tetrahydro-isochinolin-4-yl)-phenyl]-methansulfonamid;
201) (R)-1-[2-(6,8-Dichloro-2-methyl-1,2,3,4-tetrahydro-isochinolin-4-yl)-phenyl]-3-ethyl-harnstoff;
202) (S)-1-[2-(6,8-Dichloro-2-methyl-1,2,3,4-tetrahydro-isochinolin-4-yl)-phenyl]-3-ethyl-harnstoff;
203) N-[3-(6,8-Difluoro-2-methyl-1,2,3,4-tetrahydro-isochinolin-4-yl)-phenyl]-acetamid;
204) 4-(3-Bromo-phenyl)-6,8-dichloro-2-methyl-1,2,3,4-tetrahydro-isochinolin;
205) 1-[3-(6,8-Dichloro-2-methyl-1,2,3,4-tetrahydro-isochinolin-4-yl)-phenyl]-3-(2-hydroxy-ethyl)-harnstoff;
206) 3-(6,8-Dichloro-2-methyl-1,2,3,4-tetrahydro-isochinolin-4-yl)-benzoesäure-ethylester;
207) 3-(6,8-Dichloro-2-methyl-1,2,3,4-tetrahydro-isochinolin-4-yl)-benzoesäure.
sowie deren pharmazeutisch verträgliche Salze.
sowie aus deren pharmazeutisch verträglichen Salzen.

3. Verbindung der Formel I nach Anspruch 1, ausgewählt aus der Gruppe bestehend aus:
1) N-[4-(6,8-Dichloro-2-methyl-1,2,3,4-tetrahydro-isochinolin-4-yl)-phenyl]-acetamid;
2) 4-(6,8-Dichloro-2-methyl-1,2,3,4-tetrahydro-isochinolin-4-yl)-benzolsulfonamid;
3) 3-(6,8-Dichloro-2-methyl-1,2,3,4-tetrahydro-isochinolin-4-yl)-benzolsulfonamid;
4) 1-[4-(6,8-Dichloro-2-methyl-1,2,3,4-tetrahydro-isochinolin-4-yl)-phenyl]-3-methyl-thioharnstoff;
5) 1-[4-(6,8-Dichloro-2-methyl-1,2,3,4-tetrahydro-isochinolin-4-yl)-phenyl]-3-ethyl-harnstoff;
6) N-[4-(6-Bromo-8-chloro-2-methyl-1,2,3,4-tetrahydro-isochinolin-4-yl)-phenyl]-acetamid;
7) N-[3-(6,8-Dichloro-2-methyl-1,2,3,4-tetrahydro-isochinolin-4-yl)-phenyl]-acetamid;
8) N-[4-(6,8-Dichloro-2-methyl-1,2,3,4-tetrahydro-isochinolin-4-yl)-phenyl]-propionamid;
9) 1-Acetyl-piperidin-4-carbonsäure-[4-(6,8-dichloro-2-methyl-1,2,3,4-tetrahydroisochinolin-4-yl)-phenyl]-amid;
10) N-[4-(6,8-Dichloro-2-methyl-1,2,3,4-tetrahydro-isochinolin-4-yl)-phenyl]-methansulfonamid;
11) Ethansulfonsäure-[4-(6,8-dichloro-2-methyl-1,2,3,4-tetrahydro-isochinolin-4-yl)-phenyl]-amid;
12) N',N'-Dimethylamino-N-[4-(6,8-Dichloro-2-methyl-1,2,3,4-tetrahydro-isochinolin-4-yl)-phenyl]-sulfamid;
13) N-[3-(6,8-Dichloro-2-methyl-1,2,3,4-tetrahydro-isochinolin-4-yl)-phenyl]-propionamid;
14) N-[3-(6,8-Dichloro-2-methyl-1,2,3,4-tetrahydro-isochinolin-4-yl)-phenyl]-butyramid;
15) N-[3-(6,8-Dichloro-2-methyl-1,2,3,4-tetrahydro-isochinolin-4-yl)-phenyl]-isobutyramid;
16) Cyclopropancarbonsäure-[3-(6,8-dichloro-2-methyl-1,2,3,4-tetrahydro-isochinolin-4-yl)-phenyl]-amid;
17) Cyclobutancarbonsäure-[3-(6,8-dichloro-2-methyl-1,2,3,4-tetrahydro-isochinolin-4-yl)- phenyl]-amid;
18) N-[3-(6,8-Dichloro-2-methyl-1,2,3,4-tetrahydro-isochinolin-4-yl)-phenyl]-2,2,2-trifluoro-acetamid;
19) 1-Acetyl-piperidin-4-carbonsäure-[3-(6,8-dichloro-2-methyl-1,2,3,4-tetrahydroisochinolin-4-yl)-phenyl]-amid;
20) N-[3-(6,8-Dichloro-2-methyl-1,2,3,4-tetrahydro-isochinolin-4-yl)-phenyl]-nicotinamid;
21) N-[3-(6,8-Dichloro-2-methyl-1,2,3,4-tetrahydro-isochinolin-4-yl)-phenyl]-methansulfonamid;
22) Ethansulfonsäure-[3-(6,8-dichloro-2-methyl-1,2,3,4-tetrahydro-isochinolin-4-yl)-phenyl]-amid;
23) N',N'-Dimethylamino-N-[3-(6,8-Dichloro-2-methyl-1,2,3,4-tetrahydro-isochinolin-4-yl)-phenyl]-sulfamid;
24) Cyclopropancarbonsäure-[2-(6,8-dichloro-2-methyl-1,2,3,4-tetrahydro-isochinolin-4-yl)-phenyl]-amid;
25) 1-Acetyl-piperidin-4-carbonsäure-[2-(6,8-dichloro-2-methyl-1,2,3,4-tetrahydroisochinolin-4-yl)-phenyl]-amid;
26) 1-[3-(6,8-Dichloro-2-methyl-1,2,3,4-tetrahydro-isochinolin-4-yl)-phenyl]-3-ethyl-harnstoff;
27) 1-[3-(6,8-Dichloro-2-methyl-1,2,3,4-tetrahydro-isochinolin-4-yl)-phenyl]-3-methyl-thioharnstoff;
28) 1-[2-(6,8-Dichloro-2-methyl-1,2,3,4-tetrahydro-isochinolin-4-yl)-phenyl]-3-ethyl-harnstoff;
29) 1-[2-(6,8-Dichloro-2-methyl-1,2,3,4-tetrahydro-isochinolin-4-yl)-phenyl]-3-methyl-thioharnstoff;
30) N-{5-[3-(6,8-Dichloro-2-methyl-1,2,3,4-tetrahydro-isochinolin-4-yl)-phenylsulfamoyl]-4-methyl-thiazol-2-yl}-acetamid;
31) 1,2-Dimethyl-1 H-imidazole-4-sulfonsäure-[3-(6,8-dichloro-2-methyl-1,2,3,4-tetrahydro- isochinolin-4-yl)-phenyl]-amid;
32) N-[4-(6,8-Dichloro-2-methyl-1,2,3,4-tetrahydro-isochinolin-4-yl)-phenyl]-C,C,C-trifluoro-methanesulfonamid;
33) N-[3-(6,8-Dichloro-2-methyl-1,2,3,4-tetrahydro-isochinolin-4-yl)-phenyl]-C,C,C-trifluoro-methanesulfonamid;
34) N-Ethyl-N'-4-(6,8-Dichloro-2-methyl-1,2,3,4-tetrahydro-isochinolin-4-yl)-benzolsulfonyl-harnstoff;
35) N-[4-(6,8-Dichloro-2-methyl-1,2,3,4-tetrahydro- isochinolin -4-yl)-phenyl]-2-dimethylamino-acetamid;
36) 2,6-Diamino-hexansäure-[4-(6,8-dichloro-2-methyl-1,2,3,4-tetrahydro- isochinolin -4-yl)- phenyl]-amid;
37) 1 H-Pyrrole-3-carbonsäure-[4-(6,8-dichloro-2-methyl-1,2,3,4-tetrahydroisochinolin-4- yl)-phenyl]-amid;
38) 1-Methyl-piperidin-4-carbonsäure-[4-(6,8-dichloro-2-methyl-1,2,3,4-tetrahydroisochinolin-4-yl)-phenyl]-amid;
39) 1-Methansulfonyl-piperidin-4-carbonsäure-[4-(6,8-dichloro-2-methyl-1,2,3,4-tetrahydro- isochinolin-4-yl)-phenyl]-amid;
40) 1 H-Pyrazol-4- carbonsäure-[4-(6,8-dichloro-2-methyl-1,2,3,4-tetrahydroisochinolin- 4-yl)-phenyl]-amid;
41) N-[3-(6,8-Dichloro-2-methyl-1,2,3,4-tetrahydro-isochinolin-4-yl)-phenyl]-2-methylamino-acetamid;
42) N-[3-(6,8-Dichloro-2-methyl-1,2,3,4-tetrahydro-isochinolin-4-yl)-phenyl]-2-dimethylamino-acetamid;
43) 2-Amino-N-[3-(6,8-dichloro-2-methyl-1,2,3,4-tetrahydro-isochinolin-4-yl)-phenyl]-propionamid;
44) 2-Amino-N-[3-(6,8-dichloro-2-methyl-1,2,3,4-tetrahydro-isochinolin-4-yl)-phenyl]-butyramid;
45) 2,6-Diamino-hexansäure-[3-(6,8-dichloro-2-methyl-1,2,3,4-tetrahydro-isochinolin-4-yl)- phenyl]-amid;
46) 1-Methyl-piperidin-4- carbonsäure-[3-(6,8-dichloro-2-methyl-1,2,3,4-tetrahydroisochinolin-4-yl)-phenyl]-amid;
47) 1 H-Imidazol-4- carbonsäure-[3-(6,8-dichloro-2-methyl-1,2,3,4-tetrahydro-isochuinolin- 4-yl)-phenyl]-amid;
48) 1-Methansulfonyl-piperidin-4- carbonsäure-[3-(6,8-dichloro-2-methyl-1,2,3,4-tetrahydro-isochinolin-4-yl)-phenyl]-amid;
49) 3,5-Dimethyl-1 H-pyrazol-4- carbonsäure-[3-(6,8-dichloro-2-methyl-1,2,3,4-tetrahydro-isochinolin-4-yl)-phenyl]-amid;
50) 1 H-Pyrazol-4- carbonsäure-[3-(6,8-dichloro-2-methyl-1,2,3,4-tetrahydroisochinolin- 4-yl)-phenyl]-amid;
51) 3-[3-(6,8-Dichloro-2-methyl-1,2,3,4-tetrahydro-isochinolin-4-yl)-phenyl]-1,1-dimethyl-harnstoff;
52) 4-Methyl-piperazin-1-carbonsäure-[3-(6,8-dichloro-2-methyl-1,2,3,4-tetrahydroisochinolin-4-yl)-phenyl]-amid;
53) Piperidin-1-carbonsäure-[3-(6,8-dichloro-2-methyl-1,2,3,4-tetrahydro-isochinolin-4- yl)-phenyl]-amid;
54) Morpholin-4-carbonsäure-[3-(6,8-dichloro-2-methyl-1,2,3,4-tetrahydro-isochinolin-4- yl)-phenyl]-amid;
55) Pyrrolidin-1-carbonsäure-[3-(6,8-dichloro-2-methyl-1,2,3,4-tetrahydro-isochinolin-4- yl)-phenyl]-amid;
56) 3-[3-(6,8-Dichloro-2-methyl-1,2,3,4-tetrahydro-isochinolin-4-yl)-phenyl]-1,1-diethyl-harnstoff;
57) 1-[3-(6,8-Dichloro-2-methyl-1,2,3,4-tetrahydro-isochinolin-4-yl)-phenyl]-3-methyl-harnstoff;
58) 1-[3-(6,8-Dichloro-2-methyl-1,2,3,4-tetrahydro-isochinolin-4-yl)-phenyl]-3-(2-dimethylamino-ethyl)- harnstoff;
59) 1-[3-(6,8-Dichloro-2-methyl-1,2,3,4-tetrahydro-isochinolin-4-yl)-phenyl]-3-(tetrahydro-furan-3-yl)-harnstoff;
60) 1-[3-(6,8-Dichloro-2-methyl-1,2,3,4-tetrahydro-isochinolin-4-yl)-phenyl]-3-(tetrahydro-pyran-4-yl)-harnstoff;
61) 3-[3-(6,8-Dichloro-2-methyl-1,2,3,4-tetrahydro-isochinolin-4-yl)-phenyl]-1-methyl-1-(1-methyl-piperidin-4-yl)-harnstoff;
62) 3-[3-(6,8-Dichloro-2-methyl-1,2,3,4-tetrahydro-isochinolin-4-yl)-phenyl]-1-(3-dimethylamino-propyl)-1-methyl-harnstoff;
63) 3-[3-(6,8-Dichloro-2-methyl-1,2,3,4-tetrahydro-isochinolin-4-yl)-phenyl]-1-(2-dimethylamino-ethyl)-1-methyl-harnstoff;
64) 1-[3-(6,8-Dichloro-2-methyl-1,2,3,4-tetrahydro-isochinolin-4-yl)-phenyl]-3-(3-dimethylamino-propyl)-harnstoff;
65) 1-[3-(6,8-Dichloro-2-methyl-1,2,3,4-tetrahydro-isochinolin-4-yl)-phenyl]-3-(2-methoxy-ethyl)-harnstoff;
66) 1-[3-(6,8-Dichloro-2-methyl-1,2,3,4-tetrahydro-isochinolin-4-yl)-phenyl]-3-pyridin-3-yl-harnstoff;
67) 1-[3-(6,8-Dichloro-2-methyl-1,2,3,4-tetrahydro-isochinolin-4-yl)-phenyl]-3-pyridin-4-yl-harnstoff;
68) 4-Methyl-piperazin-1-carbonsäure-[2-(6,8-dichloro-2-methyl-1,2,3,4-tetrahydro-isoquinolin-4-yl)-phenyl]-amid;
69) 1-[2-(6,8-Dichloro-2-methyl-1,2,3,4-tetrahydro-isochinolin-4-yl)-phenyl]-3-methyl-harnstoff;
70) 1-[2-(6,8-Dichloro-2-methyl-1,2,3,4-tetrahydro-isochinolin-4-yl)-phenyl]-3-(2-dimethylamino-ethyl)-harnstoff;
71) 4-Methyl-piperazin-1-carbonsäure-[4-(6,8-dichloro-2-methyl-1,2,3,4-tetrahydroisochinolin-4-yl)-phenyl]-amid;
72) 1-[4-(6,8-Dichloro-2-methyl-1,2,3,4-tetrahydro-isochinolin-4-yl)-phenyl]-3-methyl-harnstoff;
73) 3-[4-(6,8-Dichloro-2-methyl-1,2,3,4-tetrahydro-isochinolin-4-yl)-phenyl]-1,1-dimethyl-harnstoff;
74) 3-[4-(6,8-Dichloro-2-methyl-1,2,3,4-tetrahydro-isochinolin-4-yl)-phenyl]-1,1-diethyl-harnstoff;
75) 1-[4-(6,8-Dichloro-2-methyl-1,2,3,4-tetrahydro-isochinolin-4-yl)-phenyl]-3-(2-dimethylamino-ethyl)-harnstoff;
76) Morpholin-4-carbonsäure-[4-(6,8-dichloro-2-methyl-1,2,3,4-tetrahydro-isochinolin-4- yl)-phenyl]-amid;
77) N-[4-(6,8-Dichloro-2-methyl-1,2,3,4-tetrahydro-isochinolin-4-yl)-phenyl]-formamid;
78) N-[3-(6,8-Dichloro-2-methyl-1,2,3,4-tetrahydro-isochinolin-4-yl)-phenyl]-formamid;
79) 1-[3-(6,8-Dichloro-2-methyl-1,2,3,4-tetrahydro-isochinolin-4-yl)-phenyl]-1,3,3-trimethyl-harnstoff;
80) 1-[3-(6,8-Dichloro-2-methyl-1,2,3,4-tetrahydro-isochinolin-4-yl)-phenyl]-1,3-dimethyl-harnstoff;
81) Morpholin-4-carbonsäure-[3-(6,8-dichloro-2-methyl-1,2,3,4-tetrahydro-isochinolin-4- yl)-phenyl]-methyl-amid;
82) 4-Methyl-piperazin-1-carbonsäure-[3-(6,8-dichloro-2-methyl-1,2,3,4-tetrahydroisochinolin-4-yl)-phenyl]-methyl-amid;
83) 1-[3-(6,8-Dichloro-2-methyl-1,2,3,4-tetrahydro-isochinolin-4-yl)-phenyl]-3-(2-dimethylamino-ethyl)-1-methyl-harnstoff;
84) [3-(6,8-Dichloro-2-methyl-1,2,3,4-tetrahydro-isochinolin-4-yl)-phenyl]-carbaminsäure-2-dimethylamino-ethyl-ester;
85) [4-(6,8-Dichloro-2-methyl-1,2,3,4-tetrahydro-isochinolin-4-yl)-phenyl]-carbaminsäure-2-dimethylamino-ethyl-ester;
86) [2-(6,8-Dichloro-2-methyl-1,2,3,4-tetrahydro-isochinolin-4-yl)-phenyl]-carbaminsäure-2-dimethylamino-ethyl-ester;
87) [3-(6,8-Dichloro-2-methyl-1,2,3,4-tetrahydro-isochinolin-4-yl)-phenyl]-carbaminsäure-methyl-ester;
88) (R oder S)-N-[3-(6,8-Dichloro-2-methyl-1,2,3,4-tetrahydro-isochinolin-4-yl)-phenyl]- methansulfonamid;
89) (R oder S)-1-[2-(6,8-Dichloro-2-methyl-1,2,3,4-tetrahydro-isochinolin-4-yl)-phenyl]-3-ethyl-harnstoff;
90) 1-[3-(6,8-Dichloro-2-methyl-1,2,3,4-tetrahydro-isochinolin-4-yl)-phenyl]-3-(2-hydroxy-ethyl)-harnstoff;
sowie aus deren pharmazeutisch verträglichen Salzen.

4. Verwendung einer Verbindung I nach einem oder mehreren der Ansprüche 1 bis 3 zur Herstellung eines Medikaments zur Behandlung oder Prophylaxe von Störungen des Atemantriebs.

5. Verwendung einer Verbindung I nach einem oder mehreren der Ansprüche 1 bis 3 zur Herstellung eines Medikaments zur Behandlung oder Prophylaxe von Atemstörungen, insbesondere Schlaf-bedingten Atemstörungen wie Schlafapnoen.

6. Verwendung einer Verbindung I nach einem oder mehreren der Ansprüche 1 bis 3 zur Herstellung eines Medikaments zur Behandlung oder Prophylaxe des Schnarchens.

7. Verwendung einer Verbindung I nach einem oder mehreren der Ansprüche 1 bis 3 zur Herstellung eines Medikaments zur Behandlung oder zur Prophylaxe von akuten und chronischen Nierenerkrankungen, besonders des akuten Nierenversagens und des chronischen Nierenversagens.

8. Verwendung einer Verbindung I nach einem oder mehreren der Ansprüche 1 bis 3 zur Herstellung eines Medikaments zur Behandlung oder Prophylaxe von Störungen der Darmfunktion.

9. Verwendung einer Verbindung I nach einem oder mehreren der Ansprüche 1 bis 3 Herstellung eines Medikaments zur Behandlung oder Prophylaxe von Störungen der Gallenfunktion.

10. Verwendung einer Verbindung I nach einem oder mehreren der Ansprüche 1 bis 3 zur Herstellung eines Medikaments zur Behandlung oder Prophylaxe von ischämischen Zuständen des peripheren und zentralen Nervensystems und des Schlaganfalls.

11. Verwendung einer Verbindung I nach einem oder mehreren der Ansprüche 1 bis 3 zur Herstellung eines Medikaments zur Behandlung oder Prophylaxe von ischämischen Zuständen peripherer Organe und Gliedmaßen.

12. Verwendung einer Verbindung I nach einem oder mehreren der Ansprüche 1 bis 3 zur Herstellung eines Medikaments zur Behandlung von Schockzuständen.

13. Verwendung einer Verbindung I nach einem oder mehreren der Ansprüche 1 bis 3 zur Herstellung eines Medikaments zu Einsatz bei chirurgischen Operationen und Organtransplantationen.

14. Verwendung einer Verbindung I nach einem oder mehreren der Ansprüche 1 bis 3 zur Herstellung eines Medikaments zur Konservierung und Lagerung von Transplantaten für chirurgische Maßnahmen.

15. Verwendung einer Verbindung I nach einem oder mehreren der Ansprüche 1 bis 3 zur Herstellung eines Medikaments zur Behandlung von Krankheiten, bei denen die Zellproliferation eine primäre oder sekundäre Ursache darstellt.

16. Verwendung einer Verbindung I nach einem oder mehreren der Ansprüche 1 bis 3 zur Herstellung eines Medikaments zur Behandlung oder Prophylaxe von Störungen des Fettstoffwechsels.

17. Verwendung einer Verbindung I nach einem oder mehreren der Ansprüche 1 bis 3 zur Herstellung eines Medikaments zur Behandlung oder Prophylaxe des Befalls durch Ektoparasiten.

18. Heilmittel, enthaltend eine wirksame Menge einer Verbindung I nach Anspruch 1.

## Claims

1. 4-Phenyltetrahydroisoquinolines of the formula I 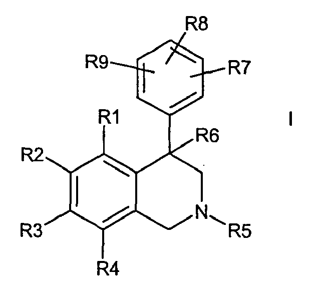 in which the meanings are:
R1, R2, R3 and R4 independently of one another H, F, Cl, Br, OH, NH₂, CₐH₂ₐ₊₁, cycloalkyl with 3, 4, 5 or 6 C atoms, OC_{b}H_{2b+1};
a and b in the groups CₐH₂ₐ₊₁ and OC_{b}H_{2b+1} independently of one another 1, 2, 3 or 4, it being possible for one or more H atoms to be replaced by F atoms;
or
R1, R2, R3 and R4 independently of one another NR11 R12;
R11 and R12 independently of one another H, CₑH₂ₑ₊₁, CᵣᵣH₂ᵣᵣ₋₁;
e 1, 2, 3 or 4,
rr 3, 4, 5 or 6,
it being possible for one or more H atoms in the groups CₑH₂ₑ₊₁ and CᵣᵣH₂ᵣᵣ₋₁ to be replaced by F atoms;
or
R11 and R12 together with the N atom to which they are bonded form a ring selected from the group consisting of pyrrolidine, piperidine, N-methylpiperazine, piperazine and morpholine;
or
R11 and R12 independently of one another COR14, CSR14, CONHR14, CSNHR14 or SO₂R14;
R14 CgH2g+1;
g 1, 2, 3 or 4, it being possible for one or more H atoms to be replaced by F atoms;
or
R1, R2, R3 and R4 independently of one another OSO₃H, SO₃H, SO₂R15;
R15 CₖH₂ₖ₊₁ or NR17R18;
k 1, 2, 3 or 4, it being possible for one or more H atoms to be replaced by F atoms;
R17 and R18 independently of one another H or CₘH₂ₘ₊₁;
m 1, 2, 3, 4 or 5, it being possible for one or more H atoms in the group CₘH₂ₘ₊₁ to be replaced by F atoms;
or
R17 and R18 together with the N atom to which they are bonded a 5- or 6-membered ring;
but where R2 must always not be equal to H;
R5 methyl or trifluoromethyl;
R6 H;
R7, R8 and R9 independently of one another OSO₃H, SO₃H or SO₂R23;
R23 CₙₙH₂ₙₙ₊₁ or NR25R26;
nn 1, 2, 3, 4 or 5,
it being possible for one or more H atoms in CₙₙH₂ₙₙ₊₁ to be replaced by F atoms;
R25 and R26 independently of one another H, CN or C_{z}H_{2z+1}, in which the first CH₂ group bonded to the nitrogen is replaced by CO or CS and the second CH₂ is replaced by NR27;
z 1, 2, 3, 4, 5 or 6;
it being possible for one or more H atoms in C_{z}H_{2z+1} to be replaced by F atoms;
R27 H or CₐₐH₂ₐₐ₊₁;
aa 1, 2, 3 or 4;
it being possible for one or more H atoms in CₐₐH₂ₐₐ₊₁ to be replaced by F atoms;
or
R25 and R26 together with the N atom to which they are bonded a 5- or 6-membered ring,
or
R27 and a CH₂ group of R25 or R26 together with the N atom to which they are bonded a 5- or 6-membered ring;
or
R7, R8 and R9 independently of one another NR32COR30, NR32CSR30 or NR32SO₂R30;
R30 H, OH, C_{cc}H_{2cc+1}, C_{yy}H_{2yy-1}, pyrrolidinyl or piperidinyl, in which rings a CH₂ group may be replaced by O or NR33;
R32 and R33 H, methyl or CF₃;
cc 1, 2, 3, 4, 5 , 6, 7 or 8;
yy 3, 4, 5 or 6;
it being possible for one or more H atoms in the groups C_{cc}H_{2cc+1} and C_{yy}H_{2yy-1} to be replaced by F atoms and for one or more CH₂ groups to be replaced by NR31 and for a CH₂ group to be replaced by O;
R31 H, methyl, ethyl, CF₃, CH₂CF₃, acetyl, propionyl, methanesulfonyl or ethanesulfonyl;
or
R31 together with a CH₂ group of R30 and the N atom to which they are jointly bonded form a 5- or 6-membered ring;
or
R30 pyridyl, imidazolyl, pyrazolyl, pyrrolyl, triazolyl, tetrazolyl, thiazolyl or oxazolyl, which are unsubstituted or substituted by a maximum of 3 substituents selected from the group consisting of F, Cl, methyl, ethyl, trifluoromethyl, NH₂, NHacetyl;
or
R7, R8 and R9 independently of one another H, F, Cl, OH, NH₂, CₑₑH₂ₑₑ₊₁, C_{ww}H_{2ww-1}, OC_{ff}H_{2ff+1}, NR40R41, CONR40R41, COOR42 or OCOR42,
ee and ff independently of one another 1, 2, 3 or 4;
ww 3, 4, 5 or 6,
it being possible for one or more H atoms in the groups CₑₑH₂ₑₑ₊₁, C_{ww}H_{2ww-1} and OC_{ff}H_{2ff+1} to be replaced by F atoms;
R40 and R41 H, CₜₜH₂ₜₜ₊₁ or C(NH)NH₂;
tt 1, 2, 3 or 4;
it being possible for one or more H atoms in the group CₜₜH₂ₜₜ₊₁ to be replaced by F atoms;
or
R40 and R41 independently of one another hydroxyethyl, N,N-dimethylaminoethyl, N,N-diethylaminoethyl, pyrrolidinoethyl, N-methylpiperazinoethyl, piperazinoethyl, morpholinoethyl or piperidinoethyl;
or
R40 and R41 together with the N atom to which they are bonded a pyrrolidine, piperidine, N-methylpiperazine, piperazine or morpholine ring;
R42 H or CₕₕH₂ₕₕ₊₁;
hh 1, 2, 3 or 4;
it being possible for one or more H atoms in the group CₕₕH₂ₕₕ₊₁ to be replaced by F atoms;
but where two substituents from the group of R7, R8 and R9 may not simultaneously be OH or OCH₃,
and where at least one of the radicals R7, R8 or R9 must be selected from the group consisting of -OᵥSO_{w}R23, NR32COR30, NR32CSR30 and NR32SO_{bb}R30; and the pharmaceutically acceptable salts and trifluoroacetates thereof.

2. Compound of the formula I **characterized in that** it is selected from the group consisting of:
1) N-[4-(6,8-dichloro-2-methyl-1,2,3,4-tetrahydro-isoquinolin-4-yl)-phenyl]-acetamide;
2) 4-(6,8-dichloro-2-methyl-1,2,3,4-tetrahydro-isoquinolin-4-yl)-benzenesulfonamide;
3) 3-(6,8-dichloro-2-methyl-1,2,3,4-tetrahydro-isoquinolin-4-yl)-benzenesulfonamide;
4) 4-(6,8-dichloro-2-methyl-1,2,3,4-tetrahydro-isoquinolin-4-yl)-N,N-dimethyl-benzenesulfonamide;
5) 4-(4-bromo-phenyl)-6,8-dichloro-2-methyl-1,2,3,4-tetrahydroisoquinoline;
6) 4-(6,8-dichloro-2-methyl-1,2,3,4-tetrahydro-isoquinolin-4-yl)-benzoic acid;
7) 4-(6,8-dichloro-2-methyl-1,2,3,4-tetrahydroisoquinolin-4-yl)-N-ethyl-benzamide;
8) 4-(6,8-dichloro-2-methyl-1,2,3,4-tetrahydroisoquinolin-4-yl)-N-propyl-benzamide;
9) 4-(6,8-dichloro-2-methyl-1,2,3,4-tetrahydro-isoquinolin-4-yl)-N-(2-dimethylamino-ethyl)-benzamide;
10) 6,8-dichloro-2-methyl-4-(4-morpholin-4-yl-phenyl)-1,2,3,4-tetrahydroisoquinoline;
11) [4-(6,8-dichloro-2-methyl-1,2,3,4-tetrahydroisoquinolin-4-yl)-phenyl]-diethyl-amine
12) 6,8-dichloro-2-methyl-4-(4-piperidin-1-yl-phenyl)-1,2,3,4-tetrahydroisoquinoline;
13) 6,8-dichloro-2-methyl-4-(4-pyrrolidin-1-yl-phenyl)-1,2,3,4-tetrahydroisoquinoline;
14) 6,8-dichloro-2-methyl-4-[4-(4-methyl-piperazin-1-yl)-phenyl]-1,2,3,4-tetrahydroisoquinoline;
15) 6,8-dichloro-2-cyclopropyl-4-phenyl-1,2,3,4-tetrahydroisoquinoline;
16) 4-(6,8-dichloro-2-methyl-1,2,3,4-tetrahydro-isoquinolin-4-yl)-phenylamine;
17) 1-[4-(6,8-dichloro-2-methyl-1,2,3,4-tetrahydro-isoquinolin-4-yl)-phenyl]-3-propylurea;
18) 1-[4-(6,8-dichloro-2-methyl-1,2,3,4-tetrahydro-isoquinolin-4-yl)-phenyl]-3-methyl-thiourea;
19) 1-[4-(6,8-dichloro-2-methyl-1,2,3,4-tetrahydro-isoquinolin-4-yl)-phenyl]-3-ethyl-urea;
20) N-[4-(6-methanesulfonyl-2-methyl-1,2,3,4-tetrahydro-isoquinolin-4-yl)-phenyl]-acetamide;
21) N-[4-(2,6,8-trimethyl-1,2,3,4-tetrahydro-isoquinolin-4-yl)-phenyl]-acetamide;
22) N-[4-(6-bromo-8-chloro-2-methyl-1,2,3,4-tetrahydro-isoquinolin-4-yl)-phenyl]-acetamide;
23) N-[4-(8-chloro-2-methyl-6-pyrrolidin-1-yl-1,2,3,4-tetrahydro-isoquinolin-4-yl)-phenyl]-acetamide;
24) N-[4-(8-chloro-2-methyl-6-morpholin-4-yl-1,2,3,4-tetrahydro-isoquinolin-4-yl)-phenyl]-acetamide;
25) N-{4-[8-chloro-2-methyl-6-(4-methyl-piperazin-1-yl)-1,2,3,4-tetrahydro-isoquinolin-4-yl]-phenyl}-acetamide;
26) N-{4-[8-chloro-6-(cyclopropylmethyl-amino)-2-methyl-1,2,3,4-tetrahydro-isoquinolin-4-yl]-phenyl}-acetamide;
27) 5-(6,8-dichloro-2-methyl-1,2,3,4-tetrahydro-isoquinolin-4-yl)-2-hydroxy-benzoic acid;
28) 5-(6,8-dichloro-2-methyl-1,2,3,4-tetrahydro-isoquinolin-4-yl)-2-hydroxy-N-methyl-benzamide;
29) 5-(6,8-dichloro-2-methyl-1,2,3,4-tetrahydro-isoquinolin-4-yl)-N-ethyl-2-hydroxybenzamide;
30) 5-(6,8-dichloro-2-methyl-1,2,3,4-tetrahydro-isoquinolin-4-yl)-N-(2-dimethylamino-ethyl)-2-hydroxy-benzamide;
31) N-[5-(6,8-dichloro-2-methyl-1,2,3,4-tetrahydro-isoquinolin-4-yl)-2-hydroxybenzoyl]-guanidine;
32) N-[3-(6,8-dichloro-2-methyl-1,2,3,4-tetrahydro-isoquinolin-4-yl)-phenyl]-acetamide;
33) 3-(6,8-dich(oro-2-methyl-1,2,3,4-tetrahydro-isoquinolin-4-yl)-phenylamine;
34) 2-(6,8-dichloro-2-methyl-1,2,3,4-tetrahydro-isoquinolin-4-yl)-phenylamine;
35) N-[4-(6,8-dichloro-2-methyl-1,2,3,4-tetrahydro-isoquinolin-4-yl)-phenyl]-propionamide;
36) N-[4-(6,8-dichloro-2-methyl-1,2,3,4-tetrahydro-isoquinolin-4-yl)-phenyl]-butyramide;
37) N-[4-(6,8-dichloro-2-methyl-1,2,3,4-tetrahydro-isoquinolin-4-yl)-phenyl]pentanamide;
38) N-[4-(6,8-dichloro-2-methyl-1,2,3,4-tetrahydro-isoquinolin-4-yl)-phenyl]-isobutyramide;
39) N-[4-(6,8-dichloro-2-methyl-1,2,3,4-tetrahydro-isoquinolin-4-yl)-phenyl]-2,2-dimethyl-propionamide;
40) N-[4-(6,8-dichloro-2-methyl-1,2,3,4-tetrahydro-isoquinolin-4-yl)-phenyl]-cyclopropanecarboxamide;
41) N-[4-(6,8-dichloro-2-methyl-1,2,3,4-tetrahydro-isoquinolin-4-yl)-phenyl]-cyclobutanecarboxamide;
42) N-[4-(6,8-dichloro-2-methyl-1,2,3,4-tetrahydro-isoquinolin-4-yl)-phenyl]-cyclopentanecarboxamide;
43) N-[4-(6,8-dichloro-2-methyl-1,2,3,4-tetrahydro-isoquinolin-4-yl)-phenyl]-2,2,2-trifluoro-acetamide;
44) N-[4-(6,8-dichloro-2-methyl-1,2,3,4-tetrahydro-isoquinolin-4-yl)-phenyl]-1-acetylpiperidine-4-carboxamide;
45) N-[4-(6,8-dichloro-2-methyl-1,2,3,4-tetrahydro-isoquinolin-4-yl)-phenyl]-nicotinamide;
46) N-[4-(6,8-dichloro-2-methyl-1,2,3,4-tetrahydro-isoquinolin-4-yl)-phenyl]-methane-sulfonamide;
47) N-[4-(6,8-dichloro-2-methyl-1,2,3,4-tetrahydro-isoquinolin-4-yl)- phenyl]-ethanesulfonamide;
48) N',N'-dimethylamino-N-[4-(6,8-dichloro-2-methyl-1,2,3,4-tetrahydro-isoquinolin-4-yl)-phenyl]-sulfamide;
49) N-[3-(6,8-dichloro-2-methyl-1,2,3,4-tetrahydro-isoquinolin-4-yl)-phenyl]-propionamide;
50) N-[3-(6,8-dichloro-2-methyl-1,2,3,4-tetrahydro-isoquinolin-4-yl)-phenyl]-butyramide;
51) N-[3-(6,8-dichloro-2-methyl-1,2,3,4-tetrahydro-isoquinolin-4-yl)-phenyl]-pentanamide;
52) N-[3-(6,8-dichloro-2-methyl-1,2,3,4-tetrahydro-isoquinolin-4-yl)-phenyl]-isobutyramide;
53) N-[3-(6,8-dichloro-2-methyl-1,2,3,4-tetrahydro-isoquinolin-4-yl)-phenyl]-2,2-dimethyl-propionamide;
54) N-[3-(6,8-dichloro-2-methyl-1,2,3,4-tetrahydro-isoquinolin-4-yl)-phenyl]-cyclopropanecarboxamide;
55) N-[3-(6,8-dichloro-2-methyl-1,2,3,4-tetrahydro-isoquinolin-4-yl)-phenyl]-cyclobutanecarboxamide;
56) N-[3-(6,8-dichloro-2-methyl-1,2,3,4-tetrahydro-isoquinolin-4-yl)-phenyl]-cyclopentanecarboxamide;
57) N-[3-(6,8-dichloro-2-methyl-1,2,3,4-tetrahydro-isoquinolin-4-yl)-phenyl]-2,2,2-trifluoro-acetamide;
58) N-[3-(6,8-dichloro-2-methyl-1,2,3,4-tetrahydro-isoquinolin-4-yl)-phenyl]-1-acetylpiperidine-4-carboxamide;
59) N-[3-(6,8-dichloro-2-methyl-1,2,3,4-tetrahydro-isoquinolin-4-yl)-phenyl]-nicotinamide;
60) N-[3-(6,8-dichloro-2-methyl-1,2,3,4-tetrahydro-isoquinolin-4-yl)-phenyl]-methane-sulfonamide;
61) N-[3-(6,8-dichloro-2-methyl-1,2,3,4-tetrahydro-isoquinolin-4-yl)- phenyl]-ethanesulfonamide;
62) N',N'-dimethylamino-N-[3-(6,8-dichloro-2-methyl-1,2,3,4-tetrahydro-isoquinolin-4-yl)-phenyl]-sulfamide;
63) N-[2-(6,8-dichloro-2-methyl-1,2,3,4-tetrahydro-isoquinolin-4-yl)-phenyl]-propionamide;
64) N-[2-(6,8-dichloro-2-methyl-1,2,3,4-tetrahydro-isoquinolin-4-yl)-phenyl]-butyramide;
65) N-[2-(6,8-dichloro-2-methyl-1,2,3,4-tetrahydro-isoquinolin-4-yl)-phenyl]-pentanamide;
66) N-[2-(6,8-dichloro-2-methyl-1,2,3,4-tetrahydro-isoquinolin-4-yl)-phenyl]-isobutyramide;
67) N-[2-(6,8-dichloro-2-methyl-1,2,3,4-tetrahydro-isoquinolin-4-yl)-phenyl]-2,2-dimethyl-propionamide;
68) N-[2-(6,8-dichloro-2-methyl-1,2,3,4-tetrahydro-isoquinolin-4-yl)-phenyl]cyclopropanecarbox-amide;
69) N-[2-(6,8-dichloro-2-methyl-1,2,3,4-tetrahydro-isoquinolin-4-yl)- phenyl]-cyclobutanecarboxamide;
70) N-[2-(6,8-dichloro-2-methyl-1,2,3,4-tetrahydro-isoquinolin-4-yl)-phenyl]-cyclopentanecarboxamide;
71) N-[2-(6,8-dichloro-2-methyl-1,2,3,4-tetrahydro-isoquinolin-4-yl)-phenyl]-2,2,2-trifluoro-acetamide;
72) N-[2-(6,8-dichloro-2-methyl-1,2,3,4-tetrahydro-isoquinolin-4-yl)-phenyl]-1-acetylpiperidine-4-carboxamide;
73) N-[2-(6,8-dichloro-2-methyl-1,2,3,4-tetrahydro-isoquinolin-4-yl)-phenyl]-methane-sulfonamide;
74) N-[2-(6,8-dichloro-2-methyl-1,2,3,4-tetrahydro-isoquinolin-4-yl)-phenyl]-ethanesulfonamide;
75) N',N'-dimethylamino-N-[2-(6,8-dichloro-2-methyl-1,2,3,4-tetrahydro-isoquinolin-4-yl)-phenyl]-sulfamide;
76) 1-[3-(6,8-dichloro-2-methyl-1,2,3,4-tetrahydro-isoquinolin-4-yl)-phenyl]-3-ethyl-urea;
77) 1-[3-(6,8-dichloro-2-methyl-1,2,3,4-tetrahydro-isoquinolin-4-yl)-phenyl]-3-methyl-thiourea;
78) 1-[2-(6,8-dichloro-2-methyl-1,2,3,4-tetrahydro-isoquinolin-4-yl)-phenyl]-3-ethyl-urea;
79) 1-[2-(6,8-dichloro-2-methyl-1,2,3,4-tetrahydro-isoquinolin-4-yl)-phenyl]-3-methyl-thiourea;
80) N-{5-[4-(6,8-dichloro-2-methyl-1,2,3,4-tetrahydro-isoquinolin-4-yl)-phenylsulfamoyl]-4-methyl-thiazol-2-yl}-acetamide;
81) N-{5-[3-(6,8-dichloro-2-methyl-1,2,3,4-tetrahydro-isoquinolin-4-yl)-phenylsulfamoyl]-4-methyl-thiazol-2-yl}-acetamide;
82) N-[4-(6,8-dichloro-2-methyl-1,2,3,4-tetrahydro-isoquinolin-4-yl)-phenyl]-1,2-dimethyl-1 H-imidazole-4-sulfonamide;
83) N-[3-(6,8-dichloro-2-methyl-1,2,3,4-tetrahydro-isoquinolin-4-yl)-phenyl]-1,2-dimethyl-1 H-imidazole-4-sulfonamide;
84) N-[4-(6,8-dichloro-2-methyl-1,2,3,4-tetrahydro-isoquinolin-4-yl)-phenyl]-5-chloro-1,3-dimethyl-1H-pyrazole-4-sulfonamide;
85) N-[3-(6,8-dichloro-2-methyl-1,2,3,4-tetrahydro-isoquinolin-4-yl)-phenyl]-5-chloro-1,3-dimethyl-1H-pyrazole-4-sulfonamide;
86) N-[4-(6,8-dichloro-2-methyl-1,2,3,4-tetrahydro-isoquinolin-4-yl)-phenyl]-5-bromothiophene-2-sulfonamide;
87) N-[3-(6,8-dichloro-2-methyl-1,2,3,4-tetrahydro-isoquinolin-4-yl)-phenyl]-5-bromothiophene-2-sulfonamide;
88) N-[4-(6,8-dichloro-2-methyl-1,2,3,4-tetrahydro-isoquinolin-4-yl)-phenyl]-C,C,C-trifluoro-methanesulfonamide;
89) N-[3-(6,8-dichloro-2-methyl-1,2,3,4-tetrahydro-isoquinolin-4-yl)-phenyl]-C,C,C-trifluoro-methanesulfonamide;
90) 4-(6,8-dichloro-2-methyl-1,2,3,4-tetrahydro-isoquinolin-4-yl)-phenyl]-2,2,2-trifluoroethanesulfonamide;
91) 3-(6,8-dichloro-2-methyl-1,2,3,4-tetrahydro-isoquinolin-4-yl)-phenyl]-2,2,2-trifluoro-ethanesulfonamide;
92) N-ethyl-N'-4-(6,8-Dichloro-2-methyl-1,2,3,4-tetrahydro-isoquinolin-4-yl)-benzenesulfonylurea;
93) 2-chloro-5-(6,8-dichloro-2-methyl-1,2,3,4-tetrahydro-isoquinolin-4-yl)-benzenesulfonamide;
94) 2-methyl-4-phenyl-6,8-bis-trifluoromethyl-1,2,3,4-tetrahydro-isoquinoline;
95) 2-amino-N-[4-(6,8-dichloro-2-methyl-1,2,3,4-tetrahydro-isoquinolin-4-yl)-phenyl]-acetamide;
96) N-[4-(6,8-dichloro-2-methyl-1,2,3,4-tetrahydro-isoquinolin-4-yl)-phenyl]-2-methylamino-acetamide;
97) N-[4-(6,8-dichloro-2-methyl-1,2,3,4-tetrahydro-isoquinolin-4-yl)-phenyl]-2-dimethylamino-acetamide;
98) 2-amino-N-[4-(6,8-dichloro-2-methyl-1,2,3,4-tetrahydro-isoquinolin-4-yl)-phenyl]-propionamide;
99) 2-amino-N-[4-(6,8-dichloro-2-methyl-1,2,3,4-tetrahydro-isoquinolin-4-yl)-phenyl]-butyramide;
100) 2,6-diamino-N-[4-(6,8-dichloro-2-methyl-1,2,3,4-tetrahydro-isoquinolin-4-yl)-phenyl]-hexanamide;
101) N-[4-(6,8-dichloro-2-methyl-1,2,3,4-tetrahydro-isoquinolin-4-yl)-phenyl]-pyrrolidine-2-carboxamide;
102) N-[4-(6,8-dichloro-2-methyl-1,2,3,4-tetrahydro-isoquinolin-4-yl)-phenyl]-isonicotinamide;
103) N-[4-(6,8-dichloro-2-methyl-1,2,3,4-tetrahydro-isoquinolin-4-yl)-phenyl]-1H-pyrrole-3-carboxamide;
104) N-[4-(6,8-dichloro-2-methyl-1,2,3,4-tetrahydro-isoquinolin-4-yl)-phenyl]-1H-pyrrole-2-carboxamide;
105) N-[4-(6,8-dichloro-2-methyl-1,2,3,4-tetrahydro-isoquinolin-4-yl)-phenyl]-1-methylpiperidine-4-carboxamide;
106) N-[4-(6,8-dichloro-2-methyl-1 ,2,3,4-tetrahydro-isoquinolin-4-yl)-phenyl]-1,4-dimethyl-1H-pyrrole-2-carboxamide;
107) N-[4-(6,8-dichloro-2-methyl-1,2,3,4-tetrahydro-isoquinolin-4-yl)-phenyl]-4-nitro-1 H-pyrrole-2-carboxamide;
108) N-[4-(6,8-dichloro-2-methyl-1,2,3,4-tetrahydro-isoquinolin-4-yl)-phenyl]-2,5-dimethyl-1 H-pyrrole-3-carboxamide;
109) N-[4-(6,8-dichloro-2-methyl-1,2,3,4-tetrahydro-isoquinolin-4-yl)-phenyl]-1H-imidazole-4-carboxamide;
110) N-[4-(6,8-dichloro-2-methyl-1,2,3,4-tetrahydro-isoquinolin-4-yl)-phenyl]-1-methanesulfonyl-piperidine-4-carboxamide;
111) N-[4-(6,8-dichloro-2-methyl-1,2,3,4-tetrahydro-isoquinolin-4-yl)-phenyl]-3,5-dimethyl-1H-pyrazole-4-carboxamide;
112) N-[4-(6,8-dichloro-2-methyl-1,2,3,4-tetrahydro-isoquinolin-4-yl)-phenyl]-1H-pyrazole-4-carboxamide;
113) N-[4-(6,8-dichloro-2-methyl-1,2,3,4-tetrahydro-isoquinolin-4-yl)-phenyl]-3-trifluoromethyl-1H-pyrazole-4-carboxamide;
114) N-[3-(6,8-dichloro-2-methyl-1,2,3,4-tetrahydro-isoquinolin-4-yl)-phenyl]-2-methylamino-acetamide;
115) N-[3-(6,8-dichloro-2-methyl-1,2,3,4-tetrahydro-isoquinolin-4-yl)-phenyl]-2-dimethylamino-acetamide;
116) 2-amino-N-[3-(6,8-dichloro-2-methyl-1,2,3,4-tetrahydro-isoquinolin-4-yl)-phenyl]-propionamide;
117) 2-amino-N-[3-(6,8-dichloro-2-methyl-1,2,3,4-tetrahydro-isoquinolin-4-yl)-phenyl]-butyramide;
118) 2,6-diamino-N-[3-(6,8-dichloro-2-methyl-1,2,3,4-tetrahydro-isoquinolin-4-yl)-phenyl]-hexanamide;
119) N-[3-(6,8-dichloro-2-methyl-1,2,3,4-tetrahydro-isoquinolin-4-yl)-phenyl]-pyrrolidine-2-carboxamide;
120) N-[3-(6,8-dichloro-2-methyl-1,2,3,4-tetrahydro-isoquinolin-4-yl)-phenyl]-isonicotinamide;
121) N-[3-(6,8-dichloro-2-methyl-1,2,3,4-tetrahydro-isoquinolin-4-yl)-phenyl]-1H-pyrrole-3-carboxamide;
122) N-[3-(6,8-dichloro-2-methyl-1,2,3,4-tetrahydro-isoquinolin-4-yl)-phenyl]-1H-pyrrole-2-carboxamide;
123) N-[3-(6,8-dichloro-2-methyl-1,2,3,4-tetrahydro-isoquinolin-4-yl)-phenyl]-1-methylpiperidine-4-carboxamide;
124) N-[3-(6,8-dichloro-2-methyl-1,2,3,4-tetrahydro-isoquinolin-4-yl)-phenyl]-1,4-dimethyl-1 H-pyrrole-2-carboxamide;
125) N-[3-(6,8-dichloro-2-methyl-1,2,3,4-tetrahydro-isoquinolin-4-yl)-phenyl]-4-nitro-1H-pyrrole-2-carboxamide;
126) N-[3-(6,8-dichloro-2-methyl-1,2,3,4-tetrahydro-isoquinolin-4-yl)-phenyl]-2,5-dimethyl-1 H-pyrrole-3-carboxamide;
127) N-[3-(6,8-dichloro-2-methyl-1,2,3,4-tetrahydro-isoquinolin-4-yl)-phenyl]-1H-imidazole-4-carboxamide;
128) N-[3-(6,8-dichloro-2-methyl-1 ,2,3,4-tetrahydro-isoquinolin-4-yl)-phenyl]-1-methanesulfonyl-piperidine-4-carboxamide;
129) N-[3-(6,8-dichloro-2-methyl-1,2,3,4-tetrahydro-isoquinolin-4-yl)-phenyl]-3,5-dimethyl-1H-pyrazole-4-carboxamide;
130) N-[3-(6,8-dichloro-2-methyl-1,2,3,4-tetrahydro-isoquinolin-4-yl)-phenyl]-1H-pyrazole-4-carboxamide;
131) N-[3-(6,8-dichloro-2-methyl-1,2,3,4-tetrahydro-isoquinolin-4-yl)-phenyl]-3-trifluoromethyl-1 H-pyrazole-4-carboxamide;
132) 1-[2-(6,8-dichloro-2-methyl-1,2,3,4-tetrahydro-isoquinolin-4-yl)-phenyl]-3-ethyl-thiourea;
133) 1-[4-(6,8-dichloro-2-methyl-1,2,3,4-tetrahydro-isoquinolin-4-yl)-phenyl]-3-ethyl-thiourea;
134) 1-[3-(6,8-dichloro-2-methyl-1,2,3,4-tetrahydro-isoquinolin-4-yl)-phenyl]-3-ethyl-thiourea;
135) 3-[3-(6,8-dichloro-2-methyl-1,2,3,4-tetrahydro-isoquinolin-4-yl)-phenyl]-1,1-dimethyl-urea;
136) N-[3-(6,8-dichloro-2-methyl-1,2,3,4-tetrahydro-isoquinolin-4-yl)-phenyl]-4-methylpiperazine-1-carboxamide;
137) N-[3-(6,8-dichloro-2-methyl-1,2,3,4-tetrahydro-isoquinolin-4-yl)-phenyl]-piperidine-1-carboxamide;
138) N-[3-(6,8-dichloro-2-methyl-1,2,3,4-tetrahydro-isoquinolin-4-yl)-phenyl]-morpholine-4-carboxamide;
139) N-[3-(6,8-dichloro-2-methyl-1,2,3,4-tetrahydro-isoquinolin-4-yl)-phenyl]-pyrrolidine-1-carboxamide;
140) 3-[3-(6,8-dichloro-2-methyl-1,2,3,4-tetrahydro-isoquinolin-4-yl)-phenyl]-1,1-diethyl-urea;
141) 1-[3-(6,8-dichloro-2-methyl-1,2,3,4-tetrahydro-isoquinolin-4-yl)-phenyl]-3-methyl-urea;
142) 1-[3-(6,8-dichloro-2-methyl-1,2,3,4-tetrahydro-isoquinolin-4-yl)-phenyl]-3-(2-dimethylamino-ethyl)-urea;
143) 1-[3-(6,8-dichloro-2-methyl-1,2,3,4-tetrahydro-isoquinolin-4-yl)-phenyl]-3-(tetrahydro-furan-3-yl)-urea;
144) 1-[3-(6,8-dichloro-2-methyl-1,2,3,4-tetrahydro-isoquinolin-4-yl)-phenyl]-3-(tetrahydro-pyran-4-yl)-urea;
145) 3-[3-(6,8-dichloro-2-methyl-1,2,3,4-tetrahydro-isoquinolin-4-yl)-phenyl]-1-methyl-1-(1-methyl-piperidin-4-yl)-urea;
146) 3-[3-(6,8-dichloro-2-methyl-1,2,3,4-tetrahydro-isoquinolin-4-yl)-phenyl]-1-(3-dimethylamino-propyl)-1-methyl-urea;
147) 3-[3-(6,8-dichloro-2-methyl-1,2,3,4-tetrahydro-isoquinolin-4-yl)-phenyl]-1-(2-dimethylamino-ethyl)-1-methyl-urea;
148) 1-[3-(6,8-dichloro-2-methyl-1,2,3,4-tetrahydro-isoquinolin-4-yl)-phenyl]-3-(3-dimethylamino-propyl)-urea;
149) 1-[3-(6,8-dichloro-2-methyl-1,2,3,4-tetrahydro-isoquinolin-4-yl)-phenyl]-3-(2-methoxy-ethyl)-urea;
150) 1-[3-(6,8-dichloro-2-methyl-1,2,3,4-tetrahydro-isoquinolin-4-yl)-phenyl]-3-pyridin-3-yl-urea;
151) 1-[3-(6,8-dichloro-2-methyl-1,2,3,4-tetrahydro-isoquinolin-4-yl)-phenyl]-3-pyridin-4-yl-urea;
152) N-[2-(6,8-dichloro-2-methyl-1,2,3,4-tetrahydro-isoquinolin-4-yl)-phenyl]-4-methylpiperazine-1-carboxamide;
153) 1-[2-(6,8-dichloro-2-methyl-1,2,3,4-tetrahydro-isoquinolin-4-yl)-phenyl]-3-methyl-urea;
154) 3-[2-(6,8-dichloro-2-methyl-1,2,3,4-tetrahydro-isoquinolin-4-yl)-phenyl]-1,1-dimethyl-urea;
155) 3-[2-(6,8-dichloro-2-methyl-1,2,3,4-tetrahydro-isoquinolin-4-yl)-phenyl]-1,1-diethyl-urea;
156) N-[2-(6,8-dichloro-2-methyl-1,2,3,4-tetrahydro-isoquinolin-4-yl)-phenyl]-piperidine-1-carboxamide;
157) N-[2-(6,8-dichloro-2-methyl-1,2,3,4-tetrahydro-isoquinolin-4-yl)-phenyl]-morpholine-4-carboxamide;
158) N-[2-(6,8-dichloro-2-methyl-1 ,2,3,4-tetrahydro-isoquinolin-4-yl)-phenyl]-pyrrolidine-1-carboxamide;
159) 1-[2-(6,8-dichloro-2-methyl-1,2,3,4-tetrahydro-isoquinolin-4-yl)-phenyl]-3-(2-dimethylamino-ethyl)-urea;
160) N-[4-(6,8-dichloro-2-methyl-1,2,3,4-tetrahydro-isoquinolin-4-yl)-phenyl]-4-methylpiperazine-1-carboxamide;
161) N-[4-(6,8-dichloro-2-methyl-1,2,3,4-tetrahydro-isoquinolin-4-yl)-phenyl]-pyrrolidine-1-carboxamide;
162) 1-[4-(6,8-dichloro-2-methyl-1,2,3,4-tetrahydro-isoquinolin-4-yl)-phenyl]-3-methyl-urea;
163) 3-[4-(6,8-dichloro-2-methyl-1,2,3,4-tetrahydro-isoquinolin-4-yl)-phenyl]-1,1-dimethyl-urea;
164) 3-[4-(6,8-dichloro-2-methyl-1,2,3,4-tetrahydro-isoquinolin-4-yl)-phenyl]-1,1-diethyl-urea;
165) 1-[4-(6,8-dichloro-2-methyl-1,2,3,4-tetrahydro-isoquinolin-4-yl)-phenyl]-3-(2-dimethylamino-ethyl)-urea;
166) N-[4-(6,8-dichloro-2-methyl-1,2,3,4-tetrahydro-isoquinolin-4-yl)-phenyl]-piperidine-1-carboxamide;
167) N-[4-(6,8-dichloro-2-methyl-1,2,3,4-tetrahydro-isoquinolin-4-yl)-phenyl]-morpholine-4-carboxamide;
168) N-[4-(6,8-dichloro-2-methyl-1,2,3,4-tetrahydro-isoquinolin-4-yl)-phenyl]-formamide;
169) [4-(6,8-dichloro-2-methyl-1,2,3,4-tetrahydro-isoquinolin-4-yl)-phenyl]-methylamine;
170) 1-[4-(6,8-dichloro-2-methyl-1,2,3,4-tetrahydro-isoquinolin-4-yl)-phenyl]-1,3-dimethyl-urea;
171) N-[4-(6,8-dichloro-2-methyl-1,2,3,4-tetrahydro-isoquinolin-4-yl)-phenyl]-methyl-4-methyl-piperazine-1-carboxamide;
172) 1-[4-(6,8-dichloro-2-methyl-1,2,3,4-tetrahydro-isoquinolin-4-yl)-phenyl]-1,3,3-trimethyl-urea;
173) N-[4-(6,8-dichloro-2-methyl-1 ,2,3,4-tetrahydro-isoquinolin-4-yl)-phenyl]-methylpiperidine-1-carboxamide;
174) N-[4-(6,8-dichloro-2-methyl-1,2,3,4-tetrahydro-isoquinolin-4-yl)-phenyl]-methylmorpholine-4-carboxamide;
175) N-[4-(6,8-dichloro-2-methyl-1,2,3,4-tetrahydro-isoquinolin-4-yl)-phenyl]-methyl-pyrrolidine-1-carboxamide;
176) 1-[4-(6,8-dichloro-2-methyl-1,2,3,4-tetrahydro-isoquinolin-4-yl)-phenyl]-3-(2-dimethylamino-ethyl)-1-methyl-urea;
177) 1-[4-(6,8-dichloro-2-methyl-1,2,3,4-tetrahydro-isoquinolin-4-yl)-phenyl]-3,3-diethyl-1-methyl-u rea;
178) N-[3-(6,8-dichloro-2-methyl-1,2,3,4-tetrahydro-isoquinolin-4-yl)-phenyl]-formamide;
179) [3-(6,8-dichloro-2-methyl-1,2,3,4-tetrahydro-isoquinolin-4-yl)-phenyl]-methylamine;
180) N-[3-(6,8-dichloro-2-methyl-1,2,3,4-tetrahydro-isoquinolin-4-yl)-phenyl]-methyl-pyrrolidine-1-carboxamide;
181) N-[3-(6,8-dichloro-2-methyl-1,2,3,4-tetrahydro-isoquinolin-4-yl)-phenyl]-methylpiperidine-1-carboxamide;
182) 1-[3-(6,8-dichloro-2-methyl-1,2,3,4-tetrahydro-isoquinolin-4-yl)-phenyl]-1,3,3-trimethyl-urea;
183) 1-[3-(6,8-dichloro-2-methyl-1,2,3,4-tetrahydro-isoquinolin-4-yl)-phenyl]-1,3-dimethyl-urea;
184) N-[3-(6,8-dichloro-2-methyl-1,2,3,4-tetrahydro-isoquinolin-4-yl)-phenyl]-methylmorpholine-4-carboxamide;
185) N-[3-(6,8-dichloro-2-methyl-1,2,3,4-tetrahydro-isoquinolin-4-yl)-phenyl]-methyl-4-methyl-piperazine-1-carboxamide;
186) 1-[3-(6,8-dichloro-2-methyl-1,2,3,4-tetrahydro-isoquinolin-4-yl)-phenyl]-3-(2-dimethylamino-ethyl)-1-methyl-urea;
187) 1-[3-(6,8-dichloro-2-methyl-1,2,3,4-tetrahydro-isoquinolin-4-yl)-phenyl]-3,3-diethyl-1-methyl-urea;
188) 2-dimethylamino-ethyl [3-(6,8-dichloro-2-methyl-1,2,3,4-tetrahydro-isoquinolin-4-yl)-phenyl]-carbamate;
189) 2-dimethylamino-ethyl [4-(6,8-dichloro-2-methyl-1,2,3,4-tetrahydro-isoquinolin-4-yl)-phenyl]-carbamate;
190) 2-dimethylamino-ethyl [2-(6,8-dichloro-2-methyl-1,2,3,4-tetrahydro-isoquinolin-4-yl)-phenyl]-carbamate;
191) methyl [3-(6,8-dichloro-2-methyl-1,2,3,4-tetrahydro-isoquinolin-4-yl)-phenyl]-carbamate;
192) ethyl [3-(6,8-dichloro-2-methyl-1,2,3,4-tetrahydro-isoquinolin-4-yl)-phenyl]-carbamate;
193) isopropyl [3-(6,8-dichloro-2-methyl-1,2,3,4-tetrahydro-isoquinolin-4-yl)-phenyl]-carbamate;
194) 2,2-dimethyl-propyl [3-(6,8-dichloro-2-methyl-1 ,2,3,4-tetrahydro-isoquinolin-4-yl)-phenyl]-carbamate;
195) methyl [4-(6,8-dichloro-2-methyl-1,2,3,4-tetrahydro-isoquinolin-4-yl)-phenyl]-carbamate;
196) isopropyl [4-(6,8-dichloro-2-methyl-1,2,3,4-tetrahydro-isoquinolin-4-yl)-phenyl]-carbamate;
197) 2,2-dimethyl-propyl [4-(6,8-dichloro-2-methyl-1 ,2,3,4-tetrahydro-isoquinolin-4-yl)-phenyl]-carbamate;
198) ethyl [4-(6,8-dichloro-2-methyl-1 ,2,3,4-tetrahydro-isoquinolin-4-yl)-phenyl]-carbamate;
199) (R)-N-[3-(6,8-dichloro-2-methyl-1,2,3,4-tetrahydro-isoquinolin-4-yl)-phenyl]-methanesulfonamide;
200) (S)-N-[3-(6,8-dichloro-2-methyl-1,2,3,4-tetrahydro-isoquinolin-4-yl)-phenyl]-methanesulfonamide;
201) (R)-1-[2-(6,8-dichioro-2-methyl-1,2,3,4-tetrahydro-isoquinolin-4-yl)-phenyl]-3-ethyl-u rea;
202) (S)-1-[2-(6,8-dichloro-2-methyl-1,2,3,4-tetrahydro-isoquinolin-4-yl)-phenyl]-3-ethyl-urea;
203) N-[3-(6,8-dichloro-2-methyl-1,2,3,4-tetrahydro-isoquinolin-4-yl)-phenyl]-acetamide;
204) 4-(3-bromo-phenyl)-6,8-dichloro-2-methyl-1,2,3,4-tetrahydro-isoquinoline;
205) 1-[3-(6,8-dichloro-2-methyl-1,2,3,4-tetrahydro-isoquinolin-4-yl)-phenyl]-3-(2-hydroxy-ethyl)-urea;
206) ethyl 3-(6,8-dichloro-2-methyl-1,2,3,4-tetrahydro-isoquinolin-4-yl)-benzoate;
207) 3-(6,8-dichloro-2-methyl-1,2,3,4-tetrahydro-isoquinolin-4-yl)-benzoic acid;
and from the pharmaceutically acceptable salts thereof.

3. Compound of the formula I according to claim 1, selected from the group consisting of:
1) N-[4-(6,8-dichloro-2-methyl-1,2,3,4-tetrahydro-isoquinolin-4-yl)-phenyl]-acetamide;
2) 4-(6,8-dichloro-2-methyl-1,2,3,4-tetrahydro-isoquinolin-4-yl)-benzenesulfonamide;
3) 3-(6,8-dichloro-2-methyl-1,2,3,4-tetrahydro-isoquinolin-4-yl)-benzenesulfonamide;
4) 1-[4-(6,8-dichloro-2-methyl-1,2,3,4-tetrahydro-isoquinolin-4-yl)-phenyl]-3-methyl-thiourea;
5) 1-[4-(6,8-dichloro-2-methyl-1,2,3,4-tetrahydro-isoquinolin-4-yl)-phenyl]-3-ethyl-urea;
6) N-[4-(6-bromo-8-chloro-2-methyl-1,2,3,4-tetrahydro-isoquinolin-4-yl)-phenyl]-acetamide;
7) N-[3-(6,8-dichloro-2-methyl-1,2,3,4-tetrahydro-isoquinolin-4-yl)-phenyl]-acetamide;
8) N-[4-(6,8-dichloro-2-methyl-1,2,3,4-tetrahydro-isoquinolin-4-yl)-phenyl]-propionamide;
9) N-[4-(6,8-dichloro-2-methyl-1,2,3,4-tetrahydro-isoquinolin-4-yl)-phenyl]-1-acetyl-piperidine-4-carboxamide;
10) N-[4-(6,8-dichloro-2-methyl-1,2,3,4-tetrahydro-isoquinolin-4-yl)-phenyl]-methane-sulfonamide;
11) N-[4-(6,8-dichloro-2-methyl-1,2,3,4-tetrahydro-isoquinolin-4-yl)-phenyl]-ethanesulfonamide;
12) N',N'-dimethylamino-N-[4-(6,8-dichloro-2-methyl-1,2,3,4-tetrahydro-isoquinolin-4-yl)-phenyl]-sulfamide;
13) N-[3-(6,8-dichloro-2-methyl-1,2,3,4-tetrahydro-isoquinolin-4-yl)-phenyl]-propionamide;
14) N-[3-(6,8-dichloro-2-methyl-1,2,3,4-tetrahydro-isoquinolin-4-yl)-phenyl]-butyramide;
15) N-[3-(6,8-dichloro-2-methyl-1,2,3,4-tetrahydro-isoquinolin-4-yl)-phenyl]-isobutyramide;
16) N-[3-(6,8-dichloro-2-methyl-1,2,3,4-tetrahydro-isoquinolin-4-yl)-phenyl]-cyclopropanecarboxamide;
17) N-[3-(6,8-dichloro-2-methyl-1,2,3,4-tetrahydro-isoquinolin-4-yl)-phenyl]-cyclobutanecarboxamide;
18) N-[3-(6,8-dichloro-2-methyl-1,2,3,4-tetrahydro-isoquinolin-4-yl)-phenyl]-2,2,2-trifluoro-acetamide;
19) N-[3-(6,8-dichloro-2-methyl-1,2,3,4-tetrahydro-isoquinolin-4-yl)-phenyl]-1-acetyl-piperidine-4-carboxamide;
20) N-[3-(6,8-dichloro-2-methyl-1,2,3,4-tetrahydro-isoquinolin-4-yl)-phenyl]-nicotinamide;
21) N-[3-(6,8-dichloro-2-methyl-1,2,3,4-tetrahydro-isoquinolin-4-yl)-phenyl]-methane-sulfonamide;
22) N-[3-(6,8-dichloro-2-methyl-1,2,3,4-tetrahydro-isoquinolin-4-yl)-phenyl]-ethanesulfonamide;
23) N',N'-dimethylamino-N-[3-(6,8-dichloro-2-methyl-1,2,3,4-tetrahydro-isoquinolin-4-yl)-phenyl]-sulfamide;
24) N-[2-(6,8-dichloro-2-methyl-1,2,3,4-tetrahydro-isoquinolin-4-yl)-phenyl]-cyclopropanecarboxamide;
25) N-[2-(6,8-dichloro-2-methyl-1,2,3,4-tetrahydro-isoquinolin-4-yl)-phenyl]-1-acetyl-piperidine-4-carboxamide;
26) 1-[3-(6,8-dichloro-2-methyl-1,2,3,4-tetrahydro-isoquinolin-4-yl)-phenyl]-3-ethyl-urea;
27) 1-[3-(6,8-dichloro-2-methyl-1,2,3,4-tetrahydro-isoquinolin-4-yl)-phenyl]-3-methyl-thiourea;
28) 1-[2-(6,8-dichloro-2-methyl-1,2,3,4-tetrahydro-isoquinolin-4-yl)-phenyl]-3-ethyl-urea;
29) 1-[2-(6,8-dichloro-2-methyl-1,2,3,4-tetrahydro-isoquinolin-4-yl)-phenyl]-3-methyl-thiourea;
30) N-{5-[3-(6,8-dichloro-2-methyl-1,2,3,4-tetrahydro-isoquinolin-4-yl)-phenylsulfamoyl]-4-methyl-thiazol-2-yl}-acetamide;
31) N-[3-(6,8-dichloro-2-methyl-1,2,3,4-tetrahydro-isoquinolin-4-yl)-phenyl]-1,2-dimethyl-1H-imidazole-4-sulfonamide;
32) N-[4-(6,8-dichloro-2-methyl-1,2,3,4-tetrahydro-isoquinolin-4-yl)-phenyl]-C,C,C-trifluoro-methanesulfonamide;
33) N-[3-(6,8-dichloro-2-methyl-1,2,3,4-tetrahydro-isoquinolin-4-yl)-phenyl]-C,C,C-trifluoro-methanesulfonamide;
34) N-ethyl-N'-4-(6,8-dichloro-2-methyl-1,2,3,4-tetrahydro-isoquinolin-4-yl)-benzenesulfonylurea;
35) N-[4-(6,8-dichloro-2-methyl-1,2,3,4-tetrahydro-isoquinolin-4-yl)-phenyl]-2-dimethylamino-acetamide;
36) 2,6-diamino-N-[4-(6,8-dichloro-2-methyl-1,2,3,4-tetrahydro-isoquinolin-4-yl)-phenyl]-hexanamide;
37) N-[4-(6,8-dichloro-2-methyl-1,2,3,4-tetrahydro-isoquinolin-4-yl)-phenyl]-1H-pyrrole-3-carboxamide;
38) N-[4-(6,8-dichloro-2-methyl-1,2,3,4-tetrahydro-isoquinolin-4-yl)-phenyl]-1-methylpiperidine-4-carboxamide;
39) N-[4-(6,8-dichloro-2-methyl-1,2,3,4-tetrahydro-isoquinolin-4-yl)-phenyl]-1-methanesulfonyl-piperidine-4-carboxamide;
40) N-[4-(6,8-dichloro-2-methyl-1,2,3,4-tetrahydro-isoquinolin-4-yl)-phenyl]-1H-pyrazole-carboxamide;
41) N-[3-(6,8-dichloro-2-methyl-1,2,3,4-tetrahydro-isoquinolin-4-yl)-phenyl]-2-methylamino-acetamide;
42) N-[3-(6,8-dichloro-2-methyl-1,2,3,4-tetrahydro-isoquinolin-4-yl)-phenyl]-2-dimethylamino-acetamide;
43) 2-amino-N-[3-(6,8-dichloro-2-methyl-1,2,3,4-tetrahydro-isoquinolin-4-yl)-phenyl]-propionamide;
44) 2-amino-N-[3-(6,8-dichloro-2-methyl-1,2,3,4-tetrahydro-isoquinolin-4-yl)-phenyl]-butyramide;
45) 2,6-diamino-N-[3-(6,8-dichloro-2-methyl-1,2,3,4-tetrahydro-isoquinolin-4-yl)-phenyl]-hexanamide;
46) N-[3-(6,8-dichloro-2-methyl-1,2,3,4-tetrahydro-isoquinolin-4-yl)-phenyl]-1-methylpiperidine-4-carboxamide;
47) N-[3-(6,8-dichloro-2-methyl-1,2,3,4-tetrahydro-isoquinolin-4-yl)-phenyl]-1H-imidazole-4-carboxamide;
48) N-[3-(6,8-dichloro-2-methyl-1,2,3,4-tetrahydro-isoquinolin-4-yl)-phenyl]-1-methanesulfonyl-piperidine-4-carboxamide;
49) N-[3-(6,8-dichloro-2-methyl-1,2,3,4-tetrahydro-isoquinolin-4-yl)-phenyl]-3,5-dimethyl-1 H-pyrazole-4-carboxamide;
50) N-[3-(6,8-dichloro-2-methyl-1,2,3,4-tetrahydro-isoquinolin-4-yl)-phenyl]-1H-pyrazole-carboxamide;
51) 3-[3-(6,8-dichloro-2-methyl-1,2,3,4-tetrahydro-isoquinolin-4-yl)-phenyl]-1,1-dimethyl-urea;
52) N-[3-(6,8-dichloro-1,2,3,4-tetrahydro-isoquinolin-4-yl)-phenyl]-4-methylpiperazine-1-carboxamide;
53) N-[3-(6,8-dichloro-2-methyl-1,2,3,4-tetrahydro-isoquinolin-4-yl)-phenyl]-piperidine-1-carboxamide;
54) N-[3-(6,8-dichloro-2-methyl-1,2,3,4-tetrahydro-isoquinolin-4-yl)-phenyl]-morpholine-4-carboxamide;
55) N-[3-(6,8-dichloro-2-methyl-1,2,3,4-tetrahydro-isoquinolin-4-yl)-phenyl]-pyrrolidine-1-carboxamide;
56) 3-[3-(6,8-dichloro-2-methyl-1,2,3,4-tetrahydro-isoquinolin-4-yl)-phenyl]-1,1-diethyl-urea;
57) 1-[3-(6,8-dichloro-2-methyl-1,2,3,4-tetrahydro-isoquinolin-4-yl)-phenyl]-3-methyl-urea;
58) 1-[3-(6,8-dichloro-2-methyl-1,2,3,4-tetrahydro-isoquinolin-4-yl)-phenyl]-3-(2-dimethylamino-ethyl)-urea;
59) 1-[3-(6,8-dichloro-2-methyl-1,2,3,4-tetrahydro-isoquinolin-4-yl)-phenyl]-3-(tetrahydro-furan-3-yl)-urea;
60) 1-[3-(6,8-dichloro-2-methyl-1,2,3,4-tetrahydro-isoquinolin-4-yl)-phenyl]-3-(tetrahydro-pyran-4-yl)-urea;
61) 3-[3-(6,8-dichloro-2-methyl-1,2,3,4-tetrahydro-isoquinolin-4-yl)-phenyl]-1-methyl-1-(1-methyl-piperidin-4-yl)-urea;
62) 3-[3-(6,8-dichloro-2-methyl-1,2,3,4-tetrahydro-isoquinolin-4-yl)-phenyl]-1-(3-dimethylamino-propyl)-1-methyl-urea;
63) 3-[3-(6,8-dichloro-2-methyl-1,2,3,4-tetrahydro-isoquinolin-4-yl)-phenyl]-1-(2-dimethylamino-ethyl)-1-methyl-urea;
64) 1-[3-(6,8-dichloro-2-methyl-1,2,3,4-tetrahydro-isoquinolin-4-yl)-phenyl]-3-(3-diethylamino-propyl)-urea;
65) 1-[3-(6,8-dichloro-2-methyl-1,2,3,4-tetrahydro-isoquinolin-4-yl)-phenyl]-3-(2-methoxy-ethyl)-urea;
66) 1-[3-(6,8-dichloro-2-methyl-1,2,3,4-tetrahydro-isoquinolin-4-yl)-phenyl]-3-pyridin-3-yl)-urea;
67) 1-[3-(6,8-dichloro-2-methyl-1,2,3,4-tetrahydro-isoquinolin-4-yl)-phenyl]-3-pyridin-4-yl-u rea;
68) N-[2-(6,8-dichloro-2-methyl-1,2,3,4-tetrahydro-isoquinolin-4-yl)-phenyl]-4-methylpiperazine-1-carboxamide;
69) 1-[2-(6,8-dichloro-2-methyl-1,2,3,4-tetrahydro-isoquinolin-4-yl)-phenyl]-3-methyl-urea;
70) 1-[2-(6,8-dichloro-2-methyl-1,2,3,4-tetrahydro-isoquinolin-4-yl)-phenyl]-3-(2-dimethylamino-ethyl)-urea;
71) N-[4-(6,8-dichloro-2-methyl-1,2,3,4-tetrahydro-isoquinolin-4-yl)-phenyl]-4-methylpiperazine-1-carboxamide;
72) 1-[4-(6,8-dichloro-2-methyl-1,2,3,4-tetrahydro-isoquinolin-4-yl)-phenyl]-3-methyl-urea;
73) 3-[4-(6,8-dichloro-2-methyl-1,2,3,4-tetrahydro-isoquinolin-4-yl)-phenyl]-1,1-dimethyl-urea;
74) 3-[4-(6,8-dichloro-2-methyl-1,2,3,4-tetrahydro-isoquinolin-4-yl)-phenyl]-1,1-diethyl-urea;
75) 1-[4-(6,8-dichloro-2-methyl-1,2,3,4-tetrahydro-isoquinolin-4-yl)-phenyl]-3-(2-dimethylamino-ethyl)-urea;
76) N-[4-(6,8-dichloro-2-methyl-1,2,3,4-tetrahydro-isoquinolin-4-yl)-phenyl]-morpholine-4-carboxamide;
77) N-4-(6,8-dichloro-2-methyl-1,2,3,4-tetrahydro-isoquinolin-4-yl)-phenyl]-formamide;
78) N-[3-(6,8-dichloro-2-methyl-1,2,3,4-tetrahydro-isoquinolin-4-yl)-phenyl]-formamide;
79) 1-[3-(6,8-dichloro-2-methyl-1,2,3,4-tetrahydro-isoquinolin-4-yl)-phenyl]-1,3,3-trimethyl-urea;
80) 1-[3-(6,8-dichloro-2-methyl-1,2,3,4-tetrahydro-isoquinolin-4-yl)-phenyl]-1,3-dimethyl-urea;
81) N-[3-(6,8-dichloro-2-methyl-1,2,3,4-tetrahydro-isoquinolin-4-yl)-phenyl]-methylmorpholine-4-carboxamide;
82) N-[3-(6,8-dichloro-2-methyl-1,2,3,4-tetrahydro-isoquinolin-4-yl)-phenyl]-methyl-4-methyl-piperazine-1-carboxamide;
83) 1-[3-(6,8-dichloro-2-methyl-1,2,3,4-tetrahydro-isoquinolin-4-yl)-phenyl]-3-(2-dimethylamino-ethyl)-1-methyl-urea;
84) 2-dimethylamine-ethyl [3-(6,8-dichloro-2-methyl-1,2,3,4-tetrahydro-isoquinolin-4-yl)-phenyl]-carbamate;
85) 2-dimethylamino-ethyl [4-(6,8-dichloro-2-methyl-1,2,3,4-tetrahydro-isoquinolin-4-yl)-phenyl]-carbamate;
86) 2-dimethylamino-ethyl [2-(6,8-dichloro-2-methyl-1,2,3,4-tetrahydro-isoquinolin-4-yl)-phenyl]-carbamate;
87) methyl [3-(6,8-dichloro-2-methyl-1,2,3,4-tetrahydro-isoquinolin-4-yl)-phenyl]-carbamate;
88) (R or S)-N-[3-(6,8-dichloro-2-methyl-1,2,3,4-tetrahydro-isoquinolin-4-yl)-phenyl]-methanesulfonamide;
89) (R or S)-1-[2-(6,8-dichloro-2-methyl-1,2,3,4-tetrahydro-isoquinolin-4-yl)-phenyl]-3-ethyl-urea;
90) 1-[3-(6,8-dichloro-2-methyl-1,2,3,4-tetrahydro-isoquinolin-4-yl)-phenyl]-3-(2-hydroxy-ethyl)-urea;
and from the pharmaceutically acceptable salts thereof.

4. Use of a compound I according to one or more of claims 1 to 3 for producing a medicament for the treatment or prophylaxis of disorders of respiratory drive.

5. Use of a compound I according to one or more of claims 1 to 3 for producing a medicament for the treatment or prophylaxis of respiratory disorders, in particular sleep-related respiratory disorders such as sleep apneas.

6. Use of a compound I according to one or more of claims 1 to 3 for producing a medicament for the treatment or prophylaxis of snoring.

7. Use of a compound I according to one or more of claims 1 to 3 for producing a medicament for the treatment or prophylaxis of acute and chronic renal disorders, particularly of acute renal failure and of chronic renal failure.

8. Use of a compound I according to one or more of claims 1 to 3 for producing a medicament for the treatment or prophylaxis of disorders of intestinal function.

9. Use of a compound I according to one or more of claims 1 to 3 for producing a medicament for the treatment or prophylaxis of disorders of biliary function.

10. Use of a compound I according to one or more of claims 1 to 3 for producing a medicament for the treatment or prophylaxis of ischemic states of the peripheral and central nervous system and of stroke.

11. Use of a compound I according to one or more of claims 1 to 3 for producing a medicament for the treatment or prophylaxis of ischemic states of peripheral organs and limbs.

12. Use of a compound I according to one or more of claims 1 to 3 for producing a medicament for the treatment of states of shock.

13. Use of a compound I according to one or more of claims 1 to 3 for producing a medicament for use in surgical operations and organ transplantations.

14. Use of a compound I according to one or more of claims 1 to 3 for producing a medicament for the preservation and storage of transplants for surgical procedures.

15. Use of a compound I according to one or more of claims 1 to 3 for producing a medicament for the treatment of disorders in which cell proliferation represents a primary or secondary cause.

16. Use of a compound I according to one or more of claims 1 to 3 for producing a medicament for the treatment or prophylaxis of disorders of lipid metabolism.

17. Use of a compound I according to one or more of claims 1 to 3 for producing a medicament for the treatment or prophylaxis of infestation by ectoparasites.

18. Medicine comprising an effective amount of a compound I according to claim 1.

## Revendications

1. 4-phényltétrahydro-isoquinoléines de formule I 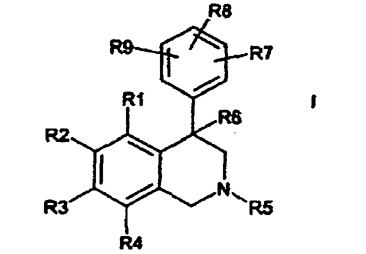 dans laquelle :
R1, R2, R3 et R4 indépendamment l'un de l'autre, signifient H, F, Cl, Br, OH, NH₂, CₐH₂ₐ₊₁, cycloalkyle comprenant 3, 4, 5 ou 6 atomes de carbone, OC_{b}H_{2b+1} ;
a et b dans les groupes CₐH₂ₐ₊₁ et OC_{b}H_{2b+1}, indépendamment l'un de l'autre, valent 1, 2, 3 ou 4, où un ou plusieurs atomes d'hydrogène peuvent être remplacés par des atomes de F ;
ou
R1, R2, R3 et R4 indépendamment l'un de l'autre, signifient NR11R12 ;
R11 et R12 indépendamment l'un de l'autre, signifient H, CₑH₂ₑ₊₁, CᵣᵣH₂ᵣᵣ₋₁ ;
e vaut 1, 2, 3 ou 4,
rr vaut 3, 4, 5 ou 6,
où, dans les groupes CₑH₂ₑ₊₁ et CᵣᵣH₂ᵣᵣ₋₁, un ou plusieurs atomes de H peuvent être remplacés par des atomes de F ;
ou
R11 et R12 ensemble avec l'atome de N auquel ils sont liés, forment un cycle, choisi dans le groupe constitué par la pyrrolidine, la pipéridine, la N-méthylpipérazine, la pipérazine et la morpholine ;
ou
R11 et R12 indépendamment l'un de l'autre, signifient COR14, CSR14, CONHR14, CSNHR14 ou SO₂R14 ;
R14 signifie C_{g}H_{2g+1} ;
g vaut 1, 2, 3 ou 4, où un ou plusieurs atomes d'hydrogène peuvent être remplacés par des atomes de F ;
ou
R1, R2, R3 et R4 indépendamment l'un de l'autre, signifient OSO₃H, SO₃H, SO₂R15 ;
R15 signifie CₖH₂ₖ₊₁ ou NR17R18 ;
k vaut 1, 2, 3 ou 4, où un ou plusieurs atomes d'hydrogène peuvent être remplacés par des atomes de F ;
R17 et R18 indépendamment l'un de l'autre, signifient H ou CₘH₂ₘ₊₁ ;
m vaut 1, 2, 3 ou 4, où, dans le groupe CₘH₂ₘ₊₁, un ou plusieurs atomes d'hydrogène peuvent être remplacés par des atomes de F ;
ou
R17 et R18 ensemble avec l'atome de N auquel ils sont liés, signifient un cycle de 5 ou 6 chaînons ;
R2 devant cependant toujours être différent de H ;
R5 signifie méthyle ou trifluorométhyle ;
R6 signifie H ;
R7, R8 et R9 indépendamment l'un de l'autre, signifient OSO₃H, SO₃H ou SO₂R23 ;
R23 signifie CₙₙH₂ₙₙ₊₁ ou NR25R26 ;
nn vaut 1, 2, 3, 4 ou 5 ;
où, dans CₙₙH₂ₙₙ₊₁, un ou plusieurs atomes d'hydrogène peuvent être remplacés par des atomes de F ;
R25 et R26 indépendamment l'un de l'autre, signifient H, CN ou C_{z}H_{2z+1}, où le premier groupe CH₂ lié à l'azote est remplacé par CO ou CS et le deuxième CH₂ est remplacé par NR27 ;
z vaut 1, 2, 3, 4, 5 ou 6 ;
où, dans C_{z}H_{2z+1}, un ou plusieurs atomes d'hydrogène peuvent être remplacés par des atomes de F ;
R27 signifie H ou CₐₐH₂ₐₐ₊₁ ;
aa vaut 1, 2, 3 ou 4 ;
où, dans CₐₐH₂ₐₐ₊₁, un ou plusieurs atomes d'hydrogène peuvent être remplacés par des atomes de F ;
ou
R25 et R26 ensemble avec l'atome de N auquel ils sont liés, signifient un cycle de 5 ou 6 chaînons, ou
R27 et un groupe CH₂ de R25 ou de R26, ensemble avec l'atome de N auquel ils sont liés, signifient un cycle de 5 ou 6 chaînons ;
ou
R7, R8 et R9 indépendamment l'un de l'autre, signifient NR32COR30, NR32CSR30 ou NR32SO₂R30 ;
R30 signifie H, OH, C_{cc}H_{2cc+1}, C_{yy}H_{2yy-1}, pyrrolidinyle ou pipéridinyle, où un groupe CH₂ dans ces cycles peut être remplacé par O ou NR33 ;
R32 et R33 signifient H, méthyle ou CF₃ ;
cc vaut 1, 2, 3, 4, 5, 6, 7 ou 8 ;
yy vaut 3, 4, 5 ou 6 ;
où, dans les groupes C_{cc}H_{2cc+1} et C_{yy}H_{2yy-1}, un ou plusieurs atomes d'hydrogène peuvent être remplacés par des atomes de F et un ou plusieurs groupes CH₂ peuvent être remplacés par NR31 et un groupe CH₂ peut être remplacé par O ;
R31 signifie H, méthyle, éthyle, CF₃, CH₂CF₃, acétyle, propionyle, méthanesulfonyle ou éthanesulfonyle ;
ou
R31 ensemble avec un groupe CH₂ de R30 et l'atome de N auquel ils sont liés ensemble, forment un cycle de 5 ou 6 chaînons ;
ou
R30 signifie pyridyle, imidazolyle, pyrazolyle, pyrrolyle, triazolyle, tétrazolyle, thiazolyle ou oxazolyle, qui sont non substitués ou substitués par au maximum 3 substituants choisis dans le groupe constitué par F, Cl, méthyle, éthyle, trifluorométhyle, NH₂, NH-acétyle ;
ou
R7, R8 et R9 indépendamment l'un de l'autre, signifient H, F, Cl, OH, NH₂, CₑₑH₂ₑₑ₊₁, C_{ww}H_{2ww-1}, OC_{ff}H_{2ff+1}, NR40R41, CONR40R41, COOR42 ou OCOR42,
ee et ff indépendamment l'un de l'autre, valent 1, 2, 3 ou 4 ;
ww vaut 3, 4, 5 ou 6,
où, dans les groupes CₑₑH₂ₑₑ₊₁, C_{ww}H_{2ww-1} et OC_{ff}H_{2ff+1}, un ou plusieurs atomes d'hydrogène peuvent être remplacés par des atomes de F ;
R40 et R41 signifient H, CₜₜH₂ₜₜ₊₁ ou C(NH)NH₂ ;
tt vaut 1, 2, 3 ou 4 ;
où, dans le groupe CₜₜH₂ₜₜ₊₁, un ou plusieurs atomes de H peuvent être remplacés par des atomes de F ;
ou
R40 et R41 indépendamment l'un de l'autre, signifient hydroxyéthyle, N,N-diméthylaminoéthyle, N,N-diéthylaminoéthyle, pyrrolidinoéthyle, N-méthylpipérazinoéthyle, pipérazinoéthyle, morpholinoéthyle ou pipéridinoéthyle ;
ou
R40 et R41 ensemble avec l'atome de N auquel ils sont liés, forment un cycle, pyrrolidine, pipéridine, N-méthylpipérazine, pipérazine ou morpholine ;
R42 signifie H ou CₕₕH₂ₕₕ₊₁ ;
hh vaut 1, 2, 3 ou 4 ;
où, dans le groupe CₕₕH₂ₕₕ₊₁, un ou plusieurs atomes d'hydrogène peuvent être remplacés par des atomes de F ;
où cependant deux substituants du groupe R7, R8 et R9 ne peuvent pas signifier simultanément OH ou OCH₃
et où au moins un des radicaux R7, R8 ou R9 doit être choisi dans le groupe constitué par -OᵥSO_{w}R23, NR32COR30, NR32CSR30 et NR32SO_{bb}R30 ; ainsi que leurs sels pharmaceutiquement acceptables et les trifluoroacétates.

2. Composé de formule I, **caractérisé**
**en ce qu'**il est choisi dans le groupe constitué par :
1) N-[4-(6,8-dichloro-2-méthyl-1,2,3,4-tétrahydro-isoquinoléin-4-yl)-phényl]-acétamide ;
2) 4-(6,8-dichloro-2-méthyl-1,2,3,4-tétrahydro-isoquinoléin-4-yl)-benzènesulfonamide ;
3) 3-(6,8-dichloro-2-méthyl-1,2,3,4-tétrahydro-isoquinoléin-4-yl)-benzènesulfonamide ;
4) 4-(6,8-dichloro-2-méthyl-1,2,3,4-tétrahydro-isoquinoléin-4-yl)-N,N-diméthylbenzènesulfonamide ;
5) 4-(4-bromophényl)-6,8-dichloro-2-méthyl-1,2,3,4-tétrahydro-isoquinoléine ;
6) acide 4-(6,8-dichloro-2-méthyl-1,2,3,4-tétrahydro-isoquinoléin-4-yl)-benzoïque ;
7) 4-(6,8-dichloro-2-méthyl-1,2,3,4-tétrahydro-isoquinoléin-4-yl)-N-éthylbenzamide ;
8) 4-(6,8-dichloro-2-méthyl-1,2,3,4-tétrahydro-isoquinoléin-4-yl)-N-propylbenzamide ;
9) 4-(6,8-dichloro-2-méthyl-1,2,3,4-tétrahydro-isoquinoléin-4-yl)-N-(2-diméthylaminoéthyl)-benzamide ;
10) 6,8-dichloro-2-méthyl-4-(4-morpholin-4-yl-phényl)-1,2,3,4-tétrahydro-isoquinoléine ;
11) [4-(6,8-dichloro-2-méthyl-1,2,3,4-tétrahydro-isoquinoléin-4-yl)-phényl]-diéthylamine ;
12) 6,8-dichloro-2-méthyl-4-(4-pipéridin-1-yl-phényl)-1,2,3,4-tétrahydro-isoquinoléine ;
13) 6,8-dichloro-2-méthyl-4-(4-pyrrolidin-1-yl-phényl)-1,2,3,4-tétrahydro-isoquinoléine ;
14) 6,8-dichloro-2-méthyl-4-[4-(4-méthylpipérazin-1-yl)-phényl]-1,2,3,4-tétrahydro-isoquinoléine ;
15) 6,8-dichloro-2-cyclopropyl-4-phényl-1,2,3,4-tétrahydro-isoquinoléine ;
16) 4-(6,8-dichloro-2-méthyl-1,2,3,4-tétrahydro-isoquinoléin-4-yl)-phénylamine ;
17) 1-[4-(6,8-dichloro-2-méthyl-1,2,3,4-tétrahydro-isoquinoléin-4-yl)-phényl]-3-propylurée ;
18) 1-[4-(6,8-dichloro-2-méthyl-1,2,3,4-tétrahydro-isoquinoléin-4-yl)-phényl]-3-méthylthio-urée ;
19) 1-[4-(6,8-dichloro-2-méthyl-1,2,3,4-tétrahydro-isoquinoléin-4-yl)-phényl]-3-éthylurée ;
20) N-[4-(6-méthanesulfonyl-2-méthyl-1,2,3,4-tétrahydro-isoquinoléin-4-yl)-phényl]-acétamide ;
21) N-[4-(2,6,8-triméthyl-1,2,3,4-tétrahydro-isoquinoléin-4-yl)-phényl]-acétamide ;
22) N-[4-(6-bromo-8-chloro-2-méthyl-1,2,3,4-tétrahydro-isoquinoléin-4-yl)-phényl]-acétamide ;
23) N-[4-(8-chloro-2-méthyl-6-pyrrolidin-1-yl-1,2,3,4-tétrahydro-isoquinoléin-4-yl)-phényl]-acétamide ;
24) N-[4-(8-chloro-2-méthyl-6-morpholin-4-yl-1,2,3,4-tétrahydro-isoquinoléin-4-yl)-phényl]-acétamide ;
25) N-{4-[8-chloro-2-méthyl-6-(4-méthylpipérazin-1-yl)-1,2,3,4-tétrahydro-isoquinoléin-4-yl]-phényl}-acétamide ;
26) N-{4-[8-chloro-6-(cyclopropylméthylamino)-2-méthyl-1,2,3,4-tétrahydro-isoquinoléin-4-yl]-phényl}-acétamide ;
27) acide 5-(6,8-dichloro-2-méthyl-1,2,3,4-tétrahydro-isoquinoléin-4-yl)-2-hydroxybenzoïque ;
28) 5-(6,8-dichloro-2-méthyl-1,2,3,4-tétrahydro-isoquinoléin-4-yl)-2-hydroxy-N-méthylbenzamide ;
29) 5-(6,8-dichloro-2-méthyl-1,2,3,4-tétrahydro-isoquinoléin-4-yl)-N-éthyl-2-hydroxybenzamide ;
30) 5-(6,8-dichloro-2-méthyl-1,2,3,4-tétrahydro-isoquinoléin-4-yl)-N-(2-diméthylaminoéthyl)-2-hydroxybenzamide ;
31) N-[5-(6,8-dichloro-2-méthyl-1,2,3,4-tétrahydro-isoquinoléin-4-yl)-2-hydroxybenzoyl]-guanidine ;
32) N-[3-(6,8-dichloro-2-méthyl-1,2,3,4-tétrahydro-isoquinoléin-4-yl)-phényl]-acétamide ;
33) 3-(6,8-dichloro-2-méthyl-1,2,3,4-tétrahydro-isoquinoléin-4-yl)-phénylamine ;
34) 2-(6,8-dichloro-2-méthyl-1,2,3,4-tétrahydro-isoquinoléin-4-yl)-phénylamine ;
35) N-[4-(6,8-dichloro-2-méthyl-1,2,3,4-tétrahydro-isoquinoléin-4-yl)-phényl]-propionamide ;
36) N-[4-(6,8-dichloro-2-méthyl-1,2,3,4-tétrahydro-isoquinoléin-4-yl)phényl]-butyramide ;
37) [4-(6,8-dichloro-2-méthyl-1,2,3,4-tétrahydro-isoquinoléin-4-yl)-phényl]-amide de l'acide pentanoïque ;
38) N-[4-(6,8-dichloro-2-méthyl-1,2,3,4-tétrahydro-isoquinoléin-4-yl)-phényl]-isobutyramide ;
39) N-[4-(6,8-dichloro-2-méthyl-1,2,3,4-tétrahydro-isoquinoléin-4-yl)-phényl]-2,2-diméthylpropionamide ;
40) [4-(6,8-dichloro-2-méthyl-1,2,3,4-tétrahydro-isoquinoléin-4-yl)-phényl]-amide de l'acide cyclopropanecarboxylique ;
41) [4-(6,8-dichloro-2-méthyl-1,2,3,4-tétrahydro-isoquinoléin-4-yl)-phényl]-amide de l'acide cyclobutanecarboxylique ;
42) [4-(6,8-dichloro-2-méthyl-1,2,3,4-tétrahydro-isoquinoléin-4-yl)- phényl]-amide de l'acide cyclopentanecarboxylique ;
43) N-[4-(6,8-dichloro-2-méthyl-1,2,3,4-tétrahydro-isoquinoléin-4-yl)-phényl]-2,2,2-trifluoroacétamide ;
44) [4-(6,8-dichloro-2-méthyl-1,2,3,4-tétrahydro-isoquinoléin-4-yl)-phényl]-amide de l'acide 1-acétylpipéridine-4-carboxylique ;
45) N-[4-(6,8-dichloro-2-méthyl-1,2,3,4-tétrahydro-isoquinoléin-4-yl)-phényl]-nicotinamide ;
46) N-[4-(6,8-dichloro-2-méthyl-1,2,3,4-tétrahydro-isoquinoléin-4-yl)-phényl]-méthanesulfonamide ;
47) [4-(6,8-dichloro-2-méthyl-1,2,3,4-tétrahydro-isoquinoléin-4-yl)-phényl]-amide de l'acide éthanesulfonique ;
48) N',N'-diméthylamino-N-[4-(6,8-dichloro-2-méthyl-1,2,3,4-tétrahydro-isoquinoléin-4-yl)-phényl]-sulfamide ;
49) N-[3-(6,8-dichloro-2-méthyl-1,2,3,4-tétrahydro-isoquinoléin-4-yl)-phényl]-propionamide ;
50) N-[3-(6,8-dichloro-2-méthyl-1,2,3,4-tétrahydro-isoquinoléin-4-yl)-phényl]-butyramide ;
51) [3-(6,8-dichloro-2-méthyl-1,2,3,4-tétrahydro-isoquinoléin-4-yl)-phényl]-amide de l'acide pentanoïque ;
52) N-[3-(6,8-dichloro-2-méthyl-1,2,3,4-tétrahydro-isoquinoléin-4-yl)-phényl]-isobutyramide ;
53) N-[3-(6,8-dichloro-2-méthyl-1,2,3,4-tétrahydro-isoquinoléin-4-yl)-phényl]-2,2-diméthylpropionamide ;
54) [3-(6,8-dichloro-2-méthyl-1,2,3,4-tétrahydro-isoquinoléin-4-yl)-phényl]-amide de l'acide cyclopropanecarboxylique ;
55) [3-(6,8-dichloro-2-méthyl-1,2,3,4-tétrahydro-isoquinoléin-4-yl)-phényl]-amide de l'acide cyclobutanecarboxylique ;
56) [3-(6,8-dichloro-2-méthyl-1,2,3,4-tétrahydro-isoquinoléin-4-yl)-phényl]-amide de l'acide cyclopentanecarboxylique ;
57) N-[3-(6,8-dichloro-2-méthyl-1,2,3,4-tétrahydro-isoquinoléin-4-yl)-phényl]-2,2,2-trifluoroacétamide ;
58) [3-(6,8-dichloro-2-méthyl-1,2,3,4-tétrahydro-isoquinoléin-4-yl)-phényl]-amide de l'acide 1-acétylpipéridine-4-carboxylique ;
59) N-[3-(6,8-dichloro-2-méthyl-1,2,3,4-tétrahydro-isoquinoléin-4-yl)-phényl]-nicotinamide ;
60) N-[3-(6,8-dichloro-2-méthyl-1,2,3,4-tétrahydro-isoquinoléin-4-yl)-phényl]-méthanesulfonamide ;
61) [3-(6,8-dichloro-2-méthyl-1,2,3,4-tétrahydro-isoquinoléin-4-yl)-phényl]-amide de l'acide éthanesulfonique ;
62) N',N'-diméthylamino-N-[3-(6,8-dichloro-2-méthyl-1,2,3,4-tétrahydro-isoquinoléin-4-yl)-phényl]-sulfamide ;
63) N-[2-(6,8-dichloro-2-méthyl-1,2,3,4-tétrahydro-isoquinoléin-4-yl)-phényl]-propionamide ;
64) N-[2-(6,8-dichloro-2-méthyl-1,2,3,4-tétrahydro-isoquinoléin-4-yl)-phényl]-butyramide ;
65) [2-(6,8-dichloro-2-méthyl-1,2,3,4-tétrahydro-isoquinoléin-4-yl)-phényl]-amide de l'acide pentanoïque ;
66) N-[2-(6,8-dichloro-2-méthyl-1,2,3,4-tétrahydro-isoquinoléin-4-yl)-phényl]-isobutyramide ;
67) N-[2-(6,8-dichloro-2-méthyl-1,2,3,4-tétrahydro-isoquinoléin-4-yl)-phényl]-2,2-diméthylpropionamide ;
68) [2-(6,8-dichloro-2-méthyl-1,2,3,4-tétrahydro-isoquinoléin-4-yl)-phényl]-amide de l'acide cyclopropanecarboxylique ;
69) [2-(6,8-dichloro-2-méthyl-1,2,3,4-tétrahydro-isoquinoléin-4-yl)-phényl]-amide de l'acide cyclobutanecarboxylique ;
70) [2-(6,8-dichloro-2-méthyl-1,2,3,4-tétrahydro-isoquinoléin-4-yl)-phényl]-amide de l'acide cyclopentanecarboxylique ;
71) N-[2-(6,8-dichloro-2-méthyl-1,2,3,4-tétrahydro-isoquinoléin-4-yl)-phényl]-2,2,2-trifluoroacétamide ;
72) [2-(6,8-dichloro-2-méthyl-1,2,3,4-tétrahydro-isoquinoléin-4-yl)-phényl]-amide de l'acide 1-acétylpipéridine-4-carboxylique ;
73) N-[2-(6,8-dichloro-2-méthyl-1,2,3,4-tétrahydro-isoquinoléin-4-yl)-phényl]-méthanesulfonamide ;
74) [2-(6,8-dichloro-2-méthyl-1,2,3,4-tétrahydro-isoquinoléin-4-yl)-phényl]-amide de l'acide éthanesulfonique ;
75) N',N'-diméthylamino-N-[2-(6,8-dichloro-2-méthyl-1,2,3,4-tétrahydro-isoquinoléin-4-yl)-phényl]-sulfamide ;
76) 1-[3-(6,8-dichloro-2-méthyl-1,2,3,4-tétrahydro-isoquinoléin-4-yl)-phényl]-3-éthylurée ;
77) 1-[3-(6,8-dichloro-2-méthyl-1,2,3,4-tétrahydro-isoquinoléin-4-yl)-phényl]-3-méthylthio-urée ;
78) 1-[2-(6,8-dichloro-2-méthyl-1,2,3,4-tétrahydro-isoquinoléin-4-yl)-phényl]-3-éthylurée ;
79) 1-[2-(6,8-dichloro-2-méthyl-1,2,3,4-tétrahydro-isoquinoléin-4-yl)-phényl]-3-méthylthio-urée ;
80) N-{5-[4-(6,8-dichloro-2-méthyl-1,2,3,4-tétrahydro-isoquinoléin-4-yl)-phénylsulfamoyl]-4-méthyl-thiazol-2-yl}-acétamide ;
81) N-{5-[3-(6,8-dichloro-2-méthyl-1,2,3,4-tétrahydro-isoquinoléin-4-yl)-phénylsulfamoyl]-4-méthylthiazol-2-yl}-acétamide ;
82) [4-(6,8-dichloro-2-méthyl-1,2,3,4-tétrahydro-isoquinoléin-4-yl)-phényl]-amide de l'acide 1,2-diméthyl-1H-imidazole-4-sulfonique ;
83) [3-(6,8-dichloro-2-méthyl-1,2,3,4-tétrahydro-isoquinoléin-4-yl)-phényl]-amide de l'acide 1,2-diméthyl-1H-imidazole-4-sulfonique ;
84) [4-(6,8-dichloro-2-méthyl-1,2,3,4-tétrahydro-isoquinoléin-4-yl)-phényl]-amide de l'acide 5-chloro-1,3-diméthyl-1H-pyrazole-4-sulfonique ;
85) [3-(6,8-dichloro-2-méthyl-1,2,3,4-tétrahydro-isoquinoléin-4-yl)-phényl]-amide de l'acide 5-chloro-1,3-diméthyl-1H-pyrazole-4-sulfonique ;
86) [4-(6,8-dichloro-2-méthyl-1,2,3,4-tétrahydro-isoquinoléin-4-yl)-phényl]-amide de l'acide 5-bromothiophène-2-sulfonique ;
87) [3-(6,8-dichloro-2-méthyl-1,2,3,4-tétrahydro-isoquinoléin-4-yl)-phényl]-amide de l'acide 5-bromothiophène-2-sulfonique ;
88) N-[4-(6,8-dichloro-2-méthyl-1,2,3,4-tétrahydro-isoquinoléin-4-yl)-phényl]-C,C,C-trifluorométhanesulfonamide ;
89) N-[3-(6,8-dichloro-2-méthyl-1,2,3,4-tétrahydro-isoquinoléin-4-yl)-phényl]-C,C,C-trifluorométhanesulfonamide ;
90) 4-(6,8-dichloro-2-méthyl-1,2,3,4-tétrahydro-isoquinoléin-4-yl)-phényl]-amide de l'acide 2,2,2-trifluoroéthanesulfonique ;
91) 3-(6,8-dichloro-2-méthyl-1,2,3,4-tétrahydro-isoquinoléin-4-yl)-phényl]-amide de l'acide 2,2,2-trifluoroéthanesulfonique ;
92) N-éthyl-N'-4-(6,8-dichloro-2-méthyl-1,2,3,4-tétrahydro-isoquinoléin-4-yl)-benzènesulfonylurée ;
93) 2-chloro-5-(6,8-dichloro-2-méthyl-1,2,3,4-tétrahydro-isoquinoléin-4-yl)-benzènesulfonamide ;
94) 2-méthyl-4-phényl-6,8-bistrifluorométhyl-1,2,3,4-tétrahydro-isoquinoléine ;
95) 2-amino-N-[4-(6,8-dichloro-2-méthyl-1,2,3,4-tétrahydro-isoquinoléin-4-yl)-phényl]-acétamide ;
96) N-[4-(6,8-dichloro-2-méthyl-1,2,3,4-tétrahydro-isoquinoléin-4-yl)-phényl]-2-méthylaminoacétamide ;
97) N-[4-(6,8-dichloro-2-méthyl-1,2,3,4-tétrahydro-isoquinoléin-4-yl)-phényl]-2-diméthylaminoacétamide ;
98) 2-amino-N-[4-(6,8-dichloro-2-méthyl-1,2,3,4-tétrahydro-isoquinoléin-4-yl)-phényl]-propionamide ;
99) 2-amino-N-[4-(6,8-dichloro-2-méthyl-1,2,3,4-tétrahydro-isoquinoléin-4-yl)-phényl]-butyramide ;
100) [4-(6,8-dichloro-2-méthyl-1,2,3,4-tétrahydro-isoquinoléin-4-yl)-phényl]-amide de l'acide 2,6-diaminohexanoïque ;
101) [4-(6,8-dichloro-2-méthyl-1,2,3,4-tétrahydro-isoquinoléin-4-yl)-phényl]-amide de l'acide pyrrolidine-2-carboxylique ;
102) N-[4-(6,8-dichloro-2-méthyl-1,2,3,4-tétrahydro-isoquinoléin-4-yl)-phényl]-isonicotinamide ;
103) [4-(6,8-dichloro-2-méthyl-1,2,3,4-tétrahydro-isoquinoléin-4-yl)-phényl]-amide de l'acide 1H-pyrrole-3-carboxylique ;
104) [4-(6,8-dichloro-2-méthyl-1,2,3,4-tétrahydro-isoquinoléin-4-yl)-phényl]-amide de l'acide 1H-pyrrole-2-carboxylique ;
105) [4-(6,8-dichloro-2-méthyl-1,2,3,4-tétrahydro-isoquinoléin-4-yl)-phényl]-amide de l'acide 1-méthylpipéridine-4-carboxylique ;
106) [4-(6,8-dichloro-2-méthyl-1,2,3,4-tétrahydro-isoquinoléin-4-yl)-phényl]-amide de l'acide 1,4-diméthyl-1H-pyrrole-2-carboxylique ;
107) [4-(6,8-dichloro-2-méthyl-1,2,3,4-tétrahydro-isoquinoléin-4-yl)-phényl]-amide de l'acide 4-nitro-1H-pyrrole-2-carboxylique ;
108) [4-(6,8-dichloro-2-méthyl-1,2,3,4-tétrahydro-isoquinoléin-4-yl)-phényl]-amide de l'acide 2,5-diméthyl-1H-pyrrole-3-carboxylique ;
109) [4-(6,8-dichloro-2-méthyl-1,2,3,4-tétrahydro-isoquinoléin-4-yl)-phényl]-amide de l'acide 1H-imidazole-4-carboxylique ;
110) [4-(6,8-dichloro-2-méthyl-1,2,3,4-tétrahydro-isoquinoléin-4-yl)-phényl]-amide de l'acide 1-méthanesulfonylpipéridine-4-carboxylique ;
111) [4-(6,8-dichloro-2-méthyl-1,2,3,4-tétrahydro-isoquinoléin-4-yl)-phényl]-amide de l'acide 3,5-diméthyl-1H-pyrazole-4-carboxylique ;
112) [4-(6,8-dichloro-2-méthyl-1,2,3,4-tétrahydro-isoquinoléin-4-yl)-phényl]-amide de l'acide 1H-pyrazole-4-carboxylique ;
113) [4-(6,8-dichloro-2-méthyl-1,2,3,4-tétrahydro-isoquinoléin-4-yl)-phényl]-amide de l'acide 3-trifluorométhyl-1H-pyrazole-4-carboxylique ;
114) N-[3-(6,8-dichloro-2-méthyl-1,2,3,4-tétrahydro-isoquinoléin-4-yl)-phényl]-2-méthylaminoacétamide ;
115) N-[3-(6,8-dichloro-2-méthyl-1,2,3,4-tétrahydro-isoquinoléin-4-yl)-phényl]-2-diméthylaminoacétamide ;
116) 2-amino-N-[3-(6,8-dichloro-2-méthyl-1,2,3,4-tétrahydro-isoquinoléin-4-yl)-phényl]-propionamide ;
117) 2-amino-N-[3-(6,8-dichloro-2-méthyl-1,2,3,4-tétrahydro-isoquinoléin-4-yl)-phényl]-butyramide ;
118) [3-(6,8-dichloro-2-méthyl-1,2,3,4-tétrahydro-isoquinoléin-4-yl)-phényl]-amide de l'acide 2,6-diaminohexanoïque ;
119) [3-(6,8-dichloro-2-méthyl-1,2,3,4-tétrahydro-isoquinoléin-4-yl)-phényl]-amide de l'acide pyrrolidine-2-carboxylique ;
120) N-[3-(6,8-dichloro-2-méthyl-1,2,3,4-tétrahydro-isoquinoléin-4-yl)-phényl]-isonicotinamide ;
121) [3-(6,8-dichloro-2-méthyl-1,2,3,4-tétrahydro-isoquinoléin-4-yl)-phényl]-amide de l'acide 1H-pyrrole-3-carboxylique ;
122) [3-(6,8-dichloro-2-méthyl-1,2,3,4-tétrahydro-isoquinoléin-4-yl)-phényl]-amide de l'acide 1H-pyrrole-2-carboxylique ;
123) [3-(6,8-dichloro-2-méthyl-1,2,3,4-tétrahydro-isoquinoléin-4-yl)-phényl]-amide de l'acide 1-méthylpipéridine-4-carboxylique ;
124) [3-(6,8-dichloro-2-méthyl-1,2,3,4-tétrahydro-isoquinoléin-4-yl)phényl]-amide de l'acide 1,4-diméthyl-1H-pyrrole-2-carboxylique ;
125) [3-(6,8-dichloro-2-méthyl-1,2,3,4-tétrahydro-isoquinoléin-4-yl)-phényl]-amide de l'acide 4-nitro-1H-pyrrole-2-carboxylique ;
126) [3-(6,8-dichloro-2-méthyl-1,2,3,4-tétrahydro-isoquinoléin-4-yl)-phényl]-amide de l'acide 2,5-diméthyl-1H-pyrrole-3-carboxylique ;
127) [3-(6,8-dichloro-2-méthyl-1,2,3,4-tétrahydro-isoquinoléin-4-yl)-phényl]-amide de l'acide 1H-imidazole-4-carboxylique ;
128) [3-(6,8-dichloro-2-méthyl-1,2,3,4-tétrahydro-isoquinoléin-4-yl)-phényl]-amide de l'acide 1-méthanesulfonylpipéridine-4-carboxylique ;
129) [3-(6,8-dichloro-2-méthyl-1,2,3,4-tétrahydro-isoquinoléin-4-yl)-phényl]-amide de l'acide 3,5-diméthyl-1H-pyrazole-4-carboxylique ;
130) [3-(6,8-dichloro-2-méthyl-1,2,3,4-tétrahydro-isoquinoléin-4-yl)-phényl]-amide de l'acide 1H-pyrazole-4-carboxylique ;
131) [3-(6,8-dichloro-2-méthyl-1,2,3,4-tétrahydro-isoquinoléin-4-yl)-phényl]-amide de l'acide 3-trifluorométhyl-1H-pyrazole-4-carboxylique ;
132) 1-[2-(6,8-dichloro-2-méthyl-1,2,3,4-tétrahydro-isoquinoléin-4-yl)-phényl]-3-éthylthio-urée ;
133) 1-[4-(6,8-dichloro-2-méthyl-1,2,3,4-tétrahydro-isoquinoléin-4-yl)-phényl]-3-éthylthio-urée ;
134) 1-[3-(6,8-dichloro-2-méthyl-1,2,3,4-tétrahydro-isoquinoléin-4-yl)-phényl]-3-éthylthio-urée ;
135) 3-[3-(6,8-dichloro-2-méthyl-1,2,3,4-tétrahydro-isoquinoléin-4-yl)-phényl]-1,1-diméthylurée ;
136) [3-(6,8-dichloro-2-méthyl-1,2,3,4-tétrahydro-isoquinoléin-4-yl)-phényl]-amide de l'acide 4-méthylpipérazine-1-carboxylique ;
137) [3-(6,8-dichloro-2-méthyl-1,2,3,4-tétrahydro-isoquinoléin-4-yl)-phényl]-amide de l'acide pipéridine-1-carboxylique ;
138) [3-(6,8-dichloro-2-méthyl-1,2,3,4-tétrahydro-isoquinoléin-4-yl)-phényl]-amide de l'acide morpholine-4-carboxylique ;
139) [3-(6,8-dichloro-2-méthyl-1,2,3,4-tétrahydro-isoquinoléin-4-yl)-phényl]-amide de l'acide pyrrolidine-1-carboxylique ;
140) 3-[3-(6,8-dichloro-2-méthyl-1,2,3,4-tétrahydro-isoquinoléin-4-yl)-phényl]-1,1-diéthylurée ;
141) 1-[3-(6,8-dichloro-2-méthyl-1,2,3,4-tétrahydro-isoquinoléin-4-yl)-phényl]-3-méthylurée ;
142) 1-[3-(6,8-dichloro-2-méthyl-1,2,3,4-tétrahydro-isoquinoléin-4-yl)-phényl]-3-(2-diméthylaminoéthyl)-urée ;
143) 1-[3-(6,8-dichloro-2-méthyl-1,2,3,4-tétrahydro-isoquinoléin-4-yl)-phényl]-3-(tétrahydrofurann-3-yl)-urée ;
144) 1-[3-(6,8-dichloro-2-méthyl-1,2,3,4-tétrahydro-isoquinoléin-4-yl)-phényl]-3-(tétrahydropyrann-4-yl)-urée ;
145) 3-[3-(6,8-dichloro-2-méthyl-1,2,3,4-tétrahydro-isoquinoléin-4-yl)-phényl]-1-méthyl-1-(1-méthylpipéridin-4-yl)-urée ;
146) 3-[3-(6,8-dichloro-2-méthyl-1,2,3,4-tétrahydro-isoquinoléin-4-yl)-phényl]-1-(3-diméthylaminopropyl)-1-méthylurée ;
147) 3-[3-(6,8-dichloro-2-méthyl-1,2,3,4-tétrahydro-isoquinoléin-4-yl)-phényl]-1-(2-diméthylaminoéthyl)-1-méthylurée ;
148) 1-[3-(6,8-dichloro-2-méthyl-1,2,3,4-tétrahydro-isoquinoléin-4-yl)-phényl]-3-(3-diméthylaminopropyl)-urée ;
149) 1-[3-(6,8-dichloro-2-méthyl-1,2,3,4-tétrahydro-isoquinoléin-4-yl)-phényl]-3-(2-méthoxyéthyl)-urée ;
150) 1-[3-(6,8-dichloro-2-méthyl-1,2,3,4-tétrahydro-isoquinoléin-4-yl)-phényl]-3-pyridin-3-ylurée ;
151) 1-[3-(6,8-dichloro-2-méthyl-1,2,3,4-tétrahydro-isoquinoléin-4-yl)-phényl]-3-pyridin-4-ylurée ;
152) [2-(6,8-dichloro-2-méthyl-1,2,3,4-tétrahydro-isoquinoléin-4-yl)-phényl]-amide de l'acide 4-méthylpipérazine-1-carboxylique ;
153) 1-[2-(6,8-dichloro-2-méthyl-1,2,3,4-tétrahydro-isoquinoléin-4-yl)-phényl]-3-méthylurée ;
154) 3-[2-(6,8-dichloro-2-méthyl-1,2,3,4-tétrahydro-isoquinoléin-4-yl)-phényl]-1,1-diméthylurée ;
155) 3-[2-(6,8-dichloro-2-méthyl-1,2,3,4-tétrahydro-isoquinoléin-4-yl)-phényl]-1,1-diéthylurée ;
156) [2-(6,8-dichloro-2-méthyl-1,2,3,4-tétrahydro-isoquinoléin-4-yl)-phényl]-amide de l'acide pipéridine-1-carboxylique ;
157) [2-(6,8-dichloro-2-méthyl-1,2,3,4-tétrahydro-isoquinoléin-4-yl)-phényl]-amide de l'acide morpholine-4-carboxylique ;
158) [2-(6,8-dichloro-2-méthyl-1,2,3,4-tétrahydro-isoquinoléin-4-yl)-phényl]-amide de l'acide pyrrolidine-1-carboxylique ;
159) 1-[2-(6,8-dichloro-2-méthyl-1,2,3,4-tétrahydro-isoquinoléin-4-yl)-phényl]-3-(2-diméthylaminoéthyl)-urée ;
160) [4-(6,8-dichloro-2-méthyl-1,2,3,4-tétrahydro-isoquinoléin-4-yl)-phényl]-amide de l'acide 4-méthylpipérazine-1-carboxylique ;
161) [4-(6,8-dichloro-2-méthyl-1,2,3,4-tétrahydro-isoquinoléin-4-yl)-phényl]-amide de l'acide pyrrolidine-1-carboxylique ;
162) 1-[4-(6,8-dichloro-2-méthyl-1,2,3,4-tétrahydro-isoquinoléin-4-yl)-phényl]-3-méthylurée ;
163) 3-[4-(6,8-dichloro-2-méthyl-1,2,3,4-tétrahydro-isoquinoléin-4-yl)-phényl]-1,1-diméthylurée ;
164) 3-[4-(6,8-dichloro-2-méthyl-1,2,3,4-tétrahydro-isoquinoléin-4-yl)-phényl]-1,1-diéthylurée ;
165) 1-[4-(6,8-dichloro-2-méthyl-1,2,3,4-tétrahydro-isoquinoléin-4-yl)-phényl]-3-(2-diméthylaminoéthyl)-urée ;
166) [4-(6,8-dichloro-2-méthyl-1,2,3,4-tétrahydro-isoquinoléin-4-yl)-phényl]-amide de l'acide pipéridine-1-carboxylique ;
167) [4-(6,8-dichloro-2-méthyl-1,2,3,4-tétrahydro-isoquinoléin-4-yl)-phényl]-amide de l'acide morpholine-4-carboxylique ;
168) N-[4-(6,8-dichloro-2-méthyl-1,2,3,4-tétrahydro-isoquinoléin-4-yl)-phényl]-formamide,
169) [4-(6,8-dichloro-2-méthyl-1,2,3,4-tétrahydro-isoquinoléin-4-yl)-phényl]-méthylamine ;
170) 1-[4-(6,8-dichloro-2-méthyl-1,2,3,4-tétrahydro-isoquinoléin-4-yl)-phényl]-1,3-diméthylurée ;
171) [4-(6,8-dichloro-2-méthyl-1,2,3,4-tétrahydro-isoquinoléin-4-yl)-phényl]-méthylamide de l'acide 4-méthylpipérazine-1-carboxylique ;
172) 1-[4-(6,8-dichloro-2-méthyl-1,2,3,4-tétrahydro-isoquinoléin-4-yl)-phényl]-1,3,3-triméthylurée ;
173) [4-(6,8-dichloro-2-méthyl-1,2,3,4-tétrahydro-isoquinoléin-4-yl)-phényl]-méthylamide de l'acide pipéridine-1-carboxylique ;
174) [4-(6,8-dichloro-2-méthyl-1,2,3,4-tétrahydro-isoquinoléin-4-yl)-phényl]-méthylamide de l'acide morpholine-4-carboxylique ;
175) [4-(6,8-dichloro-2-méthyl-1,2,3,4-tétrahydro-isoquinoléin-4-yl)-phényl]-méthylamide de l'acide pyrrolidine-1-carboxylique ;
176) 1-[4-(6,8-dichloro-2-méthyl-1,2,3,4-tétrahydro-isoquinoléin-4-yl)-phényl]-3-(2-diméthylaminoéthyl)-1-méthylurée ;
177) 1-[4-(6,8-dichloro-2-méthyl-1,2,3,4-tétrahydro-isoquinoléin-4-yl)-phényl]-3,3-diéthyl-1-méthylurée ;
178) N-[3-(6,8-dichloro-2-méthyl-1,2,3,4-tétrahydro-isoquinoléin-4-yl)-phényl]-formamide ;
179) [3-(6,8-dichloro-2-méthyl-1,2,3,4-tétrahydro-isoquinoléin-4-yl)-phényl]-méthylamine ;
180) [3-(6,8-dichloro-2-méthyl-1,2,3,4-tétrahydro-isoquinoléin-4-yl)-phényl]-méthylamide de l'acide pyrrolidine-1-carboxylique ;
181) [3-(6,8-dichloro-2-méthyl]-1,2,3,4-tétrahydro-isoquinoléin-4-yl)-phényl]-méthylamide de l'acide pipéridine-1-carboxylique ;
182) 1-[3-(6,8-dichloro-2-méthyl-1,2,3,4-tétrahydro-isoquinoléin-4-yl)-phényl]-1,3,3-triméthylurée ;
183) 1-[3-(6,8-dichloro-2-méthyl-1,2,3,4-tétrahydro-isoquinoléin-4-yl)phényl]-1,3-diméthylurée ;
184) [3-(6,8-dichloro-2-méthyl-1,2,3,4-tétrahydro-isoquinoléin-4-yl)-phényl]-méthylamide de l'acide morpholine-4-carboxylique ;
185) [3-(6,8-dichloro-2-méthyl-1,2,3,4-tétrahydro-isoquinoléin-4-yl)-phényl]-méthylamide de l'acide 4-méthylpipérazine-1-carboxylique ;
186) 1-[3-(6,8-dichloro-2-méthyl-1,2,3,4-tétrahydro-isoquinoléin-4-yl)-phényl]-3-(2-diméthylaminoéthyl)-1-méthylurée ;
187) 1-[3-(6,8-dichloro-2-méthyl-1,2,3,4-tétrahydro-isoquinoléin-4-yl)-phényl]-3,3-diéthyl-1-méthylurée ;
188) ester 2-diméthylaminoéthylique de l'acide [3-(6,8-dichloro-2-méthyl-1,2,3,4-tétrahydro-isoquinoléin-4-yl)-phényl]-carbamique ;
189) ester 2-diméthylaminoéthylique de l'acide [4-(6,8-dichloro-2-méthyl-1,2,3,4-tétrahydro-isoquinoléin-4-yl)-phényl]carbamique ;
190) ester 2-diméthylaminoéthylique de l'acide [2-(6,8-dichloro-2-méthyl-1,2,3,4-tétrahydro-isoquinoléin-4-yl)-phényl]-carbamique ;
191) ester méthylique de l'acide [3-(6,8-dichloro-2-méthyl-1,2,3,4-tétrahydro-isoquinoléin-4-yl)-phényl]-carbamique ;
192) ester éthylique de l'acide [3-(6,8-dichloro-2-méthyl-1,2,3,4-tétrahydro-isoquinoléin-4-yl)-phényl]-carbamique ;
193) ester isopropylique de l'acide[3-(6,8-dichloro-2-méthyl-1,2,3,4-tétrahydro-isoquinoléin-4-yl)-phényl]-carbamique ;
194) ester 2,2-diméthylpropylique de l'acide [3-(6,8-dichloro-2-méthyl-1,2,3,4-tétrahydro-isoquinoléin-4-yl)-phényl]-carbamique ;
195) ester méthylique de l'acide [4-(6,8-dichloro-2-méthyl-1,2,3,4-tétrahydro-isoquinoléin-4-yl)-phényl]-carbamique ;
196) ester isopropylique de l'acide [4-(6,8-dichloro-2-méthyl-1,2,3,4-tétrahydro-isoquinoléin-4-yl)-phényl]-carbamique ;
197) ester 2,2-diméthylpropylique de l'acide [4-(6,8-dichloro-2-méthyl-1,2,3,4-tétrahydro-isoquinoléin-4-yl)-phényl]-carbamique ;
198) ester éthylique de l'acide [4-(6,8-dichloro-2-méthyl-1,2,3,4-tétrahydro-isoquinoléin-4-yl)-phényl]-carbamique ;
199) (R)-N-[3-(6,8-dichloro-2-méthyl-1,2,3,4-tétrahydro-isoquinoléin-4-yl)-phényl]-méthanesulfonamide ;
200) (S)-N-[3-(6,8-dichloro-2-méthyl-1,2,3,4-tétrahydro-isoquinoléin-4-yl)-phényl]-méthanesulfonamide ;
201) (R)-1-[2-(6,8-dichloro-2-méthyl-1,2,3,4-tétrahydro-isoquinoléin-4-yl)-phényl]-3-éthylurée ;
202) (S)-1-[2-(6,8-dichloro-2-méthyl-1,2,3,4-tétrahydro-isoquinoléin-4-yl)-phényl]-3-éthylurée ;
203) N-[3-(6,8-difluoro-2-méthyl-1,2,3,4-tétrahydro-isoquinoléin-4-yl)-phényl]-acétamide ;
204) 4-(3-bromophényl)-6,8-dichloro-2-méthyl-1,2,3,4-tétrahydro-isoquinoléine ;
205) 1-[3-(6,8-dichloro-2-méthyl-1,2,3,4-tétrahydro-isoquinoléin-4-yl)-phényl]-3-(2-hydroxyéthyl)-urée ;
206) ester éthylique de l'acide 3-(6,8-dichloro-2-méthyl-1,2,3,4-tétrahydro-isoquinoléin-4-yl)-benzoïque ;
207) acide 3-(6,8-dichloro-2-méthyl-1,2,3,4-tétrahydro-isoquinoléin-4-yl)-benzoïque ;
ainsi que ses sels pharmaceutiquement acceptables.

3. Composé de formule I selon la revendication 1, choisi dans le groupe constitué par :
1) N-[4-(6,8-dichloro-2-méthyl-1,2,3,4-tétrahydro-isoquinoléin-4-yl)-phényl]-acétamide ;
2) 4-(6,8-dichloro-2-méthyl-1,2,3,4-tétrahydro-isoquinoléin-4-yl)-benzènesulfonamide ;
3) 3-(6,8-dichloro-2-méthyl-1,2,3,4-tétrahydro-isoquinoléin-4-yl)-benzènesulfonamide ;
4) 1-[4-(6,8-dichloro-2-méthyl-1,2,3,4-tétrahydro-isoquinoléin-4-yl)-phényl]-3-méthylthio-urée ;
5) 1-[4-(6,8-dichloro-2-méthyl-1,2,3,4-tétrahydro-isoquinoléin-4-yl)-phényl]-3-éthylurée ;
6) N-[4-(6-bromo-8-chloro-2-méthyl-1,2,3,4-tétrahydro-isoquinoléin-4-yl)-phényl]-acétamide ;
7) N-[3-(6,8-dichloro-2-méthyl-1,2,3,4-tétrahydro-isoquinoléin-4-yl)-phényl]-acétamide ;
8) N-[4-(6,8-dichloro-2-méthyl-1,2,3,4-tétrahydro-isoquinoléin-4-yl)-phényl]-propionamide ;
9) [4-(6,8-dichloro-2-méthyl-1,2,3,4-tétrahydro-isoquinoléin-4-yl)-phényl]-amide de l'acide 1-acétylpipéridine-4-carboxylique ;
10) N-[4-(6,8-dichloro-2-méthyl-1,2,3,4-tétrahydro-isoquinoléin-4-yl)-phényl]-méthanesulfonamide ;
11) [4-(6,8-dichloro-2-méthyl-1,2,3,4-tétrahydro-isoquinoléin-4-yl)-phényl]-amide de l'acide éthanesulfonique ;
12) N',N'-diméthylamino-N-[4-(6,8-dichloro-2-méthyl-1,2,3,4-tétrahydro-isoquinoléin-4-yl)-phényl]-sulfamide ;
13) N-[3-(6,8-dichloro-2-méthyl-1,2,3,4-tétrahydro-isoquinoléin-4-yl)-phényl]-propionamide ;
14) N-[3-(6,8-dichloro-2-méthyl-1,2,3,4-tétrahydro-isoquinoléin-4-yl)-phényl]-butyramide ;
15) N-[3-(6,8-dichloro-2-méthyl-1,2,3,4-tétrahydro-isoquinoléin-4-yl)-phényl]-isobutyramide ;
16) [3-(6,8-dichloro-2-méthyl-1,2,3,4-tétrahydro-isoquinoléin-4-yl)-phényl]-amide de l'acide cyclopropanecarboxylique ;
17) [3-(6,8-dichloro-2-méthyl-1,2,3,4-tétrahydro-isoquinoléin-4-yl)-phényl]-amide de l'acide cyclobutanecarboxylique ;
18) N-[3-(6,8-dichloro-2-méthyl-1,2,3,4-tétrahydro-isoquinoléin-4-yl)-phényl]-2,2,2-trifluoroacétamide ;
19) [3-(6,8-dichloro-2-méthyl-1,2,3,4-tétrahydro-isoquinoléin-4-yl)-phényl]-amide de l'acide 1-acétylpipéridine-4-carboxylique ;
20) N-[3-(6,8-dichloro-2-méthyl-1,2,3,4-tétrahydro-isoquinoléin-4-yl)-phényl]-nicotinamide ;
21) N-[3-(6,8-dichloro-2-méthyl-1,2,3,4-tétrahydro-isoquinoléin-4-yl)-phényl]-méthanesulfonamide ;
22) [3-(6,8-dichloro-2-méthyl-1,2,3,4-tétrahydro-isoquinoléin-4-yl)-phényl]-amide de l'acide éthanesulfonique ;
23) N',N'-diméthylamino-N-[3-(6,8-dichloro-2-méthyl-1,2,3,4-tétrahydro-isoquinoléin-4-yl)-phényl]-sulfamide ;
24) [2-(6,8-dichloro-2-méthyl-1,2,3,4-tétrahydro-isoquinoléin-4-yl)-phényl]-amide de l'acide cyclopropanecarboxylique ;
25) [2-(6,8-dichloro-2-méthyl-1,2,3,4-tétrahydro-isoquinoléin-4-yl)-phényl]-amide de l'acide 1-acétylpipéridine-4-carboxylique ;
26) 1-[3-(6,8-dichloro-2-méthyl-1,2,3,4-tétrahydro-isoquinoléin-4-yl)-phényl]-3-éthylurée ;
27) 1-[3-(6,8-dichloro-2-méthyl-1,2,3,4-tétrahydro-isoquinoléin-4-yl)-phényl]-3-méthylthio-urée ;
28) 1-[2-(6,8-dichloro-2-méthyl-1,2,3,4-tétrahydro-isoquinoléin-4-yl)-phényl]-3-éthylurée ;
29) 1-[2-(6,8-dichloro-2-méthyl-1,2,3,4-tétrahydro-isoquinoléin-4-yl)-phényl]-3-méthylthio-urée ;
30) N-{5-[3-(6,8-dichloro-2-méthyl-1,2,3,4-tétrahydro-isoquinoléin-4-yl)-phénylsulfamoyl]-4-méthylthiazol-2-yl}-acétamide ;
31) [3-(6,8-dichloro-2-méthyl-1,2,3,4-tétrahydro-isoquinoléin-4-yl)-phényl]-amide de l'acide 1,2-diméthyl-1H-imidazole-4-sulfonique ;
32) N-[4-(6,8-dichloro-2-méthyl-1,2,3,4-tétrahydro-isoquinoléin-4-yl)-phényl]-C,C,C-trifluorométhanesulfonamide ;
33) N-[3-(6,8-dichloro-2-méthyl-1,2,3,4-tétrahydro-isoquinoléin-4-yl)-phényl]-C,C,C-trifluorométhanesulfonamide ;
34) N-éthyl-N'-4-(6,8-dichloro-2-méthyl-1,2,3,4-tétrahydro-isoquinoléin-4-yl)-benzènesulfonylurée ;
35) N-[4-(6,8-dichloro-2-méthyl-1,2,3,4-tétrahydro-isoquinoléin-4-yl)-phényl]-2-diméthylaminoacétamide ;
36) [4-(6,8-dichloro-2-méthyl-1,2,3,4-tétrahydro-isoquinoléin-4-yl)-phényl]-amide de l'acide 2,6-diaminohexanoïque ;
37) [4-(6,8-dichloro-2-méthyl-1,2,3,4-tétrahydro-isoquinoléin-4-yl)-phényl]-amide de l'acide 1H-pyrrole-3-carboxylique ;
38) [4-(6,8-dichloro-2-méthyl-1,2,3,4-tétrahydro-isoquinoléin-4-yl)-phényl]-amide de l'acide 1-méthylpipéridine-4-carboxylique ;
39) [4-(6,8-dichloro-2-méthyl-1,2,3,4-tétrahydro-isoquinoléin-4-yl)-phényl]-amide de l'acide 1-méthanesulfonylpipéridine-4-carboxylique ;
40) [4-(6,8-dichloro-2-méthyl-1,2,3,4-tétrahydro-isoquinoléin-4-yl)-phényl]-amide de l'acide 1H-pyrazole-4-carboxylique ;
41) N-[3-(6,8-dichloro-2-méthyl-1,2,3,4-tétrahydro-isoquinoléin-4-yl)-phényl]-2-méthylaminoacétamide ;
42) N-[3-(6,8-dichloro-2-méthyl-1,2,3,4-tétrahydro-isoquinoléin-4-yl)-phényl]-2-diméthylaminoacétamide ;
43) 2-amino-N-[3-(6,8-dichloro-2-méthyl-1,2,3,4-tétrahydro-isoquinoléin-4-yl)-phényl]-propionamide ;
44) 2-amino-N-[3-(6,8-dichloro-2-méthyl-1,2,3,4-tétrahydro-isoquinoléin-4-yl)-phényl]-butyramide ;
45) [3-(6,8-dichloro-2-méthyl-1,2,3,4-tétrahydro-isoquinoléin-4-yl)-phényl]-amide de l'acide 2,6-diaminohexanoïque ;
46) [3-(6,8-dichloro-2-méthyl-1,2,3,4-tétrahydro-isoquinoléin-4-yl)-phényl]-amide de l'acide 1-méthylpipéridine-4-carboxylique ;
47) [3-(6,8-dichloro-2-méthyl-1,2,3,4-tétrahydro-isoquinoléin-4-yl)-phényl]-amide de l'acide 1H-imidazole-4-carboxylique ;
48) [3-(6,8-dichloro-2-méthyl-1,2,3,4-tétrahydro-isoquinoléin-4-yl)-phényl]-amide de l'acide 1-méthanesulfonylpipéridine-4-carboxylique ;
49) [3-(6,8-dichloro-2-méthyl-1,2,3,4-tétrahydro-isoquinoléin-4-yl)-phényl]-amide de l'acide 3,5-diméthyl-1H-pyrazole-4-carboxylique ;
50) [3-(6,8-dichloro-2-méthyl-1,2,3,4-tétrahydro-isoquinoléin-4-yl)-phényl]-amide de l'acide 1H-pyrazole-4-carboxylique ;
51) 3-[3-(6,8-dichloro-2-méthyl-1,2,3,4-tétrahydro-isoquinoléin-4-yl)-phényl]-1,1-diméthylurée ;
52) [3-(6,8-dichloro-2-méthyl-1,2,3,4-tétrahydro-isoquinoléin-4-yl)-phényl]-amide de l'acide 4-méthylpipérazine-1-carboxylique ;
53) [3-(6,8-dichloro-2-méthyl-1,2,3,4-tétrahydro-isoquinoléin-4-yl)-phényl]-amide de l'acide pipéridine-1-carboxylique ;
54) [3-(6,8-dichloro-2-méthyl-1,2,3,4-tétrahydro-isoquinoléin-4-yl)-phényl]-amide de l'acide morpholine-4-carboxylique ;
55) [3-(6,8-dichloro-2-méthyl-1,2,3,4-tétrahydro-isoquinoléin-4-yl)-phényl]-amide de l'acide pyrrolidine-1-carboxylique ;
56) 3-[3-(6,8-dichloro-2-méthyl-1,2,3,4-tétrahydro-isoquinoléin-4-yl)-phényl]-1,1-diéthylurée ;
57) 1-[3-(6,8-dichloro-2-méthyl-1,2,3,4-tétrahydro-isoquinoléin-4-yl)-phényl]-3-méthylurée ;
58) 1-[3-(6,8-dichloro-2-méthyl-1,2,3,4-tétrahydro-isoquinoléin-4-yl)-phényl]-3-(2-diméthylaminoéthyl)-urée ;
59) 1-[3-(6,8-dichloro-2-méthyl-1,2,3,4-tétrahydro-isoquinoléin-4-yl)-phényl]-3-(tétrahydrofurann-3-yl)-urée ;
60) 1-[3-(6,8-dichloro-2-méthyl-1,2,3,4-tétrahydro-isoquinoléin-4-yl)-phényl]-3-(tétrahydropyrann-4-yl)-urée ;
61) 3-[3-(6,8-dichloro-2-méthyl-1,2,3,4-tétrahydro-isoquinoléin-4-yl)-phényl]-1-méthyl-1-(1-méthylpipéridin-4-yl)-urée ;
62) 3-[3-(6,8-dichloro-2-méthyl-1,2,3,4-tétrahydro-isoquinoléin-4-yl)-phényl]-1-(3-diméthylaminopropyl)-1-méthylurée ;
63) 3-[3-(6,8-dichloro-2-méthyl-1,2,3,4-tétrahydro-isoquinoléin-4-yl)-phényl]-1-(2-diméthylaminoéthyl)-1-méthylurée ;
64) 1-[3-(6,8-dichloro-2-méthyl-1,2,3,4-tétrahydro-isoquinoléin-4-yl)-phényl]-3-(3-diméthylaminopropyl)-urée ;
65) 1-[3-(6,8-dichloro-2-méthyl-1,2,3,4-tétrahydro-isoquinoléin-4-yl)-phényl]-3-(2-méthoxyéthyl)-urée ;
66) 1-[3-(6,8-dichloro-2-méthyl-1,2,3,4-tétrahydro-isoquinoléin-4-yl)-phényl]-3-pyridin-3-ylurée ;
67) 1-[3-(6,8-dichloro-2-méthyl-1,2,3,4-tétrahydro-isoquinoléin-4-yl)-phényl]-3-pyridin-4-ylurée ;
68) [2-(6,8-dichloro-2-méthyl-1,2,3,4-tétrahydro-isoquinoléin-4-yl)-phényl]-amide de l'acide 4-méthylpipérazine-1-carboxylique ;
69) 1-[2-(6,8-dichloro-2-méthyl-1,2,3,4-tétrahydro-isoquinoléin-4-yl)-phényl]-3-méthylurée ;
70) 1-[2-(6,8-dichloro-2-méthyl-1,2,3,4-tétrahydro-isoquinoléin-4-yl)-phényl]-3-(2-diméthylaminoéthyl)-urée ;
71) [4-(6,8-dichloro-2-méthyl-1,2,3,4-tétrahydro-isoquinoléin-4-yl)-phényl]-amide de l'acide 4-méthylpipérazine-1-carboxylique ;
72) 1-[4-(6,8-dichloro-2-méthyl-1,2,3,4-tétrahydro-isoquinoléin-4-yl)-phényl]-3-méthylurée ;
73) 3-[4-(6,8-dichloro-2-méthyl-1,2,3,4-tétrahydro-isoquinoléin-4-yl)-phényl]-1,1-diméthylurée ;
74) 3-[4-(6,8-dichloro-2-méthyl-1,2,3,4-tétrahydro-isoquinoléin-4-yl)-phényl]-1,1-diéthylurée ;
75) 1-[4-(6,8-dichloro-2-méthyl-1,2,3,4-tétrahydro-isoquinoléin-4-yl)-phényl]-3-(2-diméthylaminoéthyl)-urée ;
76) [4-(6,8-dichloro-2-méthyl-1,2,3,4-tétrahydro-isoquinoléin-4-yl)-phényl]-amide de l'acide morpholine-4-carboxylique ;
77) N-[4-(6,8-dichloro-2-méthyl-1,2,3,4-tétrahydro-isoquinoléin-4-yl)-phényl]-formamide ;
78) N-[3-(6,8-dichloro-2-méthyl-1,2,3,4-tétrahydro-isoquinoléin-4-yl)-phényl]-formamide ;
79) 1-[3-(6,8-dichloro-2-méthyl-1,2,3,4-tétrahydro-isoquinoléin-4-yl)-phényl]-1,3,3-triméthylurée ;
80) 1-[3-(6,8-dichloro-2-méthyl-1,2,3,4-tétrahydro-isoquinoléin-4-yl)-phényl]-1,3-diméthylurée ;
81) [3-(6,8-dichloro-2-méthyl-1,2,3,4-tétrahydro-isoquinoléin-4-yl)-phényl]-méthylamide de l'acide morpholine-4-carboxylique ;
82) [3-(6,8-dichloro-2-méthyl-1,2,3,3-tétrahydro-isoquinoléin-4-yl)-phényl]-méthylamide de l'acide 4-méthylpipérazine-1-carboxylique ;
83) 1-[3-(6,8-dichloro-2-méthyl-1,2,3,4-tétrahydro-isoquinoléin-4-yl)-phényl]-3-(2-diméthylaminoéthyl)-1-méthylurée ;
84) ester 2-diméthylaminoéthylique de l'acide [3-(6,8-dichloro-2-méthyl-1,2,3,4-tétrahydro-isoquinoléin-4-yl)-phényl]-carbamique ;
85) ester 2-diméthylaminoéthylique de l'acide [4-(6,8-dichloro-2-méthyl-1,2,3,4-tétrahydro-isoquinoléin-4-yl)-phényl]-carbamique ;
86) ester 2-diméthylaminoéthylique de l'acide [2-(6,8-dichloro-2-méthyl-1,2,3,4-tétrahydro-isoquinoléin-4-yl)-phényl]-carbamique ;
87) ester méthylique de l'acide [3-(6,8-dichloro-2-méthyl-1,2,3,4-tétrahydro-isoquinoléin-4-yl)-phényl]-carbamique ;
88) (R ou S)-N-[3-(6,8-dichloro-2-méthyl-1,2,3,4-tétrahydro-isoquinoléin-4-yl)-phényl]-méthanesulfonamide ;
89) (R ou S)-1-[2-(6,8-dichloro-2-méthyl-1,2,3,4-tétrahydro-isoquinoléin-4-yl)-phényl]-3-éthylurée ;
90) 1-[3-(6,8-dichloro-2-méthyl-1,2,3,4-tétrahydro-isoquinoléin-4-yl)-phényl]-3-(2-hydroxyéthyl)-urée ;
ainsi que ses sels pharmaceutiquement acceptables.

4. Utilisation d'un composé I selon l'une ou plusieurs des revendications 1 à 3 pour la préparation d'un médicament destiné au traitement ou à la prophylaxie de troubles de la fonction respiratoire.

5. Utilisation d'un composé I selon l'une ou plusieurs des revendications 1 à 3 pour la préparation d'un médicament destiné au traitement ou à la prophylaxie de troubles respiratoires, en particulier les troubles respiratoires liés au sommeil, tels que les apnées du sommeil.

6. Utilisation d'un composé I selon l'une ou plusieurs des revendications 1 à 3 pour la préparation d'un médicament destiné au traitement ou à la prophylaxie du ronflement.

7. Utilisation d'un composé I selon l'une ou plusieurs des revendications 1 à 3 pour la préparation d'un médicament destiné au traitement ou à la prophylaxie de maladies rénales aiguës et chroniques, en particulier de l'insuffisance rénale aiguë et de l'insuffisance rénale chronique.

8. Utilisation d'un composé I selon l'une ou plusieurs des revendications 1 à 3 pour la préparation d'un médicament destiné au traitement ou à la prophylaxie de troubles de la fonction intestinale.

9. Utilisation d'un composé I selon l'une ou plusieurs des revendications 1 à 3 pour la préparation d'un médicament destiné au traitement ou à la prophylaxie de troubles de la fonction biliaire.

10. Utilisation d'un composé I selon l'une ou plusieurs des revendications 1 à 3 pour la préparation d'un médicament destiné au traitement ou à la prophylaxie d'états ischémiques du système nerveux périphérique et central et l'attaque.

11. Utilisation d'un composé I selon l'une ou plusieurs des revendications 1 à 3 pour la préparation d'un médicament destiné au traitement ou à la prophylaxie d'états ischémiques d'organes périphériques et des membres.

12. Utilisation d'un composé I selon l'une ou plusieurs des revendications 1 à 3 pour la préparation d'un médicament destiné au traitement des états de choc.

13. Utilisation d'un composé I selon l'une ou plusieurs des revendications 1 à 3 pour la préparation d'un médicament destiné à une utilisation lors d'opérations chirurgicales et de transplantations d'organes.

14. Utilisation d'un composé I selon l'une ou plusieurs des revendications 1 à 3 pour la préparation d'un médicament destiné à la conservation et à l'entreposage de transplants en vue d'interventions chirurgicales.

15. Utilisation d'un composé I selon l'une ou plusieurs des revendications 1 à 3 pour la préparation d'un médicament destiné au traitement de maladies où la prolifération cellulaire représente une cause primaire ou secondaire.

16. Utilisation d'un composé I selon l'une ou plusieurs des revendications 1 à 3 pour la préparation d'un médicament destiné au traitement ou à la prophylaxie de troubles du métabolisme.

17. Utilisation d'un composé I selon l'une ou plusieurs des revendications 1 à 3 pour la préparation d'un médicament destiné au traitement ou à la prophylaxie de l'infestation par des ectoparasites.

18. Médicament, contenant une quantité active d'un composé I selon la revendication 1.
